# EUROPÄISCHE PATENTSCHRIFT

(11) **EP 2 188 254 B1**
(45) Veröffentlichungstag und Bekanntmachung des Hinweises auf die Patenterteilung: **21.03.2012**
(21) Anmeldenummer: 08803289.1
(22) Anmeldetag: 28.08.2008
(51) Int. Cl.: C07D 209/42, C07D 213/64, C07D 213/82, C07D 233/90, C07D 237/24, C07D 239/28, C07D 239/34, C07D 239/36, C07D 239/42, C07D 261/18, C07D 263/34, C07D 277/56, C07D 307/68, A61P 43/00, C07C 237/24, C07C 317/28, C07C 317/44, C07K 5/06

(54) **NEUE BRADYKININ B1-ANTAGONISTEN**
NOVEL BRADYKININ B1-ANTAGONISTS
NOUVEAUX ANTAGONISTES DE LA BRADYKININE B1

(30) Priorität: 29.08.2007 DE 102007041042
(43) Veröffentlichungstag der Anmeldung: 26.05.2010
(73) Patentinhaber: Boehringer Ingelheim International GmbH, 55216 Ingelheim am Rhein (DE)
(72) Erfinder: HAUEL, Norbert, 55216 Ingelheim am Rhein (DE); CECI, Angelo, 55216 Ingelheim am Rhein (DE); CEREDA, Enzo, 55216 Ingelheim Am Rhein (DE); DOODS, Henri, 55216 Ingelheim am Rhein (DE); KONETZKI, Ingo, 55216 Ingelheim am Rhein (DE); MACK, Juergen, 55216 Ingelheim am Rhein (DE); PRIEPKE, Henning, 55216 Ingelheim am Rhein (DE); SCHULER-METZ, Annette, 55216 Ingelheim am Rhein (DE); WALTER, Rainer, 55216 Ingelheim am Rhein (DE)
(74) Vertreter: Hammann, Heinz
(86) Internationale Anmeldenummer: PCT/EP2008/061263
(87) Internationale Veröffentlichungsnummer: WO 2009/027450

(56) Entgegenhaltungen:
- WO-A-03/065789
- WO-A-03/066577
- KUDUK, SCOTT D. ET AL: "Development of Orally Bioavailable and CNS Penetrant Biphenylaminocyclopropane Carboxamide Bradykinin B1 Receptor Antagonists" JOURNAL OF MEDICINAL CHEMISTRY , 50(2), 272-282 CODEN: JMCMAR; ISSN: 0022-2623, 2007, XP002506929
- KUDUK ET AL: "Bradykinin B1 antagonists: Biphenyl SAR studies in the cyclopropanecarboxamide series" BIOORGANIC & MEDICINAL CHEMISTRY LETTERS, PERGAMON, ELSEVIER SCIENCE, GB, Bd. 17, Nr. 13, 1. Juli 2007 (2007-07-01), Seiten 3608-3612, XP022114547 ISSN: 0960-894X
- KUDUK ET AL: "Bradykinin B1 antagonists: SAR studies in the 2,3-diaminopyridine series" BIOORGANIC & MEDICINAL CHEMISTRY LETTERS, PERGAMON, ELSEVIER SCIENCE, GB, Bd. 15, Nr. 17, 1. September 2005 (2005-09-01), Seiten 3925-3929, XP005000609 ISSN: 0960-894X

## Beschreibung

Gegenstand der vorliegenden Erfindung sind Verbindungen der allgemeinen Formel I

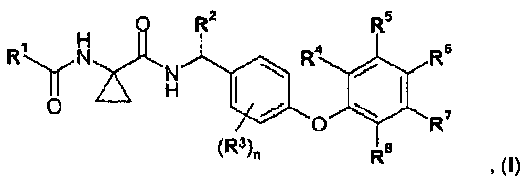

In der **n**, **R¹**, **R²**, **R³**, **R⁴**, **R⁵**, **R⁶**, **R⁷** und **R⁸** wie nachstehend beschrieben definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

Im Stand der Technik werden bereits Verbindungen mit B1-antagonistischen Eigenschaften beschrieben: WO 03/066577, WO 03/065789 und J. Med. Chem. 2007, 50(2), 272-282 (Scott D. Kuduk et al.). Die Verbindungen der vorliegenden Anmeldung unterscheiden sich von den voranstehend genannten Dokumenten vorangig dadurch, dass die Biphenylgruppe durch eine Phenoxyphenylgruppe ersetzt wurde.

Ein weiteres Dokument. Bioorg. & Med. Chem. Letters 2007, Pergamon, Elsevier Science, GB, Band 17. Nr. 13, 3608-3612 (Scott D. Kuduk et al.) beschreibt SAR-Studien der voranstehend genannten Verbindungsklasse, bei denen der Biphenylteil Jedoch nicht variiert wurde.

Eine weitere Publikation. Bioorg. & Med. Chem. Letters 2005, Pergamon, Elsevier Science, GB. Band 15, Nr. 17, 3925-3929 (Scott D. Kuduk et al.), beschreibt SAR-Studien einer Verbindungsklasse, die neben einer Biphenylgruppe einen 2,3-Diaminopyridinteil trägt. Auch hier wurde der Biphenylteil nicht variiert.

### DETAILLIERTE BESCHREIBUNG DER ERFINDUNG

In der obigen allgemeinen Formel **I** bedeuten in einer ersten Ausführungsform
- **R¹**: (a) eine gegebenenfalls mit einem Rest **R^{1.1}** substituierte C₁₋₆-Alkylgruppe,
(b) eine C₁₋₃-Alkylgruppe, in der jede Methylengruppe mit bis zu zwei Fluoratomen und jede Methylgruppe mit bis zu drei Fluoratomen substituiert ist,
(c) eine gegebenenfalls mit einem Rest **R^{1.2}** substituierte C₃₋₆-Cycloalkylgruppe,
(d) eine C₂₋₆-Alkenylgruppe,
(e) eine C₂₋₈-Alkinylgruppe,
(f) eine gegebenenfalls mit 1, 2 oder 3 Resten **R^{1.3}** substituierte Aryl-C₀₋₂-alkylengruppe,
(g) ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält und der zusätzlich benzo-kondensiert sein kann,
(h) ein gegebenenfalls mit einem Rest **R^{1.4}** substituierter sechsgliedriger Heteroaryl-Rest, der ein, zwei oder drei N-Atome enthält und der zusätzlich benzo-kondensiert sein kann
(i) -O-**R^{1.1.1}** oder
(j) -N**R^{1.1.3}R^{1.1.4}**,
- **R^{1.1}**: Halogen, -NO₂, -CN, C₃₋₆-Cycloalkyl, -O**R^{1.1.1}**, -S**R^{1.1.1}**, -C(O)**R^{1.1.1}**, -S(O)₂**R^{1.1.2}**, -O-S(O)₂-**R^{1.1.1}**, -CO₂**R^{1.1.1}**, -O-C(O)-**R^{1.1.1}**, -N**R^{1.1.3}R^{1.1.4}**, -N**R^{1.1.3}**-C(O)-**R^{1.1.1}**, -N**R^{1.1.3}**-C(O)-**R^{1.1.1}**, -N**R^{1.1.3}**-CO₂-**R^{1.1.1}** oder -C(O)-N**R^{1.1.3}R^{1.1.4}**,
- **R^{1.1.1}**: (a) H,
(b) C₁-₄-Alkyl,
(c) C₁-₃-Alkyl, in der jede Methylengruppe mit bis zu zwei Fluoratomen und jede Methylgruppe mit bis zu drei Fluoratomen substituiert ist,
(d) eine gegebenenfalls mit 1, 2 oder 3 Resten **R^{1.1.1.1}** substituierte Phenylgruppe,
(e) C₃₋₆-Cycloalkyl oder
(f) eine gegebenenfalls mit 1, 2 oder 3 Resten **R^{1.1.1.2}** substituierte Pyridylgruppe,
- **R^{1.1.1.1}**: unabhängig voneinander
(a) Halogen, -NO₂, -CN, -OH, -O-C₁₋₄-Alkyl, C₃₋₆-Cycloalkyl, C₁-₄-Alkyl oder
(b) C₁-₃-Alkyl, in der jede Methylengruppe mit bis zu zwei Fluoratomen und jede Methylgruppe mit bis zu drei Fluoratomen substituiert ist,
- **R^{1.1.2}**: unabhängig voneinander Halogen oder C₁₋₄-Alkyl,
- **R^{1.1.2}**: (a) C₁₋₄-Alkyl,
(b) C₁₋₃-Alkyl, in der jede Methylengruppe mit bis zu zwei Fluoratomen und jede Methylgruppe mit bis zu drei Fluoratomen substituiert ist,
(c) -O-C₁₋₄-Alkyl oder
(d) eine gegebenenfalls mit 1, 2 oder 3 Resten **R^{1.1.1.1}** substituierte Phenylgruppe,
- **R^{1.1.3}**, **R^{1.1.4}**: unabhängig voneinander
(a) H,
(b) eine gegebenenfalls mit 1, 2 oder 3 Resten **R^{1.1.4.1}** substituierte C₁₋₄-Alkylgruppe,
(c) eine gegebenenfalls mit 1, 2 oder 3 Resten **R^{1.1.1.1}** substituierte Phenylgruppe,
(d) C₃₋₆-Cycloalkyl, oder
- **R^{1.1.3}** und 6-gliedrigen ausgewählt: **R^{1.1.4}** zusammen mit dem N-Atom, an das sie gebunden sind, einen 4-, 5- oder heterocyclischen Ring bilden, der zusätzlich ein weiteres Heteroatom aus N, O und S enthalten kann, oder
- **R^{1.1.3}** und Imid: **R^{1.1.4}** zusammen mit dem N-Atom, an das sie gebunden sind, ein cyclisches bilden,
- **R^{1.1.4.1}**: unabhängig voneinander Halogen, -NH₂, -NH(C₁₋₄-Alkyl), -N(C₁₋₄-Alkyl)₂ oder -SO₂-**R^{1.1.2}**,
- **R^{1.2}**: Halogen, -NO₂, -CN oder Phenyl,
- **R^{1.3}**: (a) Halogen, -NO₂, -CN, -O**R^{1.1.1}**, -S**R^{1.1.1}**, -CO₂**R^{1.1.1}**, C₁₋₆-Alkyl oder
(b) C₁₋₃-Alkyl, in der jede Methylengruppe mit bis zu zwei Fluoratomen und jede Methylgruppe mit bis zu drei Fluoratomen substituiert ist,
- **R^{1.4}**: unabhängig voneinander
(a) Halogen, -NO₂ -CN, -O**R^{1.1.1}**, -S**R^{1.1.1}**, -S(O)-**R^{1.1.2}**, -S(O)₂-**R^{1.1.2}**, -N**R^{1.1.3}R^{1.1.4}**, C₁₋₆-Alkyl,
(b) C₁₋₃-Alkyl, in der jede Methylengruppe mit bis zu zwei Fluoratomen und jede Methylgruppe mit bis zu drei Fluoratomen substituiert ist, oder
(c) eine Oxo-Gruppe,
- **R²**: (a) H oder C₁₋₄-Alkyl,
(b) C₁₋₃-Alkyl, in der jede Methylengruppe mit bis zu zwei Fluoratomen und jede Methylgruppe mit bis zu drei Fluoratomen substituiert ist,
- **R³**: unabhängig voneinander
(a) H, Halogen, -CN, -OH, C₁₋₆-Alkyl, C₃₋₇-Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl, oder
(b) C₁₋₃-Alkyl, in der jede Methylengruppe mit bis zu zwei Fluoratomen und jede Methylgruppe mit bis zu drei Fluoratomen substituiert ist,
- **R⁴**, **R⁵**, **R⁶**, **R⁷**, **R⁸**: unabhängig voneinander
(a) H, Halogen, -CN, -OH,
(b) C₁₋₆-Alkyl, wobei zwei benachbarte Substituenten gemeinsam eine Trimethylen- oder Tetramethylengruppe darstellen können,
(c) C₁₋₃-Alkyl, in der jede Methylengruppe mit bis zu zwei Fluoratomen und jede Methylgruppe mit bis zu drei Fluoratomen substituiert ist,
(d) C₃₋₇-Cycloalkyl,
(e) -O-C₁₋₆-Alkyl, wobei zwei benachbarte Substituenten eine Methylendioxy- oder Ethylendioxygruppe darstellen können,
(f) -O-CF₃, -O-C₃₋₇-Cycloalkyl,
(g) -NH₂, -NH(C₁₋₃-Alkyl), -N(C₁₋₃-Alkyl)₂,
(h) -C(O)-**R^{8.1}**,
(i) -SO₂-**R^{8.2}**,
(j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, lsoxazolyl, Oxadiazolyl, Thiazolyl, Isothiazolyl, Thiadiazolyl, Imidazolyl, Pyrazolyl, Triazolyl und Tetrazolyl, oder
(k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
- **R⁸⁻¹**: -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇-Cycloalkyl,
- **R^{8.2}**: -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
- **n**: eine der Ziffern 0, 1, 2, 3 oder 4,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R²**, **R³**, **R⁴**, **R⁵**, **R⁶**, **R⁷**, **R⁸** und n wie voranstehend unter der ersten Ausführungsform erwähnt definiert sind und
- **R¹**: (a) eine gegebenenfalls mit einem Rest **R^{1.1}** substituierte C₁₋₆-Alkylgruppe,
(b) eine C₁₋₃-Alkylgruppe, in der jede Methylengruppe mit bis zu zwei Fluoratomen und jede Methylgruppe mit bis zu drei Fluoratomen substituiert ist,
(c) eine gegebenenfalls mit einem Rest **R^{1.2}** substituierte C₃₋₆-Cycloalkylgruppe,
(d) eine gegebenenfalls mit 1, 2 oder 3 Resten **R^{1.3}** substituierte Phenylgruppe,
(e) ein gegebenenfalls mit einem Rest **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält, oder
(f) ein gegebenenfalls mit einem Rest **R^{1.4}** substituierter sechsgliedriger Heteroaryl-Rest, der ein, zwei oder drei N-Atome enthält,
- **R^{1.1}**: C₃₋₆-Cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂-C₁₋₆-Alkyl oder -O-S(O)₂-C₁₋₆-Alkyl,
- **R^{1.1.1}**: (a) H,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist, oder
(d) C₃₋₆-Cycloalkyl,
- **R^{1.1.3}**, **R^{1.1.4}**: unabhängig voneinander
(a) H,
(b) C₁₋₄-Alkyl oder
(c) C₃₋₆-Cycloalkyl, und

- **R^{1.2}**: Halogen, -NO₂ -CN oder Phenyl,
- **R^{1.3}**: (a) Halogen, -NO₂ -CN, -O**R^{1.1.1}**, -S**R^{1.1.1}**, -CO₂**R^{1.1.1}**, C₁₋₆-Alkyl oder
(b) C₁₋₃-Alkyl, in der jede Methylengruppe mit bis zu zwei Fluoratomen und jede Methylgruppe mit bis zu drei Fluoratomen substituiert ist, und
- **R^{1.4}**: (a) Halogen, -NO₂, -CN, -O**R^{1.1.1}**, -S**R^{1.1.1}**, -N**R^{1.1.3}R^{1.1.4}**, C₁₋₆-Alkyl,
(b) C₁₋₃-Alkyl, in der jede Methylengruppe mit bis zu zwei Fluoratomen und jede Methylgruppe mit bis zu drei Fluoratomen substituiert ist, oder
(c) eine Oxo-Gruppe bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen
- **R**¹: (a) eine gegebenenfalls mit einem Rest **R^{1.1}** substituierte C₁₋₆-Alkylgruppe,
(b) eine C₁₋₃-Alkylgruppe, in der jede Methylengruppe mit bis zu zwei Fluoratomen und jede Methylgruppe mit bis zu drei Fluoratomen substituiert ist,
(c) eine gegebenenfalls mit einem Rest **R^{1.2}** substituierte C₃₋₆-Cycloalkylgruppe,
(d) eine gegebenenfalls mit 1, 2 oder 3 Resten **R^{1.3}** substituierte Phenylgruppe,
(e) ein gegebenenfalls mit einem Rest **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält, oder
(f) ein gegebenenfalls mit einem Rest **R^{1.4}** substituierter sechsgliedriger Heteroaryl-Rest, der ein, zwei oder drei N-Atome enthält,
- **R^{1.1}**: C₃₋₆-Cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂-C₁₋₆-Alkyl oder -O-S(O)₂-C₁₋₆-Alkyl,
- **R^{1.1.1}**: (a) H,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist, oder
(d) C₃₋₆-Cycloalkyl, unabhängig voneinander

- **R^{1.1.3} R^{1.1.4}**: (a) H,
(b) C₁₋₄-Alkyloder
(c) C₃₋₆-Cycloalkyl,
- **R^{1.2}**: Halogen, -NO₂, -CN oder Phenyl,
- **R^{1.3}**: (a) Halogen, -NO₂, -CN, -O**R^{1.1.1}**, -S**R^{1.1.1}**, -CO₂**R^{1.1.1}**, C₁₋₆-Alkyl oder
(b) C₁₋₃-Alkyl, in der jede Methylengruppe mit bis zu zwei Fluoratomen und jede Methylgruppe mit bis zu drei Fluoratomen substituiert ist,
- **R^{1.4}**: (a) Halogen, -NO₂, -CN, -O**R^{1.1.1}**, -S**R^{1.1.1}**, -N**R^{1.1.3}R^{1.1.4}**, C₁₋₆-Alkyl,
(b) C₁₋₃-Alkyl, in der jede Methylengruppe mit bis zu zwei Fluoratomen und jede Methylgruppe mit bis zu drei Fluoratomen substituiert ist, oder
(c) eine Oxo-Gruppe
- **R²**: H oder C₁₋₃-Alkyl,
- **R³**: unabhängig voneinander
(a) H, Halogen, C₁₋₃-Alkyl, -O-C₁₋₃-Alkyl oder
(b) C₁₋₃-Alkyl, in der jede Methylengruppe mit bis zu zwei Fluoratomen und jede Methylgruppe mit bis zu drei Fluoratomen substituiert ist,
- **R⁴**: H oder Halogen,
- **R⁵**: (a) H, Halogen,
(b) C₁₋₃-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -C(O)-O-C₁₋₃-Alkyl oder -C(O)-NH₂,

- **R⁶**: (a) H, Halogen,
(b) C₁₋₃-Alkylen-**R^{6.1}**,
(c) C₁₋₃-Alkyl, in der jede Methylengruppe mit bis zu zwei Fluoratomen und jede Methylgruppe mit bis zu drei Fluoratomen substituiert ist,
(d) -OH, -O-C₁₋₄-Alkyl,
(e) -O-CHF₂, -O-CF₃,
(f) -C(O)-O-**R^{6.2}**, -CN, -C(O)-CH₃, -C(O)-NH₂ oder
(g) Pyrrolyl,
- **R^{6.1}**: H, -OH,
- **R^{6.2}**: H, C₁₋₃-Alkyl,
- **R⁷**: (a) H, Halogen,
(b) C₁₋₃-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -C(O)-NH₂ oder -C(O)-Pyrrolidinyl, und
- **R⁸**: (a) H, Halogen,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -O-CF₃,
(f) -CN, -C(O)-NH₂ oder
(g) Pyrrolyl,
bedeuten, deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R²**, **R³**, **R⁴**, **R⁵**, **R⁶**, **R⁷**, **R⁸** und n wie voranstehend unter der ersten Ausführungsform erwähnt definiert sind und
- **R¹**: (a) eine gegebenenfalls mit einem Rest **R^{1.1}** substituierte C₁₋₆-Alkylgruppe,
(b) eine C₁₋₃-Alkylgruppe, in der jede Methylengruppe mit bis zu zwei Fluoratomen und jede Methylgruppe mit bis zu drei Fluoratomen substituiert ist,
(c) eine gegebenenfalls mit 1, 2 oder 3 Resten **R^{1.3}** substituierte Phenylgruppe,
(d) ein gegebenenfalls mit einem Rest **R^{1.4}** substituierter fünfgliedriger Heteroaryl-Rest, der ausgewählt ist aus der Gruppe bestehend aus Furanyl, Isoxazolyl, Isothiazolyl, Oxazolyl, Thiazolyl, Pyrazolyl, Thiadiazolyl, Oxadiazolyl, Triazolyl, Tetrazolyl, Imidazolyl, Indolyl, Thienyl, Pyrrolyl, Benzimidazolyl, Benzoxazolyl, Benzthiazolyl und Benzisoxazinyl, oder
(e) ein gegebenenfalls mit einem Rest **R^{1.4}** substituierter sechsgliedriger Heteroaryl-Rest, der ausgewählt ist aus der Gruppe bestehend aus Pyridyl, Pyrimidinyl, Pyridazinyl, Pyrazinyl, Triazinyl, Chinazolinyl und Chinoxazinyl,
- **R^{1.1}**: C₃₋₆-Cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂C₁₋₆-Alkyl oder -O-S(O)₂-C₁₋₆-Alkyl,
- **R^{1.1.1}**: (a) H,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist, oder
(d) C₃₋₆-Cycloalkyl,
- **R^{1.1.3}**, **R^{1.1.4}**: unabhängig voneinander
(a) H,
(b) C₁₋₄-Alkyl oder
(c) C₃₋₆-Cycloalkyl,
- **R^{1.3}**: (a) Halogen, -NO₂, -CN, -O**R^{1.1.1}**, -S**R^{1.1.1}**, -CO₂**R^{1.1.1}**, C₁₋₆-Alkyl oder
(b) C₁₋₃-Alkyl, in der jede Methylengruppe mit bis zu zwei Fluoratomen und jede Methylgruppe mit bis zu drei Fluoratomen substituiert ist, und
- **R^{1.4}**: (a) Halogen, -NO₂, -CN, -O**R^{1.1.1}**, -S**R^{1.1.1}**, C₁₋₆-Alkyl,
(b) C₁₋₃-Alkyl, in der jede Methylengruppe mit bis zu zwei Fluoratomen und jede Methylgruppe mit bis zu drei Fluoratomen substituiert ist, oder
(c) eine Oxo-Gruppe bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R²**, **R³**, **R⁴**, **R⁵**, **R⁶**, **R⁷**, **R⁸** und n wie voranstehend unter der ersten Ausführungsform erwähnt definiert sind und
- **R¹**: eine Gruppe ausgewählt aus 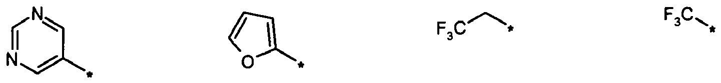 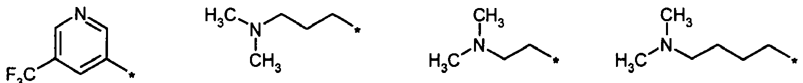 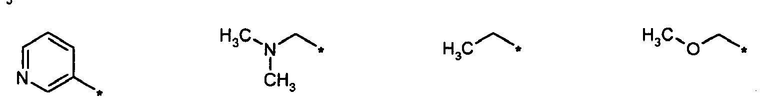 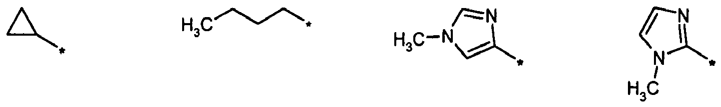 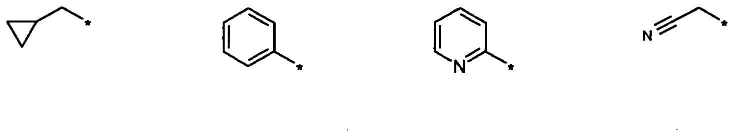 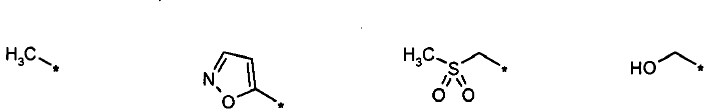 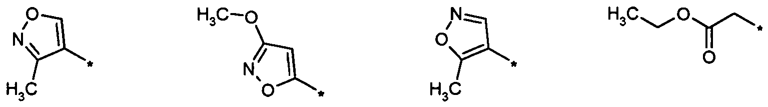 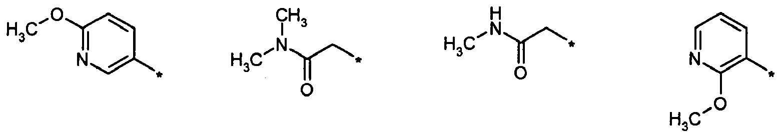 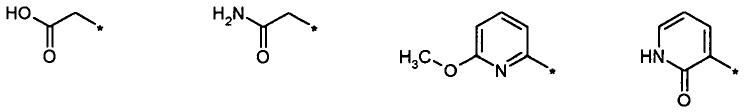 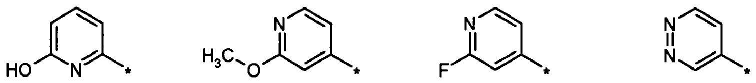 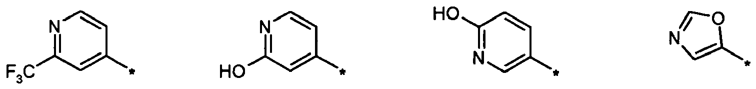 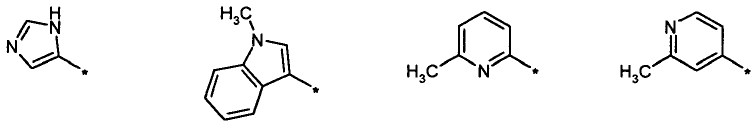 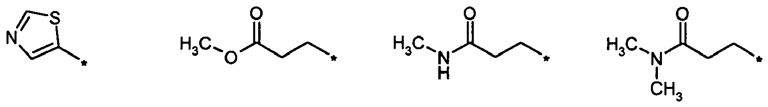 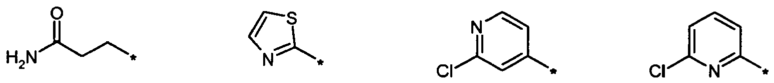 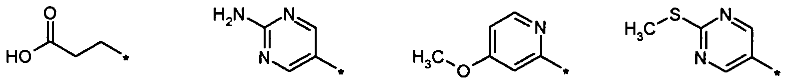 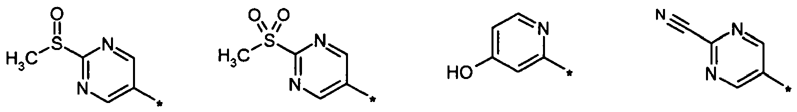
bedeutet, deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R³**, **R⁴**, **R⁵**, **R⁶**, **R⁷**, **R⁸** und n wie voranstehend unter der ersten Ausführungsform erwähnt definiert sind und
- **R²**: H oder C₁₋₃-Alkyl bedeutet,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R²**, **R⁴**, **R⁵**, **R⁶**, **R⁷**, **R⁸** und n wie voranstehend unter der ersten Ausführungsform erwähnt definiert sind und
- **R³**: unabhängig voneinander
(a) H, Halogen, C₁₋₃-Alkyl, -O-C₁₋₃-Alkyl oder
(b) C₁₋₃-Alkyl, in der jede Methylengruppe mit bis zu zwei Fluoratomen und jede Methylgruppe mit bis zu drei Fluoratomen substituiert ist, bedeutet,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R²**, **R³**, **R⁵**, **R⁶**, **R⁷**, **R⁸** und n wie voranstehend unter der ersten Ausführungsform erwähnt definiert sind und
- **R⁴**: H oder Halogen bedeutet,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R²**, **R³**, **R⁴**, **R⁶**, **R⁷**, **R⁸** und n wie voranstehend unter der ersten Ausführungsform erwähnt definiert sind und
- **R⁵**: (a) H, Halogen,
(b) C₁₋₃-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -C(O)-O-C₁₋₃-Alkyl oder -C(O)-NH₂ bedeutet,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R²**, **R³**, **R⁴**, **R⁵**, **R⁷**, **R⁸** und n wie voranstehend unter der ersten Ausführungsform erwähnt definiert sind und
- **R⁶**: (a) H, Halogen,
(b) C₁₋₃-Alkylen-**R^{6.1}**
(c) C₁₋₃-Alkyl, in der jede Methylengruppe mit bis zu zwei Fluoratomen und jede Methylgruppe mit bis zu drei Fluoratomen substituiert ist,
(d) -OH, -O-C₁₋₄-Alkyl,
(e) -O-CHF₂, -O-CF₃,
(f) -C(O)-O-**R^{6.2}**, -CN, -C(O)-CH₃, -C(O)-NH₂ oder
(g) Pyrrolyl,
- **R^{6.1}**: H oder -OH und

- **R^{6.2}**: H oder C₁₋₃-Alkyl bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R²**, **R³**, **R⁴**, **R⁵**, **R⁶**, **R⁸** und n wie voranstehend unter der ersten Ausführungsform erwähnt definiert sind und
- **R⁷**: (a) H, Halogen,
(b) C₁₋₃-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -C(O)-NH₂ oder -C(O)-Pyrrolidinyl bedeutet,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in denen **R¹**, **R²**, **R³**, **R⁴**, **R⁵**, **R⁶**, **R⁷** und n wie voranstehend unter der ersten Ausführungsform erwähnt definiert sind und
- **R⁸**: (a) H, Halogen,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(f) -CN, -C(O)-NH₂ oder
(g) Pyrrolyl bedeutet,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel I, in der
- **R¹**: eine Gruppe ausgewählt aus

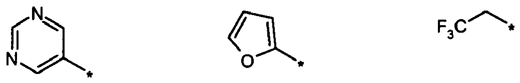

- **R²**: H oder -CH₃,
- **R³**: H, F, -CF₃, -CH₃ oder -O-CH₃,
- **R⁴**: H oder Cl,
- **R⁵**: H, Cl, C₁₋₃-Alkyl, -CF₃, -O-C₁₋₃-Alkyl, -C(O)-O-C₁₋₃-Alkyl oder -C(O)-NH₂,
- **R⁶**: H, F, Cl, C₁₋₃-Alkyl, -CF₃, -O-C₁₋₄-Alkyl, -OCF₃, -C(O)-NH₂ oder Pyrrolyl,
- **R⁷**: H, F, Cl, C₁₋₃-Alkyl, -CF₃, -O-C₁₋₃-Alkyl, -C(O)-NH₂ oder -C(O)-Pyrrolidinyl und
- **R⁸**: H, F, Cl, Br, C₁₋₄-Alkyl, -CF₃, -O-C₁₋₃-Alkyl, -OCF₃, -C(O)-NH₂, -OCF₃ oder Pyrrolyl
bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der obigen allgemeinen Formel **I**, in der

**R¹** eine Gruppe ausgewählt aus 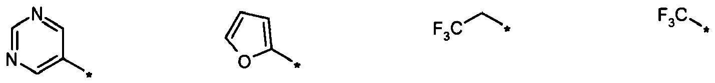 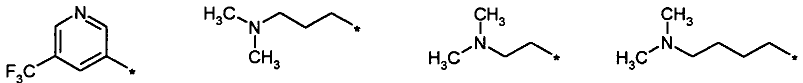 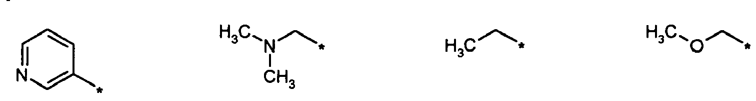 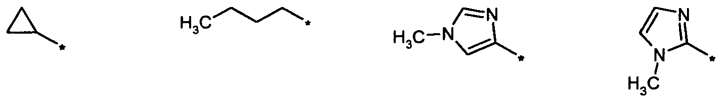 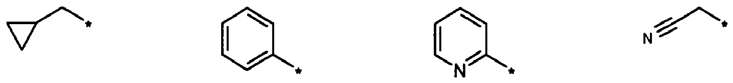 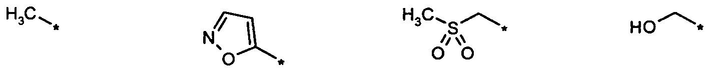 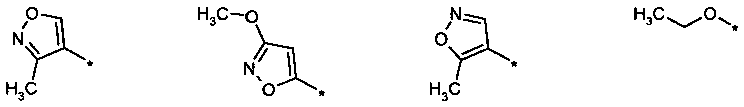 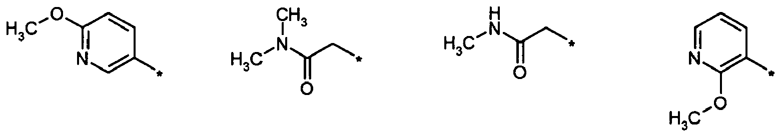 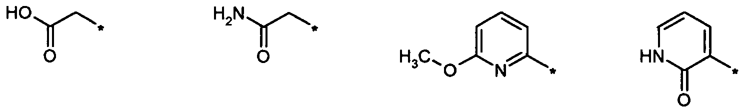 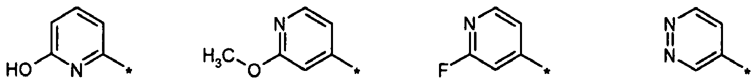 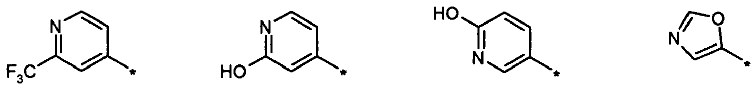 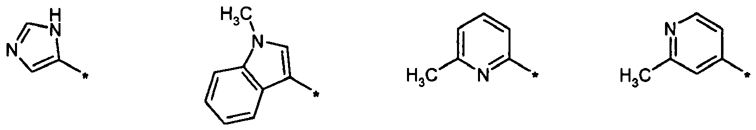 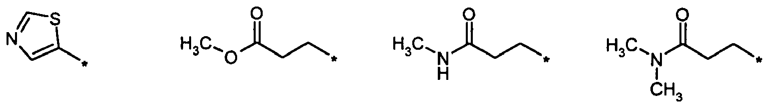 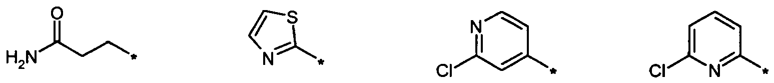 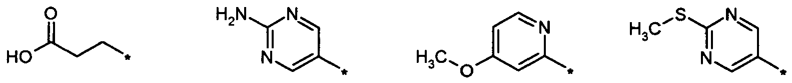 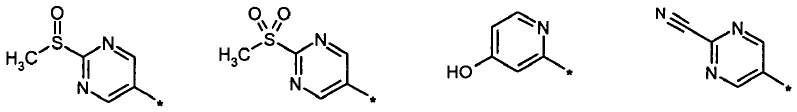
- **R²**: H, -CH₃ oder -C₂H₅,
- **R³**: H, F, Cl, -CF₃, -CH₃ oder -O-CH₃,
- **R⁴**: H oder Cl,
- **R⁵**: H, Cl, C₁₋₃-Alkyl, -CF₃, -O-C₁₋₃-Alkyl, -C(OhO-C₁₋₃-Alkyl oder -C(O)-NH₂,
- **R⁶**: H, F, Cl, Br, -CN, C₁₋₃-Alkyl, -CF₃, -COOH, -COO-C₁₋₃-Alkyl, -CH(OH)CH₃, -OH, -O-C₁₋₄-Alkyl, -OCF₃, -OCHF₂, -C(O)-CH₃, -C(O)-NH₂ oder Pyrrolyl,
- **R⁷**: H, F, Cl, C₁₋₃-Alkyl, -CF₃, -O-C₁₋₃-Alkyl, -C(O)-NH₂ oder -C(O)-Pyrrolidinyl und
- **R⁸**: H, F, Cl, Br, -CN, C₁₋₄-Alkyl, -CF₃, -O-C₁₋₃-Alkyl, -OCF₃, -C(O)-NH₂, -OCF₃ oder Pyrrolyl,
bedeuten, deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der allgemeinen Formel la, 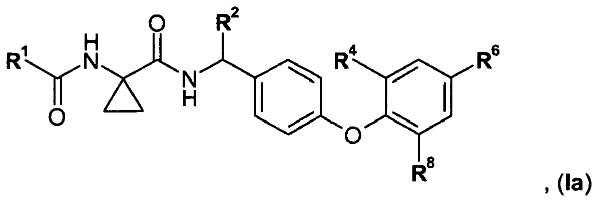 in der
- **R¹**: eine gegebenenfalls mit einem Rest **R^{1.1}** substituierte C₁₋₆-Alkylgruppe,
- **R^{1.1}**: Halogen, -NO₂, -CN, C₃₋₆-Cycloalkyl, -O**R^{1.1.1}**, -S**R^{1.1.1}**, -C(O)**R^{1.1.1}**, -S(O)₂-**R^{1.1.2}**, -O-S(O)₂-**R^{1.1.1}**, -CO₂**R^{1.1.1}**, -O-C(O)-**R^{1.1.1}**, -N**R^{1.1.3}R^{1.1.4}**, -N**R^{1.1.3}**-C(O)-**R^{1.1.1}**, -N**R^{1.1.3}**-C(O)-**R^{1.1.1}**, -N**R^{1.1.3}**-CO₂-**R^{1.1.1}** oder -C(O)-N**R^{1.1.3}R^{1.1.4}**,
- **R^{1.1.1}**: (a) H,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist, oder
(d) C₃₋₆-Cycloalkyl,
- **R^{1.1.2}**: (a) C₁₋₄-Alkyl,
(b) C₁₋₃-Alkyl, in der jede Methylengruppe mit bis zu zwei Fluoratomen und jede Methylgruppe mit bis zu drei Fluoratomen substituiert ist, oder
(c) -O-C₁₋₄-Alkyl,
- **R^{1.1.3}**, **R^{1.1.4}**: unabhängig voneinander
(a) H,
(b) eine gegebenenfalls mit 1, 2 oder 3 Resten **R^{1.1.4.1}** substituierte C₁₋₄-Alkylgruppe oder
(c) C₃₋₆-Cycloalkyl
- **R²**: (a) H, C₁₋₄-Alkyl oder
(b) C₁₋₃-Alkyl, in der jede Methylengruppe mit bis zu zwei Fluoratomen und jede Methylgruppe mit bis zu drei Fluoratomen substituiert ist,
- **R⁴**: H oder Halogen bedeutet,
- **R⁶**: (a) H, Halogen,
(b) C₁₋₃-Alkylen-**R^{6.1}**,
(c) C₁₋₃-Alkyl, in der jede Methylengruppe mit bis zu zwei Fluoratomen und jede Methylgruppe mit bis zu drei Fluoratomen substituiert ist,
(d) -OH, -O-C₁₋₄-Alkyl,
(e) -O-CHF₂, -O-CF₃,
(f) -C(O)-O-**R^{6.2}**, -CN, -C(O)-CH₃, -C(O)-NH₂ oder
(g) Pyrrolyl bedeutet,
- **R^{6.1}**: H, -OH,
- **R^{6.2}**: H, C₁₋₃-Alkyl, und
- **R⁸**: (a) H, Halogen,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -O-CF₃,
(f) -CN, -C(O)-NH₂ oder
(g) Pyrrolyl bedeuten,
deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der allgemeinen Formel la, in der
- **R¹**: eine Gruppe ausgewählt aus

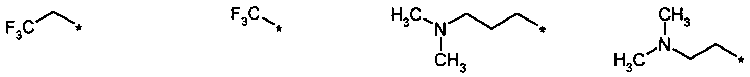

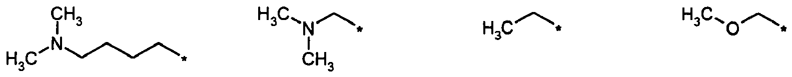

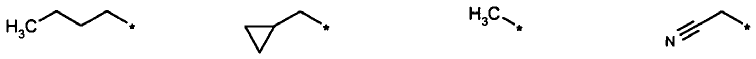

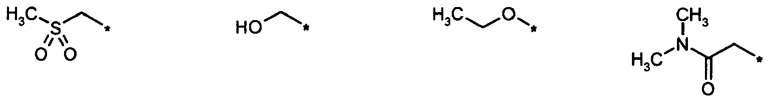

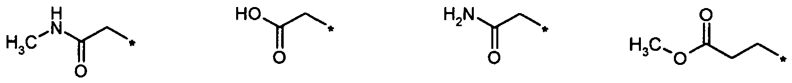

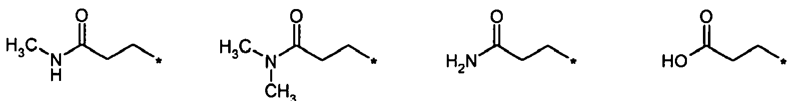

- **R²**: H, -CH₃ oder -C₂H₅,
- **R⁴**: H oder Cl,
- **R⁶**: Cl oder -O-C₁₋₄-Alkyl, und
- **R⁸**: F oder -CF₃,
bedeuten, deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der allgemeinen Formel **la**, 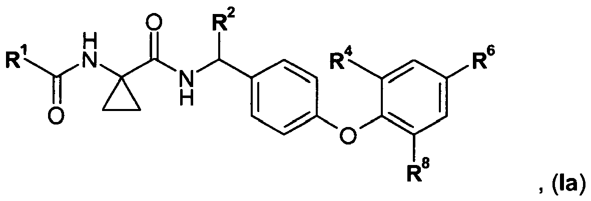 in der
- **R¹**: (a) ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält und der zusätzlich benzo-kondensiert sein kann, oder
(b) ein gegebenenfalls mit einem Rest **R^{1.4}** substituierter sechsgliedriger Heteroaryl-Rest, der ein, zwei oder drei N-Atome enthält und der zusätzlich benzo-kondensiert sein kann,
- **R^{1.4}**: unabhängig voneinander
(a) Halogen, -NO₂, -CN, -O**R^{1.1.1}**, -S**R^{1.1.1}**, -S(O)-**R^{1.1.2}**, -S(O)₂-**R^{1.1.2}**, -N**R^{1.1.3}R^{1.1.3}**, C₁₋₆-Alkyl oder
(b) C₁₋₃-Alkyl, in der jede Methylengruppe mit bis zu zwei Fluoratomen und jede Methylgruppe mit bis zu drei Fluoratomen substituiert ist,
- **R^{1.1.1}**: (a) H,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist, oder
(d) C₃₋₆-Cycloalkyl,
- **R^{1.1.2}**: (a) C₁₋₄-Alkyl,
(b) C₁₋₃-Alkyl, in der jede Methylengruppe mit bis zu zwei Fluoratomen und jede Methylgruppe mit bis zu drei Fluoratomen substituiert ist, oder
(c) -O-C₁₋₄-Alkyl,
- **R^{1.1.3}**, **R^{1.1.4}**: unabhängig voneinander
(a) H,
(b) eine gegebenenfalls mit 1, 2 oder 3 Resten **R^{1.1.4.1}** substituierte C₁₋₄-Alkylgruppe,
(c) C₃₋₆-Cycloalkyl, oder
- **R²**: (a) H, C₁₋₄-Alkyl oder
(b) C₁₋₃-Alkyl, in der jede Methylengruppe mit bis zu zwei Fluoratomen und jede Methylgruppe mit bis zu drei Fluoratomen substituiert ist,
- **R⁴**: H oder Halogen bedeutet,
- **R⁶**: (a) H, Halogen,
(b) C₁₋₃-Alkylen-**R^{6.1}**,
(c) C₁₋₃-Alkyl, in der jede Methylengruppe mit bis zu zwei Fluoratomen und jede Methylgruppe mit bis zu drei Fluoratomen substituiert ist,
(d) -OH, -O-C₁₋₄-Alkyl,
(e) -O-CHF₂, -O-CF₃,
(f) -C(O)-O-**R^{6.2}**, -CN, -C(O)-CH₃, -C(O)-NH₂ oder
(g) Pyrrolyl bedeutet,
- **R^{6.1}**: H, -OH,
- **R^{6.2}**: H, C₁₋₃-Alkyl, und
- **R⁸**: (a) H, Halogen,
(b) C₁₋₄-Alkyl,
(c) C₁₋₃-Alkyl, in der jede Methylengruppe mit bis zu zwei Fluoratomen und jede Methylgruppe mit bis zu drei Fluoratomen substituiert ist,
(d) -O-C₁₋₃-Alkyl,
(e) -O-CF₃,
(f) -CN, -C(O)-NH₂ oder
(g) Pyrrolyl bedeuten,

deren Enantiomere, deren Diastereomere, deren Gemische und deren Salze, insbesondere deren physiologisch verträgliche Salze mit organischen oder anorganischen Säuren oder Basen.

Eine weitere Ausführungsform der vorliegenden Erfindung besteht in den Verbindungen der allgemeinen Formel la, in der
- **R¹**: eine Gruppe ausgewählt aus

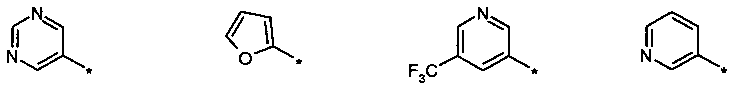

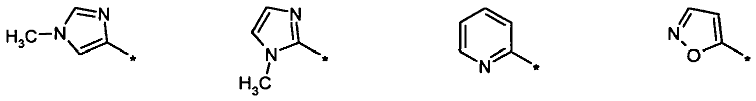

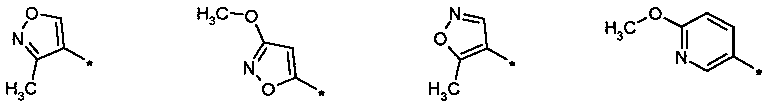

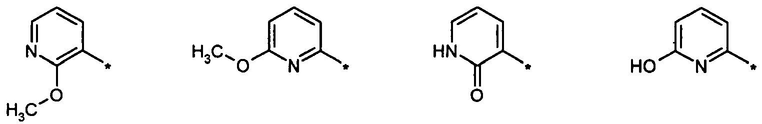

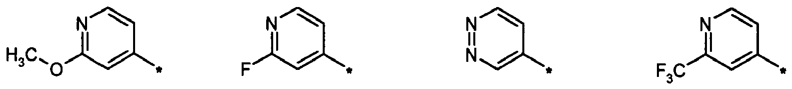

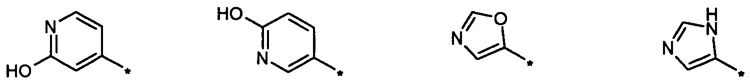

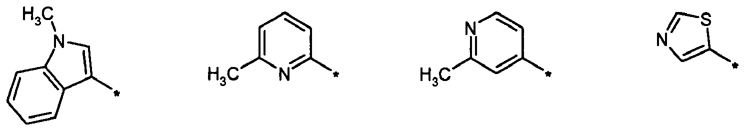

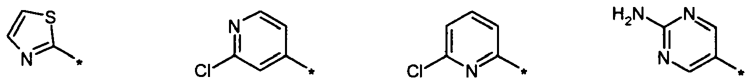

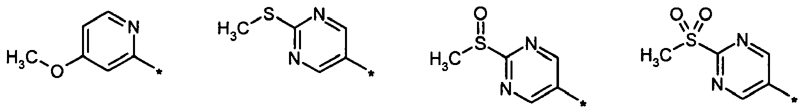

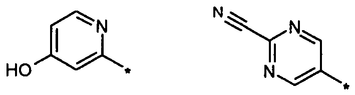

- **R²**: H, -CH₃ oder -C₂H₅,
- **R⁴**: H oder Cl,
- **R⁶**: F, Cl, Br, -CN, -CH₃, -CF₃, -COOH, -COO-CH₃, -CH(OH)CH₃, -OH, -O-CH₃, -OCF₃, -OCHF₂, -C(O)-CH₃, -C(O)-NH₂ oder Pyrrolyl, und
- **R⁸**: H, F, Cl, Br, -CN, C₁₋₄-Alkyl, -CF₃, -O-C₁₋₃-Alkyl, -OCF₃, -C(O)-NH₂, -OCF₃ oder Pyrrolyl,
bedeuten,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel I seien beispielsweise folgende genannt:

| Nr. | Struktur |
|---|---|
| (1) | 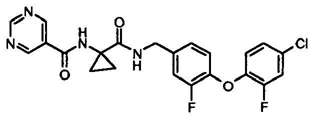 |
| (2) | 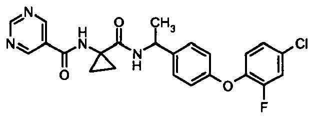 |
| (3) | 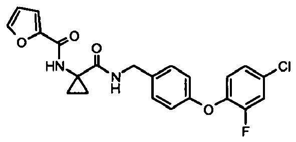 |
| (4) | 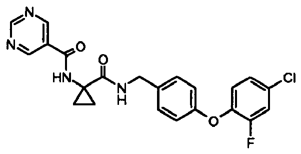 |
| (5) | 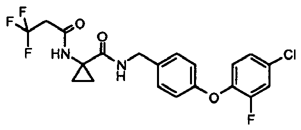 |
| (6) | 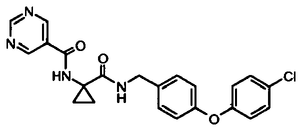 |
| (7) | 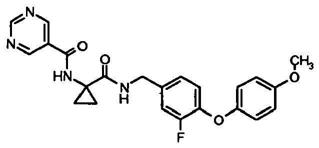 |
| (8) | 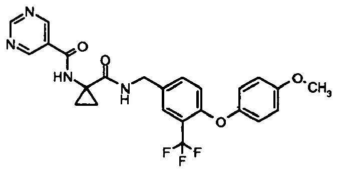 |
| (9) | 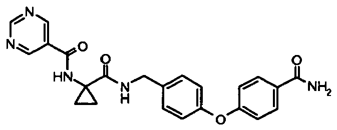 |
| (10) | 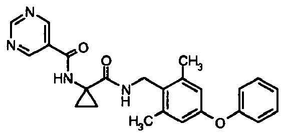 |
| (11) | 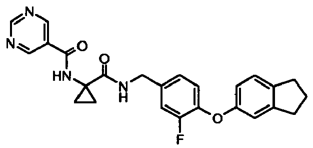 |
| (12) | 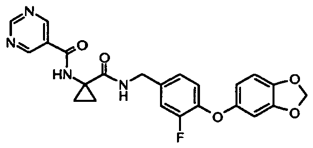 |
| (13) | 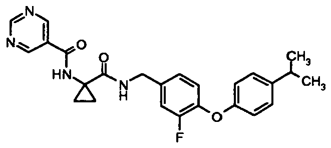 |
| (14) | 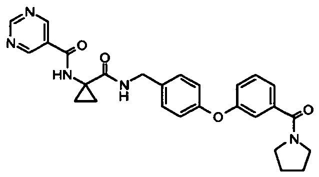 |
| (15) | 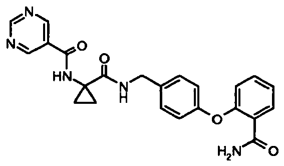 |
| (16) | 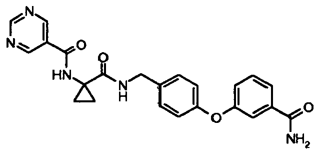 |
| (17) | 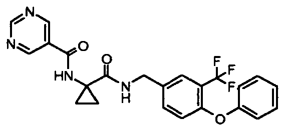 |
| (18) | 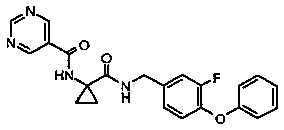 |
| (19) | 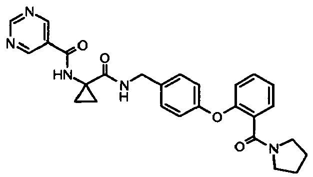 |
| (20) | 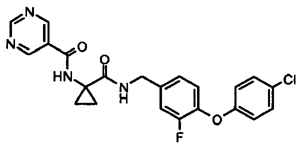 |
| (21) | 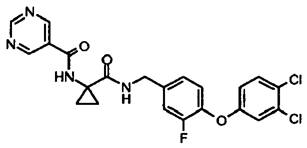 |
| (22) | 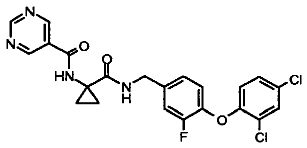 |
| (23) | 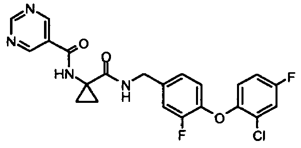 |
| (24) | 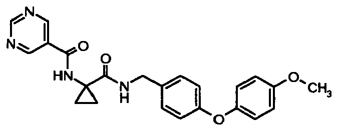 |
| (25) | 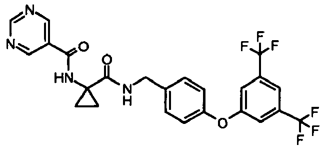 |
| (26) | 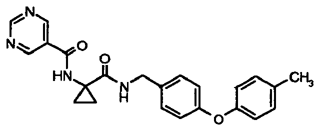 |
| (27) | 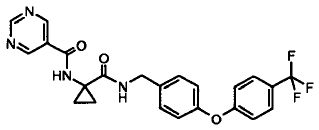 |
| (28) | 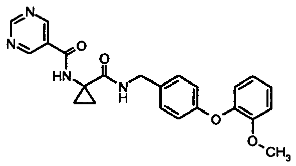 |
| (29) | 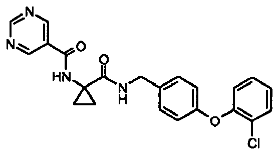 |
| (30) | 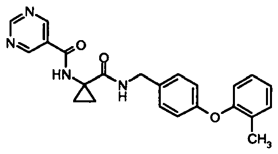 |
| (31) | 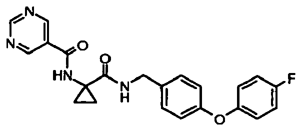 |
| (32) | 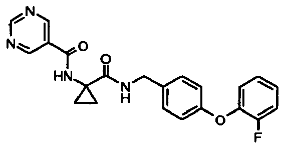 |
| (33) | 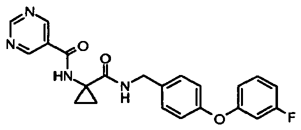 |
| (34) | 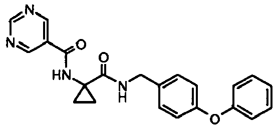 |
| (35) | 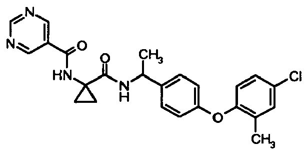 |
| (36) | 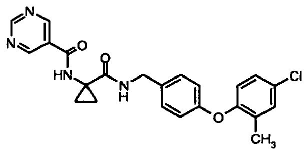 |
| (37) | 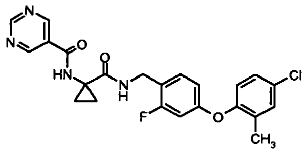 |
| (38) | 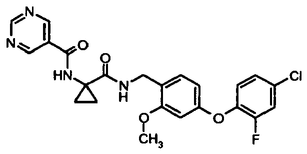 |
| (39) | 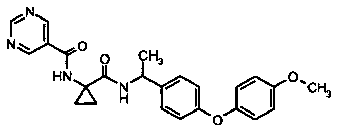 |
| (40) | 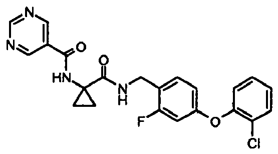 |
| (41) | 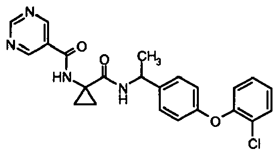 |
| (42) | 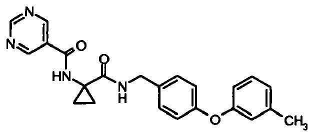 |
| (43) | 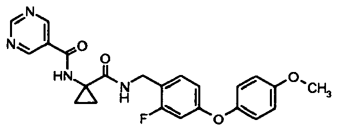 |
| (44) | 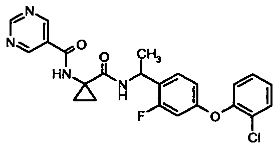 |
| (45) | 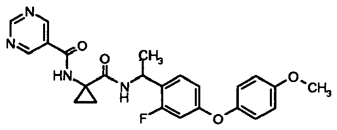 |
| (46) | 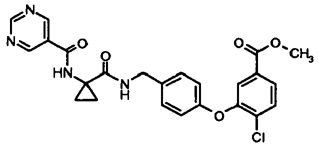 |
| (47) | 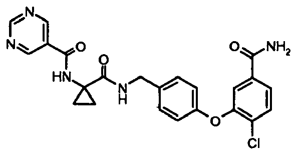 |
| (48) | 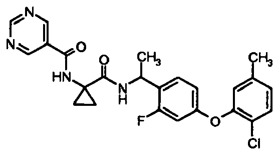 |
| (49) | 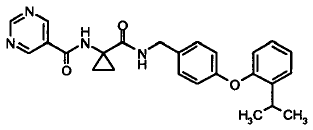 |
| (50) | 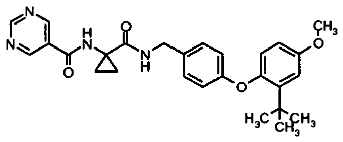 |
| (51) | 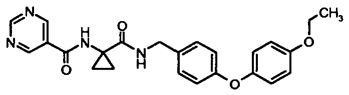 |
| (52) | 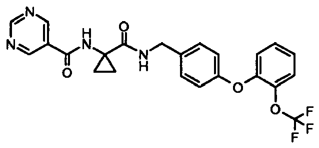 |
| (53) | 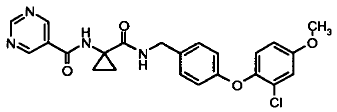 |
| (54) | 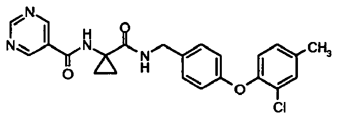 |
| (55) | 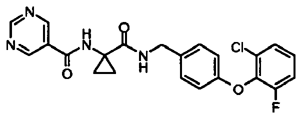 |
| (56) | 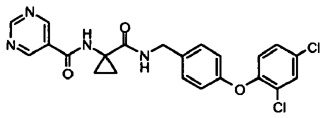 |
| (57) | 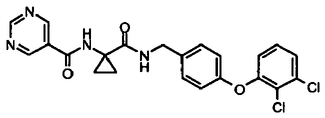 |
| (58) | 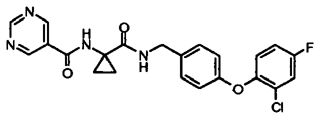 |
| (59) | 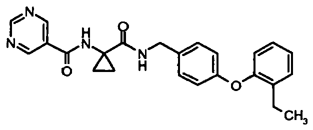 |
| (60) | 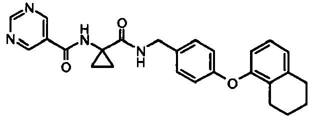 |
| (61) | 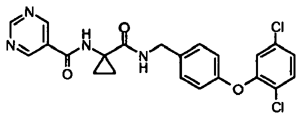 |
| (62) | 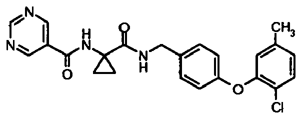 |
| (63) | 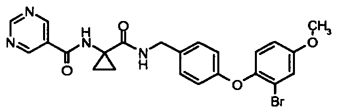 |
| (64) | 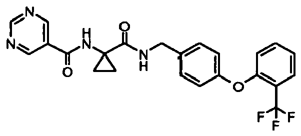 |
| (65) | 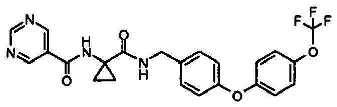 |
| (66) | 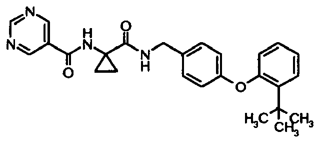 |
| (67) | 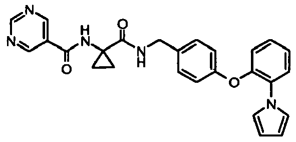 |
| (68) | 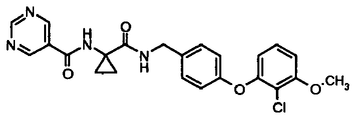 |
| (69) | 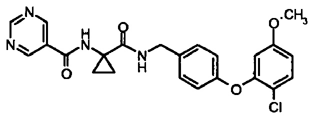 |
| (70) | 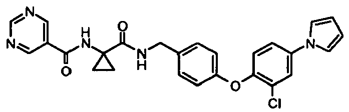 |
| (71) | 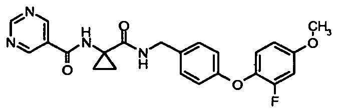 |
| (72) | 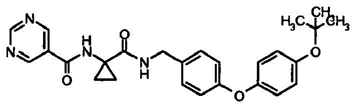 |
| (73) | 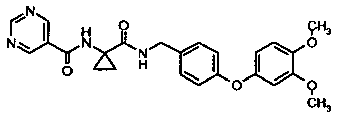 |
| (74) | 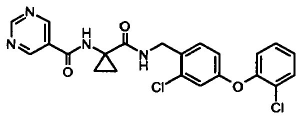 |
| (75) | 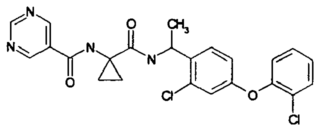 |
| (76) | 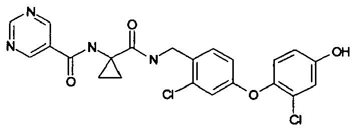 |
| (77) | 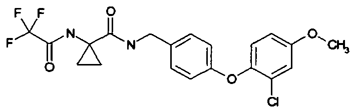 |
| (78) | 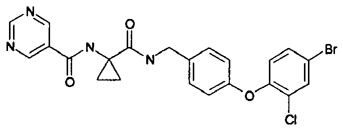 |
| (79) | 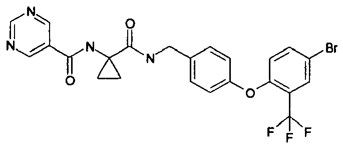 |
| (80) | 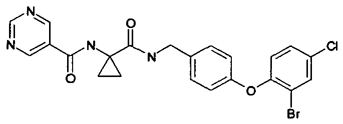 |
| (81) | 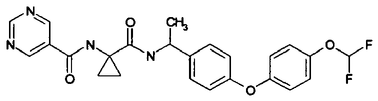 |
| (82) | 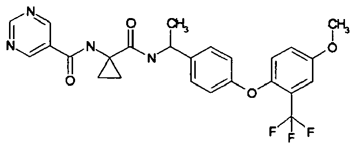 |
| (83) | 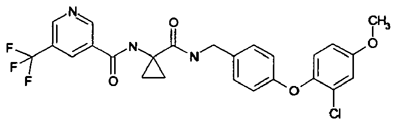 |
| (84) | 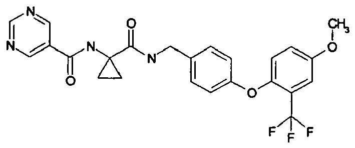 |
| (85) | 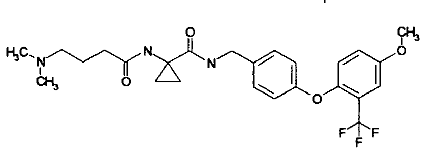 |
| (86) | 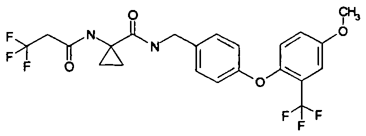 |
| (87) | 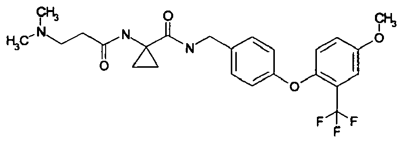 |
| (88) | 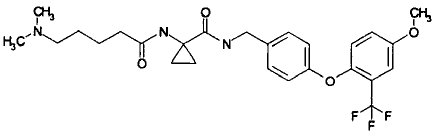 |
| (89) | 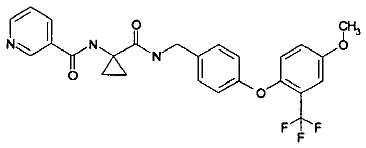 |
| (90) | 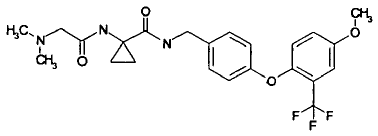 |
| (91) | 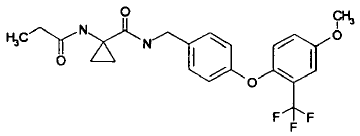 |
| (92) | 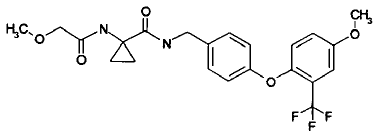 |
| (93) | 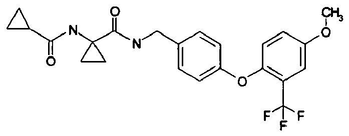 |
| (94) | 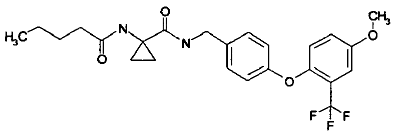 |
| (95) | 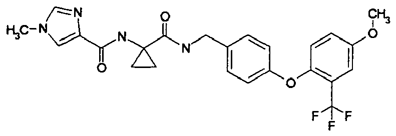 |
| (96) | 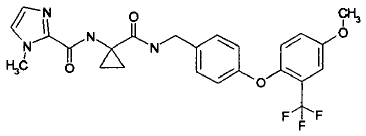 |
| (97) | 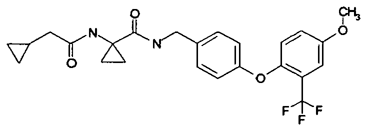 |
| (98) | 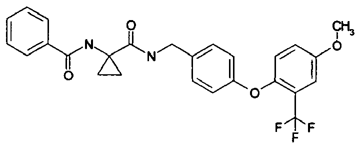 |
| (99) | 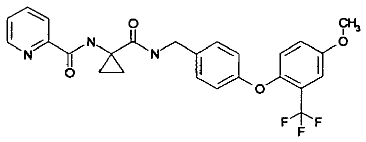 |
| (100) | 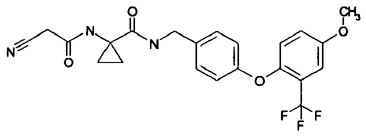 |
| (101) | 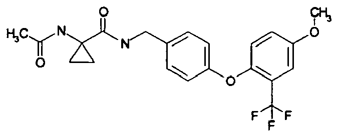 |
| (102) | 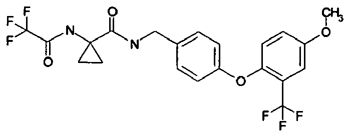 |
| (103) | 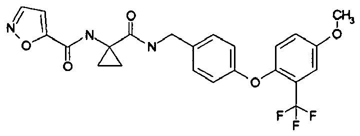 |
| (104) | 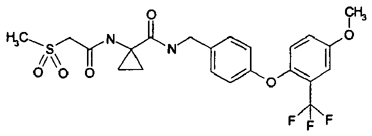 |
| (105) | 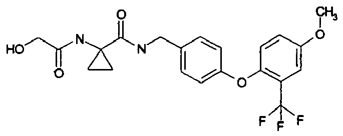 |
| (106) | 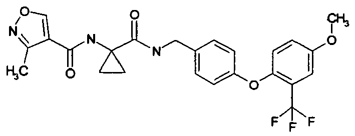 |
| (107) | 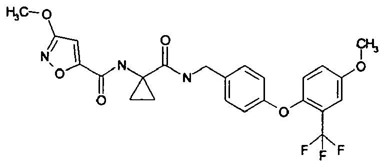 |
| (108) | 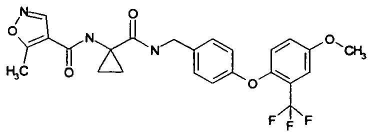 |
| (109) | 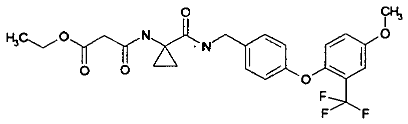 |
| (110) | 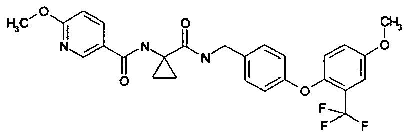 |
| (111) | 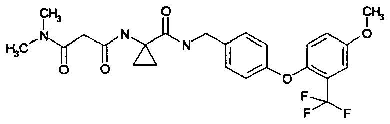 |
| (112) | 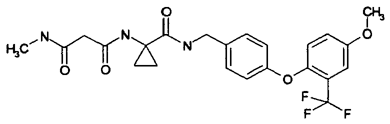 |
| (113) | 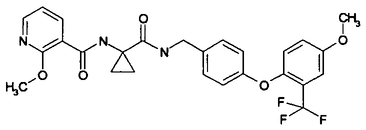 |
| (114) | 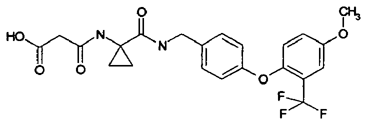 |
| (115) | 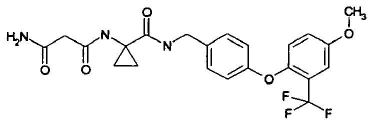 |
| (116) | 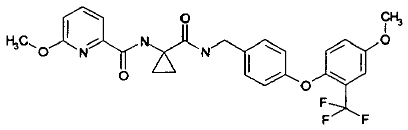 |
| (117) | 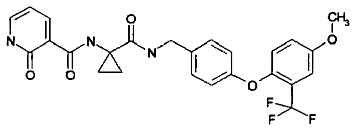 |
| (118) | 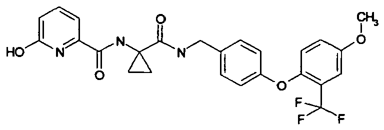 |
| (119) | 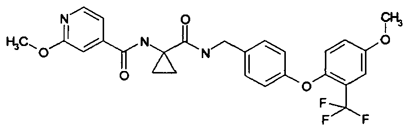 |
| (120) | 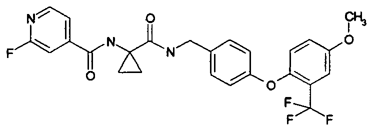 |
| (121) | 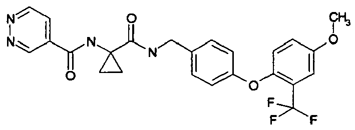 |
| (122) | 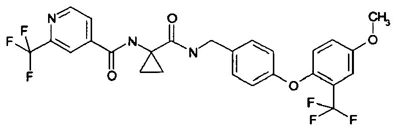 |
| (123) | 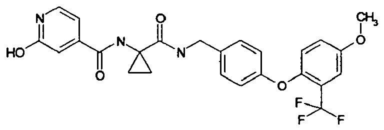 |
| (124) | 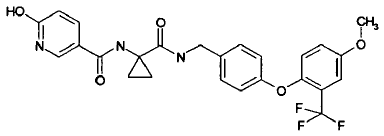 |
| (125) | 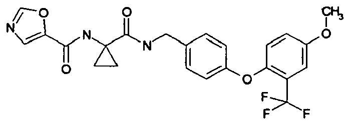 |
| (126) | 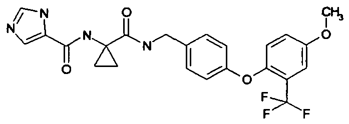 |
| (127) | 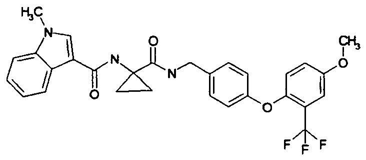 |
| (128) | 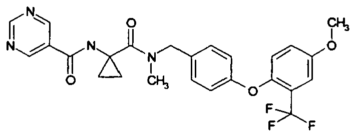 |
| (129) | 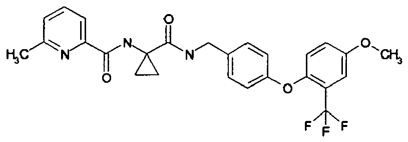 |
| (130) | 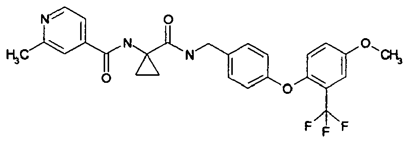 |
| (131) | 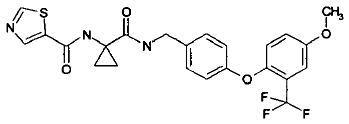 |
| (132) | 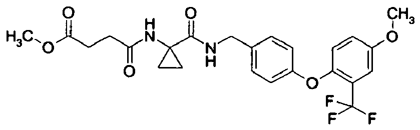 |
| (133) | 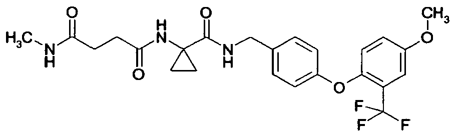 |
| (134) | 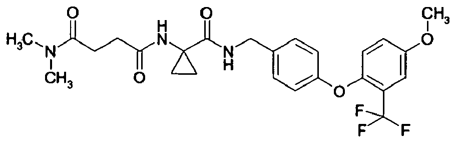 |
| (135) | 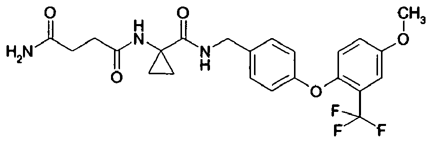 |
| (136) | 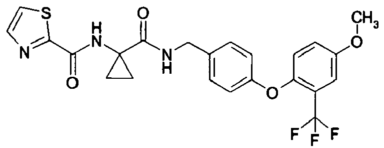 |
| (137) | 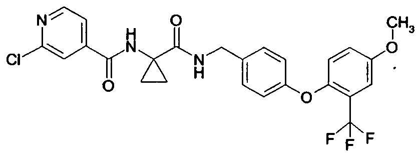 |
| (138) | 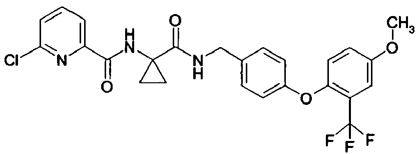 |
| (139) | 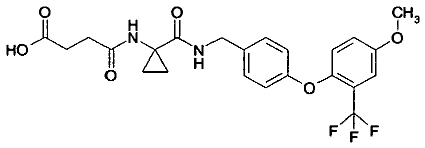 |
| (140) | 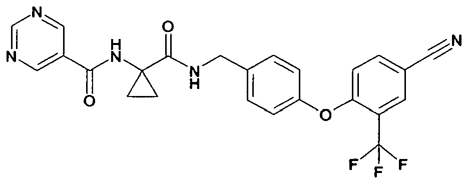 |
| (141) | 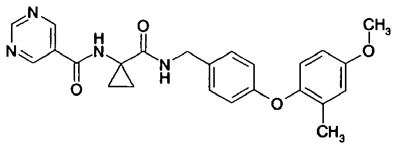 |
| (142) | 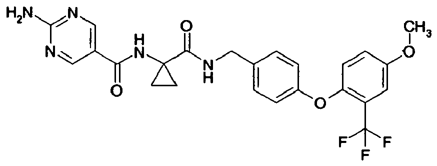 |
| (143) | 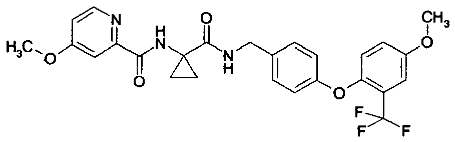 |
| (144) | 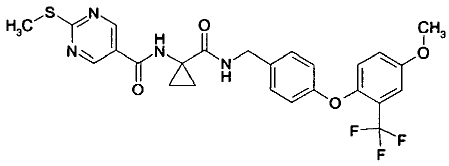 |
| (145) | 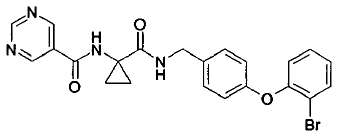 |
| (146) | 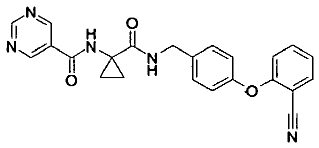 |
| (147) | 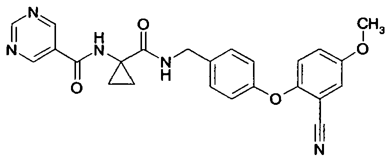 |
| (148) | 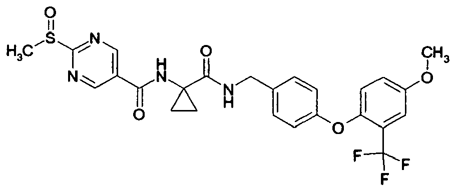 |
| (149) | 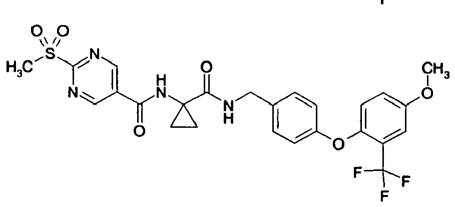 |
| (150) | 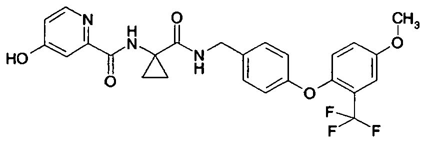 |
| (151) | 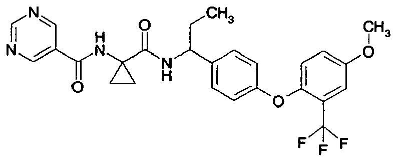 |
| (152) | 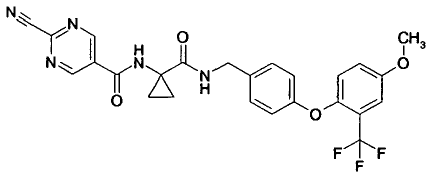 |
| (153) | 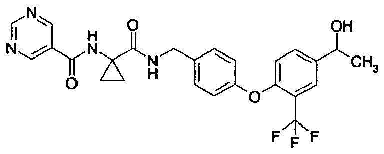 |
| (154) | 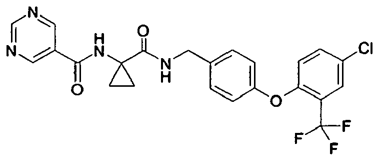 |
| (155) | 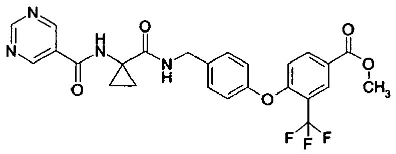 |
| (156) | 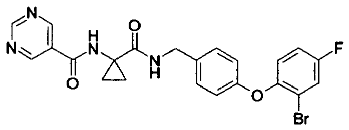 |
| (157) | 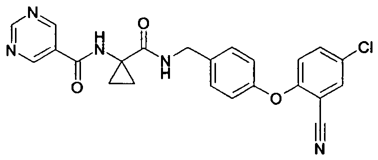 |
| (158) | 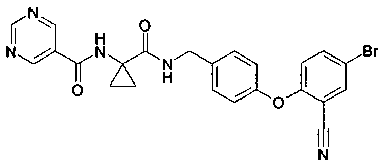 |
| (159) | 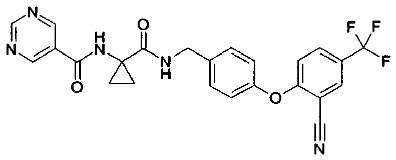 |
| (160) | 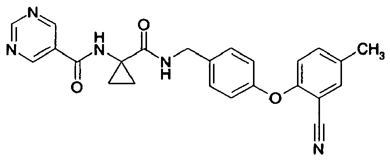 |
| (161) | 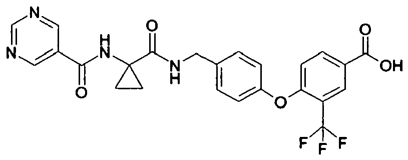 |
| (162) | 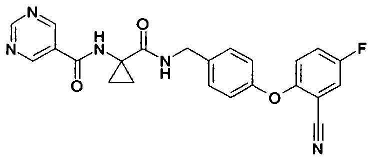 |
| (163) | 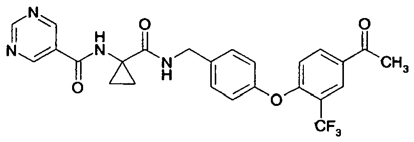 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Soweit nicht anders angegeben, sind alle Substituenten voneinander unabhängig. Sollten an einer Gruppe z.B. mehrere C₁₋₆-Alkylgruppen als Substituenten sein, so könnte im Fall von drei Substituenten C₁₋₆-Alkyl unabhängig voneinander einmal Methyl, einmal n-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden Alkylgruppen mit 1 bis 3 Kohlenstoffatomen, unter dem Begriff "C₁₋₄-Alkyl" verzweigte und unverzweigte Alkylgruppen mit 1 bis 4 Kohlenstoffatomen, unter dem Begriff "C₁₋₆-Alkyl" verzweigte und unverzweigte Alkylgruppen mit 1 bis 6 Kohlenstoffatomen und unter dem Begriff "C₁₋₆-Alkyl" verzweigte und unverzweigte Alkylgruppen mit 1 bis 8 Kohlenstoffatomen verstanden. Beispielsweise werden hierfür genannt: Methyl, Ethyl, *n-*Propyl, *i*so-Propyl, *n*-Butyl, *iso*-Butyl, *sec*-Butyl, *tert*-Butyl, *n*-Pentyl, Neopentyl, *n*-Hexyl, *n*-Heptyl und *n*-Qctyl. Gegebenenfalls werden für die vorstehend genannten Gruppen auch die Abkürzungen Me, Et, *n*-Pr, *i*-Pr, *n*-Bu, *i*-βu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, sec-Butyl und *tert*-Butyl.

Unter dem Begriff "C₀₋₂-Alkylen" werden verzweigte und unverzweigte Alkylengruppen mit 0 bis 2 Kohlenstoffatomen verstanden, wobei eine C₀-Alkylengruppe eine Bindung darstellt. Beispielsweise werden hierfür genannt: Methylen, Ethylen und Ethan-1,1-diyl.

Unter dem Begriff "C₃₋₇-Cycloalkyl" (auch soweit sie Bestandteil anderer Reste sind) werden cyclische Alkylgruppen mit 3 bis 7 Kohlenstoffatomen und unter dem Begriff "C₃₋₆-Cycloalkyl" cyclische Alkylgruppen mit 3 bis 6 Kohlenstoffatomen verstanden. Beispielsweise werden hierfür genannt: Cyclopropyl, Cyclobutyl, Cyclopentyl, Cyclohexyl oder Cycloheptyl.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen verstanden, soweit sie mindestens eine Doppelbindung aufweisen. Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl, Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-Butenyl, 2-Butenyl und 3-Butenyl, 1-Methyl-1-propenyl, 1-Methyl-2-propenyl etc.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen verstanden, soweit sie mindestens eine Dreifachbindung aufweisen. Bevorzugt sind Alkinylgruppen mit 2 bis 4 Kohlenstoffatomen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-Butinyl, 2-Butinyl und 3-Butinyl, 1-Methyl-1-propinyl, 1-Methyl-2-propinyl etc.

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Unter dem Begriff "heterocyclische Ringe" werden stabile 4-, 5- oder 6-gliedrige monocyclische heterocyclische Ringsysteme verstanden, die sowohl gesättigt als auch einfach ungesättigt sein können und neben Kohlenstoffatomen ein oder zwei Heteroatome tragen können, die ausgewählt sind aus der Gruppe bestehend aus Stickstoff, Sauerstoff und Schwefel. Dabei können sowohl Stickstoff- als auch Schwefelheteroatome optional oxidiert sein. Die voranstehend genannten Heterocyclen können über ein Kohlenstoffatom oder über ein Stickstoffatom mit dem restlichen Molekül verknüpft sein. Als Beispiele seien folgende Verbindungen genannt:

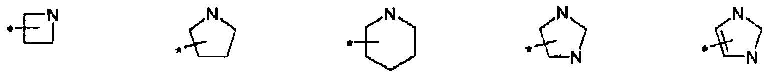

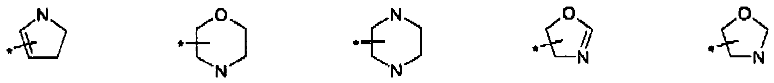

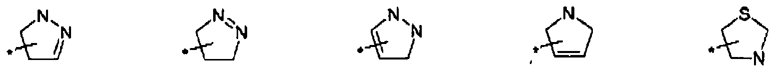

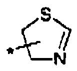

"Cyclische Imide" umfassen beispielsweise Succinimid, Maleimid und Phthalimid.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür Phenyl, 1-Naphthyl oder 2-Naphthyl genannt: bevorzugter Arylrest ist Phenyl.

Unter dem Begriff "Heteroaryl" werden fünf- oder sechsgliedrige heterocyclische Aromaten verstanden, die ein, zwei, drei oder vier Heteroatome, ausgewählt aus der Gruppe Sauerstoff, Schwefel und Stickstoff, enthalten können und zusätzlich so viele konjugierte Doppelbindungen enthalten, dass ein aromatisches System gebildet wird. Diese Heteroaryle können zusätzlich mit einem Phenylring benzo-kondensiert sein, so dass neun- oder zehngliedrige bicyclische Heteroaryle gebildet werden. Als Beispiele für fünf- oder sechsgliedrige heterocyclische Aromaten seien genannt:

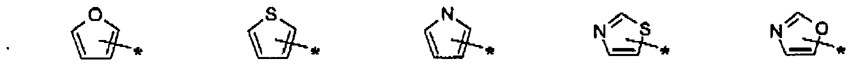

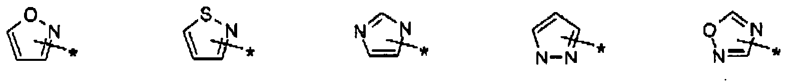

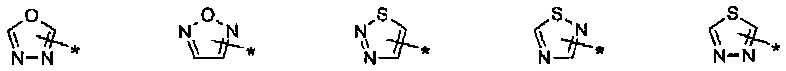

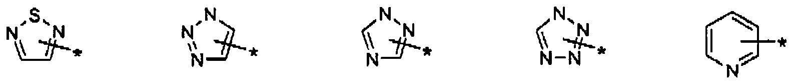

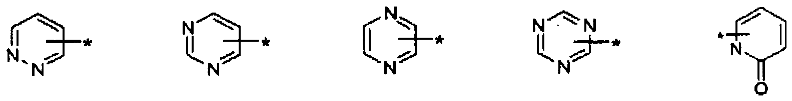

Als Beispiele für neun- oder zehngliedrige heterocyclische Aromaten seien genannt:

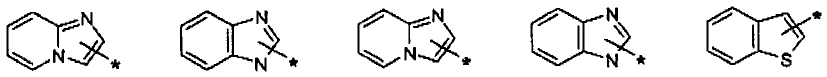

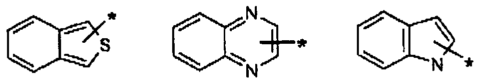

Weiterhin kann ein im Heteroarylrest vorhandenes Stickstoffatom oxidiert sein, wodurch ein N-Oxid entsteht.

Unter dem Begriff "Oxo-Gruppe" wird ein Sauerstoff-Substituent an einem Kohlenstoffatom verstanden, was zur Ausbildung einer Carbonylgruppe -C(O)- führt. Das Einführen einer Oxo-Gruppe als Substituent an einem nichtaromatischen Kohlenstoffatom führt zu einer Umwandlung einer -CH₂-Gruppe in eine -C(O)-Gruppe. Das Einführen einer Oxo-Gruppe an einem aromatischen Kohlenstoffatom führt zur Umwandlung einer -CH-Gruppe in eine -C(O)-Gruppe und kann den Verlust der Aromatizität zur Folge haben.

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder *p*-Toluolsulfonsäure in Frage, als organische Säuren kommen beispielsweise Äpfelsäure, Bernsteinsäure. Essigsäure, Fumarsäure, Maleinsäure, Mandelsäure, Milchsäure. Weinsäure oder Zitronensäure in Betracht.

Weiterhin lassen sich die Verbindungen der allgemeinen Formel **I**, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dlcyclohexylamin in Betracht

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enatiomeren, aus denen sich die erwähnten Racemate zusammensetzen.

### HERSTELLVERFAHREN

Erfindungsgemäß erhält man die Verbindungen der allgemeinen Formel **I** nach an sich dem Fachmann bekannten Verfahren, beispielsweise nach folgenden Verfahren:
(A) Amid-Kupplung: 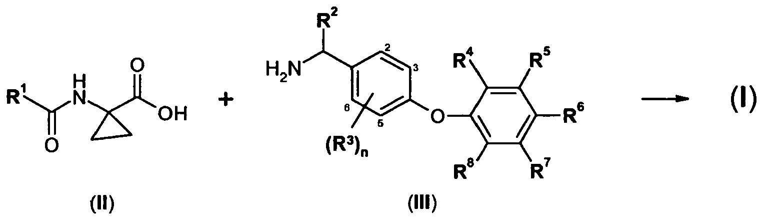
(B) Amid-Kupplung: 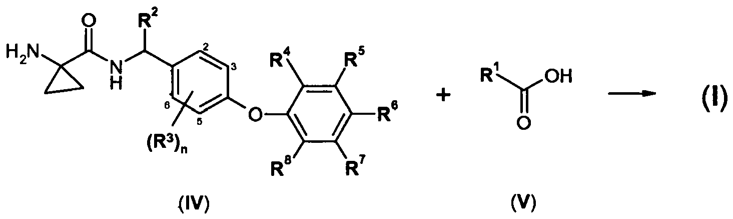
(C) Reduktive Aminierung der Aldehyde oder Ketone; Reduktion der zuvor aus den Aldehyden oderKetonen gebildeten Oxime: 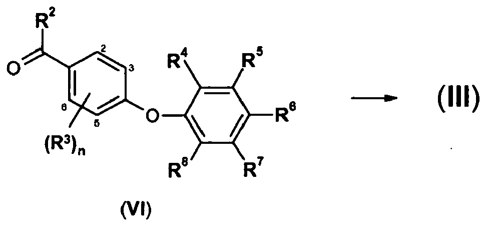
(D) nucleophile Substitution an 4-Fluor-Aldehyden oder Ketonen: 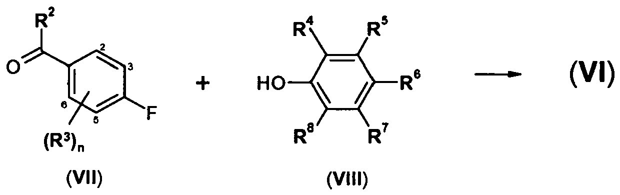
(E) Reduktion der Nitrilgruppe: 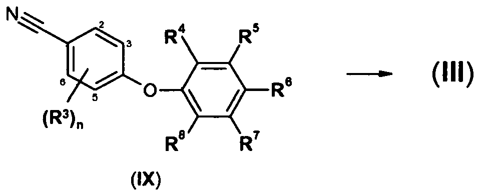
(F) nucleophile Substitution an 4-Fluor-Benzonitrilen:

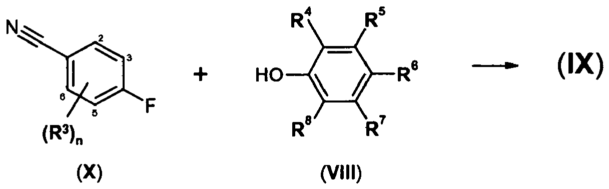

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden. Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen** / **Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz.
   Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Unter dem Ausdruck "Behandlung" oder "Therapie" ist eine therapeutische Behandlung von Patienten mit manifester, akuter oder chronischer Indikation zu verstehen, wobei einerseits die symptomatische (palliative) Behandlung zur Linderung der Krankheitssymptome und andererseits die kausale oder kurative Behandlung der Indikation mit dem Ziel, den pathologischen Zustand zu beenden, den Schweregrad des pathologischen Zustands zu vermindern oder die Progression des pathologischen Zustands zu verzögern, abhängig von der Art oder Schwere der Indikation, eingeschlossen sind.

Ein weiterer Gegenstand der vorliegenden Erfindung ist die Verwendung einer Verbindung der allgemeinen Formel I zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen. Dabei ist die Verwendung dadurch gekennzeichnet, dass sie die Verabreichung einer wirksamen Menge einer Verbindung der allgemeinen Formel **I** oder eines physiologisch verträglichen Salzes davon an einen Patienten beinhaltet, der einer solchen Behandlung bedarf.

Neben der Eignung als Humantherapeutika, sind diese Substanzen auch nützlich in der veterinärmedizinischen Therapie von Haustieren, exotischen Tieren und Nutztieren.

### KOMBINATIONEN

Zur Behandlung von Schmerzen kann es vorteilhaft sein, die erfindungsgemäßen Verbindungen mit belebenden Stoffen wie Koffein oder anderen schmerzlindernden Wirkstoffen zu kombinieren. Stehen zur Behandlung der Ursache der Schmerzen, geeignete Wirkstoffe zur Verfügung, so können diese mit den erfindungsgemäßen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemäßen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, Mesalazin, Olsalazin) und Pyrazolone (Apazon, Bezpiperylon, Feprazon, Mofebutazon, Oxyphenbutazon, Phenylbutazon, Propyphenazon und Metamizol) und Coxibe (Celecoxib, Valecoxib, Rofecoxib, Etoricoxib).
Opiat Rezeptor Agonisten wie z. B. Morphin, Propoxyphen (Darvon), Tramadol, Buprenorphin.
Cannabinoid Agonisten wie z.B. GW-1000, KDS-2000, SAB-378, SP-104, NVP001-GW-843166, GW-842166X, PRS-211375.
Natriumkanalblocker wie z.B. Carbamazepin, Mexiletin, Lamotrigin, Pregabalin, Tectin, NW-1029, CGX-1002.
N-Typ Calciumkanalblocker wie z.B. Ziconitid, NMED-160, SP1-860.
Serotonerge und noradrenerge Modulatoren wie z.B. SR-57746, Paroxetin, Duloxetin, Clonidin, Amitriptylin, Citalopram.
Corticosteroide wie z.B. Betamethason, Budesonid, Cortison, Dexamethason, Hydrocortison, Methylprednisolon, Prednisolon, Prednison und Triamcinolon.
Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin, Cetirizin, Desloratadin, fexofenadin, Levocetirizin.
Histamin H2-Rezeptor Antagonisten wie z.B. Cimetidin, Famotidin, und Ranitidin. Protonenpumpenhemmer wie z.B. Omeprazol, Pantoprazol, Esomeprazol.
Leukotrien Antagonisten und 5-Lipoxygenasehemmer wie z.B. Zafirlukast, Montelukast, Pranlukast und Zileuton.
Lokalanästhetika wie z.B. Ambroxol, Lidocain.
VR1 Agonisten und Antagonisten wie z.B. NGX-4010, WL-1002, ALGRX-4975, WL-10001, AMG-517.
Nikotinrezeptor Agonisten wie z.B. ABT-202, A-366833, ABT-594, BTG-102, A-85380, CGX1204.
P2X3-Rezeptor Antagonisten wie z.B. A-317491, ISIS-13920, AZD-9056.
NGF Agonisten und Antagonisten wie z.B. RI-724, RI-1024, AMG-819, AMG-403, PPH 207.
NK1 und NK2 Antagonisten wie z.B. DA-5018, R-116301, CP-728663, ZD-2249. NMDA Antagonisten wie z.B. NER-MD-11, CNS-5161, EAA-090, AZ-756, CNP-3381. Kaliumkanal Modulatoren wie z.B. CL-888, ICA-69673, Retigabin.
GABA Modulatoren wie z.B. Lacosamid.
Serotonerge und noradrenerge Modulatoren wie z.B. SR-57746, Paroxetin, Duloxetin, Clonidin, Amitriptylin, Citalopram, Flibanserin.
Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel Massenspektren und/oder ¹H-NMR-Spektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX*^{™}*, 35 bis 70 µm) oder Alox (E. Merck, Darmstadt, Aluminiumoxid 90 standardisiert, 63 bis 200 µm, Artikel-Nr: 1.01097.9050) verwendet.

In den Versuchsbeschreibungen werden die folgenden Abkürzungen verwendet:
- DC: Dünnschichtchromatogramm
- DIPEA: Diisopropylethylamin
- DMA: *N,N*-Dimethylacetamid
- DMAP: 4-Dimethylaminopyridin
- DMF: *N,N*-Dimethylformamid
- DMSO: Dimethylsulfoxid
- HATU: *O*-(7-Azabenzotriazol-1-yl)-*N,N,N',N'*-tetramethyluroniumhexafluorphosphal
- *tert*: tertiär
- TBTU: 2-(1*H*-Benzotriazol-1-yl)-1,1,3,3-tetramethyluronium-tetrafluorborat
- THF: Tetrahydrofuran

Folgende analytische HPLC-Methoden wurden verwendet:
Methode 1:
Säule: XTerra^{™} MS C18, 2.5 µM, 4.6 x 30 mm
Detektion: 210 - 420 nm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 3.1 | 2.0 | 98.0 | 1.0 |
| 4.5 | 2.0 | 98.0 | 1.0 |
| 5.0 | 95.0 | 5.0 | 1.0 |

Methode 2:
Säule: Microsorb C18, 3 µM, 4.6 x 50 mm
Detektion: 220 - 320 nm
Fließmittel A: Wasser / 0.1 % TFA
Fließmittel B: Acetonitril / 0.1% TFA
Gradient:

| Zeit in min | %A | %B | Flussrate in mL/min |
|---|---|---|---|
| 0.0 | 95.0 | 5.0 | 1.5 |
| 0.5 | 95.0 | 5.0 | 1.5 |
| 3.8 | 2.0 | 98.0 | 1.5 |
| 4.3 | 2.0 | 98.0 | 1.5 |
| 4.35 | 95.0 | 5.0 | 1.5 |
| 4.6 | 95.0 | 5.0 | 1.5 |

Methode 3:
Säule: XTerra^{™} MS C18, 3.5 µM, 4.6 x 50 mm
Detektion: 210 - 420 nm
Fließmittel A: Wasser / 0.1 % Ameisensäure
Fließmittel B: Acetonitril / 0.1% Ameisensäure
Gradient:

| Zeit in min | %A | %B | Flussrate in mL/min |
|---|---|---|---|
| 0.0 | 95.0 | 5.0 | 1.0 |
| 0.1 | 95.0 | 5.0 | 1.0 |
| 7.1 | 2.0 | 98.0 | 1.0 |
| 7.9 | 2.0 | 98.0 | 1.0 |
| 8.0 | 95.0 | 5.0 | 1.0 |

Methode 4:
Säule: Zorbax Stable Bond C18, 3.5 µM, 4.6 x 75 mm
Detektion: 230 - 360 nm
Fließmittel A: Wasser / 0.1 % Ameisensäure
Fließmittel B: Acetonitril / 0.1% Ameisensäure
Gradient:

| Zeit in min | %A | %B | Flussrate in mL/min |
|---|---|---|---|
| 0.0 | 95.0 | 5.0 | 1.6 |
| 0.1 | 95.0 | 5.0 | 1.6 |
| 4.5 | 10.0 | 90.0 | 1.6 |
| 5.09 | 10.0 | 90.0 | 1.6 |
| 5.5 | 90.0 | 10.0 | 1.6 |

Methode 5:
Säule: Interchim Strategy C18, 5 µM, 4.6 x 50 mm
Detektion: 220 - 320 nm
Fließmittel A: Wasser / 0.1 % TFA
Fließmittel B: Acetonitril
Gradient:

| Zeit in min | %A | %B | Flussrate in mL/min |
|---|---|---|---|
| 0.0 | 95.0 | 5.0 | 3.0 |
| 0.3 | 95.0 | 5.0 | 3.0 |
| 2.0 | 2.0 | 98.0 | 3.0 |
| 2.4 | 2.0 | 98.0 | 3.0 |
| 2.45 | 95.0 | 5.0 | 3.0 |
| 2.8 | 95.0 | 5.0 | 3.0 |

Methode 6:
Säule: Merck Cromolith Speed ROD RP18e, 4.6 x 50 mm
Detektion: 190 - 400 nm
Fließmittel A: Wasser / 0.1 % Ameisensäure
Fließmittel B: Acetonitril / 0.1% Ameisensäure
Gradient:

| Zeit in min | %A | %B | Flussrate in mL/min |
|---|---|---|---|
| 0.0 | 90.0 | 10.0 | 1.5 |
| 4.5 | 10.0 | 90.0 | 1.5 |
| 5.0 | 10.0 | 90.0 | 1.5 |
| 5.5 | 90.0 | 10.0 | 1.5 |

Folgende präparative Methoden wurden für die Umkehrphasen-Chromatographie verwendet:
Methode 1:
Säule: AXIA Gemini C18 10 µM, 100 x 30 mm
Detektion: 210 - 50D nm
Fließmittel A: Wasser / 0.1 % Trifluoressigsäure
Fließmittel B: Acetonitril
Gradient:

| Zeit in min | %A | %B | Flussrate in mL/min |
|---|---|---|---|
| 0.0 | 90.0 | 10.0 | 50 |
| 0.6 | 90.0 | 10.0 | 50 |
| 1.5 | 90.0 | 10.0 | 50 |
| 8.0 | 5.0 | 95.0 | 50 |
| 9.0 | 5.0 | 95.0 | 50 |
| 9.2 | 90.0 | 10.0 | 50 |
| 10.0 | 90.0 | 10.0 | 50 |

Methode 2:
Säule: Atlantis C18 5 µM, 100 x 30 mm
Detektion: 210 - 500 nm
Fließmittel A: Wasser / 0.1 % Trifluoressigsäure
Fließmittel B: Acetonitril
Gradient:

| Zeit in min | %A | %B | Flussrate in mL/min |
|---|---|---|---|
| 0.0 | 95.0 | 5.0 | 5 |
| 0.5 | 95.0 | 5.0 | 50 |
| 8.0 | 5.0 | 95.0 | 50 |
| 9.0 | 5.0 | 95.0 | 50 |
| 9.5 | 95.0 | 5.0 | 50 |
| 10.0 | 95.0 | 5.0 | 50 |
| 10.1 | 95.0 | 5.0 | 5 |

Folgende Mikrowellen-Apparaturen wurde verwendet: Biotage EmrysOptimizer^{™}, CEM Explorer^{™}, CEM Discover^{™}

### Beispiel 1: Pyrimidin-5-carbonsäure {1-[4-(4-chlor-2-fluor-phenoxy)-3-fluor-benzyl-carbamoyl]-cyclopropyl}-amid

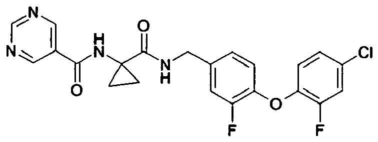

### 1 a) 1-[(Pyrimidin-5-carbonyl)-amino]-cyclopropancarbonsäureethylester

Eine Lösung von 6.80g (54.8 mMol) Pyrimidin-5-carbonsäure, 18.82 mL (135 mMol) Triethylamin und 19.27g (60 mMol) TBTU in 200 mL THF wurde 30 Minuten bei Raumtemperatur gerührt. Dann wurden 9.11g (55 mMol) 1-Amino-cyclopropancarbonsäureethylester-Hydrochlorid hinzugefügt und über Nacht weiter gerührt. Anschließen wurde das Gemisch eingedampft, der Rückstand mit 200 mL Wasser verrührt und das Rohprodukt mit Essigsäureethylester extrahiert. Durch Säulenchromatographie (Kieselgel, Dichlormethan + 0-4% Methanol) wurde das Zwischenprodukt gereinigt.

| | |
|---|---|
| Ausbeute: | 88% der Theorie |
| C₁₁H₁₃N₃O₃ | (235.24) |
| Massenspektrum: | [M+H]⁺ = 236 |

### 1b) 1-[(Pyrimidin-5-carbonyl)-amino]-cyclopropancarbonsäure

Zu einer Lösung von 11.0g (46.76 mMol) 1-[(Pyrimidin-5-carbonyl)-amino]-cyclopropancarbonsäureethylester in 200 mL Methanol wurden 65 mL einer 2N Natronlauge gegeben und das Gemisch bei 50°C eine Stunde gerührt. Dann wurde mit konzentrierter Essigsäure neutralisiert und im Vakuum bis zur Trockne eingedampft. Das so erhaltene Rohprodukt wurde chromatographisch gereinigt.

| | |
|---|---|
| Ausbeute: | 52% der Theorie |
| C₉H₉N₃O₃ | (207.19) |
| Massenspektrum: | [M+H]⁺ = 208 |
| | [M-H]⁻ = 206 |

### 1 c) 4-(4-Chlor-2-fluor-phenoxy)-3-fluor-benzonitril

Eine Lösung von 1.6 mL (15 mMol) 4-Chlor-2-fluorphenol und 1.68g (15 mMol) Kaliumtert.butylat in 10 mL DMSO wurde eine Stunde bei Raumtemperatur gerührt. Dann wurden 2.1g (15 mMol) 3,4-Difluor-bezonitril hinzugegeben und über Nacht bei 60°C gerührt. Das Gemisch wurde anschließend mit ca. 50 mL Wasser versetzt, dann dreimal mit je 30 ml Essigsäureethylester extrahiert. Die organischen Extrakte wurden mit Natriumchloridlösung gewaschen, über Natriumsulfat getrocknet und eingedampft. Das so erhaltene Produkt wurde ohne weitere Reinigung weiter umgesetzt.

| | |
|---|---|
| Ausbeute: | 98% der Theorie. |
| C₁₃H₆ClF₂NO | (265.64) |
| R_{f} = 0.90 | Dünnschichtchromatographie (Kieselgel, Dichlormethan + Ethanol 50:1): |

### 1d) 4-(4-Chlor-2-fluor-phenoxy)-3-fluor-benzylamin

1.0g (3.76 mMol) 4-(4-Chlor-2-fluor-phenoxy)-3-fluor-benzonitril wurden in 30 mL methanolischer Ammoniak-Lösung unter Zusatz von Raney-Nickel bei 50°C und einem Wasserstoff-Druck von 50 psi hydriert. Anschließend wrde der Katalysator abfiltriert und das Filtrat zur Trockne eingedampft. Das so erhaltene Rohprodukt wurde ohne weitere Reinigung weiter umgesetzt.

| | |
|---|---|
| Ausbeute: | 99% der Theorie |
| C₁₃H₁₀ClF₂NO | (269.67) |
| Massenspektrum: | [M+H]⁺ = 270/72 |

### 1e) Pyrimidin-5-carbonsäure-{1-[4-(4-chlor-2-fluor-phenoxy)-3-fluor-benzylcarbamoyl]-cyclopropyl}-amid Hydrochlorid

Zu einer Lösung von 250mg (1.2 mMol) 1-[(Pyrimidin-5-carbonyl)-amino]-cyclopropancarbonsäure (aus 1 b) in 15 mL Tetrahydrofuran wurden 0.5 mL (3.6 mMol) Triethylamin, 0.433g (1.35 mMol) TBTU und 325.5mg (1.2 mMol) 4-(4-Chlor-2-fluor-phenoxy)-3-fluor-benzylamin gegeben und das Gemisch über Nacht bei Raumtemperatur gerührt. Anschließend wurde das Gemisch bis zur Trockne eingeengt und das so erhaltene Rohprodukt chromatographisch gereinigt.

Das gereinigte Produkt wurde in ca. 4 mL Essigsäureethylester gelöst und durch Zutropfen von etherischer Salzsäure-Lösung wurde das Hydrochlorid gefällt, abfiltriert und getrocknet.

| | |
|---|---|
| Ausbeute: | 56% der Theorie |
| C₂₂H₁₇ClF₂N₄O₃ × HCl | (496.31) |
| Massenspektrum: | [M-H]⁻ = 457/59 |

Dünnschichtchromatographie (Kieselgel, Dichlormethan + Ethanol 9:1): R_{f} = 0.48

### Beispiel 2: Pyrimidin-5-carbonsäure-(1-{1-[4-(4-chlor-2-fluor-phenoxy)-phenyl]-ethyl-carbamoyl}-cyclopropyl)-amid

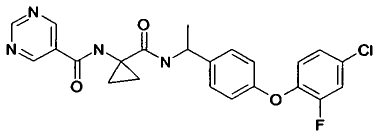

### 2a) 1-[4-(4-Chlor-2-fluor-phenoxy)-phenyl]-ethanon

Eine Lösung von 2.45g (17.7mMol) 4-Fluor-acetophenon und 2.60g (17.7 mMol) 4-Chlor-2-fluorphenol in 40 mL DMSO wurde mit 8.0g (57.9 mMol) Kaliumcarbonat versetzt und 32 Stunden bei 120°C gerührt. Dann wurde im Vakuum bis zur Trockne eingeengt, der Rückstand mit ca. 50 mL Wasser versetzt und dreimal mit je 40 mL Essigsäuremethylester extrahiert. Die Extrakte wurden mit 2N Kaliumcarbonatlösung gewaschen, über Natriumsulfat getrocknet und eingeengt. Das so erhaltene Rohprodukt wurde durch Säulenchromatographie gereinigt (Kieselgel, Dichlormethan).

| | |
|---|---|
| Ausbeute: | 72% der Theorie |
| C₁₄H₁₀ClFO₂ | (264.68) |
| Massenspektrum: | [M+H]⁺ = 265 |
| R_{f} = 0.18 | Dünnschichtchromatographie (Aluminiumoxid, Petrolether + Dichlormethan 4:1) |

### 2b) 1-[4-(4-Chlor-2-fluor-phenoxy)-phenyl]-ethanon-oxim

Ein Gemisch aus 3.4g (12.8 mMol) 1-[4-(4-Chlor-2-fluor-phenoxy)-phenyl]-ethanon und 3.0 mL Hydroxylamin-Lösung (50% in Wasser) in 100 mL Ethanol wurde fünf Stunden zum Rückfluss erhitzt. Dann wurde das Gemisch zur Trockne eingedampft, der Rückstand mit ca. 15 mL Wasser versetzt und dreimal mit je 10 mL Essigsäureethylester extrahiert. Die Extrakte wurden mit gesättigter Natriumchloridlösung gewaschen, getrocknet und eingeengt. Das so erhaltene Produkt wurde ohne weitere Reinigung weiter umgesetzt.

| | |
|---|---|
| Ausbeute: | 72% der Theorie |
| C₁₄H₁₁ClFNO₂ | (279.69) |
| Massenspektrum: | [M+H]⁺ = 280 |
| R_{f} = 0.55 | Dünnschichtchromatographie (Kieselgel, Dichlormethan + Methanol 50:1) |

### 2c) 1-[4-(4-Chlor-2-fluor-phenoxy)-phenyl]-ethylamin

2.60g (9.3 mMol) 1-[4-(4-Chlor-2-fluor-phenoxy)-phenyl]-ethanon-oxim wurden in 20 mL Methanol gelöst, dann mit 30 mL 7N methanolischer Ammoniak-Lösung versetzt und nach Zugabe von 0.2g Raney-Nickel bei Raumtemperatur und 50 psi Wasserstoff-Druck hydriert. Anschließend wurde der Katalysator abfiltriert und das Filtrat eingedampft. Das so erhaltene Rohprodukt wurde ohne weitere Reinigung weiter umgesetzt.

| | |
|---|---|
| Ausbeute: | 89% der Theorie |
| C₁₄H₁₃ClFNO | (265.71) |
| Massenspektrum: | [M-NH2]⁺ = 249/51 |
| R_{f} = 0.36 | Dünnschichtchromatographie (Kieselgel, Dichlormethan + Methanol 9:1) |

### 2d) Pyrimidin-5-carbonsäure-(1-{1-[4-(4-chlor-2-fluor-phenoxy)-phenyl]-ethyl-carbamoyl}-cyclopropyl)-amid

Analog Beispiel (1e) wurde die Titelverbindung aus 1-[(Pyrimidin-5-carbonyl)-amino]-cyclopropancarbonsäure und 1-[4-(4-Chlor-2-fluor-phenoxy)-phenyl]-ethylamin hergestellt.

| | |
|---|---|
| Ausbeute: | 29% der Theorie |
| C₂₃H_{2O}ClFN₄O₃ | (454.88) |
| Massenspektrum: | [M+H]⁺ = 455/57 |
| R_{f} = 0.44 | Dünnschichtchromatographie (Kieselgel, Dichlormethan + Methanol 9:1) |

### Beispiel 7: Pyrimidin-5-carbonsäure {1-[3-fluor-4-(4-methoxy-phenoxy)-benzylcarbamoyl]-cyclopropyl}-amid

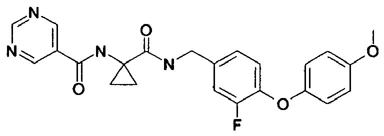

### 7a) 3-Fluor-4-(4-methoxy-phenoxy)-benzaldehyd

4.9 g (40mMol) 4-Methoxy-phenol und 4.4ml (40mMol) 3,4-Difluorbenzaldehyd wurden in 40ml DMA gelöst und in einer Mikrowelle (CEM Explorer) 15 Minuten bei 110°C gerührt, dann über basisches Alox filtriert, mit DMF nachgewaschen und einrotiert. Der Rückstand wurde über eine KG-Säule mit Gradient (Cyclohexan + 10-25% Essigester) getrennt und einrotiert.

| | |
|---|---|
| Ausbeute: | 57% der Theorie |
| C₁₄H₁₁FO₃ | (246.24) |
| Massenspektrum: | [M+H]⁺ = 247 |

### 7b) 3-Fluor-4-(4-methoxy-phenoxy)-benzylamin

73.9 mg (0.3 mMol) 3-Fluor-4-(4-methoxy-phenoxy)-benzaldehyd werden in 5ml methanolischem Ammoniak gelöst, mit Ra-Ni versetzt und etwa 9 Stunden bei 35°C und 3 bar H₂-Druck geschüttelt.

Der Katalysator wurde abgesaugt und die Lösung am Vakuum eingeengt, in 3 ml DMF gelöst und chromatographisch gereinigt.

| | |
|---|---|
| Ausbeute: | 67% der Theorie |
| C₁₄H₁₄FNO₂ | (247.27) |
| Massenspektrum: | [M-NH₂]⁺ = 231 |

### 7c) Pyrimidin-5-carbonsäure-{1-[3-fluor-4-(4-methoxy-phenoxy)-benzylcarbamoyl]-cyclopropyl}-amid

24.7 mg (0.1 mMol) 3-Fluor-4-(4-methoxy-phenoxy)-benzylamin wurden in 1 ml DMF gelöst. 20.7 mg (0.1 mMol) 1-[(Pyrimidin-5-carbonyl)-amino]-cyclopropancarbonsäure (hergestellt in 1 b) wurden ebenfalls in DMF gelöst und 35.3 mg (0.11 mMol) und 21 µl Triethylamin (0.15 mMol) zugesetzt. Über Nacht bei Raumtemperatur geschüttelt und mit Reversed-Phase Chromatographie gereinigt.

| | |
|---|---|
| Ausbeute: | 65% der Theorie |
| C₂₃H₂₁FN₄O₄ | (436.44) |
| Massenspektrum: | [M+H]⁺ = 437 |

### Beispiel 9: Pyrimidin-5-carbonsäure {1-[4-(4-carbamoyl-phenoxy)-benzylcarbamoyl]-cyclopropyl}-amid

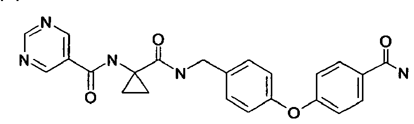

### 9a) 4-(4-Formyl-phenoxy)-benzamid

3.1 g (23 mMol) 4-Hydroxybenzamid und 2.8 g (23 mMol) 4-Fluorbenzaldehyd wurden in DMSO gelöst, mit 4.4 g (32 mMol) K₂CO₃ versetzt und bei 140°C über Nacht gerührt, über basisches Alox filtriert, mit DMF nachgewaschen, einrotiert und chromatographisch gereinigt (Kieselgelsäule, Dichlormethan mit Gradient 10-20% Methanol). Da noch DMF enthalten, wurde mit Wasser verrieben, abgesaugt und getrocknet.

| | |
|---|---|
| Ausbeute: | 57% der Theorie |
| C₁₄H₁₁NO₃ | (241.25) |
| Massenspektrum | (El):M^{+.} = 241 |

### 9b) 4-(4-Aminomethyl-phenoxy)-benzamid

Analog Beispiel (7b) wurde von 4-(4-Formyl-phenoxyl)-benzamid ausgegangen.

| | |
|---|---|
| C₁₄H₁₄N₂O₂ | (242.28) |
| Massenspektrum: | [M-NH₂]⁺ = 226 |

### 9c) Pyrimidin-5-carbonsäure-{1-[4-(4-carbamoyl-phenoxy)-benzylcarbamoyl]-cyclopropyl}-amid

Analog Beispiel (7c) wurde die Titelverbindung ausgehend von 4-(4-Aminomethyl-phenoxy)-benzamid hergestellt.

| | |
|---|---|
| Ausbeute: | 69% der Theorie |
| C₂₃H₂₁N₅O₄ | (431.54) |
| Massenspektrum: | [M+H]⁺ = 432 |

### Beispiel 10: Pyrimidin-5-carbonsäure [1-(2,6-dimethyl-4-phenoxy-benzylcarbamoyl)-cyclopropyl]-amid

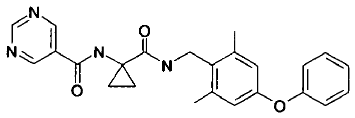

### 10a) 2,6-Dimethyl-4-phenoxy-benzaldehyd

2.3 g (25 mMol) Phenol und 3.8 g (25 mMol) 2,6-Dimethyl-4-fluorbenzaldehyd wurden in 60 ml DMA gelöst, mit 4.8 g (35 mMol) K₂CO₃ versetzt und in einer Mikrowelle (CEM Discoverer) bei 150°C für 5 min gerührt, über basisches Alox filtriert, mit DMF nachgewaschen und einrotiert. In Acetonitril/Wasser-Gemisch aufgenommen. Die Substanz kristallisiert, wurde abfiltriert und getrocknet.

| | |
|---|---|
| Ausbeute: | 83% der Theorie |
| C₁₅H₁₄O₂ | (226.28) |
| Massenspektrum: | [M+H]⁺ = 227 |

### 10b) 2,6-Dimethyl-4-phenoxy-benzylamin

Analog Beispiel (7b) wurde von 2,6-Dimethyl-4-phenoxy-benzaldehyd ausgegangen.

| | |
|---|---|
| C₁₅H₁₇NO | (227.31) |
| Massenspektrum: | [M-NH₂]⁺ = 211 |

### 10c) Pyrimidin-5-carbonsäure [1-(2,6-dimethyl-4-phenoxy-benzylcarbamoyl)-cyclopropyl]-amid

Analog Beispiel (7c) wurde die Titelverbindung ausgehend von 2,6-Dimethyl-4-phenoxy-benzylamin hergestellt.

| | |
|---|---|
| Ausbeute: | 53% der Theorie |
| C₂₄H₂₄N₄O₃ | (416.48) |
| Massenspektrum: | [M+H]⁺ = 417 |

### Beispiel 14: Pyrimidin-5-carbonsäure (1-{4-[3-(pyrrolidin-1-carbonyl)-phenoxy]-benzylcarbamoyl}-cyclopropyl)-amid

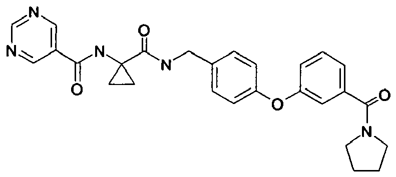

### 14a) 3-(4-Formyl-phenoxy)-benzoesäure

5.3 g (35 mMol) 3-Hydroxy-benzoesäuremethylester und 4.3 g (35 mMol) 4-Fluor-benzaldehyd wurden in DMSO gelöst und 5.8 g (42 mMol) K₂CO₃ zugesetzt und bei 80°C 6 h gerührt. Danach über basisches Alox mit Celite filtriert, einrotiert und chromatographisch gereinigt (Kieselgelsäule, Cyclohexan mit Gradient 5-30% Essigester). Dann wurde mit 50 ml Methanol und 24 ml 2M NaOH versetzt, 2 Stunden bei Raumtemperatur gerührt und das Methanol abgezogen. Mit Wasser verdünnt und mit 44 ml 1 M Salzsäure versetzt, wobei das Produkt ausfällt. Mit Wasser gewaschen und getrocknet.

| | |
|---|---|
| Ausbeute: | 63% der Theorie |
| C₁₄H₁₀O₄ | (242.23) |
| Massenspektrum: | [M-H]⁻ = 241 |

### 14b) 4-[3-(Pyrrolidin-1-carbonyl)-phenoxy]-benzaldehyd

1 g (4.1 mMol) 3-(4-Formyl-phenoxy)-benzoesäure wurden in 25 ml DMF gelöst und mit 0.86 ml (4.95 mMol) DIPEA und 1.32 g (4.1 mMol) TBTU versetzt, 5 min bei Raumtemperatur gerührt und dann 0.29 g (4.1 mMol) Pyrrolidin zugegeben. Bei Raumtemperatur gerührt, über basisches Alox filtriert, einrotiert und chromatographisch gereinigt (Kieselgelsäule, Dichlormethan mit Gradient 0-10% Methanol).

| | |
|---|---|
| Ausbeute: | 41 % der Theorie. |
| C₁₈H₁₇NO₃ | (295.34) |
| Massenspektrum: | [M+H]⁺ = 296 |

### 14c) [3-(4-Aminomethyl-phenoxy)-phenyl]-pyrrolidin-1-yl-methanon

Analog Beispiel (9b) wurde von 4-[3-(Pyrrolidin-1-carbonyl)-phenoxy]-benzaldehyd ausgegangen.

| | |
|---|---|
| C₁₈H₂₀N₂O₂ | (296.37) |
| Massenspektrum: | [M-NH₂]⁺ = 280 |

### 14d) Pyrimidin-5-carbonsäure (1-{4-[3-(pyrrolidin-1-carbonyl)-phenoxy]-benzylcarbamoyl}-cyclopropyl)-amid

Analog Beispiel (7c) wurde die Titelverbindung ausgehend von [3-(4-Aminomethyl-phenoxy)-phenyl]-pyrrolidin-1-yl-methanon hergestellt.

| | |
|---|---|
| Ausbeute: | 77% der Theorie |
| C₂₇H₂₇N₅O₄ | (485.54) |
| Massenspektrum: | [M+H]⁺ = 486 |

### Beispiel 39: Pyrimidin-5-carbonsäure-(1-{1-[4-(4-methoxy-phenoxy)-phenyl]-ethyl-carbamoyl}-cyclopropyl)-amid

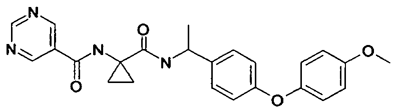

### 39a) 1-[4-(4-Methoxy-phenoxyl)-phenyl]-ethanon

138 mg (1 mMol) 4-Fluoracetophenon wurden vorgelegt und 124 mg (1 mMol) 4-Methoxyphenol wurden in DMSO gelöst und zugegeben, mit 200 mg (1.45 mMol) K₂CO₃ versetzt und bei 80°C für 8 h gerührt, dann 6 h bei 100°C, dann 3 h bei 120°C und 3 h bei 140°C. Das Reaktionsgemisch über basisches Alox filtriert, mit DMF/Methanol = 9/1 nachgewaschen und einrotiert. Die Substanz wurde über Umkehrphasen-Chromatographie aufgereinigt.

| | |
|---|---|
| Ausbeute: | 32% der Theorie |
| C₁₅H₁₄O₃ | (242.27) |
| Massenspektrum: | [M+H]⁺ = 243 |

### 39b) 1-[4-(4-Methoxy-phenoxy)-phenyl]-ethylamin

78 mg (0.32 mMol) 1-[4-(4-Methoxy-phenoxy)-phenyl]-ethanon in 10 ml 7M methanolischem Ammoniak gelöst und mit 50 mg Raney-Nickel versetzt. 6 h bei 50°C und 3 bar Wasserstoffdruck geschüttelt. Noch zweimal Raney-Nickel zugesetzt und für 2 h und 6 h unter den gleichen Bedingungen weiterhydriert. Vom Katalysator abgesaugt und eingeengt.

| | |
|---|---|
| Ausbeute: | 73% der Theorie |
| C₁₅H₁₇NO₂ | (243.31) |
| Massenspektrum: | [M-NH₂]⁺ = 227 |

### 39c) Pyrimidin-5-carbonsäure-(1-{1-[4-(4-methoxy-phenoxy)-phenyl]-ethylcarbamoyl}-cyclopropyl)-amid

Analog Beispiel (7c) wurde die Titelverbindung ausgehend von 1-[4-(4-Methoxy-phenoxy)-phenyl]-ethylamin hergestellt.

| | |
|---|---|
| Ausbeute: | 32% der Theorie |
| C₂₄H₂₄N₄O₄ | (432.48) |
| Massenspektrum: | [M+H]⁺ = 433 |

### Beispiel 40: Pyrimidin-5-carbonsäure-{1-[4-(2-chlor-phenoxy)-2-fluor-benzylcarbamoyl]-cyclopropyl}-amid

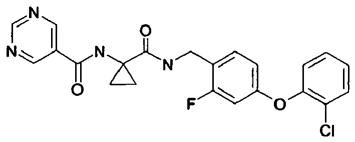

### 40a) 4-(2-Chlorphenoxy)-2-fluorbenzaldehyd

142 mg (1 mMol) 2,4-Difluorbenzaldehyd wurden in 5 ml DMSO gelöst und vorgelegt und mit 200 mg (1.45 mMol) K₂CO₃ versetzt. 128 mg (1 mMol) 2-Chlorphenol wurden in 10 ml DMSO gelöst und zugegeben und bei Raumtemperatur 2 Tage gerührt. Das Reaktionsgemisch wurde über basisches Alox filtriert, mit DMF nachgewaschen und einrotiert. Die Substanz wurde über Umkehrphasen-Chromatographie aufgereinigt.

| | |
|---|---|
| Ausbeute: | 48% der Theorie. |
| C₁₃H₈ClFO₂ | (250.66) |
| Massenspektrum: | [M+H]⁺ = 251/253 |

### 40b) 4-(2-Chlorphenoxy)-2-fluorbenzylamin

148 mg (0.59 mMol) 4-(2-Chlorphenoxy)-2-fluorbenzaldehyd in 10 ml 7M methanolischem Ammoniak gelöst und mit 50 mg Raney-Nickel versetzt. 4 h bei 35°C und 3 bar Wasserstoffdruck geschüttelt. Vom Katalysator abgesaugt, eingeengt und über Umkehrphasen-Chromatographie gereinigt.

| | |
|---|---|
| Ausbeute: | 43% der Theorie |
| C₁₃H₁₁ClFNO | (251.69) |
| Massenspektrum: | [M+H]⁺ = 252/254 |

### 40c) Pyrimidin-5-carbonsäure-{1-[4-(2-chlor-phenoxy)-2-fluor-benzylcarbamoyl]-cyclopropyl}-amid

Analog Beispiel (7c) wurde die Titelverbindung ausgehend von 4-(2-Chlorphenoxy)-2-fluorbenzylamin hergestellt.

| | |
|---|---|
| Ausbeute: | 67% der Theorie |
| C₂₂H₁₈ClFN₄O₃ | (440.86) |
| Massenspektrum: | [M+H]⁺ = 441/443 |

### Beispiel 43: Pyrimidin-5-carbonsäure-{1-[2-fluor-4-(4-methoxy-phenoxy)-benzylcarbamoyl]-cyclopropyl}-amid

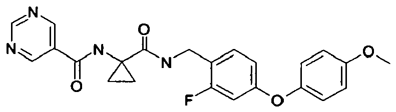

### 43a) 2-Fluor-4-(4-methoxv-phenoxy)-benzaldehyd

Analog Beispiel (40a) wurde von 2,4-Difluorbenzaldehyd und 4-Methoxyphenol ausgegangen.

| | |
|---|---|
| Ausbeute: | 47% der Theorie |
| C₁₄H₁₁FO₃ | (246.24) |
| Massenspektrum: | [M+H]⁺ = 247 |

### 43b) 2-Fluoro-4-(4-methoxy-phenoxy)-benzylamin

180 mg (0.73 mMol) 2-Fluor-4-(4-methoxy-phenoxy)-benzaldehyd mit 180 µl (3 mMol) Hydroxylamin-Lösung (50% in Wasser) in 5 ml DMF bei 100°C für 1 h gerührt und dann bei Raumtemperatur über Nacht gerührt. Eingeengt, in 10 ml 7M methanolischem Ammoniak gelöst und mit 50 mg Raney-Nickel versetzt. 4 h bei Raumtemperatur und 3 bar Wasserstoffdruck geschüttelt. Vom Katalysator abgesaugt, eingeengt und mit Umkehrphasen-Chromatographie gereinigt.

| | |
|---|---|
| Ausbeute: | 52% der Theorie |

| | |
|---|---|
| C₁₄H₁₄FNO₂ | (247.27) |
| Massenspektrum: | [M-NH₂]⁺ = 231 |

### 43c) Pyrimidin-5-carbonsäure-{1-[2-fluor-4-(4-methoxy-phenoxy)-benzylcarbamoyl]-cyclopropyl}-amid

Analog Beispiel (7c) wurde die Titelverbindung ausgehend von 2-Fluoro-4-(4-methoxy-phenoxy)-benzylamin hergestellt.

| | |
|---|---|
| Ausbeute: | 64% der Theorie |
| C₂₃H₂₁FN₄O₄ | (436.45) |
| Massenspektrum: | [M+H]⁺ = 437 |

### Beispiel 44: Pyrimidin-5-carbonsäure (1-{1-[4-(2-chlor-phenoxy)-2-fluor-phenyl]-ethyl-carbamoyl}-cyclopropyl)-amid

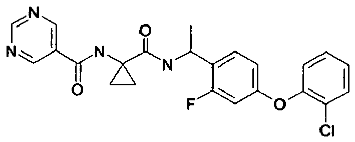

### 44a) 1-[4-(2-Chlor-phenoxy)-2-fluor-phenyl]-ethanon

156 mg (1 mMol) 2,4-Difluoracetophenon wurden in 5 ml DMSO gelöst, vorgelegt und mit 200 mg (1.45 mMol) K₂CO₃ versetzt. 128 mg (1 mMol) 2-Chlorphenol wurden in 5 ml DMSO gelöst und zugegeben und bei Raumtemperatur für 2 Tage gerührt. Das Reaktionsgemisch wurde über basisches Alox filtriert, mit DMF nachgewaschen und einrotiert. Die Substanz wurde über Umkehrphasen-Chromatographie aufgereinigt.

| | |
|---|---|
| Ausbeute: | 35% der Theorie |
| C₁₄H₁₀ClFO₂ | (264.69) |
| Massenspektrum: | [M+H]⁺ = 265/267 |

### 44b) 1-[4-(2-Chlor-phenoxy)-2-fluor-phenyl]-ethylamin

Analog Beispiel (43b) wurde von 1-[4-(2-Chlor-phenoxy)-2-fluor-phenyl]-ethanon ausgegangen.

| | |
|---|---|
| Ausbeute: | 49% der Theorie |
| C₁₄H₁₃ClFNO | (265.72) |
| Massenspektrum: | [M-NH₂]⁺ = 249/251 |

### 44c) Pyrimidin-5-carbonsäure (1-{1-[4-(2-chlor-phenoxy)-2-fluor-phenyl]-ethylcarbamoyl}-cyclopropyl)-amid

Analog Beispiel (7c) wurde die Titelverbindung ausgehend von 1-[4-(2-Chlor-phenoxy)-2-fluor-phenyl]-ethylamin hergestellt.

| | |
|---|---|
| Ausbeute: | 56% der Theorie |
| C₂₃H₂₀ClFN₄O₃ | (454.89) |
| Massenspektrum: | [M+H]⁺ = 455/457 |

### Beispiel 49: Pyrimidin-5-carbonsäure-{1-[4-(2-isopropyl-phenoxy)-benzylcarbamoyl]-cyclopropyl}-amid

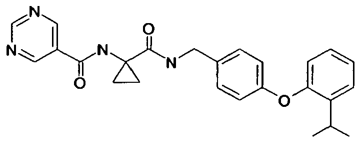

### 49a) 4-(2-Isopropyl-phenoxy)-benzylamin

27 mg (0.2 mMol) 2-lsopropyl-phenol wurden in 2 ml DMSO gelöst. Dann wurde mit 41.4 mg (0.3 mMol) K₂CO₃ und 1 ml einer 0.2 M Lösung von 4-Fluor-benzaldehyd in DMSO versetzt und über Nacht bei 100°C geschüttelt. Danach über basisches Alox filtriert, mit DMF nachgewaschen und einrotiert. Der Rückstand wurde in 2ml Methanol gelöst und 7 ml 7M methanolischem Ammoniak zugegeben, mit Ra-Ni versetzt und 7 Stunden bei 55°C und 3.5 bar H₂-Druck geschüttelt. Der Katalysator wurde abgesaugt, mit Methanol nachgewaschen und die Lösung am Vakuum eingeengt, in 2 ml DMF gelöst und chromatographisch gereinigt.

| | |
|---|---|
| Ausbeute: | 14% laut UV-Chromatogramm im LCMS |
| C₁₆H₁₉NO | (241.34) |
| Massenspektrum: | [M-NH₂]⁺ = 225 |

### 49b) Pyrimidin-5-carbonsäure-{1-[4-(2-isopropyl-phenoxy)-benzylcarbamoyl]-cyclopropyl}-amid

Analog Beispiel (7c) wurde die Titelverbindung aus 1-[(Pyrimidin-5-carbonyl)-amino]-cyclopropancarbonsäure und 4-(2-lsopropyl-phenoxy)-benzylamin hergestellt.

| | |
|---|---|
| Ausbeute: | 89% der Theorie |
| C₂₅H₂₆N₄O₃ | (430.51) |
| Massenspektrum: | [M+H]⁺ = 431 |

### Beispiel 74: Pyrimidin-5-carbonsäure {1-[2-chlor-4-(2-chlor-phenoxy)-benzyl-carbamoyl]-cyclopropyl}-amid

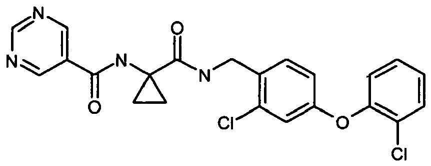

### 74a) 2-Chlor-4-(2-chlor-phenoxy)-benzonitril

Eine Lösung von 1.17 g (7.54 mMol) 2-Chlor-4-fluorbenzonitril und 0.97 g (7.54 mMol) 2-Chlorphenol in 40 mL DMSO wurde mit 3.2 g (23.16 mMol) Kaliumcarbonat versetzt und über Nacht bei 120°C gerührt. Dann wurde im Vakuum bis zur Trockne eingeengt, der Rückstand mit Wasser versetzt und mit Essigsäureethylester extrahiert. Die Extrakte wurden mit Wasser und gesättigter Natriumchloridlösung gewaschen, getrocknet und eingeengt. Das so erhaltene Rohprodukt wurde durch Säulenchromatographie gereinigt (Kieselgel, Dichlormethan).

| | |
|---|---|
| Ausbeute: | 60% der Theorie |
| C₁₃H₇Cl₂NO | (264.106) |
| Massenspektrum: | [M+NH₄]⁺ = 281/3/5 |

### 74b) 2-Chlor-4-(2-chlor-phenoxy)-benzylamin

1.2 g (4.54 mMol) 2-Chlor-4-(2-chlor-phenoxy)-benzonitril in 30 mL Methanol gelöst und 30 ml 7M methanolischem Ammoniak zugegeben, anschließend mit 150 mg Raney-Nickel versetzt. Es wurde bei 50°C und 50 psi Wasserstoffdruck geschüttelt. Vom Katalysator abgesaugt und eingeengt.

| | |
|---|---|
| Ausbeute: | 98% der Theorie |
| C₁₃H₁₁Cl₂NO | (268.14) |
| Massenspektrum: | [M+H]⁺ = 268/70/2 |

### 74c) Pyrimidin-5-carbonsäure {1-[2-chlor-4-(2-chlor-phenoxy)-benzyl-carbamoxy]-cyclopropyl}-amid

Zu einer Lösung von 80 mg (0.38 mMol) 1-[(Pyrimidin-5-carbonyl)-amino]-cyclopropancarbonsäure (aus 1b) in 30 mL Tetrahydrofuran und 5 mL DMF wurden 85 mg (0.84 mMol) Triethylamin, 130 mg (0.40 mMol) TBTU und 100 mg (0.37 mMol) 2-Chlor-4-(2-chlor-phenoxy)-benzylamin gegeben und das Gemisch über Nacht bei Raumtemperatur gerührt. Anschließend wurde das Gemisch bis zur Trockne eingeengt und den Rückstand mit 2 M Kaliumcarbonatlösung versetzt. Es wurde mit Essigsäureethylester extrahiert und die organische Phase mit Wasser und gesättigter Natriumchloridlösung gewaschen, getrocknet und eingeengt. Das so erhaltene Rohprodukt wurde über Reversed-Phase chromatographisch gereinigt. Die Fraktionen wurden bis auf die Wasserphase eingeengt, mit Ammoniak basisch gestellt und mit Essigsäureethylester extrahiert. Die organische Phase wurde mit gesättigter Natriumchloridlösung gewaschen, getrocknet und eingeengt. Den Rückstand mit Ether nachgedampft und getrocknet.

| | |
|---|---|
| Ausbeute: | 29% der Theorie |
| C₂₂H₁₈Cl₂N₄O₃ | (457.31) |
| Massenspektrum: | [M+H]⁺ = 457/9/61 |
| R_{f} = 0.7 | Dünnschichtchromatographie (Kieselgel, Dichlormethan + Methanol 9:1) |

### Beispiel 75: Pyrimidin-5-carbonsäure (1-{1-[2-chlor-4-(2-chlor-phenoxy)-phenyl]-ethyl-carbamoyl}-cyclopropyl)-amid

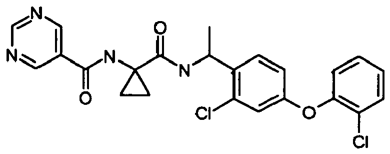

### 75a) 1-[2-Chlor-4-(2-chlor-phenoxy)-phenyl]-ethanon

Analog Beispiel 74a) wurde von 2-Chlor-4-fluoracetophenon und 2-Chlorphenol ausgegangen.

| | |
|---|---|
| Ausbeute: | 77% der Theorie |
| C₁₄H₁₀Cl₂O₂ | (281.13) |
| Massenspektrum: | [M+H]⁺ = 281/3/5 |

### 75b) 1-[2-Chlor-4-(2-chlor-phenoxy)-phenyl]-ethanon-oxim

Ein Gemisch aus 2.4 g (8.54 mMol) 1-[2-Chlor-4-(2-chlor-phenoxy)-phenyl]-ethanon und 0.76 mL Hydroxylamin-Lösung (50% in Wasser) in 60 mL Ethanol wurde 24 Stunden zum Rückfluss erhitzt. Dann wurde das Gemisch zur Trockne eingedampft, der Rückstand wurde chromatographisch gereinigt (Kieselgelsäule, Methylenchlorid).

| | |
|---|---|
| Ausbeute: | 73% der Theorie |
| C₁₄H₁₁Cl₂NO₂ | (296.15) |
| Massenspektrum: | [M+H]⁺ = 296/8/300 |

### 75c) 1-[2-Chlor-4-(2-chlor-phenoxy)-phenyl]-ethylamin

1.8 g (6.08 mMol) 1-[2-Chlor-4-(2-chlor-phenoxy)-phenyl]-ethanon-oxim in 50 mL Methanol gelöst und 50 mL 7M methanolischem Ammoniak zugegeben, anschließend mit 400 mg Raney-Nickel versetzt. Es wurde bei RT und 50 psi Wasserstoffdruck geschüttelt. Vom Katalysator abgesaugt und eingeengt. Der Rückstand wurde chromatographisch gereinigt (Kieselgelsäule, Dichlormethan:Methanol = 50:1 bis 9:1)

| | |
|---|---|
| Ausbeute: | 42% der Theorie |
| C₁₄H₁₃Cl₂NO | (282.16) |
| Massenspektrum: | [M+H]⁺ = 282/4/6 |

### 75d) Pyrimidin-5-carbonsäure (1-{1-[2-chlor-4-(2-chlor-phenoxy)-phenyl]-ethyl-carbamoyl}-cyclopropyl)-amid

Analog Beispiel (74c) wurde die Titelverbindung ausgehend von 1-[2-Chlor-4-(2-chlor-phenoxy)-phenyl]-ethylamin hergestellt.

| | |
|---|---|
| Ausbeute: | 35% der Theorie |
| C₂₃H₂₀ClN₄O₃ (471.34) | |
| Massenspektrum: | [M+H]⁺ = 471/473/475 |
| R_{f} = 0.15 | Dünnschichtchromatographie (Kieselgel, Dichlormethan + Methanol 19:1) |

### Beispiel 76: Pyrimidin-5-carbonsäure {1-[2-chlor-4-(2-chlor-4-hydroxy-phenoxy)-benzyl-carbamoyl]-cyclopropyl}-amid

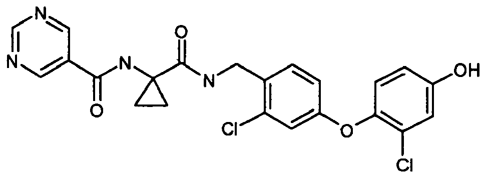

### 76a) 2-Chlor-4-(2-chlor-4-methoxy-phenoxy)-benzonitril

Eine Lösung von 3.0 g (19.28 mMol) 2-Chlor-4-fluorbenzonitril und 3.06 g (19.28 mMol) 2-Chlor-4-methoxyphenol in 77 mL DMSO wurde mit 5.32 g (38.57 mMol) Kaliumcarbonat versetzt und 2 h bei 120°C gerührt. Dann wurde im Vakuum bis zur Trockne eingeengt, der Rückstand in Dichlormethan aufgenommen und einmal mit halbgesättigter Kaliumcarbonat Lösung und zweimal mit Wasser gewaschen. Die organische Phase wurde über Natriumsulfat getrocknet und eingeengt.

| | |
|---|---|
| Ausbeute: | 100% der Theorie |
| C₁₄H₉Cl₂NO₂ | (294.13) |
| Massenspektrum: | [M-H]⁻ = 292/4/6 |

### 76b) 2-Chlor-4-(2-chlor-4-hydroxy-phenoxy)-benzonitril

0,5 g (1.7 mMol) 2-Chlor-4-(2-chlor-4-methoxy-phenoxy)-benzonitril und 2.21 mL (2.21 mMol) Bortribromid 1 M in Dichlormethan wurden in 5.5 mL Dichlormethan für 5 Tage bei RT gerührt. Der Ansatz wurde vorsichtig mit Methanol versetzt und anschließend Wasser und Dichlormethan zugegeben. Die Phasen wurden getrennt und die Wasserphase noch zweimal mit Dichlormethan extrahiert. Die organische Phase wurde mit Natriumsulfat getrocknet und eingeengt.

| | |
|---|---|
| Ausbeute: | 100% der Theorie |
| C₁₃H₇Cl₂NO₂ | (280.11) |
| Massenspektrum: | [M-H]⁻ = 278/80/2 |

### 76c) 4-(4-Aminomethyl-3-chlor-phenoxy)-3-chlor-phenol-trifluoressigsäuresalz

74 mg (1.96 mMol) Lithiumaluminiumhydrid wurden vorgelegt und bei 0°C 3 mL THF zugetropft. Anschließend wurde 498 mg (1.78 mMol) 2-Chlor-4-(2-chlor-4-hydroxy-phenoxy)-benzonitril in 3 mL THF langsam bei 0°C zugetropft. Reaktionsgemisch über Nacht bei RT gerührt, dann mit 2M Natronlauge bei 0°C zersetzt und über Celite filtriert. Es wurde mit THF nachgewaschen und das Filtrat mit Natriumsulfat getrocknet und eingeengt. Der Rückstand wurde chromatographisch (Reversed Phase) gereinigt.

| | |
|---|---|
| Ausbeute: | 21 % der Theorie |
| C₁₃H₁₁Cl₂NO₂ | (284.14) |
| Massenspektrum: | [M-NH₂]⁺ = 267/9/71 |

### 76d) Pyrimidin-5-carbonsäure {1-[2-chlor-4-(2-chlor-4-hydroxy-phenoxy)-benzylcarbamoyl]-cyclopropyl}-amid

Zu einer Lösung von 100 mg (0.48 mMol) 1-[(Pyrimidin-5-carbonyl)-amino]-cyclopropancarbonsäure (aus 1 b) in 5 mL DMF wurden 269 µl (1.93 mMol) Triethylamin und 166 mg (0.52 mMol) TBTU gegeben und 10 min bei RT rühren gelassen. Anschließend wurde 147 mg (0.37 mMol) 4-(4-Aminomethyl-3-chlor-phenoxy)-3-chlor-phenol-trifluoressigsäuresalz gelöst in 30 mL THF zugegeben. Das Reaktionsgemisch wurde 2 h bei RT gerührt und bis zur Trockne eingeengt. Der Rückstand wurde chromatographisch (Reversed Phase) gereinigt.

| | |
|---|---|
| Ausbeute: | 43% der Theorie |
| C₂₂H₁₈C1₂N₄O₄ | (473.31) |
| Massenspektrum: | [M+H]⁺ = 473/5/7 |

### Beispiel 77: 1-(2,2,2-Trifluor-acetylamin)-cyclopropancarbonsäure 4-(2-chlor-4-methoxy-phenoxy)-benzylamid

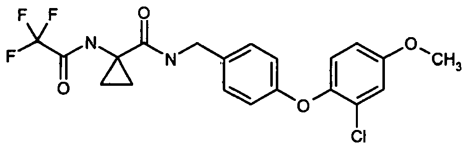

### 77a) 1-(2,2,2-Trifluor-acetylamin)-cyclopropancarbonsäure 4-(2-chlor-4-methoxy-phenoxy)-benzylamid

150 mg (0.43 mMol) 1-Amino-cyclopropancarbonsäure 4-(2-chlor-4-methoxy-phenoxy)-benzylamid wurde in 15 mL Dichlormethan gelöst, 90 µL (0.65 mMol) Triethylamin und 72 µL (0.52 mMol) Trifluoressigsäure wurden zugegeben. Das Reaktionsgemisch wurde 2 h bei RT gerührt und anschließend mit Wasser versetzt und eingeengt. Der Rückstand wurde in DMF gelöst und chromatographisch (Reversed Phase) gereinigt.

| | |
|---|---|
| Ausbeute: | 29% der Theorie |
| C₂₀H₁₈ClF₃N₂O₄ | (442.82) |
| Massenspektrum: | [M-H]⁻ = 441/3 |

### Beispiel 78: Pyrimidin-5-carbonsäure {1-[4-(4-brom-2-chlor-phenoxy)-benzylcarbamoyl]-cyclopropyl}-amid

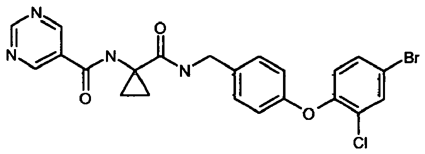

### 78a) 4-(4-Brom-2-chlor-phenoxy)-benzaldehyd

Eine Lösung von 50 mg (0.40 mMol) 4-Fluorbenzaldehyd und 83.6 mg (0.40 mMol) 4-Brom-2-chlorphenol in 2 mL DMSO wurde mit 80 mg (0.58 mMol) Kaliumcarbonat versetzt und über Nacht bei 120°C gerührt. Das Reaktionsgemisch wurde über Alox B filtriert, mit Dichlormethan nach gewaschen und eingeengt. Der Rückstand wurde chromatographisch (Reversed Phase) gereinigt.

| | |
|---|---|
| Ausbeute: | 16% der Theorie |
| C₁₃H₈BrClO₂ | (311.56) |
| Massenspektrum: | [M+H]⁺ = 311/3/5 |

### 78b) 4-(4-Brom-2-chlor-phenoxy)-benzaldehyd-oxim

20 mg (0.06 mMol) 4-(4-Brom-2-chior-phenoxy)-benzaldehyd mit 20 µL (0.33 mMol) Hydroxylamin-Lösung (50% in Wasser) in 2 mL DMF bei 80°C für 1 h gerührt. Das Reaktionsgemisch wurde chromatographisch (Reversed Phase) gereinigt.

| | |
|---|---|
| Ausbeute: | 86% der Theorie |
| C₁₃H₉BrClNO₂ | (326.57) |
| Massenspektrum: | [M+H]⁺ = 326/8/30 |

### 78c) 4-(4-Brom-2-chlor-Phenoxy)-benzylamin

18 mg (0.06 mMol) 4-(4-Brom-2-chlor-phenoxy)-benzaldehyd-oxim wurde in 2 mL Methanol gelöst, anschließend wurden 37 mg (0.15 mMol) Nickel(II)chloridhexahydrat und 21 mg (0.56 mMol) Natriumborhydrid langsam zugegeben. Das Reaktionsgemisch wurde bei RT über Nacht gerührt und anschließend chromatographisch (Reversed Phase) gereinigt.

| | |
|---|---|
| Ausbeute: | 46% der Theorie |
| C₁₃H₁₁BrClN0 | (312.59) |
| Massenspektrum: | [M+H]⁺ = 295/7/9 |

### 78d) Pyrimidin-5-carbonsäure {1-[4-(4-brom-2-chlor-phenoxy)-benzylcarbamoyl]-cyclopropyl}-amid

Zu einer Lösung von 5.4 mg (0.03 mMol) 1-[(Pyrimidin-5-carbonyl)-amino]-cyclo-propan-carbonsäure (aus 1 b) in 1 mL DMF wurden 7 µL (0.05 mMol) Triethylamin und 8.4 mg (0.03 mMol) TBTU gegeben und 5 min bei RT gerührt. Anschließend wurde 8 mg (0.03 mMol) 4-(4-Brom-2-chlor-phenoxy)-benzylamin zugegeben und das Gemisch über Nacht bei Raumtemperatur gerührt. Das Reaktionsgemisch wurde chromatographisch (Reversed Phase) gereinigt.

| | |
|---|---|
| Ausbeute: | 84% der Theorie |
| C₂₂H₁₈BrClN₄O₃ | (501.76) |
| Massenspektrum: | [M-H]⁻ = 499/501/3 |

### Beispiel 79: Pyrimidin-5-carbonsäure {1-[4-(4-brom-2-trifluormethyl-phenoxy)-benzyl-carbamoyl]-cyclopropyl}-amid

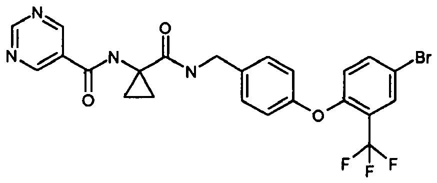

### 79a) 4-(4-Brom-2-trifluormethyl-phenoxy)-benzaldehyd

Analog Beispiel 78a) wurde von 4-Fluorbenzaldehyd und 4-Brom-2-(trifluormethyl)-benzenol ausgegangen.

| | |
|---|---|
| Ausbeute: | 30% der Theorie |
| C₁₄H₈BrF3O₂ | (345.11) |
| Massenspektrum: | [M+H]⁺ = 345/7 |

### 79b) 4-(4-Brom-2-trifluormethyl-phenoxy)-benzaldehyd-oxim

Analog 78b) wurde von 4-(4-Brom-2-trifluormethyl-phenoxy)-benzaldehyd ausgegangen, es wurde direkt weiter umgesetzt ohne chromatographische Aufreinigung.

| | |
|---|---|
| Ausbeute: | 100% der Theorie |
| C₁₄H₉BrF₃NO₂ | (360.13) |
| Massenspektrum: | [M+H]⁺ = 360/2 |

### 79c) 4-(4-Brom-2-trifluoromethyl-phenoxy)-benzylamin-trifluoressiasäuresalz Analog 78c) wurde von 4-(4-Brom-2-trifluormethyl-phenoxy)-benzaldehyd-oxim ausgegangen, die Reaktionszeit betrug aber nur 10 min.

| | |
|---|---|
| Ausbeute: | 52% der Theorie |
| C₁₄H₁₁BrF₃NO | (346.15) |
| Massenspektrum: | [M+H]⁺ = 346/8 |

### 79d) Pyrimidin-5-carbonsäure {1-[4-(4-brom-2-trifluormethyl-phenoxy) -benzylcarbamoyl]-cyclopropyl}-amid

Analog Beispiel (78d) wurde die Titelverbindung ausgehend von 4-(4-Brom-2-trifluormethyl-phenoxy)-benzylamin-trifluoressiasäuresalz hergestellt.

| | |
|---|---|
| Ausbeute: | 80% der Theorie |
| C₂₃H₁₈BrF₃N₄O₃ | (535.32) |
| Massenspektrum: | [M+H]⁺ = 535/7 |

### Beispiel 81: Pyrimidin-5-carbonsäure (1-{1-[4-(4-difluormethoxy-phenoxy)-phenyl]-ethylcarbamoyl}-cyclopropyl)-amid

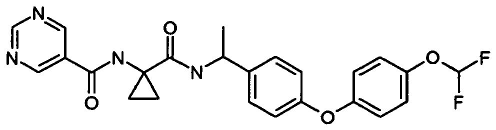

### 81 a) 1-[4-(4-Difluormethoxy)-phenoxy-phenyl]-ethanon

Eine Lösung von 666 µL (5.52 mMol) 4-Fluoracetophenon und 883 mg (5.52 mMol) 4-(Di-fluormethoxy)phenol in 10 mL DMSO wurde mit 1.91 g (13.78 mMol) Kaliumcarbonat versetzt und über Nacht bei 100°C gerührt. Das Reaktionsgemisch wurde auf 250 mL halbgesättigter Natriumchloridlösung gegossen und zweimal mit Tert-butylmethylether extrahiert. Die organische Phase wurde getrocknet und eingeengt. Der Rückstand wurde chromatographisch (Kieselgel, Petrolether/Essigsäureethylester) gereinigt.

| | |
|---|---|
| Ausbeute: | 89% der Theorie |
| C₁₅H₁₂F₂O₃ | (278.25) |
| Massenspektrum: | [M+H]⁺ = 279 |

### 81 b) 1-[4-(4-Difluormethoxy-phenoxy)-phenyl]-ethanon-oxim

Ein Gemisch aus 1.37 g (4.92 mMol) 1-[4-(4-Difluormethoxy-phenoxy)-phenyl]-ethanon und 1.16 mL Hydroxylamin-Lösung (50% in Wasser) in 5 mL Ethanol wurde 3 Stunden zum Rückfluss erhitzt. Dann wurde das Gemisch eingeengt und mehrere Male mit Ethanol nachgedampft, der Rückstand wurde chromatographisch gereinigt (Kieselgelsäule, Petrolether/Essigsäurethylester).

| | |
|---|---|
| Ausbeute: | 94% der Theorie |
| C₁₅H₁₃F₂NO₃ | (293.27) |
| Massenspektrum: | [M+H]⁺ = 294 |

### 81 c) 1-[4-(4-Difluormethoxy-phenoxy)-phenyl]-ethylamin

1.35 g (4.60 mMol) 1-[4-(4-Difluormethoxy-phenoxy)-phenyl]-ethanon-oxim in 50 ml 7M methanolischem Ammoniak gelöst, anschließend mit 100 mg Raney-Nickel versetzt. Es wurde bei RT und 50 psi Wasserstoffdruck über Nacht geschüttelt. Vom Katalysator abgesaugt und eingeengt.

| | |
|---|---|
| Ausbeute: | 99% der Theorie |
| C₁₅H₁₅F₂NO₂ | (279.28) |
| Massenspektrum: | [M-NH₂]⁺ = 263 |

### 81d) Pyrimidin-5-carbonsäure (1-{1-[4-(4-difluormethoxy-phenoxy)-phenyl]-ethylcarbamoyl}-cyclopropyl)-amid

Zu einer Lösung von 0.28 g (1.35 mMol) 1-[(Pyrimidin-5-carbonyl)-amino]-cyclo-propan-carbonsäure (aus 1 b) in 5 mL DMF wurden 376 µL (2.70 mMol) Triethylamin und 0.52 g (1.62 mMol) TBTU gegeben und 5 min bei RT gerührt. Anschließend wurde 0.42 g (1.50 mMol) 1-[4-(4-Difluormethoxy-phenoxy)-phenyl]-ethylamin zugegeben und das Gemisch 30 min bei Raumtemperatur gerührt. Das Reaktionsgemisch wurde chromatographisch (Reversed Phase) gereinigt. Das Acetonitril wurde abdestilliert, die Wasserphase wurde mit Ammoniak basisch gestellt und mit Dichlormethan extrahiert. Die organische Phase wurde getrocknet und eingeengt.

| | |
|---|---|
| Ausbeute: | 66% der Theorie |
| C₂₄H₂₂F₂N₄O₄ | (468.45) |
| Massenspektrum: | [M-H]⁻ = 467 |

### Beispiel 82: Pyrimidin-5-carbonsäure (1-{1-[4-(4-methoxy-2-trifluormethyl-phenoxy)-phenyl]-ethylcarbamoyl}-cyclopropyl)-amid

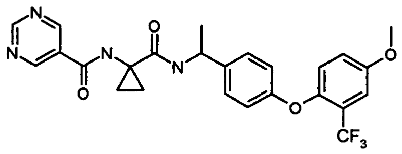

### 82a) 1-Benzyloxy-4-methoxy-2-trifluormethyl-benzol

Zu einer Lösung von 6.7 g (167.50 mMol) Natriumhydrid in 148 mL NMP wurde langsam 18 g (166.67 mMol) Benzylalkohol gegeben. Das Reaktionsgemisch wurde 30 min bei RT gerührt, anschließend wurde eine Lösung von 27 g (139.09 mMol) 1-Fluor-4-methoxy-2-trifluormethyl-benzol in 515 mL NMP zugegeben, dies wurde 30 min bei RT und 2 h bei 100°C gerührt. Nach Abkühlen auf RT wurde mit Wasser verdünnt und mit Essigsäureethylester extrahiert, die organische Phase wurde mit gesättigter Natriumchloridlösung gewaschen, mit Natriumsulfat getrocknet und eingeengt. Der Rückstand wurde chromatographisch (Kieselgel, Petrolether/Essigsäureethylester) gereinigt.

| | |
|---|---|
| Ausbeute: | 76% der Theorie |
| C₁₅H₁₃F₃O₂ | (282.26) |
| Massenspektrum: | [M+] = 282 |
| R_{f} = 0.4 | Dünnschichtchromatographie (Kieselgel, Essigester / Petrolether 7:93) |

### 82b) 4-Methoxy-2-trifluormethyl-phenol

30 g (106.28 mMol) 1-Benzyloxy-4-methoxy-2-trifluormethyl-benzol in 60 mL Essigsäureethylester gelöst und anschließend mit 3 g Palladium/C 10% versetzt. Es wurde bei RT und 50 psi Wasserstoffdruck für 4 h geschüttelt. Das Reaktionsgemisch wurde über Celite abgesaugt und das Filtrat eingeengt. Der Rückstand wurde chromatographisch (Kieselgel, Petrolether/Essigester) gereinigt.

| | |
|---|---|
| Ausbeute: | 97% der Theorie |
| C₈H₇F₃O₂ | (192.14) |
| Massenspektrum: | [M-H]⁻ = 191 |
| R_{f} = 0.2 | Dünnschichtchromatographie (Kieselgel, Essigester / Petrolether 1:10) |

### 82c) 4-(4-Methoxy-2-trifluormethyl-phenoxy)-benzonitril

Eine Lösung von 4 g (20.82 mMol) 4-Methoxy-2-trifluoromethyl-phenol und 2.52 g (20.82 mMol) 4-Fluorbenzonitril in 60 mL DMSO wurde mit 5.75 g (41.64 mMol) Kaliumcarbonat versetzt und 3 h bei 120°C gerührt. Das Reaktionsgemisch wurde mit Wasser verdünnt, und dreimal mit Dichlormethan extrahiert. Die organische Phase wurde zweimal mit Wasser gewaschen, mit Natriumsulfat getrocknet und eingeengt. Der Rückstand wurde chromatographisch (Reversed Phase) gereinigt.

| | |
|---|---|
| Ausbeute: | 52% der Theorie |
| C₁₅H_{1O}F₃NO₂ | (293.24) |
| Massenspektrum: | [M-H]⁻ = 292 |

### 82d) 1-[4-(4-Methoxy-2-trifluormethyl-phenoxy)-phenyl]-ethanon

4.87 mL (6.82 mMol) Methylmagnesiumbromid (1.4 M in THF) wurde unter Stickstoffatmosphäre vorgelegt und auf -20°C abgekühlt, dann wurde 0.5 g (1.71 mMol) 4-(4-Methoxy-2-trifluormethyl-phenoxy)-benzonitril in 2.5 mL Diethylether zugetropft. Nach der Zugabe noch 15 min bei -20°C gerührt und dann auf RT erwärmen lassen. Das Reaktionsgemisch wurde langsam auf eine Eis/Ammoniumchlorid Kältemischung gegeben und anschließend mit Diethylether extrahiert. Die organische Phase wurde getrocknet und eingeengt. Der Rückstand wurde chromatographisch (Kieselgel, Petrolether/Essigsäurethylester) gereinigt.

| | |
|---|---|
| Ausbeute: | 62% der Theorie |
| C₁₆H₁₃F₃O₃ | (310.27) |
| Massenspektrum: | [M+H]⁺ = 311 |

### 82e) 1-[4-(4-Methoxy-2-trifluormethyl-phenoxy)-phenyl]-ethanon-oxim

Analog Beispiel 81b) wurde von 1-[4-(4-Methoxy-2-trifluormethyl-phenoxy)-phenyl]-ethanon ausgegangen.

| | |
|---|---|
| Ausbeute: | 95% der Theorie |
| C₁₆H₁₄F₃NO₃ | (325.28) |
| Massenspektrum: | [M+H]⁺ = 326 |

### 82f) 1-[4-(4-Methoxy-2-trifluormethyl-phenoxy)-phenyl]-ethylamin

Analog Beispiel 2c) wurde von 1-[4-(4-Methoxy-2-trifluormethyl-phenoxy)-phenyl]-ethanon-oxim ausgegangen.

| | |
|---|---|
| Ausbeute: | 100% der Theorie |
| C₁₆H₁₆F₃NO₂ | (311.30) |
| Massenspektrum (El): | [M*+] = 311 |

### 82g) Pyrimidin-5-carbonsäure (1-{1-[4-(4-methoxy-2-trifluormethyl-phenoxy)-phenyl]-ethylcarbamoyl}-cyclopropyl)-amid

Analog Beispiel 76d) wurde von 1-[4-(4-Methoxy-2-trifluormethyl-phenoxy)-phenyl]-ethylamin ausgegangen.

| | |
|---|---|
| Ausbeute: | 53% der Theorie |
| C₂₅H₂₃F₃N₄O₄ | (500.47) |
| Massenspektrum: | [M+H]⁺ = 501 |

### Beispiel 83: N-{1-[4-(2-Chlor-4-methoxy-phenoxy)-benzylcarbamoyl]-cyclopropyl}-5-trifluormethyl-nicotinamid

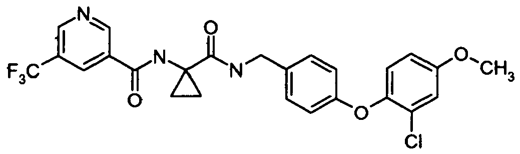

### 83a) N-{1-[4-(2-Chlor-4-methoxl-phenoxy)-benzylcarbamoyl]-cyclopropyl}-5-trifluormethyl-nicotinamid

Zu einer Lösung von 73.7 mg (0.37 mMol) 1-tert-butoxycarbonylamino-cyclopropancarbonsäure in 6 mL DMF wurden 102 µL (0.73 mMol) Triethylamin und 117 mg (0.36 mMol) TBTU gegeben und 5 min bei RT gerührt. Anschließend wurde 96.8 mg (0.37 mMol) 4-(2-Chlor-4-methoxy-phenoxy)-benzylamin_zugegeben und das Gemisch über Nacht bei Raumtemperatur gerührt. Das Reaktionsgemisch wurde über Alox B filtriert, mit DMF nachgewaschen und das Filtrat eingeengt. Zum Rückstand wurden 10 ml Dichlormethan/Trifluoressigsäure = 1/1 gegeben und 1 h bei RT geschüttelt. Das Reaktionsgemisch wurde eingeengt und der Rückstand wurde chromatographisch (Reversed Phase) gereinigt. Die entsprechenden Fraktionen wurden gefriergetrocknet. 35-mg (0.18 mMol) 5-(Trifluormethyl)nicotinsäure wurde in 2 ml DMF gelöst und mit 52 µL (0.37 mMol) Triethylamin und 59 mg (0.18 mMol) TBTU gegeben und 10 min bei RT gerührt. Anschließend wurde das gefriergetrocknete 1-Amino-cyclopropancarbonsäure-4-(2-chlor-4-methoxy-phenoxy)-benzylamid zugegeben und über Nacht bei RT gerührt. Das Reaktionsgemisch wurde direkt chromatographisch (Reversed Phase) gereinigt.

| | |
|---|---|
| Ausbeute: | 21% der Theorie |
| C₂₅H₂₁C1F₃N₃O₄ | (519.90) |
| Massenspektrum: | [M+H]⁺ = 520 |

### Beispiel 84: Pyrimidin-5-carbonsäure {1-[4-(4-methoxy-2-trifluormethyl-phenoxy)-benzylcarbamoyl]-cyclopropyl}-amid

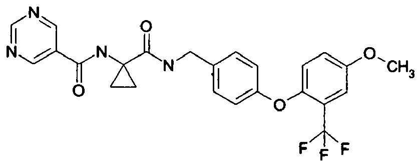

### 84a) 4-(4-Methoxy-2-trifluormethyl-phenoxy)-benzylamin

2.53 g (8.63 mMol) 4-(4-Methoxy-2-trifluormethyl-phenoxy)-benzonitril (aus 82c) in 100 ml 7M methanolischem Ammoniak gelöst, anschließend mit 250 mg Raney-Nickel versetzt. Es wurde bei RT und 50 psi Wasserstoffdruck für 3 h geschüttelt. Vom Katalysator abgesaugt und eingeengt.

| | |
|---|---|
| Ausbeute: | 90% der Theorie |
| C₁₅H₁₄F₃NO₂ | (297.27) |
| Massenspektrum: | [M-NH2]⁺ = 281 |

### 84b) Pyrimidin-5-carbonsäure {1-[4-(4-methoxy-2-trifluormethyl-phenoxy)-benzyl-carbamoyl]1-cyclopropyl}-amid

Analog Beispiel 78d) wurde von 4-(4-Methoxy-2-trifluormethyl-phenoxy)-benzylamin ausgegangen.

| | |
|---|---|
| C₂₄H₂₁F₃N₄O₄ | (486.44) |
| Massenspektrum: | [M+H]⁺ = 487 |

### Beispiel 85: 1-(4-Dimethylamino-butyrylamino)-cyclopropancarbonsäure 4-(4-methoxy-2-trifluormethyl-phenoxy)-benzylamid

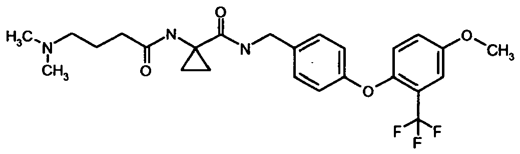

### 85a) 1 -Amino-cyclopropancarbonsäure 4-(4-methoxy-2-trifluormethyl-phenoxy)-benzylamid

Zu einer Lösung von 541.5 mg (2.69 mMol) 1-tert-butoxycarbonylamino-cyclopropancarbonsäure in 45 mL DMF wurden 540 µL (3.85 mMol) Triethylamin und 864 mg (2.69 mMol) TBTU gegeben und 5 min bei RT gerührt. Anschließend wurde 800 mg (2.69 mMol) 4-(4-Methoxy-2-trifiuormethyl-phenoxy)-benzylamin (aus 84a) zugegeben und das Gemisch 2 h bei Raumtemperatur gerührt. Das Reaktionsgemisch wurde über Alox B filtriert, mit DMF/MeOH=9/1 nach gewaschen und das Filtrat eingeengt. Zum Rückstand wurden 20 ml Dichlormethan/Trifluoressigsäure/Wasser = 50/45/5 gegeben und 1 h bei RT gerührt. Das Reaktionsgemisch wurde eingeengt, der Rückstand in Dichlormethan gelöst und mit 10 mL 1 M Natronlauge ausgeschüttelt. Die Dichlormethanphase wurde abgetrennt und eingeengt.

| | |
|---|---|
| Ausbeute: | 79% der Theorie |
| C₁₉H₁₉F₃N₂O₃ | (380.36) |
| Massenspektrum: | [M+H]⁺ = 381 |

### 85b) 1-(4-Dimethylamino-butyrylamino)-cyclopropancarbonsäure 4-(4-methoxy-2-trifluormethyl-phenoxy)-benzylamid

1.44 mg (11 µMol) 4-Dimethylamino-buttersäure wurde in 100 µL DMF gelöst, 3.5 µL (24.95 µmol) Triethylamin und 3.37 mg (10.5 µmol) TBTU in 100 µL DMF wurden zugegeben und 10 min bei RT gerührt. Anschließend wurde 3.80 mg (10 µmol) 1-Amino-cyclopropancarbonsäure 4-(4-methoxy-2-trifluormethyl-phenoxy)-benzylamid in 100 µL DMF zugegeben und das Gemisch 3 Tage bei Raumtemperatur geschüttelt. Über Alox B filtriert, mit DMF/MeOH=9/1 nachgewaschen und das Filtrat eingeengt.

| | |
|---|---|
| Ausbeute: | 73% der Theorie |
| C₂₅H₃₀F₃N₃ | (493.53) |
| Massenspektrum: | [M+H]⁺ = 494 |

### Beispiel 100: 1-(2-Cyano-acetylamino)-cyclopropancarbonsäure 4-(4-methoxy-2-trifluormethyl-phenoxy)-benzylamid

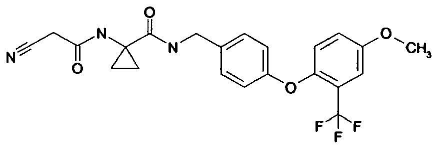

### 100a) 1-(2-Cyano-acetylamino)-cyclopropancarbonsäure 4-(4-methoxy-2-trifluormethyl-phenoxy)-benzylamid

Analog Beispiel 85b) wurde von Cyanessigsäure und 1-Amino-cyclopropancarbonsäure 4-(4-methoxy-2-trifluormethyl-phenoxy)-benzylamid (aus 85a) ausgegangen. Außerdem wurde der Rückstand am Ende chromatographisch (Reversed Phase) gereinigt.

| | |
|---|---|
| Ausbeute: | 25% der Theorie |
| C₂₂H₂₀F₃N₃ | (447.42) |
| Massenspektrum: | [M+H]⁺ = 448 |

### Beispiel 106: 3-Methyl-isoxazol-4-carbonsäure {1-[4-(4-methoxy-2-trifluormethyl-phenoxy)-benzylcarbamoyl]-cyclopropyl}-amid

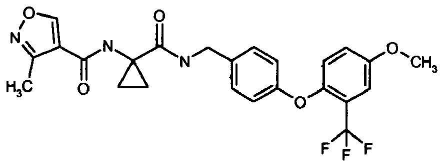

### 106a) 3-Methyl-isoxazol-4-carbonsäure {1-[4-(4-methoxy-2-trifluormethyl-phenoxy)-benzylcarbamoyl]-cyclonropyl}-amid

Zu einer Lösung von 33.4 mg (0.26 mMol) 3-Methylisoxazole-4-carbonsäure in 5 mL DMF wurden 37 µL (0.26 mMol) Triethylamin und 84.4 mg (0.26 mMol) TBTU gegeben und 10 min bei RT gerührt. Anschließend wurde 100 mg (0.26 mMol) 1-Amino-cyclopropancarbonsäure 4-(4-methoxy-2-trifluormethyl-phenoxy)-benzylamid (aus 85a) zugegeben und das Gemisch 4 h bei Raumtemperatur und über Nacht bei 40°C gerührt. Das Reaktionsgemisch wurde chromatographisch (Reversed Phase) gereinigt.

| | |
|---|---|
| Ausbeute: | 12% der Theorie |
| C₂₄H₂₂F₃N₃O₅ | (489.44) |
| Massenspektrum: | [M+H]⁺ = 490 |

### Beispiel 107: 3-Methoxy-isoxazol-5-carbonsäure {1-[4-(4-methoxy-2-trifluoromethyl-phenoxy)-benzylcarbamoyl]-cyclopropyl}-amid

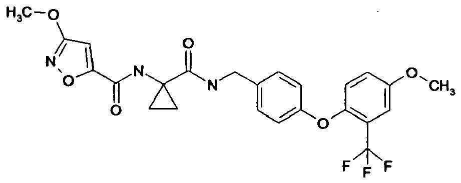

### 107a) 3-Methoxy-isoxazol-5-carbonsäure {1-[4-(4-methoxy-2-trifluoromethyl-phenoxy)-benzylcarbamoyl]-cyclopropyl}-amid

Analog Beispiel 106a) wurde von 3-Methoxy-isoxazol-5-carbonsäure ausgegangen. Die Reaktionszeit betrug 4 h bei RT.

| | |
|---|---|
| Ausbeute: | 25% der Theorie |
| C₂₄H₂₂F₃N₃O₆ | (505.44) |
| Massenspektrum: | [M+H]⁺ = 506 |

### Beispiel 108: 5-Methyl-isoxazol-4-carbonsäure {1-[4-(4-methoxy-2-trifluormethyl-phenoxy)-benzylcarbamoyl]-cyclopropyl}-amid

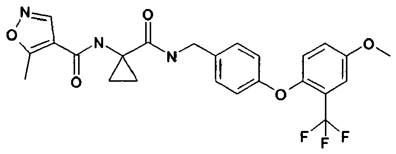

### 108a) 5-Methyl-isoxazol-4-carbonsäure {1-[4-(4-methoxy-2-trifluormethyl-phenoxy)-benzylcarbamoyl]-cyclopropyl}-amid

Analog Beispiel 106a) wurde von 3-Methyl-isoxazol-4-carbonsäure ausgegangen. Die Reaktionszeit betrug über Nacht bei RT.

| | |
|---|---|
| Ausbeute: | 45% der Theorie |
| C₂₄H₂₂F₃N₃O | (489.44) |
| Massenspektrum: | [M+H]⁺ = 490 |

### Beispiel 109: N-{1-[4-(4-Methoxy-2-trifluormethyl-phenoxy)-benzylcarbamoyl]-cyclopropyl}-malonsäuremonoamid monoethylester

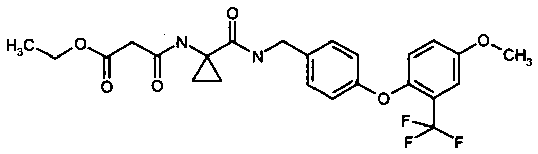

### 109a) N-{1-[4-(4-Methoxy-2-trifluoromethyl-phenoxy)-benzylcarbamoyl]-cyclopropyl}-malonsäuremonoamid monoethylester

Zu einer Lösung von 33.27 mg (0.25 mMol) Mono-ethylmalonat in 100 µL DMF wurden 87 µL (0.50 mMol) DIPEA und 80.9 mg (0.25 mMol) TBTU gegeben und 5 min bei RT gerührt. Anschließend wurde 83 mg (0.17 mMol) 1-Amino-cyclopropancarbonsäure 4-(4-methoxy-2-trifluormethyl-phenox)-benzylamid-trifluoressigsäuresalz (aus 85a) zugegeben und das Gemisch 1.5 h bei Raumtemperatur gerührt. Das Reaktionsgemisch wurde chromatographisch (Reversed Phase) gereinigt.

| | |
|---|---|
| Ausbeute: | 76% der Theorie |
| C₂₄H₂₅F₃N₂O₆ | (494.46) |
| Massenspektrum: | [M+H]⁺ = 495 |

### Beispiel 113: 2-Methoxy-N-{1-[4-(4-methoxy-2-trifluormethyl-phenoxy)-benzylcarbamoyl]-cyclopropyl}-nicotinamid

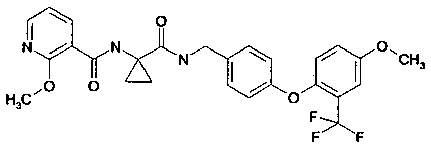

### 113a) 2-Methoxy-N-{1-[4-(4-methoxy-2-trifluormethyl-phenoxy)-benzylcarbamoyl]-cyclopropyl}-nicotinamid

Analog Beispiel 109a) wurde von 2-Methoxynicotinsäure ausgegangen. Die Reaktionszeit betrug 24 h bei RT.

| | |
|---|---|
| Ausbeute: | 61% der Theorie |
| C₂₆H₂₄F₃N₃O₅ | (515.48) |
| Massenspektrum: | [M+H]⁺ = 516 |

### Beispiel 114: N-{1-[4-(4-Methoxy-2-trifluormethyl-phenoxy)-benzylcarbamoyl]-cyclopropyl}-malonsäuremonoamid

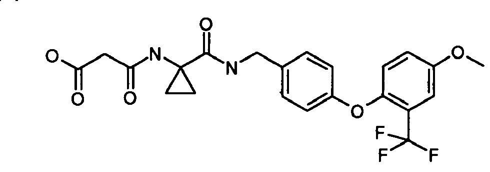

### 114a) N-{1-[4-(4-Methoxy-2-trifluormethyl-phenoxy)-benzylcarbamoyl]-cyclopropyl} malonsäuremonoamid

33 mg (0.067 mmol) N-{1-[4-(4-Methoxy-2-trifluoromethyl-phenoxy)-benzylcarbamoyl]-cyclopropyl}-malonsäuremonoamid monoethylester (aus 109a) wurde zusammen mit 1 mL Natronlauge 1 M in 20 mL THF für 1 h bei RT gerührt. Das Reaktionsgemisch wurde eingeengt und der Rückstand wurde chromatographisch (Reversed Phase) gereinigt.

| | |
|---|---|
| Ausbeute: | 26% der Theorie |
| C₂₂H₂₁F₃N₂O₆ | (466.41) |
| Massenspektrum: | [M+H]⁺ = 467 |

### Beispiel 116: 6-Methoxy-pyridin-2-carbonsäure {1-[4-(4-methoxy-2-trifluormethyl-phenoxy)benzylcarbamoyl]-cyclopropyl}-amid

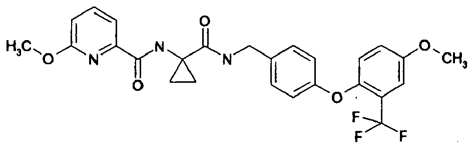

### 116a) 6-Methoxy-pyridin-2-carbonsäure {1-[4-(4-methoxy-2-trifluormethyl-phenoxy) benzylcarbamoyl]-cyclopropyl}-amid

Analog Beispiel 109a) wurde von 6-Methoxy-2-pyridincarbonsäure ausgegangen. Die Reaktionszeit betrug über Nacht bei RT.

| | |
|---|---|
| Ausbeute: | 53% der Theorie |
| C₂₆H₂₄F₃N₃O₅ | (515.48) |
| Massenspektrum: | [M+H]⁺ = 516 |

### Biespiel 118:6-Hydroxy-pyridin-2-carbonsäure {1-[4-(4-methoxy-2-trifluormethyl-phenoxy)-benzylcarbamoyl]-cyclopropyl]-cyclopropyl}amid

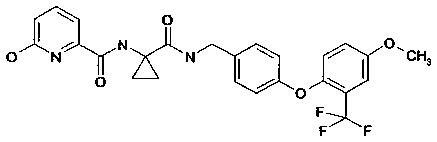

### 118a) 6-Hydroxy-pyridin-2-carbonsäure {1-[4-(4-methoxy-2-trifluormethyl-phenoxy)-benzylcarbamoyl]-cyclopropy}-amid

Zu einer Lösung von 20 mg (0.14 mmol) 6-Hydroxypicolinsäure in 100 µL DMF wurden 74 µL (0.43 mmol) DIPEA und 57.7 mg (0.18 mmol) TBTU gegeben und 5 min bei RT gerührt. Anschließend wurde 71 mg (0.14 mmol) 1-Amino-cyclopropancarbonsäure 4-(4-methoxy-2-trifluormethyl-phenoxy)-benzylamid-trifluoressigsäuresal (aus 84a) zugegeben und das Gemisch 3 Tage bei Raumtemperatur gerührt. Das Reaktionsgemisch wurde chromatographisch (Reversed Phase) gereinigt.

| | |
|---|---|
| Ausbeute: | 52% der Theorie |
| C₂₅H₂₂F₃N₃O₅ | (501.46) |
| Massenspektrum: | [M+H]⁺ = 502 |

### Beispiel 119: 2-Methoxy-N-{1-[4-(4-methoxy-2-trifluormethyl-phenoxy)-benzylcarbamoyl]-cyclopropyl}-isonicotinamid

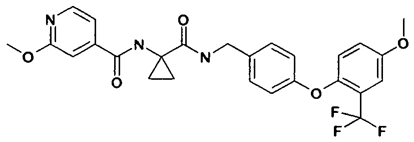

### 119a) 2-Methoxy-N-{1-[4-(4-methoxy-2-trifluormethyl-phenoxy)-benzylcarbamoyl]-cyclopropyl}l-isonicotinamid

Analog Beispiel 118a) wurde von 2-Methoxy-4-pyridincarbonsäure ausgegangen. Die Reaktionszeit betrug 2 h bei RT.

| | |
|---|---|
| Ausbeute: | 51 % der Theorie |
| C₂₆H₂₄F₃N₃O₅ | (515.48) |
| Massenspektrum: | [M+H]⁺ = 516 |

### Beispiel 120: 2-Fluor-N-{1-[4-(4-methoxy-2-trifluormethyl-phenoxy)-benzylcarbamoyl]-cyclopropyl}-isonicotinamid

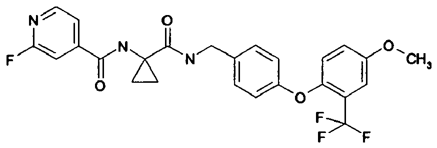

### 120a) 2-Fluor-N-{1-[4-(4-methoxy-2-trifluormethyl-phenoxy)-benzylcarbamoyl]-cyclopropyl}-isonicotinamid

Zu einer Lösung von 23 mg (0.16 mmol) 6-Fluor-nicotinsäure in 2 mL DMF wurden 68 µL (0.48 mmol) TEA und 57 mg (0.19 mmol) TBTU gegeben und 5 min bei RT gerührt. Anschließend wurde 80 mg (0.16 mmol) 1-Amino-cyclopropancarbonsäure 4-(4-methoxy-2-trifluormethyl-phenoxy)-benzylamid-trifluoressigsäuresalz (aus 85a) zugegeben und das Gemisch 2 h bei Raumtemperatur gerührt. Es wurde noch mal 57 mg (0.19 mmol) TBTU, 68 µL (0.48 mmol) TEA und 23 mg (0.16 mmol) 6-Fluor-nicotinsäure zugegeben und über Nacht bei RT gerührt. Das Reaktionsgemisch wurde chromatographisch (Reversed Phase) gereinigt.

| | |
|---|---|
| Ausbeute: | 33% der Theorie |
| C₂₅H₂₁F₄N₃O₄ | (503.46) |
| Massenspektrum: | [M+H]⁺ = 504 |

### Beispiel 125: Oxazol-5-carbonsäure {1-[4-(4-methoxy-2-trifluoromethyl-phenoxy)-benzyl-carbamoyl]-cyclopropyl}-amid

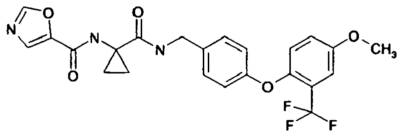

### 125a) Oxazol-5-carbonsäure {1-[4-(4-methoxy-2-trifluoromethyl-phenoxy)-benzyl-carbamoyl]-cyclopropyl}-amid

Zu einer Lösung von 15.4 mg (0.14 mmol) Oxazol-5-carbonsäure in 1.5 mL DMF wurden 57.5 µL (0.41 mmol) TEA und 48 mg (0.15 mmol) TBTU gegeben und 5 min bei RT gerührt. Anschließend wurde 67.5 mg (0.14 mmol) 1-Amino-cyclopropancarbonsäure 4-(4-methoxy-2-trifluormethyl-phenoxy)-benzylamid-trifluoressigsäuresalz (aus 85a) zugegeben und das Gemisch 2 h bei Raumtemperatur gerührt. Das Reaktionsgemisch wurde chromatographisch (Reversed Phase) gereinigt.

| | |
|---|---|
| Ausbeute: | 25% der Theorie |
| C₂₃H₂₀F₃N₃O₅ | (475.42) |
| Massenspektrum: | [M+H]⁺ = 476 |

### Beispiel 129: 6-Methyl-pyridin-2-carbonsäure {1-[4-(4-methoxy-2-trifluormethyl-phenoxy)-benzylcarbamoyl]-cyclopropyl}-amid

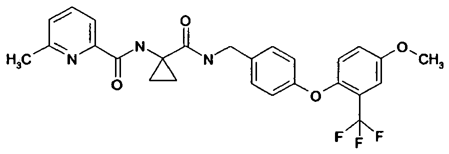

### 129a) 6-Methyl-pyridin-2-carbonsäure{1-[4-(4-methoxy-2-trifluormethyl-phenoxy)-benzylcarbamoyl]-cyclopropyl}-amid

Zu einer Lösung von 27.7 mg (0.20 mmol) 6-Methylpicolinsäure in 5 mL DMF wurden 28 µL (0.20 mmol) TEA und 118.8 mg (0.30 mmol) HATU gegeben und 5 min bei RT gerührt. Anschließend wurden 112 µL (0.81 mmol) TEA und 100 mg (0.20 mmol) 1-Amino-cyclopropancarbonsäure 4-(4-methoxy-2-trifluormethyl-phenoxy)-benzylamid-trifluoressigsäuresalz (aus 85a) zugegeben und das Gemisch über Nacht bei RT gerührt. Das Reaktionsgemisch wurde chromatographisch (Reversed Phase) gereinigt.

| | |
|---|---|
| Ausbeute: | 80% der Theorie |
| C₂₆H₂₄F₃N₃O₄ | (499.48) |
| Massenspektrum: | [M+H]⁺ = 500 |

### Beispiel 139: N-{1-[4-(4-Methoxy-2-trifluormethyl-phenoxy)-benzylcarbamoyl]-cyclopropyl}-bernsteinsäure-monoamid

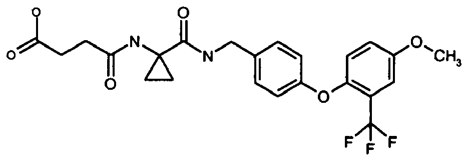

Eine Lösung von 82 mg (0.166 mmol) N-{1-[4-(4-Methoxy-2-trifluormethyl-phenoxy)-benzylcarbamoyl]-cyclopropyl}-bernsteinsäure-monoamid-methylester (Produkt aus Beispiel 132) und 2 mL Natronlauge (1 N) in 10 mL THF wurde zwei Stunden bei Raumtemperatur gerührt, dann mit 0.1 N Salzsäure neutralisiert und eingedampft. Der feste Rückstand wurde in ca. 3 mL Dichlormethan aufgerührt, abfiltriert und erneut eingedampft.

| | |
|---|---|
| Ausbeute: | 98% der Theorie |
| C₂₃H₂₃F₃N_{2P}Oₑ | (480.43) |
| Massenspektrum: | [M+H]⁺ = 481 |

### Beispiel 140: Pyrimidin-5-carbonsäure {1-[4-(4-cyano-2-trifluormethyl-phenoxy)-benzyl-carbamoyl]-cyclopropyl}-amid

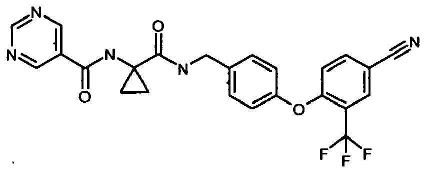

Eine Lösung von 100 mg (0.32 mmol) Pyrimidin-5-carbonsäure [1-(4-hydroxy-benzyl-carbamoyl)-cyclopropyl]-amid, 60.5 mg (0.32 mmol) 4-Fluor-3-trifluormethyl-benzonitril und 110 mg (0.8 mmol) Kaliumcarbonat in 5 mL DMF wurde zwei Stunden bei 110°C gerührt. Nach dem Abkühlen wurde das Gemisch mit ca. 3 mL Aceton verdünnt, filtriert und eingedampft. Das so erhaltene Rohprodukt wurde durch Säulenchromatographie (Reversed Phase) gereinigt.

| | |
|---|---|
| Ausbeute: | 82% der Theorie |
| C₂₄H₁₈F₃N₅O₃ | (481.43) |
| Massenspektrum: | [M+H]⁺ = 481 |

### Beispiel 148: 2-Methansulfinyl-pyrimidin-5-carbonsäure {1-[4-{4-methoxy-2-trifluormethyl-phenoxy)-benzylcarbamoyl]-cyclopropyl}amid

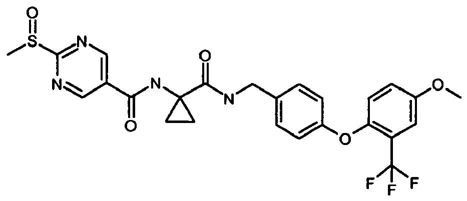

Zu einer Lösung von 575 mg (1.08 mmol) 2-Methylthio-pyrimidin-5-carbonsäure {1-[4-(4-methoxy-2-trifluormethyl-phenoxy)-benzylcarbamoyl]-cyclopropyl}-amid (Produkt aus Beispiel 144) in 10 mL Dichlormethan wurden bei ca. 5°C 186 mg (1.08 mmol) 3-Chlorperbenzoesäure gegeben. Danach wurde die Kühlung entfernt und das Reaktionsgemisch weitere drei Stunden bei Raumtemperatur gerührt. Die Hälfte der Lösung wurde eingedampft und das so erhaltene Rohprodukt chromatographisch (Reversed Phase HPLC) gereinigt.

| | |
|---|---|
| Ausbeute: | 6% der Theorie |
| C₂₅H₂₃F₃N₄O₅S | (548.54) |
| Massenspektrum: | [M+H]⁺ = 549 |

### Beispiel 149: 2-Methansulfonyl-pyrimidin-5-carbonsäure {1-[4-(4-methoxy-2-trifluormethyl-phenoxy)-benzylcarbamoyl]-cyclopropyl}-amid

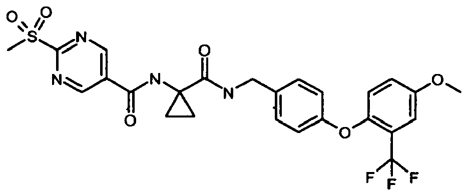

Die Hälfte des rohen Produktgemisches aus Beispiel 148 wurde mit weiteren 186 mg 3-Chlorperbenzoesäure versetzt und zwei Stunden bei Raumtemperatur gerührt. Nach dem Eindampfen der Lösung wurde das so erhaltene Rohprodukt chromatographisch (Reversed Phase HPLC) gereinigt.

| | |
|---|---|
| Ausbeute: | 47% der Theorie |
| C₂₅H₂₃F₃N₄O₆S | (564.54) |
| Massenspektrum: | [M+H]⁺ = 565 |

### Beispiel 152: 2-Cyano-pyrimidin-5-carbonsäure {1-[4-(4-methoxy-2-trifluormethyl-phenoxy)-benzylcarbamoyl]cyclopropyl}-amid

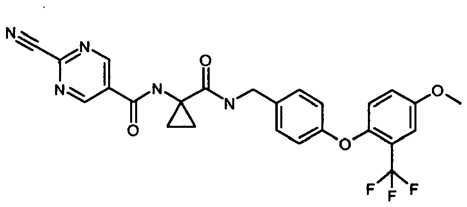

Eine Lösung von 50 mg (0.089 mmol) 2-Methansulfonyl-pyrimidin-5-carbonsäure {1-[4-(4-methoxy-2-trifluormethyl-phenoxy)-benzylcarbamoyl]-cyclopropyl}-amid (Produkt aus Beispiel 149) in 2 mL DMF wurde mit 9 mg (0.13 mmol) Kaliumcyanid versetzt und anschließend in einer Mikrowellenapparatur 10 Minuten auf 100°C erhitzt. Das Gemisch wurde dann mit 2 mL konzentrierter Ammoniaklösung versetzt und mit Dichlormethan extrahiert. Das nach dem Eindampfen erhaltene Rohprodukt wurde chromatographisch (Reversed Phase HPLC) gereinigt.

| | |
|---|---|
| Ausbeute: | 51 % der Theorie |
| C₂₅H₂₀F₃N₅O₄ | (511.45) |
| Massenspektrum: | [M+H]⁺ = 512 |

### Beispiel 163: Pyrimidin-5-carbonsäure {1-[4-(4-acetyl-2-trifluormethyl-phenoxy)-benzyl-carbamoyl]-cyclopropyl}-amid

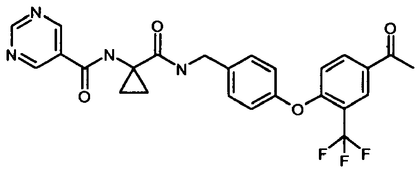

Eine Lösung von 70 mg (0.14 mmol) Pyrimidin-5-carbonsäure (1-{4-[4-(1-hydroxyethyl)-2-trifluoromethyl-phenoxy]-benzylcarbamoyl}-cyclopropyl)-amid (Produkt aus Beispiel 153) in 10 mL Dichlormethan wurde mit 300 mg (3.5 mmol) Mangandioxid versetzt und drei Tage bei Raumtemperatur gerührt. Das Gemisch wurde dann filtriert und eingedampft. Das so erhaltene Rohprodukt wurde chromatographisch (Reversed Phase HPLC) gereinigt.

| | |
|---|---|
| Ausbeute: | 21 % der Theorie |
| C₂₅H₂₁F₃N₄O₄ | (498.45) |
| Massenspektrum: | [M+H]⁺ = 499 |

Die übrigen Verbindungen werden analog der voranstehend erwähnten Beispiele hergestellt.

Tabelle der Endverbindungen:

| **Nr.** | **Struktur** | **Edukt (Benzylamin)** | **Herstellvorschrift** | **LCMS** |
|---|---|---|---|---|
| (1) | 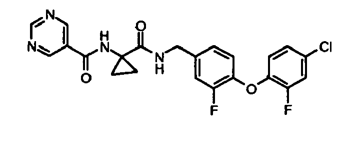 | 1d) | 1e) | [M-H]- = 457/459 |
| (2) | 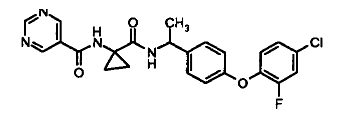 | 2c) | analog 1e) | [M+H]+ = 455/457 |
| (3) | 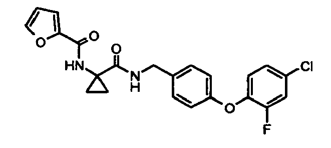 | 3b) | analog 1e) | [M+H]+ = 429/431 |
| (4) | 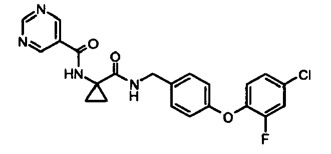 | 3b) | analog 1e) | [M+H]+ = 441/443 |
| (5) | 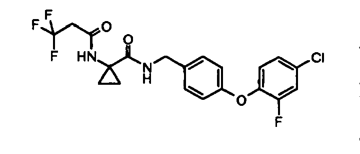 | 3b) | analog 1e) | [M+H]+ = 445/447 |
| (6) | 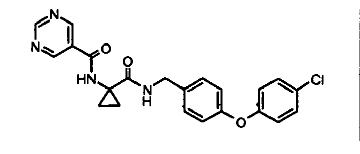 | 6b) | analog 1e) | [M+H]+ = 423/425 |
| (7) | 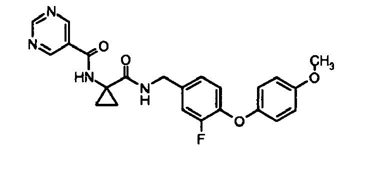 | 7b) | 7c) | [M+H]+ = 437 |
| (8) | 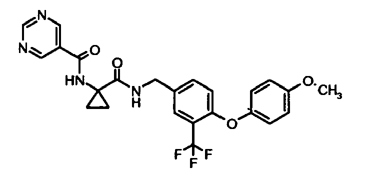 | 8b) | analog 7c) | [M+H]+ = 487 |
| (9) | 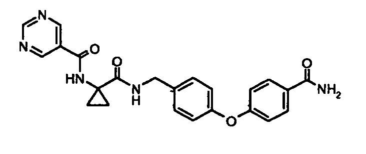 | 9b) | analog 7c) | [M+H]+ = 432 |
| (10) | 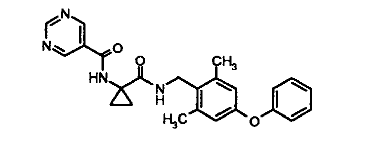 | 10b) | analog 7c) | [M+H]+ = 417 |
| (11) | 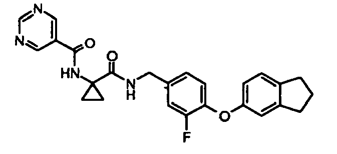 | 11b) | analog 7c) | [M+H]+ = 447 |
| (12) | 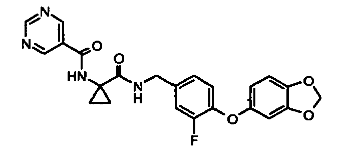 | 12b) | analog 7c) | [M+H]+ = 451 |
| (13) | 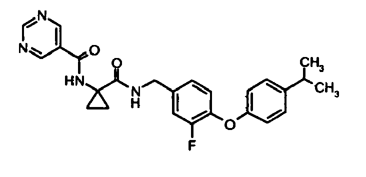 | 13b) | analog 7c) | [M+H]+ = 449 |
| (14) | 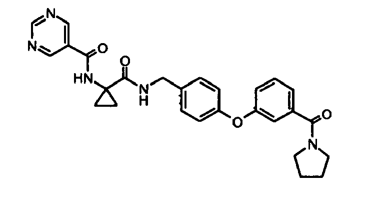 | 14c) | analog 7c) | [M+H]+ = 486 |
| (15) | 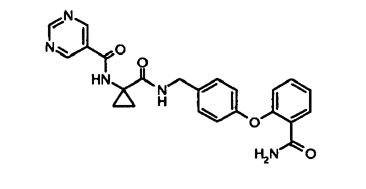 | 15c) | analog 7c) | [M+H]+ = 432 |
| (16) | 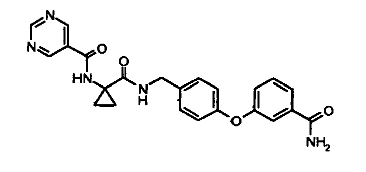 | 16c) | analog 7c) | [M+H]+ = 432 |
| (17) | 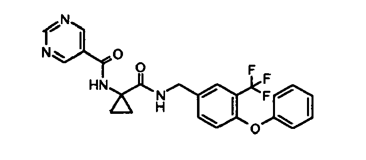 | 17b) | analog 7c) | [M+H]+ = 457 |
| (18) | 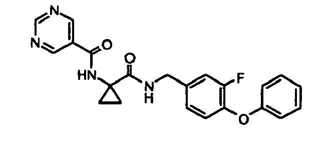 | 18b) | analog 7c) | [M+H]+ = 407 |
| (19) | 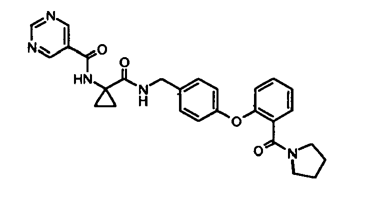 | 19c) | analog 7c) | [M+H]+ = 486 |
| (20) | 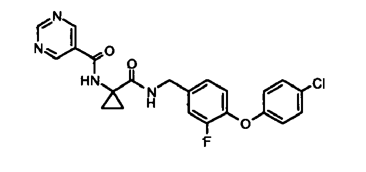 | 20b) | analog 7c) | [M+H]+ = 441/443 |
| (21) | 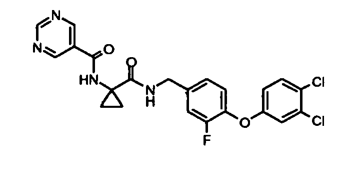 | 21b) | analog 7c) | [M+H]+ = 475/477/479 |
| (22) | 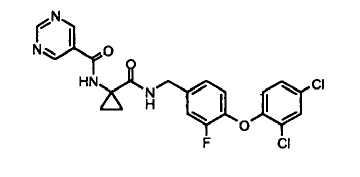 | 22b) | analog 7c) | [M+H]+ = 475/477/479 |
| (23) | 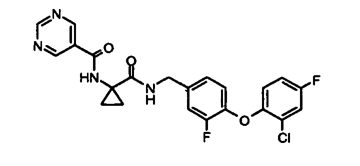 | 23b) | analog 7c) | [M+H]+ = 459/461 |
| (24) | 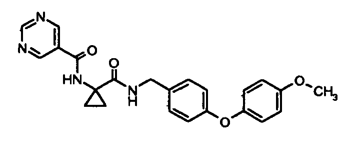 | kommerziell erhältlich | analog 7c) | [M+H]+ = 419 |
| (25) | 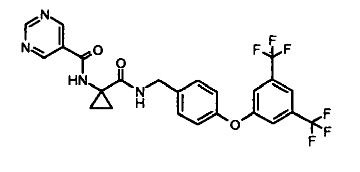 | kommerziell erhältlich | analog 7c) | [M+H]+ = 525 |
| (26) | 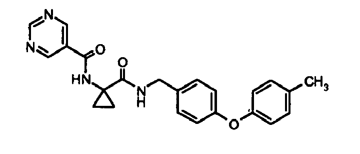 | kommerziell erhältlich | analog 7c) | [M+H]+ = 403 |
| (27) | 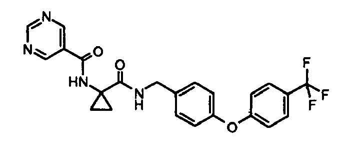 | kommerziell erhältlich | analog 7c) | [M+H]+ = 457 |
| (28) | 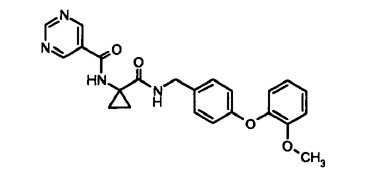 | kommerziell erhältlich | analog 7c) | [M+H]+ = 419 |
| (29) | 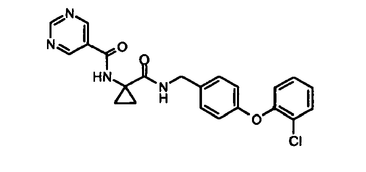 | kommerziell erhältlich | analog 7c) | [M+H]+ = 423/425 |
| (30) | 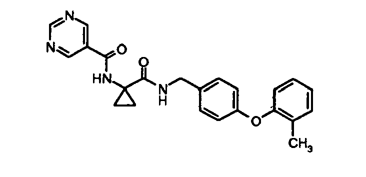 | kommerziell erhältlich | analog 7c) | [M+H]+ = 403 |
| (31) | 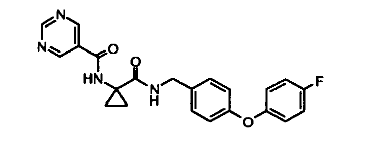 | kommerziell erhältlich | analog 7c) | [M+H]+ = 407 |
| (32) | 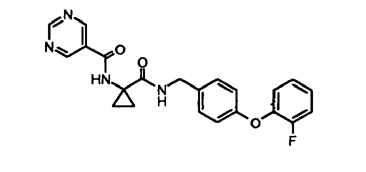 | kommerziell erhältlich | analog 7c) | [M+H]+ = 407 |
| (33) | 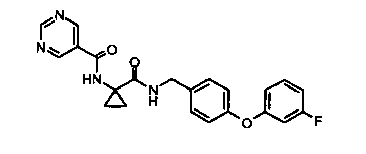 | kommerziell erhältlich | analog 7c) | [M+H]+ = 407 |
| (34) | 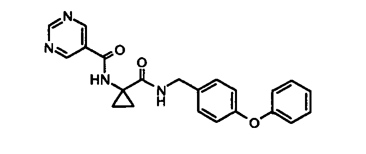 | kommerziell erhältlich | analog 7c) | [M-H]- = 387 |
| (35) | 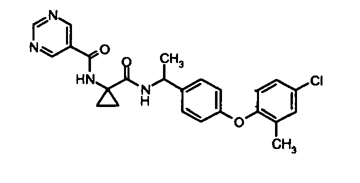 | kommerziell erhältlich | analog 7a,b,c) | [M+H]+ = 451 |
| (36) | 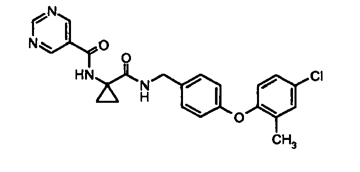 | kommerziell erhältlich | analog 7a,b,c) | [M+H]+ = 437 |
| (37) | 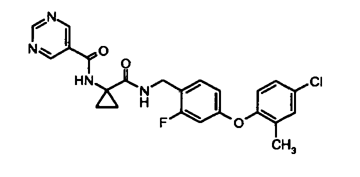 | kommerziell erhältlich | analog 7a,b,c) | [M+H]+ = 455/457 |
| (38) | 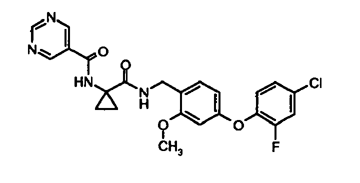 | kommerziell erhältlich | analog 7a,b,c) | [M+H]+ = 471/473 |
| (39) | 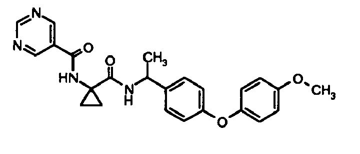 | 39b) | analog 7c) | [M+H]+ = 433 |
| (40) | 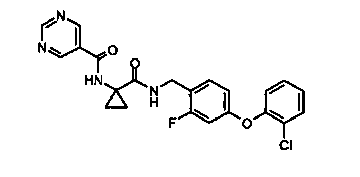 | 40b) | analog 7c) | [M+H]+ = 441 /443 |
| (41) | 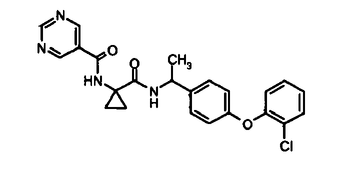 | 41b) | analog 7c) | [M+H]+ = 437/439 |
| (42) | 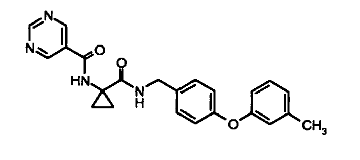 | kommerziell erhältlich | analog 7c) | [M+H]+ = 403 |
| (43) | 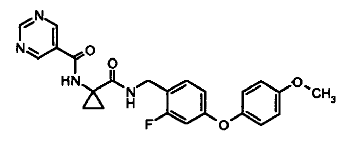 | 43b) | analog 7c) | [M+H]+ = 437 |
| (44) | 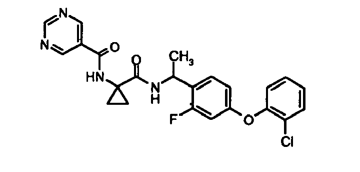 | 44b) | analog 7c) | [M+H]+ = 455/457 |
| (45) | 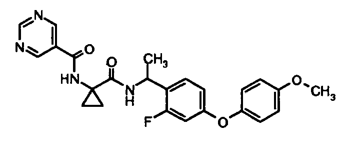 | 45b) | analog 7c) | [M+H]+ = 451 |
| (46) | 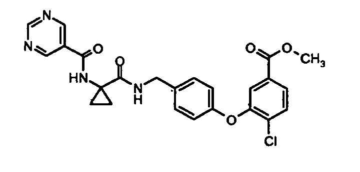 | 46b) | analog 1e) | [M+H]+ = 481 /483 |
| (47) | 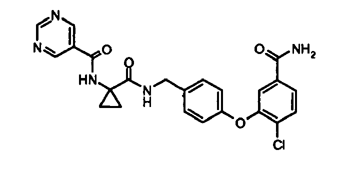 | 47b) | analog 1e) | [M+H]+ = 466/468 |
| (48) | 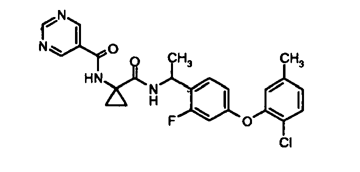 | 48b) | analog 1e) | [M+H]+ = 467/469 |
| (49) | 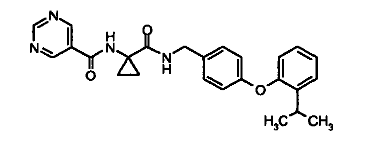 | 49a) | analog 7c) | [M+H]+ = 431 |
| (50) | 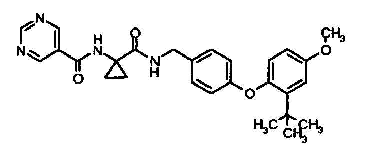 | 50a) | analog 7c) | [M+H]+ = 475 |
| (51) | 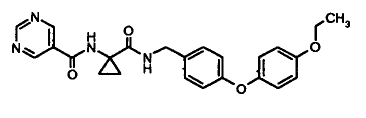 | 51a) | analog 7c) | [M+H]+ = 433 |
| (52) | 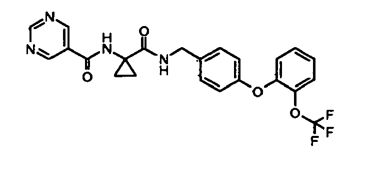 | 52a) | analog 7c) | [M+H]+ = 473 |
| (53) | 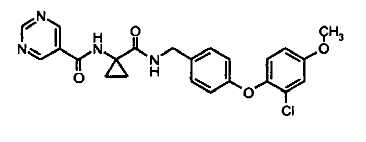 | 53a) | analog 7c) | [M+H]+ = 453/455 |
| (54) | 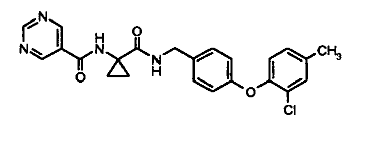 | 54a) | analog 7c) | [M+H]+ = 437/439 |
| (55) | 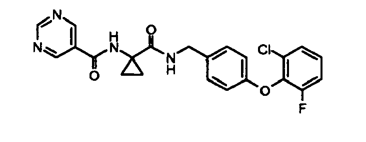 | 55a) | analog 7c) | [M+H]+= 441/443 |
| (56) | 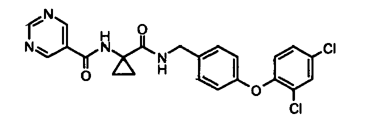 | 56a) | analog 7c) | [M+H]+ = 457/459/461 |
| (57) | 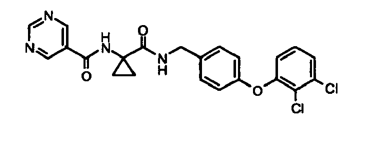 | 57a) | analog 7c) | [M+H]+ = 457/459/461 |
| (58) | 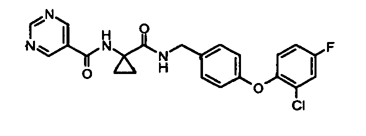 | 58a) | analog 7c) | [M+H]+ = 441/443 |
| (59) | 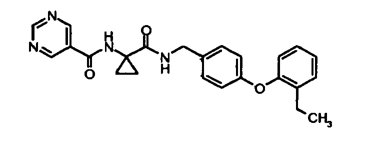 | 59a) | analog 7c) | [M+H]+ = 417 |
| (60) | 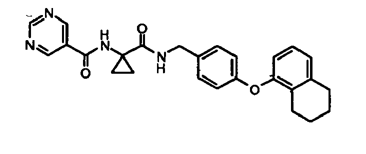 | 60a) | analog 7c) | [M+H]+=443 |
| (61) | 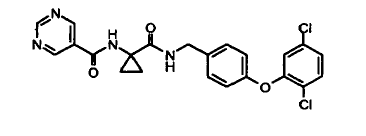 | 61a) | analog 7c) | [M+H]+ = 457/459/461 |
| (62) | 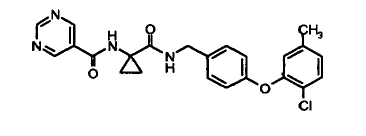 | 62a) | analog 7c) | [M+H]+ = 437/439 |
| (63) | 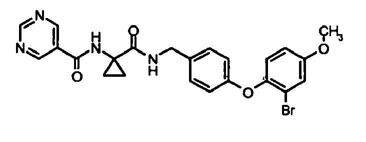 | 63a) | analog 7c) | [M+H]+ = 497/499 |
| (64) | 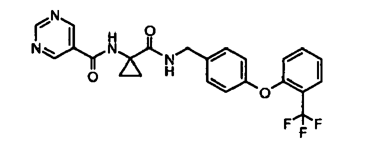 | 64a) | analog 7c) | [M+H]+ = 457 |
| (65) | 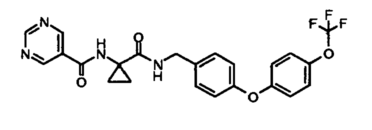 | 65a) | analog 7c) | [M+H]+ = 473 |
| (66) | 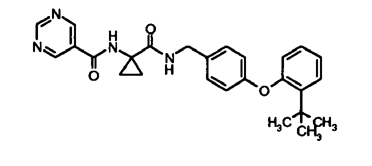 | 66a) | analog 7c) | [M+H]+ = 445 |
| (67) | 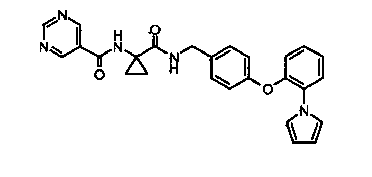 | 67a) | analog 7c) | [M+H]+ = 454 |
| (68) | 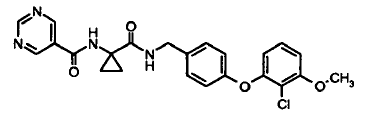 | 68a) | analog 7c) | [M+H]+ = 453/455 |
| (69) | 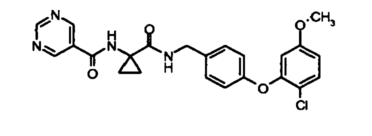 | 69a) | analog 7c) | [M+H]+ = 453/455 |
| (70) | 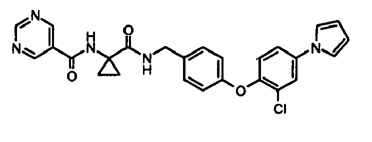 | 70a) | analog 7c) | [M+H]+ = 488/490 |
| (71) | 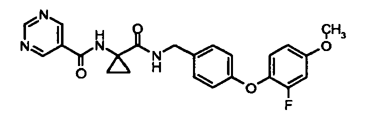 | 71a) | analog 7c) | [M+H]+ = 437/439 |
| (72) | 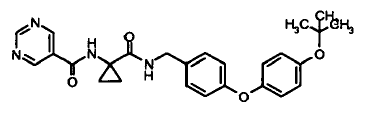 | 72a) | analog 7c) | [M+H]+ = 461 |
| (73) | 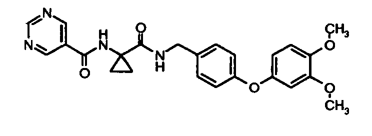 | 73a) | analog 7c) | [M+H]+ = 449 |
| (74) | 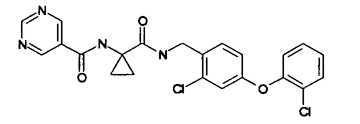 | 74b) | 74c) | [M+H]+ = 457/9/61 |
| (75) | 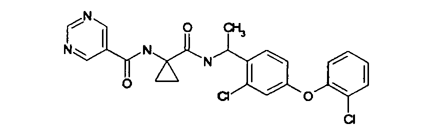 | 75c) | analog 74c) | [M+H]+ = 471/3/5 |
| (76) | 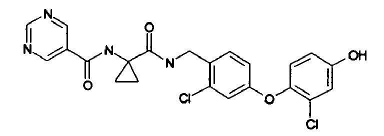 | 76c) | 76d) | [M+H]+ = 473/5/7 |
| (77) | 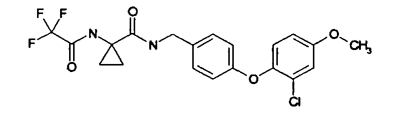 | 53a) | 77a) | 441/443 |
| (78) | 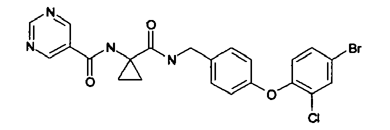 | 78c) | 78d) | [M-H]- = 499/501/3 |
| (79) | 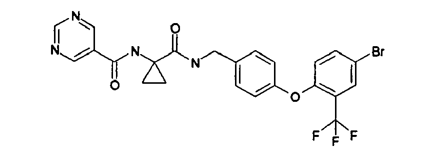 | 79c) | analog 78d) | [M+H]+ = 535/7 |
| (80) | 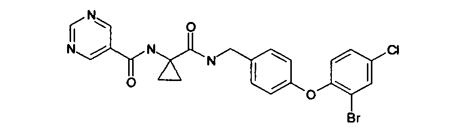 | 80c) | analog 78d) | [M+H]+ = 501/3/5 |
| (81) | 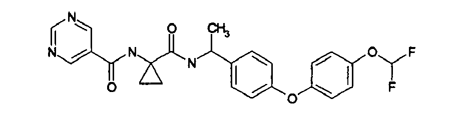 | 81c) | 81d) | [M-H]- = 467 |
| (82) | 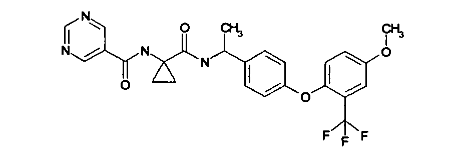 | 82f) | analog 76d) | [M+H]+ = 501 |
| (83) | 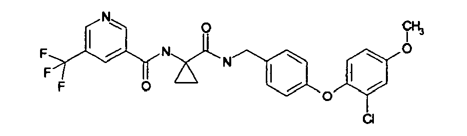 | 53a) | 83a) | [M+H]+ = 520 |
| (84) | 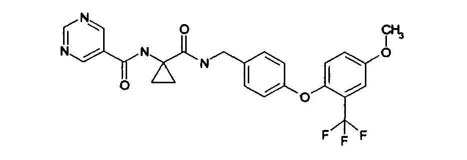 | 84a) | 84b) | [M+H]+ = 487 |
| (85) | 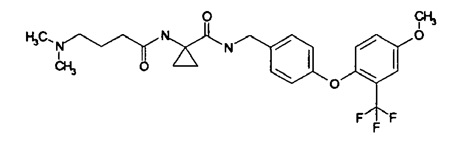 | 84a) | 85b) | [M+H]+ = 494 |
| (86) | 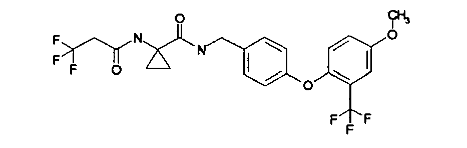 | 84a) | analog 85b) | [M+H]+ = 491 |
| (87) | 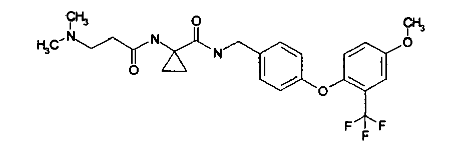 | 84a) | analog 85b) | [M+H]+ = 480 |
| (88) | 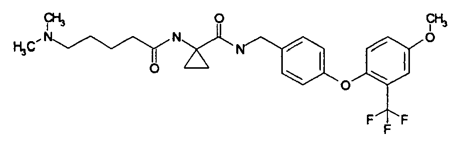 | 84a) | analog 85b) | [M+H]+ = 508 |
| (89) | 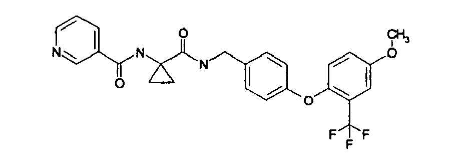 | 84a) | analog 85b) | [M+H]+ = 486 |
| (90) | 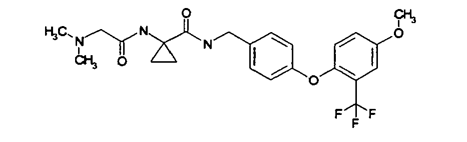 | 84a) | analog 85b) | [M+H]+ = 466 |
| (91) | 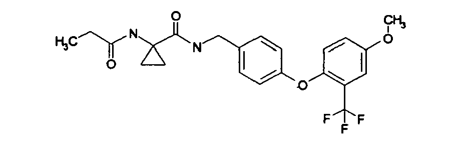 | 84a) | analog 85b) | [M+H]+ = 437 |
| (92) | 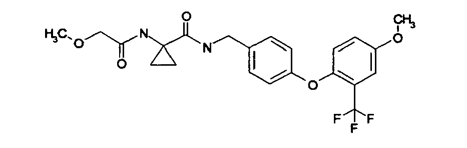 | 84a) | analog 85b) | [M+H]+ = 453 |
| (93) | 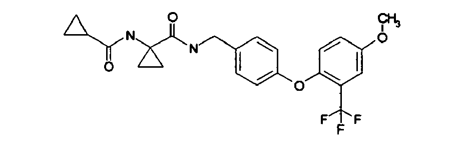 | 84a) | analog 85b) | [M+H]+ = 449 |
| (94) | 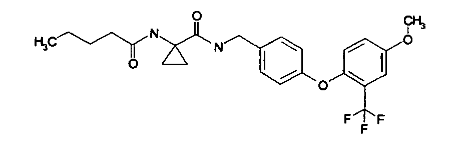 | 84a) | analog 85b) | [M+H]+ = 465 |
| (95) | 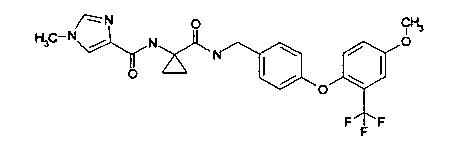 | 84a) | analog 85b) | [M+H]+ = 489 |
| (96) | 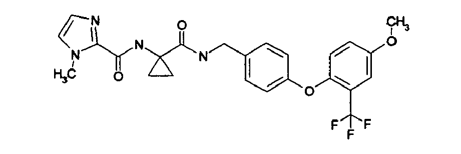 | 84a) | analog 85b) | [M+H]+ = 489 |
| (97) | 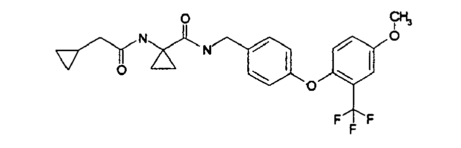 | 84a) | analog 85b) | [M+H]+ = 463 |
| (98) | 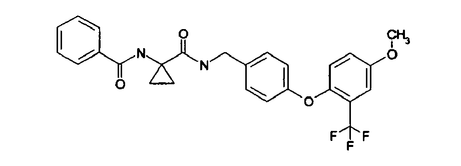 | 84a) | analog 85b) | [M+H]+ = 485 |
| (99) | 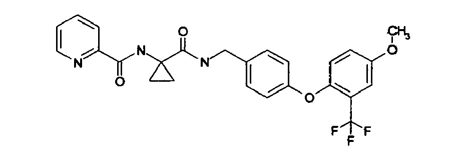 | 84a) | analog 85b) | [M+H]+ = 486 |
| (100) | 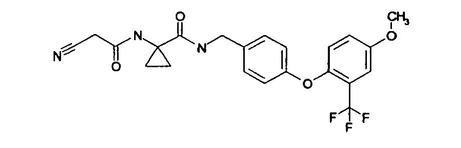 | 84a) | 100a) | [M+H]+ = 448 |
| (101) | 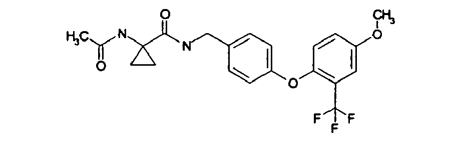 | 84a) | analog 100a) | [M+H]+ = 423 |
| (102) | 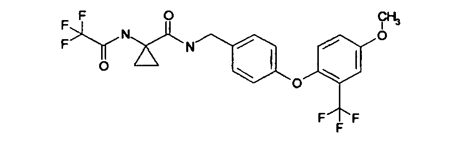 | 84a) | analog 100a) | [M+H]+ = 477 |
| (103) | 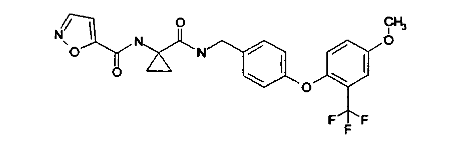 | 84a) | analog 100a) | [M+H]+ = 476 |
| (104) | 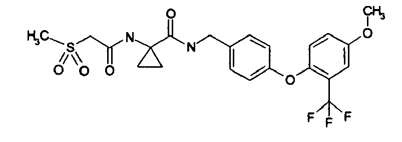 | 84a) | analog 78d) | [M+H]+ = 501 |
| (105) | 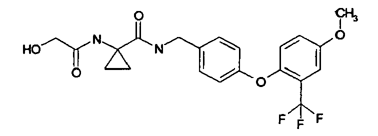 | 84a) | analog 78d) | [M+H]+ = 439 |
| (106) | 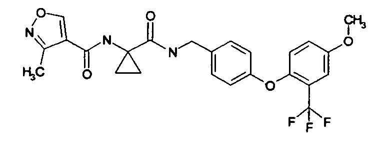 | 84a) | 106a) | [M+H]+ = 490 |
| (107) | 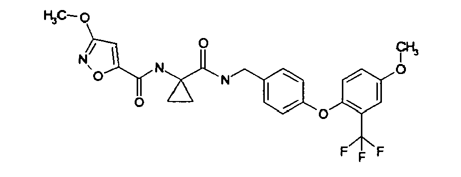 | 84a) | 107a), analog 106a) | [M+H]+ = 506 |
| (108) | 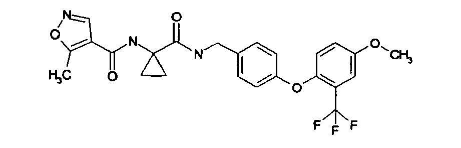 | 84a) | 108a), analog 106a) | [M+H]+ = 490 |
| (109) | 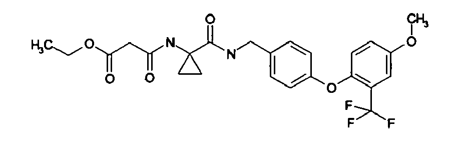 | 84a) | 109a) | [M+H]+ = 495 |
| (110) | 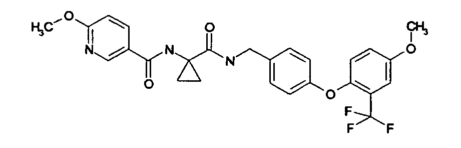 | 84a) | analog 109a) | [M+H]+ = 516 |
| (111) | 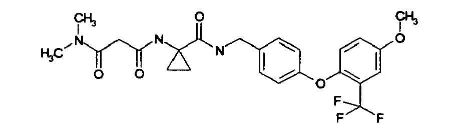 | 84a) | analog 109a) | [M+H]+ = 494 |
| (112) | 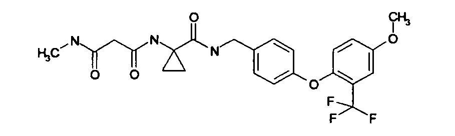 | 84a) | analog 109a) | [M+H]+ = 480 |
| (113) | 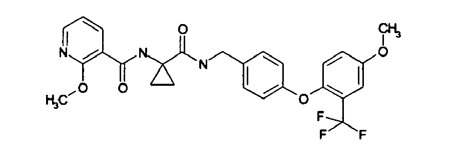 | 84a) | 113a) | [M+H]+ = 516 |
| (114) | 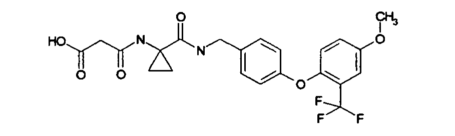 | 84a) | 114a) | [M+H]+ = 467 |
| (115) | 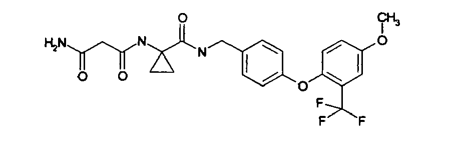 | 84a) | analog 109a) | [M+H]+ = 466 |
| (116) | 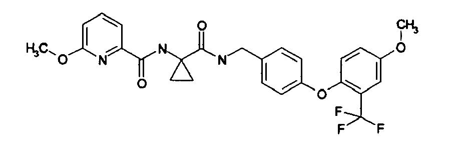 | 84a) | 116a), analog 109a) | [M+H]+ = 516 |
| (117) | 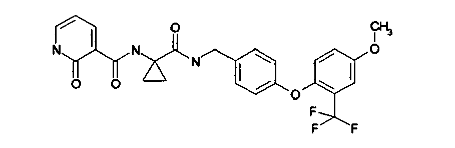 | 84a) | analog 107a) | [M+H]+ = 502 |
| (118) | 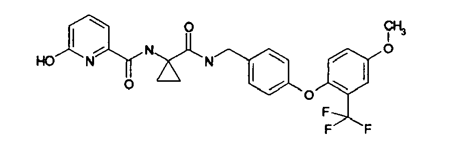 | 84a) | 118a) | [M+H]+ = 502 |
| (119) | 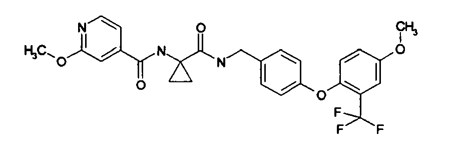 | 84a) | 119a), analog 118a) | [M+H]+ = 516 |
| (120) | 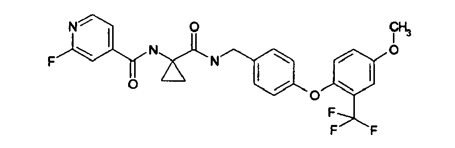 | 84a) | 120a) | [M+H]+ = 504 |
| (121) | 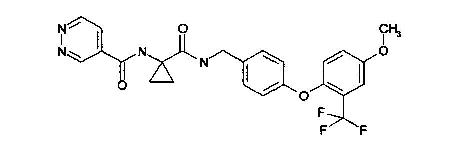 | 84a) | analog 120a) | [M+H]+ = 487 |
| (122) | 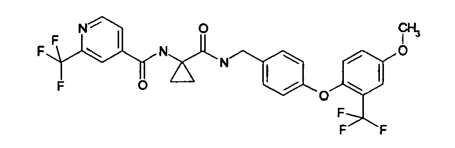 | 84a) | analog 120a) | [M+H]+ = 554 |
| (123) | 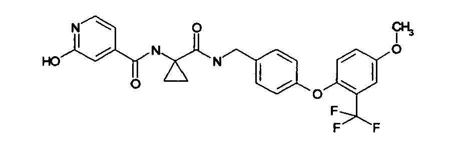 | 84a) | analog 78d) | [M+H]+ = 502 |
| (124) | 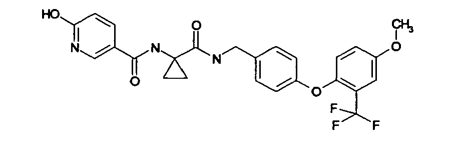 | 84a) | analog 78d) | [M+H]+ = 502 |
| (125) | 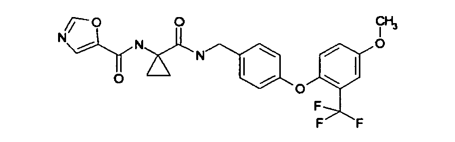 | 84a) | 125a) | [M+H]+ = 476 |
| (126) | 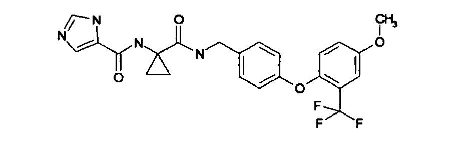 | 84a) | analog 120a) | [M+H]+ = 475 |
| (127) | 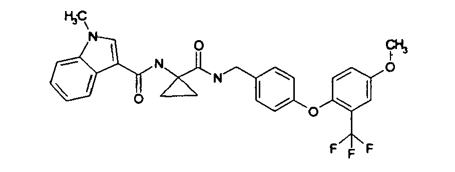 | 84a) | analog 78d) | [M+H]+ = 538 |
| (128) | 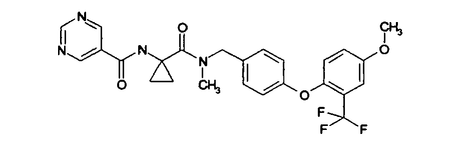 | 84a) | analog 78d) | [M+H]+ = 501 |
| (129) | 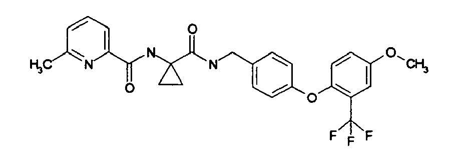 | 84a) | 129a) | [M+H]+ = 500 |
| (130) | 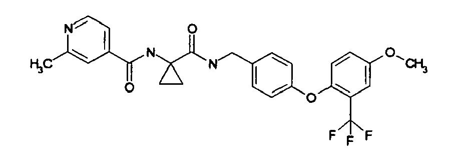 | 84a) | analog 129a) | [M+H]+ = 500 |
| (131) | 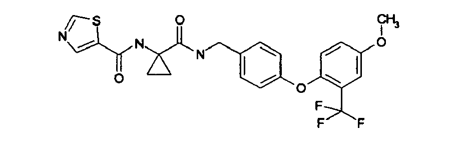 | 84a) | analog 125a) | [M+H]+ = 492 |
| (132) | 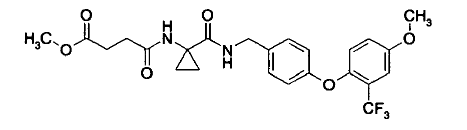 | 84a) | analog 109 | [M+H]+ = 495 |
| (133) | 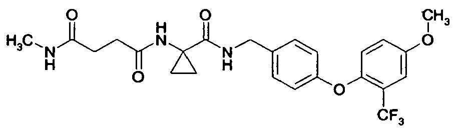 | 84a) | analog 109 | [M+H]+ = 494 |
| (134) | 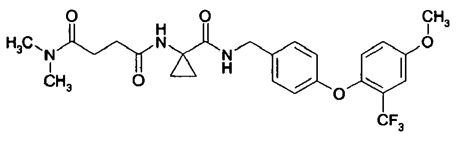 | 84a) | analog 109 | [M+H]+ = 508 |
| (135) | 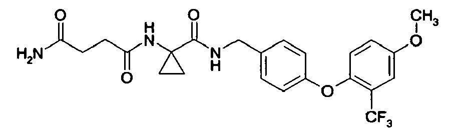 | 84a) | analog 109 | [M+H]+ = 480 |
| (136) | 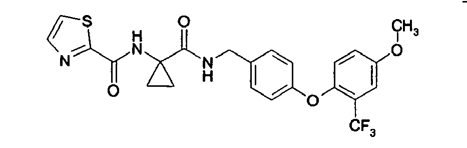 | 84a) | analog 129 | [M+H]+ = 492 |
| (137) | 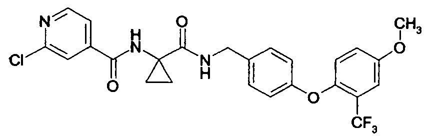 | 84a) | analog 129 | [M+H]+ = 520 |
| (138) | 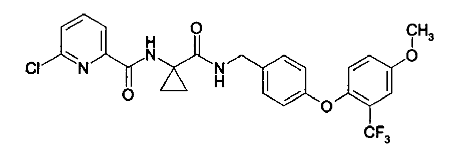 | 84a) | analog 129 | [M+H]+ = 520 |
| (139) | 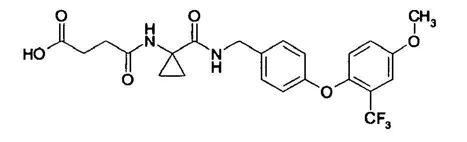 | | | [M+H]+ = 481 |
| (140) | 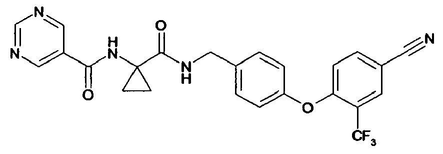 | | | [M+H]+ = 482 |
| (141) | 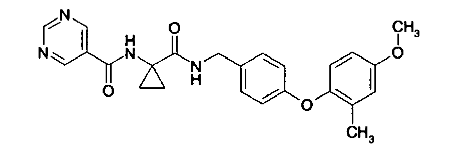 | 85 | analog 1 e) | [M+H]+ = 433 |
| (142) | 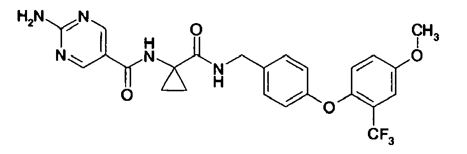 | 84a) | analog 129 | [M+H]+ = 502 |
| (143) | 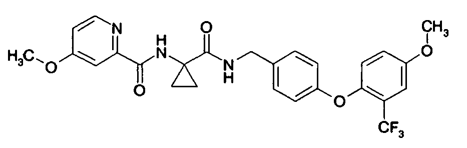 | 84a) | analog 129 | [M+H]+ = 516 |
| (144) | 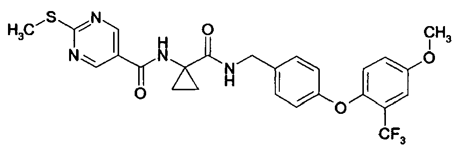 | 84a) | analog 129 | [M+H]+ = 533 |
| (145) | 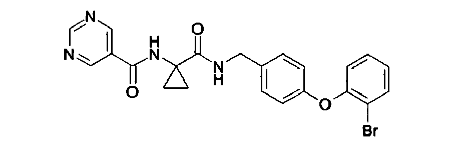 | 86 | analog 1e) | [M+H]+ = 467 |
| (146) | 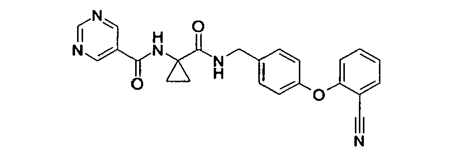 | | analog 140 | [M+H]+ = 414 [M-H]- = 412 |
| (147) | 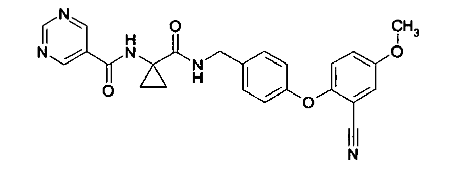 | | analog 140 | [M+H]+ = 444 [M-H]- = 442 |
| (148) | 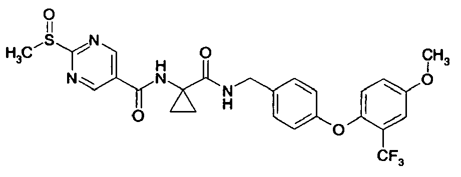 | | | [M+H]+ = 549 |
| (149) | 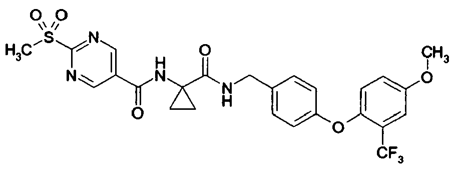 | | | [M+H]+ = 565 |
| (150) | 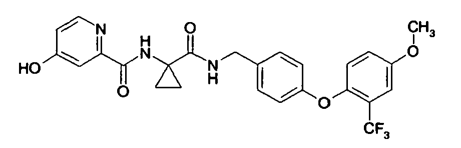 | 84a) | analog 129 | [M+H]+ = 502 |
| (151) | 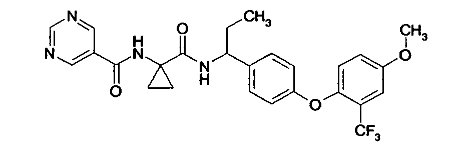 | 87 | analog 1e) | [M+H]+ = 515 |
| (152) | 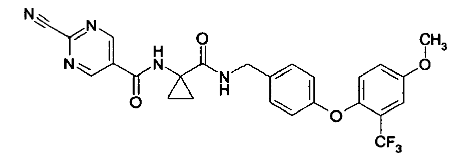 | | | [M+H]+ = 512 |
| (153) | 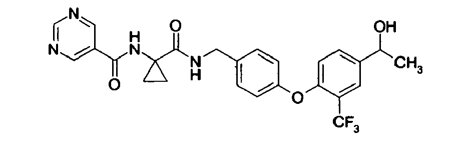 | 88 | analog 1e) | [M+H]+ = 501 |
| (154) | 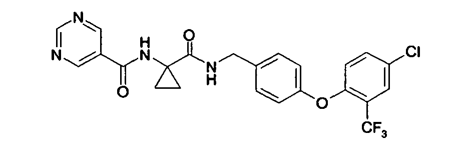 | 89 | analog 1e) | [M+H]+ = 491 |
| (155) | 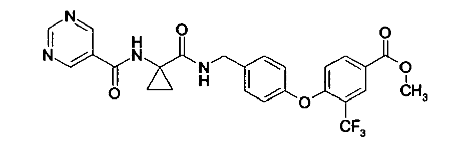 | 90 | analog 1e) | [M+H]+ = 515 |
| (156) | 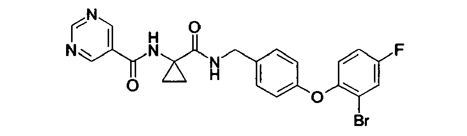 | 91 | analog 1e) | [M+H]+= 485 [M-H]- = 483 |
| (157) | 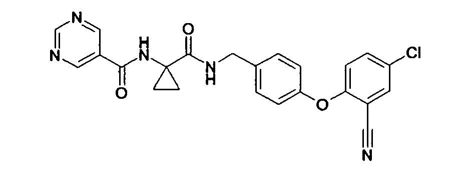 | | analog 140 | [M+H]+ = 448 |
| (158) | 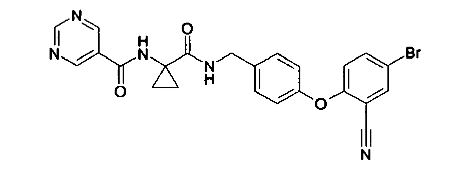 | | analog 140 | [M+H]+ = 492/94 |
| (159) | 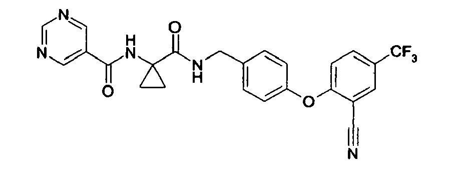 | | analog 140 | [M+H]+ = 482 |
| (160) | 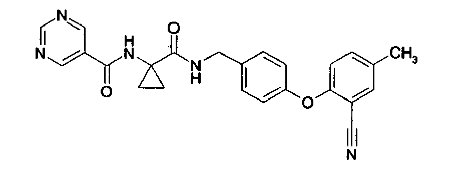 | | analog 140 | [M+H]+ = 428 |
| (161) | 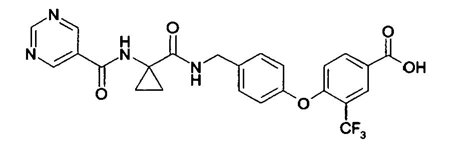 | | analog 139 | [M+H]+ = 501 [M-H]- = 499 |
| (162) | 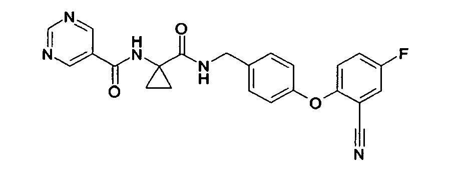 | | analog 140 | [M+H]+ = 432 [M-H]- = 430 |
| (163) | 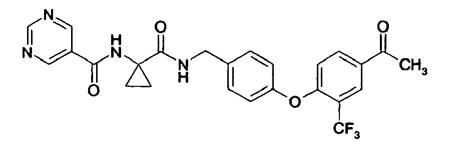 | | | [M+H]+ = 499 |

Tabelle der Zwischenverbindungen **III**

| | | | |
|---|---|---|---|
| | | 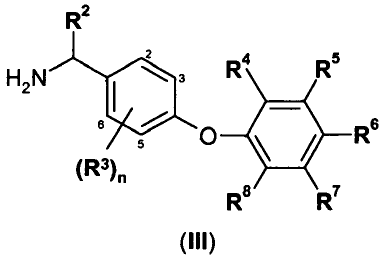 | |

| **Nr.** | **Struktur** | **Herstell-vorschrift** | **LCMS** |
|---|---|---|---|
| (1d) | 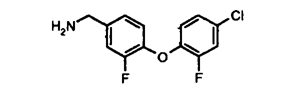 | (1d) | [M+H]+ = 270/272 |
| (2c) | 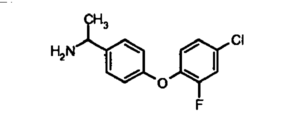 | (2c) | [M-NH2]- = 249/251 |
| (3b) | 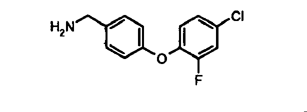 | analog (1 d) | [M+H]+ = 252/254 |
| (7b) | 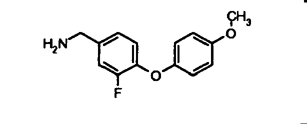 | (7a,b) | [M-NH2]+ =231 |
| (8b) | 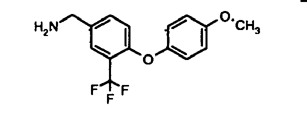 | analog (7a,b) | [M-NH2]+ =281 |
| (9b) | 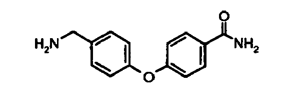 | (9a,b) | [M-NH2]+ =226 |
| (10b) | 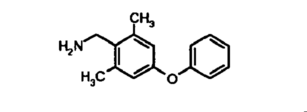 | (10a,b) | [M-NH2]+ =211 |
| (11b) | 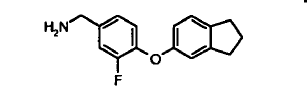 | analog (7a,b) | [M-NH2]+ =241 |
| (12b) | 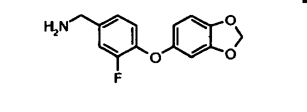 | analog (7a,b) | [M-NH2]+ =245 |
| (13b) | 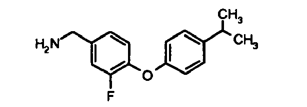 | analog (7a,b) | [M-NH2]+ =243 |
| (14c) | 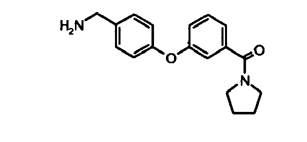 | analog (9a,b) | [M-NH2]+ =280 |
| (15c) | 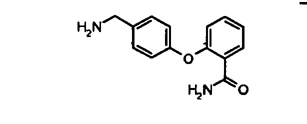 | analog (9a,b) | [M-NH2]+ =226 |
| (16c) | 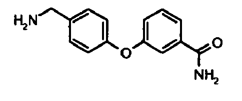 | analog (9a,b) | [M-NH2]+ =226 |
| (17b) | 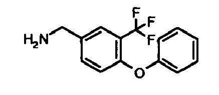 | analog (8a,b) | [M-NH2]+ =251 |
| (18b) | 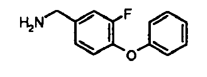 | analog (7a,b) | [M-NH2]+ =201 |
| (19c) | 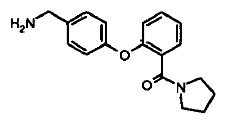 | analog (9a,b) | [M-NH2]+ =280 |
| (20b) | 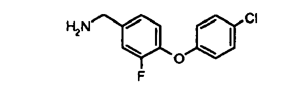 | analog (7a,b) | [M-NH2]+ =235 |
| (21b) | 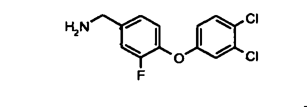 | analog (7a,b) | [M+H]+ = 286/288/290 |
| (22b) | 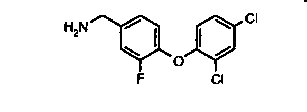 | analog (7a,b) | [M-NH2]+ = 269/71/73 |
| (23b) | 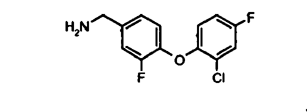 | analog (7a,b) | [M+H]+ = 270/272 |
| (39b) | 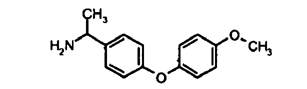 | (39a,b) | [M-NH2]+ = 269/71/73 |
| (40b) | 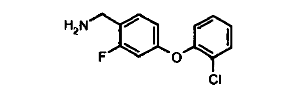 | (40a,b) | [M+H]+ = 252/254 |
| (41b) | 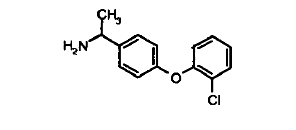 | analog (39a,b) | [M-NH2]+ = 231/233 |
| (43b) | 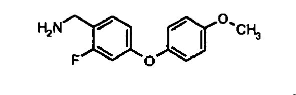 | (43a,b) | [M-NH2]+ = 231 |
| (44b) | 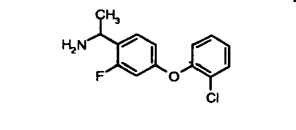 | (44a,b) | [M-NH2]+ = 249/251 |
| (45b) | 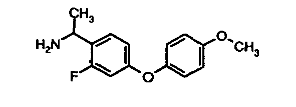 | analog (44a,b) | [M-NH2]+ = 245 |
| (46b) | 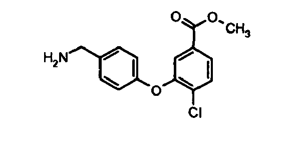 | analog (1d) | [M-NH2]+ = 275/277 |
| (49a) | 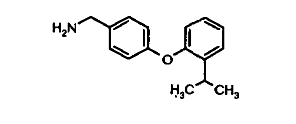 | (49a) | [M-NH2]+ =225 |
| (50a) | 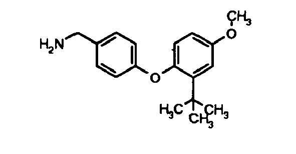 | analog (49a) | [M-NH2]+ =269 |
| (51 a) | 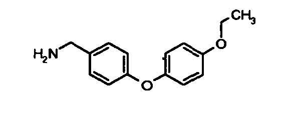 | analog (49a) | [M-NH2]+ =227 |
| (52a) | 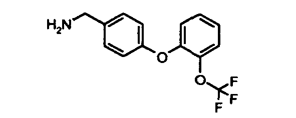 | analog (49a) | [M-NH2]+ =267 |
| (53a) | 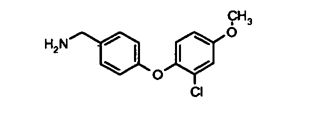 | analog (49a) | [M-NH2]+ =247 |
| (54a) | 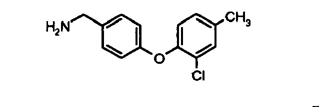 | analog (49a) | [M-NH2]+ =231 |
| (55a) | 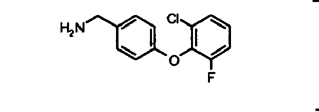 | analog (49a) | [M-NH2]+ =235 |
| (56a) | 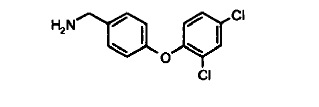 | analog (49a) | [M-NH2]+ =251/253 |
| (57a) | 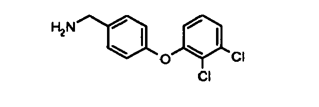 | analog (49a) | [M-NH2]+ =251/253 |
| (58a) | 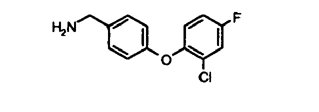 | analog (49a) | [M-NH2]+ =235 |
| (59a) | 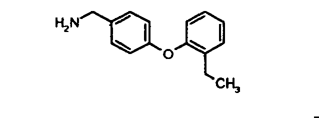 | analog (49a) | [M-NH2]+ =211 |
| (60a) | 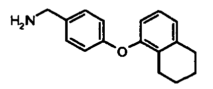 | analog (49a) | [M-NH2]+ =237 |
| (61a) | 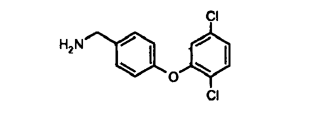 | analog (49a) | [M-NH2]+ =251 /253 |
| (62a) | 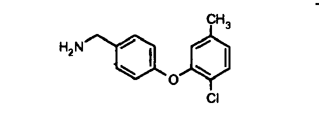 | analog (49a) | [M-NH2]+ =231 |
| (63a) | 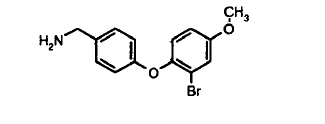 | analog (49a) | [M-N H2]+ =291 /293 |
| (64a) | 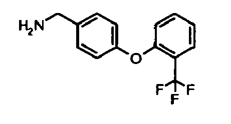 | analog (49a) | [M-NH2]+ =251 |
| (65a) | 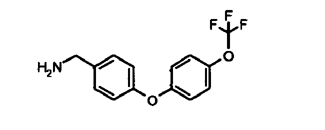 | analog (49a) | [M-NH2]+ =267 |
| (66a) | 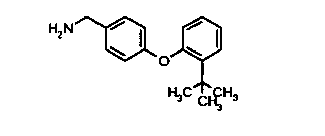 | analog (49a) | [M-NH2]+ =239 |
| (67a) | 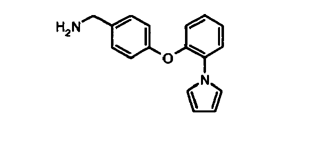 | analog (49a) | [M-NH2]+ =248 |
| (68a) | 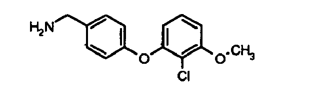 | analog (49a) | [M-NH2]+ =247 |
| (69a) | 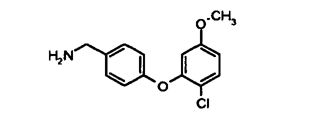 | analog (49a) | [M-NH2]+ =247 |
| (70a) | 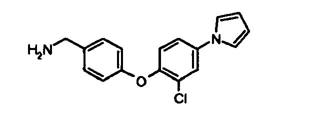 | analog (49a) | [M-NH2]+ =282 |
| (71a) | 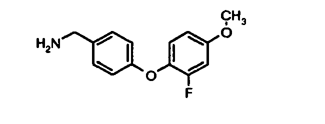 | analog (49a) | [M-NH2]+ =231 |
| (72a) | 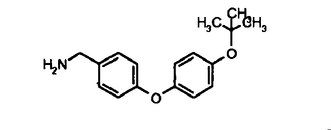 | analog (49a) | [M-NH2]+ =255 |
| (73a) | 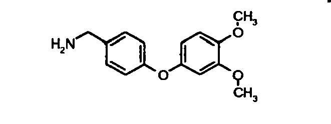 | analog (49a) | [M-NH2]+ =243 |
| (74b) | 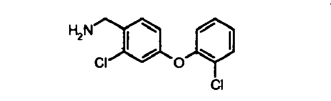 | (74a,b) | [M+H]+= 268/70/2 |
| (75c) | 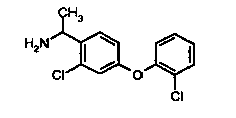 | (75a,b,c) | [M+H]+= 282/4/6 |
| (76c) | 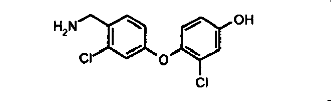 | (76a,b,c) | [M-NH4]+= 267/9/71 |
| (78c) | 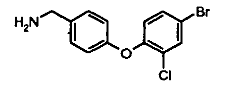 | (78a,b,c) | [M+H]+= 295/7/9 |
| (79c) | 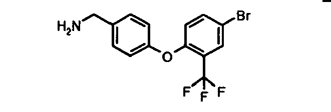 | (79a,b,c) | [M+H]+= 346/8 |
| (80c) | 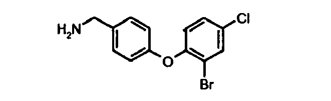 | analog (79a,b,c) | [M-NH2]+= 295/7/9 |
| (81c)) | 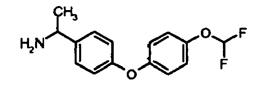 | (81a,b,c) | [M+H-NH3]+= 263 |
| (82f)) | 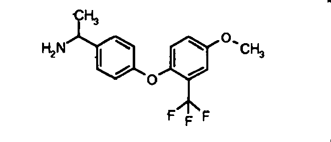 | (82c,d,e,f) | M*+=311 |
| (84a)) | 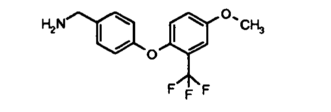 | analog (82c) und (84a) | [M-NH2]+= 281 |
| (85) | 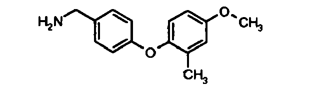 | analog (49a) | [M+H-NH3]+= 227 |
| (86) | 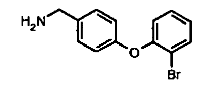 | analog (49a) | |
| (87) | 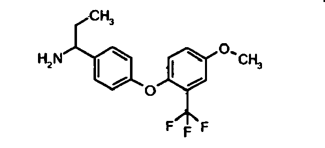 | analog (49a) | [M+H-NH3]+= 309 |
| (88) | 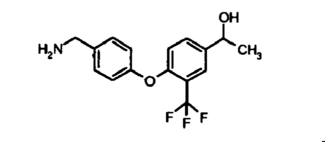 | | [M+H-NH3]+= 295 |
| (89) | 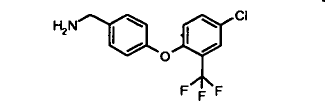 | analog (49a) | [M+H-NH3]+= 285 [M+H]+ = 302 |
| (90) | 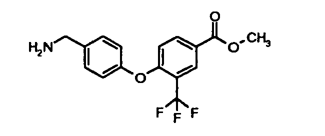 | | [M+H-NH3]+= 309 |
| (91) | 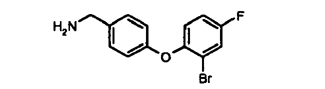 | analog (49a) | [M+H]+ = 296 |

Die nachfolgenden Beispiele beschreiben pharmazeutische Darreichungsformen, die als Wirkstoff eine beliebige Verbindung der allgemeinen Formel I enthalten, ohne jedoch den Umfang der vorliegenden Erfindung darauf einzuschränken:

### Beispiel I

Trockenampulle mit 75 mg Wirkstoff pro 10 ml

Zusammensetzung:

| | |
|---|---|
| Wirkstoff | 75.0 mg |
| Mannitol | 500 mg |
| Wasser für Injektionszwecke ad | 10.0 ml |

Herstellung:
Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

Herstellung:
(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel III

Tablette mit 350 mg Wirkstoff

Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

Herstellung:
(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
   Durchmesser der Tabletten: 12 mm.

### Beispiel IV

Kapseln mit 50 mg Wirkstoff

Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

Herstellung:
(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

### Beispiel V

### Kapseln mit 350 mg Wirkstoff

Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

Herstellung:
(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Gr6Be 0 abgefühlt.

### Beispiel VI

Suppositorien mit 100 mg Wirkstoff
1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel **I** 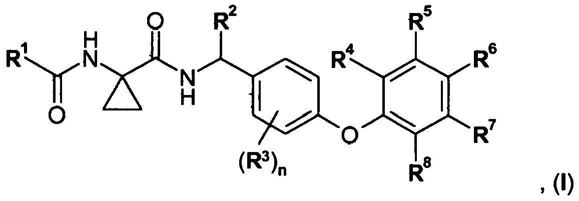 in der
**R¹** (a) eine gegebenenfalls mit einem Rest **R^{1.1}** substituierte C₁₋₆-Alkylgruppe, (b) eine C₁₋₃-Alkylgruppe, in der jede Methylengruppe mit bis zu zwei Fluoratomen und jede Methylgruppe mit bis zu drei Fluoratomen substituiert ist, (c) eine gegebenenfalls mit einem Rest **R^{1.2}** substituierte C₃₋₆-Cycloalkylgruppe, (d) eine C₂₋₆-Alkenylgruppe, (e) eine C₂₋₆-Alkinylgruppe, (f) eine gegebenenfalls mit 1, 2 oder 3 Resten **R^{1.3}** substituierte Aryl-C₀₋₂alkylengruppe, (g) ein gegebenenfalls mit 1, 2 oder 3 Resten **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält und der zusätzlich benzo-kondensiert sein kann, (h) ein gegebenenfalls mit einem Rest **R^{1.4}** substituierter sechsgliedriger Heteroaryl-Rest, der ein, zwei oder drei N-Atome enthält und der zusätzlich benzo-kondensiert sein kann, (i) -O-**R^{1.1.1}** oder (j) -**NR^{1.1.2}R^{1.1.4}**
**R^{1.1}** Halogen, -NO₂, -CN, C₃₋₆-Cycloalkyl, -O**R^{1.1.1}**, -SR^{1.1.1}, -C(O)**R^{1.1.1}**, -S(O)₂-**R^{1.21.2}**, -O-S(O)₂-**R^{1.1.1}**, -CO₂**R^{1.1.1}**, -O-C(O)-**R^{1.1.1}**, -N**R^{1.1.3}R^{1.1.4}**, -N**R^{1.1.3}**-C(O)-**R^{1.1.1}**, -N**R^{1.1.3}**-C(O)-**R^{1.1.1}** -N**R^{1.1.3}**-CO₂-**R^{1.1.1}** oder -C(O)N**R^{1.1.3}R^{1.1.4}**,
**R^{1.1.1}** (a) H, (b) C₁₋₄-Alkyl,
**R^{1.1.1.1}** (c) C₁₋₃-Alkyl, in der jede Methylengruppe mit bis zu zwei Fluoratomen und jede Methylgruppe mit bis zu drei Fluoratomen substituiert ist, (d) eine gegebenenfalls mit 1, 2 oder 3 Resten **R^{1.1.1.1}** substituierte Phenylgruppe, (e) C₃₋₆₋Cycloalkyl oder (f) eine gegebenenfalls mit 1, 2 oder 3 Resten **R^{1.1.1.2}** substituierte Pyridylgruppe, unabhängig voneinander (a) Halogen, -NO₂, -CN, -OH, -O-C₁₋₄-Alkyl, C₃₋₆-Cycloalkyl, C₁₋₄-Alkyl oder (b) C₁₋₃-Alkyl, in der jede Methylengruppe mit bis zu zwei Fluoratomen und jede Methylgruppe mit bis zu drei Fluoratomen substituiert ist,
**R^{1.1.1.2}** unabhängig voneinander Halogen oder C₁₋₄-Alkyl,
**R^{1.1.2}** (a) C₁₋₄-Alkyl, (b) C₁₋₃-Alkyl, in der jede Methylengruppe mit bis zu zwei Fluoratomen und jede Methylgruppe mit bis zu drei Fluoratomen substituiert ist, (c) -O-C₁₋₄-Alkyl oder (d) eine gegebenenfalls mit 1, 2 oder 3 Resten **R^{1.1.1.1}** substituierte Phenylgruppe,
**R^{1.1.3}**, **R^{1.1.4}** unabhängig voneinander (a) H, (b) eine gegebenenfalls mit 1, 2 oder 3 Resten **R^{1.1.4.1}** substituierte C₁₋₄-Alkylgruppe, (c) eine gegebenenfalls mit 1, 2 oder 3 Resten **R^{1.1.1.1}** substituierte Phenylgruppe, (d) C₃₋₆Cycloalkyl, oder
**R^{1.1.3}** 6-gliedrigen ausgewählt und **R^{1.1.4}** zusammen mit dem N-Atom, an das sie gebunden sind, einen 4-, 5- oder heterocyclischen Ring bilden, der zusätzlich ein weiteres Heteroatom aus N, O und S enthalten kann, oder
**R^{1.1.3}** Imid und **R^{1.1.4}** zusammen mit dem N-Atom, an das sie gebunden sind, ein cyclisches bilden,
**R^{1.1.4.1}** unabhängig voneinander Halogen, -NH₂, -NH(C₁₋₄-Alkyl), -N(C₁₋₄-Alkyl)₂ oder -SO₂-**R^{1.1.2}**,
**R^{1.2}** Halogen, -NO₂, -CN oder Phenyl,
**R^{1.3}** (a) Halogen, -NO₂, -CN, -O**R^{1.1.1}**, -S**R^{1.1.1}**, -CO₂**R^{1.1.1}**, C₁₋₆-Alkyl oder (b) C₁₋₃-Alkyl, in der jede Methylengruppe mit bis zu zwei Fluoratomen und jede Methylgruppe mit bis zu drei Fluoratomen substituiert ist,
**R^{1.4}** unabhängig voneinander (a) Halogen, -NO₂, -CN, -O**R^{1.1.1}**, -S**R^{1.1.1}**, -S(O)-**R^{1.1.2}**, -S(O)₂**R^{1.1.2}**, -N**R^{1.1.3}R^{1.1.4}**, C₁₋₆-Alkyl, (b) C₁₋₃-Alkyl, in der jede Methylengruppe mit bis zu zwei Fluoratomen und jede Methylgruppe mit bis zu drei Fluoratomen substituiert ist, oder (c) eine Oxo-Gruppe,
**R²** (a) H, C₁₋₄-Alkyl oder (b) C₁₋₃-Alkyl, in der jede Methylengruppe mit bis zu zwei Fluoratomen und jede Methylgruppe mit bis zu drei Fluoratomen substituiert ist,
**R³** unabhängig voneinander (a) H, Halogen, -CN, -OH, C₁₋₆-Alkyl, C₃₋₇Cycloalkyl, -O-C₁₋₄-Alkyl, -O-CF₃, -O-C₃₋₆-Cycloalkyl, -N(C₁₋₃-Alkyl)₂, -C(O)-NH₂, -(SO₂)NH₂, -SO₂-C₁₋₃-Alkyl oder (b) C₁₋₃-Alkyl, in der jede Methylengruppe mit bis zu zwei Fluoratomen und jede Methylgruppe mit bis zu drei Fluoratomen substituiert ist,
**R⁴, R⁵, R⁶, R⁷, R⁸** unabhängig voneinander (a) H, Halogen, -CN, -OH, (b) C₁₋₆-Alkyl, wobei zwei benachbarte Substituenten gemeinsam eine Trimethylen- oder Tetramethylengruppe darstellen können, (c) C₁₋₃-Alkyl, in der jede Methylengruppe mit bis zu zwei Fluoratomen und jede Methylgruppe mit bis zu drei Fluoratomen substituiert ist, (d) C₃₋₇-Cycloalkyl, (e) -O-C₁₋₆-Alkyl, wobei zwei benachbarte Substituenten eine Methylendioxy- oder Ethylendioxygruppe darstellen können, (f) -O-CF₃, -O-C₃₋₇Cycloalkyl, (g) -NH₂, -NH(C₁₋₃-Alkyl), -N(C₁₋₃-Alkyl)₂, (h) -C(O)-**R^{8.1}**, (i) -SO₂-**R^{8.2}**, (j) eine gegebenenfalls mit einer oder zwei C₁₋₃-Alkylgruppen substituierte fünfgliedrige Heteroarylgruppe, die ausgewählt ist aus der Gruppe bestehend aus Pyrrolyl, Oxazolyl, Isoxazolyl, Oxadiazolyl, Thiazolyl, Isothiazolyl, Thiadiazolyl, Imidazolyl, Pyrazolyl, Triazolyl und Tetrazolyl, oder (k) eine gegebenenfalls mit einer oder zwei C₁₋₃-Alkylgruppen substituierte sechsgliedrige Heteroarylgruppe, die ausgewählt ist aus der Gruppe bestehend aus Pyridyl, Pyrimidinyl, Pyrazinyl, Pyridazinyl und Triazinyl,
**R^{8.1}** -NH₂, -NH(C₁₋₆-Alkyl),-N(C₁₋₆-Alkyl)₂, *N*-Acetidinyl, *N*-Pyrrolidinyl, *N*-Piperidinyl, *N*-Morpholinyl, -OH, -O-C₁₋₈-Alkyl oder -O-C₃₋₇Cycloalkyl,
**R^{8.2}** -NH₂, -NH(C₁₋₆-Alkyl),-N(C₁₋₆-Alkyl)₂, *N*-Acetidinyl, *N*-Pyrrolidinyl, *N*-Piperidinyl oder *N*-Morpholinyl und
**n** eine der Ziffern 0, 1, 2, 3 oder 4 bedeuten,
deren Enantiomere, deren Diastereomere, deren Gemische und deren Salze, insbesondere deren physiologisch verträgliche Salze mit organischen oder anorganischen Säuren oder Basen.

2. Verbindungen der allgemeinen Formel **I** gemäß Anspruch 1, in der **R²**, **R³**, **R⁴**, **R⁵**, **R⁶**, **R⁷**, **R⁸** und n wie in Anspruch 1 definiert sind und
**R¹** (a) eine gegebenenfalls mit einem Rest **R^{1.1}** substituierte C₁₋₆-Alkylgruppe, (b) eine C₁₋₃-Alkylgruppe, in der jede Methylengruppe mit bis zu zwei Fluoratomen und jede Methylgruppe mit bis zu drei Fluoratomen substituiert ist, (c) eine gegebenenfalls mit einem Rest **R^{1.2}** substituierte C₃₋₆-Cycloalkylgruppe,
**R^{1.1}** (d) eine gegebenenfalls mit 1, 2 oder 3 Resten **R^{1.3}** substituierte Phenylgruppe, (e) ein gegebenenfalls mit einem Rest **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält, oder (f) ein gegebenenfalls mit einem Rest **R^{1.4}** substituierter sechsgliedriger Heteroaryl-Rest, der ein, zwei oder drei N-Atome enthält, C₃₋₆-Cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂C₁₋₆-Alkyl oder -O-S(O)₂-C₁₋₆-Alkyl,
**R^{1.1.1}** (a) H, (b) C₁₋₄-Alkyl, (c) C₁₋₃-Alkyl, in der jede Methylengruppe mit bis zu zwei Fluoratomen und jede Methylgruppe mit bis zu drei Fluoratomen substituiert ist, oder (d) C₃₋₆-Cycloalkyl,
**R^{1.1.3}**, **R^{1.1.4}** unabhängig voneinander (a) H, (b) C₁₋₄-Alkyl oder (c) C₃₋₆-Cycloalkyl,
**R^{1.2}** Halogen, -NO₂, -CN oder Phenyl,
**R^{1.3}** (a) Halogen, -NO₂, -CN, -O**R^{1.1.1}**, -S**R^{1.1.1}**, -CO₂**R^{1.1.1}**, C₁₋₆-Alkyl oder (b) C₁₋₃-Alkyl, in der jede Methylengruppe mit bis zu zwei Fluoratomen und jede Methylgruppe mit bis zu drei Fluoratomen substituiert ist, und
**R^{1.4}** (a) Halogen, -NO₂, -CN, -O**R^{1.1.1}**, -S**R^{1.1.1}**, -N**R^{1.1.3}R^{1.1.4}**, C₁₋₆-Alkyl, (b) C₁₋₃-Alkyl, in der jede Methylengruppe mit bis zu zwei Fluoratomen und jede Methylgruppe mit bis zu drei Fluoratomen substituiert ist, oder (c) eine Oxo-Gruppe bedeuten,
deren Enantiomere, deren Diastereomere, deren Gemische und deren Salze, insbesondere deren physiologisch verträgliche Salze mit organischen oder anorganischen Säuren oder Basen.

3. Verbindungen der allgemeinen Formel **I** gemäß Anspruch 1, in der
**R¹** (a) eine gegebenenfalls mit einem Rest **R^{1.1}** substituierte C₁₋₆-Alkylgruppe, (b) eine C₁₋₃-Alkylgruppe, in der jede Methylengruppe mit bis zu zwei Fluoratomen und jede Methylgruppe mit bis zu drei Fluoratomen substituiert ist, (c) eine gegebenenfalls mit einem Rest **R^{1.2}** substituierte C₃₋₆-Cycloalkylgruppe, (d) eine gegebenenfalls mit 1, 2 oder 3 Resten **R^{1.3}** substituierte Phenylgruppe, (e) ein gegebenenfalls mit einem Rest **R^{1.4}** substituierter fünfgliedriger Heteroaryl-Rest, der mindestens ein N-, O- oder S-Atom enthält und der gegebenenfalls zusätzlich ein, zwei oder drei weitere N-Atome enthält, oder (f) ein gegebenenfalls mit einem Rest **R^{1.4}** substituierter sechsgliedriger Heteroaryl-Rest, der ein, zwei oder drei N-Atome enthält,
**R^{1.1}** C₃₋₆-Cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂C₁₋₆-Alkyl oder -O-S(O)₂-C₁₋₆-Alkyl,
**R^{1.1.1}** (a) H, (b) C₁₋₄-Alkyl, (c) C₁₋₃-Alkyl, in der jede Methylengruppe mit bis zu zwei Fluoratomen und jede Methylgruppe mit bis zu drei Fluoratomen substituiert ist, oder (d) C₃₋₆-Cycloalkyl,
**R^{1.1.3}**, **R^{1.1.4}** unabhängig voneinander (a) H, (b) C₁₋₄-Alkyl oder (c) C₃₋₆-Cycloalkyl,
**R^{1.2}** Halogen, -NO₂, -CN oder Phenyl,
**R^{1.3}** (a) Halogen, -NO₂, -CN, -O**R^{1.1.1}**, -S**R^{1.1.1}**, -CO₂**R^{1.1.1}**, C₁₋₆-Alkyl oder (b) C₁₋₃-Alkyl, in der jede Methylengruppe mit bis zu zwei Fluoratomen und jede Methylgruppe mit bis zu drei Fluoratomen substituiert ist,
**R^{1.4}** (a) Halogen, -NO₂, -CN, -O**R^{1.1.1}**, -S**R^{1.1.1}**, -N**R^{1.1.3}R^{1.1.4}**, C₁₋₆-Alkyl oder (b) C₁₋₃-Alkyl, in der jede Methylengruppe mit bis zu zwei Fluoratomen und jede Methylgruppe mit bis zu drei Fluoratomen substituiert ist, oder (c) eine Oxo-Gruppe
**R²** H oder C₁₋₃-Alkyl,
**R³** unabhängig voneinander (a) H, Halogen, C₁₋₃-Alkyl, -O-C₁₋₃-Alkyl oder (b) C₁₋₃-Alkyl, in der jede Methylengruppe mit bis zu zwei Fluoratomen und jede Methylgruppe mit bis zu drei Fluoratomen substituiert ist,
**R⁴** H oder Halogen,
**R⁵** (a) H, Halogen, (b) C₁₋₃-Alkyl, (c) C₁₋₃-Alkyl, in der jede Methylengruppe mit bis zu zwei Fluoratomen und jede Methylgruppe mit bis zu drei Fluoratomen substituiert ist, (d) -O-C₁₋₃-Alkyl, (e) -C(O)-O-C₁₋₃-Alkyl oder-C(O)-NH2,
**R⁶** (a) H, Halogen, (b) C₁₋₃-Alkylen-**R^{6.1}**, (c) C₁₋₃-Alkyl, in der jede Methylengruppe mit bis zu zwei Fluoratomen und jede Methylgruppe mit bis zu drei Fluoratomen substituiert ist, (d) -OH, -O-C₁₋₄-Alkyl, (e) -O-CHF₂, -O-CF₃, (f) -C(O)-O-**R^{6.2}**, -CN, -C(O)-CH₃, -C(O)-NH₂ oder (g) Pyrrolyl,
**R^{6.1}** H, -OH,
**R^{6.2}** H, C₁₋₃-Alkyl,
**R⁷** (a) H, Halogen, (b) C₁₋₃-Alkyl, (c) C₁₋₃-Alkyl, in der jede Methylengruppe mit bis zu zwei Fluoratomen und jede Methylgruppe mit bis zu drei Fluoratomen substituiert ist, (d) -O-C₁₋₃-Alkyl, (e) -C(O)-NH₂ oder -C(O)-Pyrrolidinyl, und
**R⁸** (a) H, Halogen, (b) C₁₋₄-Alkyl, (c) C₁₋₃-Alkyl, in der jede Methylengruppe mit bis zu zwei Fluoratomen und jede Methylgruppe mit bis zu drei Fluoratomen substituiert ist, (d) -O-C₁₋₃-Alkyl, (e) -O-CF₃, (f) -CN, -C(O)-NH₂ oder (g) Pyrrolyl
bedeuten,
deren Enantiomere, deren Diastereomere, deren Gemische und deren Salze, insbesondere deren physiologisch verträgliche Salze mit organischen oder anorganischen Säuren oder Basen.

4. Verbindungen der allgemeinen Formel **I** gemäß Anspruch 1, in der **R²**, **R³**, **R⁴** , **R⁵**, **R⁶**, **R⁷**, **R⁸** und n wie in Anspruch 1 definiert sind und
**R¹** (a) eine gegebenenfalls mit einem Rest **R^{1.1}** substituierte C₁₋₆-Alkylgruppe, (b) eine C₁₋₃-Alkylgruppe, in der jede Methylengruppe mit bis zu zwei Fluoratomen und jede Methylgruppe mit bis zu drei Fluoratomen substituiert ist, (c) eine gegebenenfalls mit 1, 2 oder 3 Resten **R^{1.3}** substituierte Phenylgruppe,
**R^{1.1}** (d) ein gegebenenfalls mit einem Rest **R^{1.4}** substituierter fünfgliedriger Heteroaryl-Rest, der ausgewählt ist aus der Gruppe bestehend aus Furanyl, Isoxazolyl, Isothiazolyl, Oxazolyl, Thiazolyl, Pyrazolyl, Thiadiazolyl, Oxadiazolyl, Triazolyl, Tetrazolyl, Imidazolyl, Indolyl, Thienyl, Pyrrolyl, Benzimidazolyl, Benzoxazolyl, Benzthiazolyl und Benzisoxazinyl, oder (e) ein gegebenenfalls mit einem Rest **R^{1.4}** substituierter sechsgliedriger Heteroaryl-Rest, der ausgewählt ist aus der Gruppe bestehend aus Pyridyl, Pyrimidinyl, Pyridazinyl, Pyrazinyl, Triazinyl, Chinazolinyl und Chinoxazinyl, C₃₋₆-Cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂-C₁₋₆-Alkyl oder -O-S(O)₂-C₁₋₆-Alkyl,
**R^{1.1.1}** (a) H, (b) C₁₋₄-Alkyl, (c) C₁₋₃-Alkyl, in der jede Methylengruppe mit bis zu zwei Fluoratomen und jede Methylgruppe mit bis zu drei Fluoratomen substituiert ist, oder (d) C₃₋₆-Cycloalkyl,
**R^{1.1.3}**, **R^{1.1.4}** unabhängig voneinander (a) H, (b) C₁₋₄-Alkyl oder (c) C₃₋₆-Cycloalkyl,
**R^{1.3}** (a) Halogen, -NO₂, -CN, -O**R^{1.1.1}**, -S**R^{1.1.1}**, -CO₂**R^{1.1.1}**, C₁₋₆-Alkyl oder (b) C₁₋₃-Alkyl, in der jede Methylengruppe mit bis zu zwei Fluoratomen und jede Methylgruppe mit bis zu drei Fluoratomen substituiert ist, und
**R^{1.4}** (a) Halogen, -NO₂, -CN, -O**R^{1.1.1}**, -S**R^{1.1.1}**, C₁₋₆-Alkyl, (b) C₁₋₃-Alkyl, in der jede Methylengruppe mit bis zu zwei Fluoratomen und jede Methylgruppe mit bis zu drei Fluoratomen substituiert ist, oder (c) eine Oxo-Gruppe bedeuten,
deren Enantiomere, deren Diastereomere, deren Gemische und deren Salze, insbesondere deren physiologisch verträgliche Salze mit organischen oder anorganischen Säuren oder Basen.

5. Verbindungen der allgemeinen Formel **I** gemäß Anspruch 1, in der **R²**, **R³**, **R⁴**, **R⁵**, **R⁶**, **R⁷**, **R⁸** und n wie in Anspruch 1 definiert sind und
**R¹** eine Gruppe ausgewählt aus 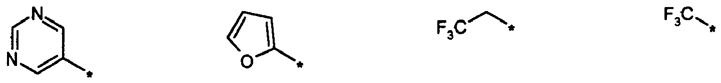 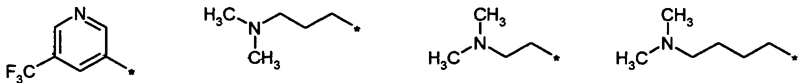 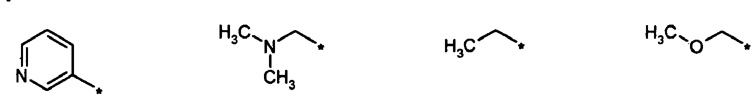 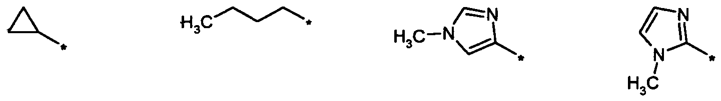 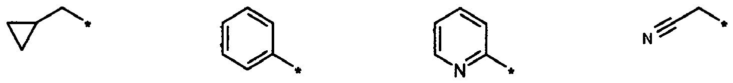 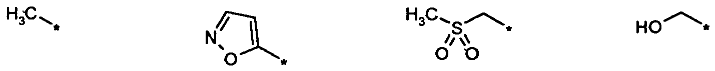 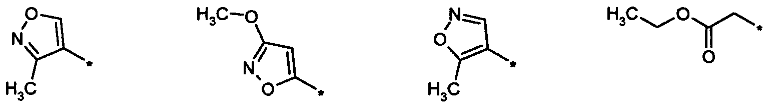 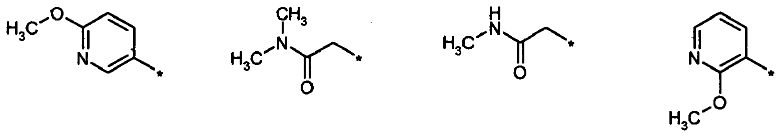 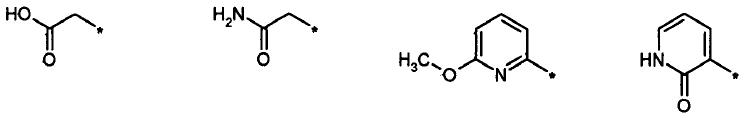 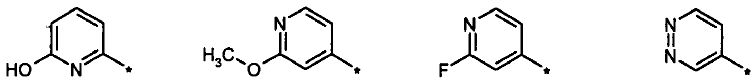 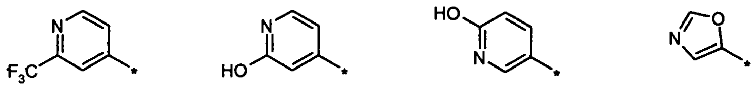 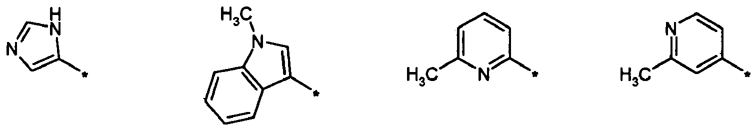 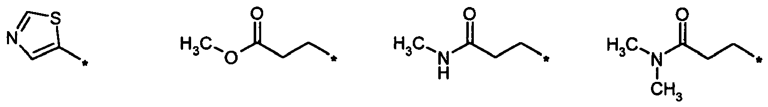 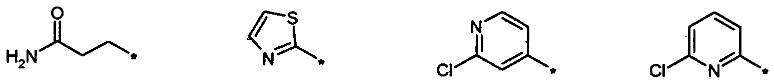 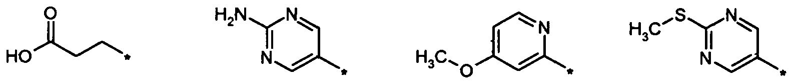 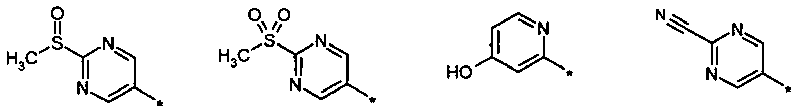
bedeutet,
deren Enantiomere, deren Diastereomere, deren Gemische und deren Salze, insbesondere deren physiologisch verträgliche Salze mit organischen oder anorganischen Säuren oder Basen.

6. Verbindungen der allgemeinen formel **I** gemäß Anspruch 1, in der **R¹**, **R³**, **R⁴**, **R⁵**, **R⁶**, **R⁷**, **R⁸** und **n** wie in Anspruch 1 definiert sind und
**R²** H oder C₁₋₃-Alkyl bedeutet,
deren Enantiomere, deren Diastereomere, deren Gemische und deren Salze, insbesondere deren physiologisch verträgliche Salze mit organischen oder anorganischen Säuren oder Basen.

7. Verbindungen der allgemeinen Formel **I** gemäß Anspruch 1, in der **R¹**, **R²**, **R⁴**, **R⁵**, **R⁶**, **R⁷**, **R⁸** und n wie in Anspruch 1 definiert sind und
**R³** unabhängig voneinander (a) H, Halogen, C₁₋₃-Alkyl, -O-C₁₋₃-Alkyl oder (b) C₁₋₃-Alkyl, in der jede Methylengruppe mit bis zu zwei Fluoratomen und jede Methylgruppe mit bis zu drei Fluoratomen substituiert ist, bedeutet,
deren Enantiomere, deren Diastereomere, deren Gemische und deren Salze, insbesondere deren physiologisch verträgliche Salze mit organischen oder anorganischen Säuren oder Basen.

8. Verbindungen der allgemeinen Formel **I** gemäß Anspruch 1, in der **R¹**, **R²**, **R³**, **R⁵**, **R⁶**, **R⁷**, **R⁸** und n wie in Anspruch 1 definiert sind und
R**⁴** H oder Halogen bedeutet,
deren Enantiomere, deren Diastereomere, deren Gemische und deren Salze, insbesondere deren physiologisch verträgliche Salze mit organischen oder anorganischen Säuren oder Basen.

9. Verbindungen der allgemeinen Formel **I** gemäß Anspruch 1, in der **R¹**, **R²**, **R³**, **R⁴**, **R⁶**, **R⁷**, **R⁸** und **n** wie in Anspruch 1 definiert sind und
**R⁵** (a) H, Halogen, (b) C₁₋₃-Alkyl, (c) C₁₋₃-Alkyl, in der jede Methylengruppe mit bis zu zwei Fluoratomen und jede Methylgruppe mit bis zu drei Fluoratomen substituiert ist, (d) -O-C₁₋₃-Alkyl, (e) -C(O)-O-C₁₋₃-Alkyl oder -C(O)-NH₂ bedeutet,
deren Enantiomere, deren Diastereomere, deren Gemische und deren Salze, insbesondere deren physiologisch verträgliche Salze mit organischen oder anorganischen Säuren oder Basen.

10. Verbindungen der allgemeinen Formel I gemäß Anspruch 1, in der **R¹**, **R²**, **R³**, **R⁴**, **R⁵**, **R⁷**, **R⁸** und **n** wie in Anspruch 1 definiert sind und
**R⁶** (a) H, Halogen, (b) C₁₋₃-Alkylen-**R^{6.1},** (c) C₁₋₃-Alkyl, in der jede Methylengruppe mit bis zu zwei Fluoratomen und jede Methylgruppe mit bis zu drei Fluoratomen substituiert ist, (d) -OH, -O-C₁₋₄-Alkyl, (e) -OCHF₂, -O-CF₃, (f) -C(O)-O-**R^{6.2}**, -CN, -C(O)-CH₃, -C(O)-NH₂ oder (g) Pyrrolyl,
**R^{6.1}** H oder -OH, und
**R^{6.2}** H oder C₁₋₃-Alkyl, bedeuten,
deren Enantiomere, deren Diastereomere, deren Gemische und deren Salze, insbesondere deren physiologisch verträgliche Salze mit organischen oder anorganischen Säuren oder Basen.

11. Verbindungen der allgemeinen Formel **I** gemäß Anspruch 1, in der **R¹**, **R²**, **R³**, **R⁴**, **R⁵**, **R⁶**, **R⁸** und **n** wie in Anspruch 1 definiert sind und
**R⁷** (a) H, Halogen, (b) C₁₋₃-Alkyl, (c) C₁₋₃-Alkyl, in der jede Methylengruppe mit bis zu zwei Fluoratomen und jede Methylgruppe mit bis zu drei Fluoratomen substituiert ist, (d) -O-C₁₋₃-Alkyl, (e) -C(O)-NH₂ oder -C(O)-Pyrrolidinyl bedeutet,
deren Enantiomere, deren Diastereomere, deren Gemische und deren Salze, insbesondere deren physiologisch verträgliche Salze mit organischen oder anorganischen Säuren oder Basen.

12. Verbindungen der allgemeinen Formel **I** gemäß Anspruch 1, in der **R¹**, **R²**, **R³**, **R⁴**, **R⁵**, **R⁶**, **R⁷** und **n** wie in Anspruch 1 definiert sind und
**R⁸** (a) H, Halogen, (b) C₁₋₄-Alkyl, (c) C₁₋₃-Alkyl, in der jede Methylengruppe mit bis zu zwei Fluoratomen und jede Methylgruppe mit bis zu drei Fluoratomen substituiert ist, (d) -O-C₁₋₃-Alkyl, (e) -O-CF₃, (f) -CN, -C(O)-NH₂ oder (g) Pyrrolyl bedeutet,
deren Enantiomere, deren Diastereomere, deren Gemische und deren Salze, insbesondere deren physiologisch verträgliche Salze mit organischen oder anorganischen Säuren oder Basen.

13. Verbindungen der allgemeinen Formel I gemäß Anspruch 1, in der
**R¹** eine Gruppe ausgewählt aus 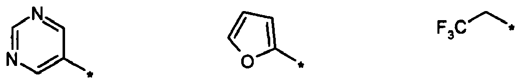
**R²** H oder -CH₃,
**R³** H, F, -CF₃, -CH₃ oder -O-CH₃,
**R⁴** H oder Cl,
**R⁵** H, Cl, C₁₋₃-Alkyl, -CF₃, -O-C₁₋₃-Alkyl, -C(O)-O-C₁₋₃-Alkyl oder -C(O)-NH₂,
**R⁶** H, F, Cl, C₁₋₃-Alkyl, -CF₃, -O-C₁₋₄-Alkyl, -OCF₃, -C(O)-NH₂ oder Pyrrolyl,
**R⁷** H, F, Cl, C₁₋₃-Alkyl, -CF₃, -O-C₁₋₃-Alkyl, -C(O)-NH₂ oder -C(O)-Pyrrolidinyl, und
**R⁸** H, F, Cl, Br, C₁₋₄-Alkyl, -CF₃, -O-C₁₋₃-Alkyl, -OCF₃, -C(O)-NH₂, -OCF₃ oder Pyrrolyl,
bedeuten,
deren Enantiomere, deren Diastereomere, deren Gemische und deren Salze, insbesondere deren physiologisch verträgliche Salze mit organischen oder anorganischen Säuren oder Basen.

14. Verbindungen der allgemeinen Formel **I** gemäß Anspruch 1, in der
**R¹** eine Gruppe ausgewählt aus 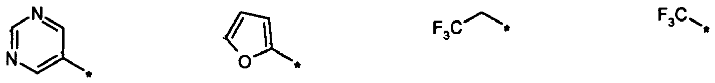 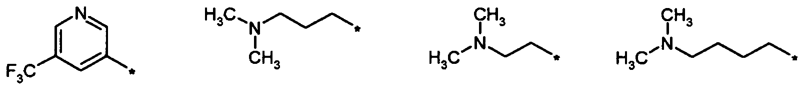 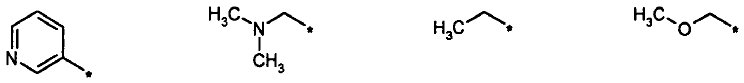 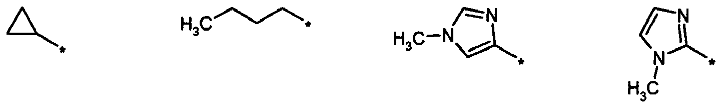 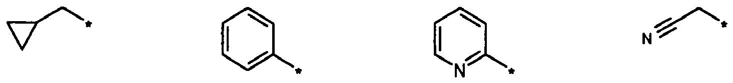 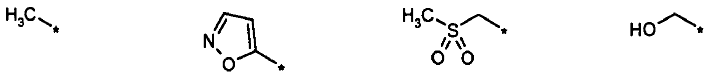 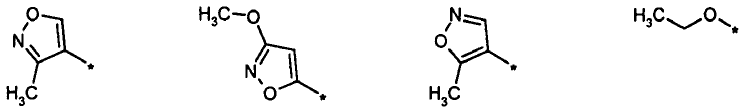 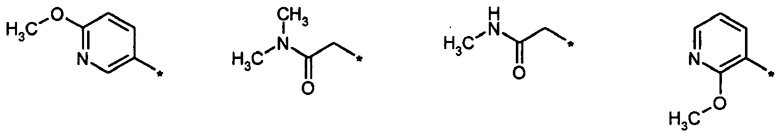 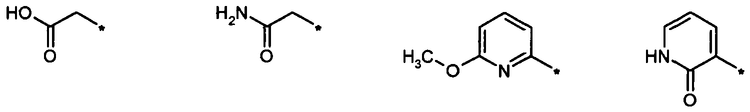 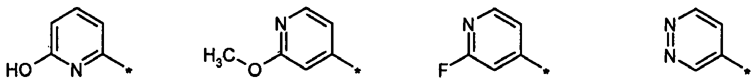 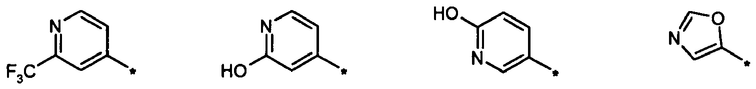 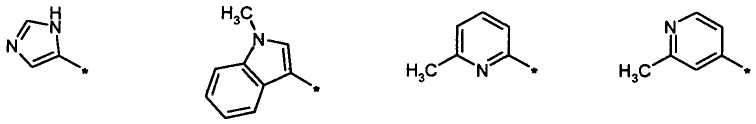 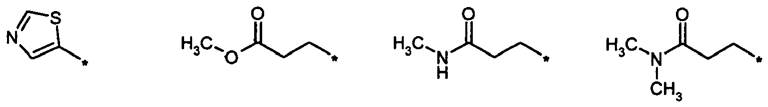 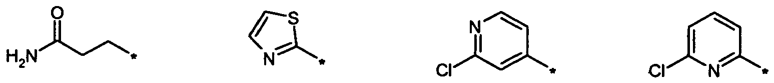 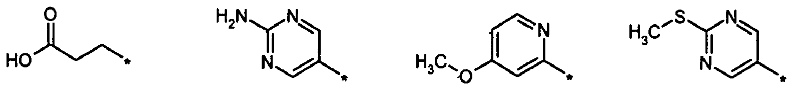 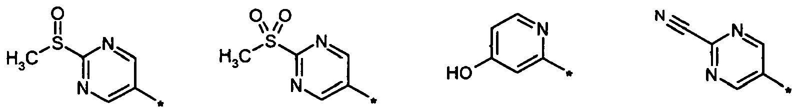
**R²** H, -CH₃ oder -C₂H₅,
**R³** H, F, Cl, -CF₃, -CH₃ oder -O-CH₃,
**R⁴** H oder Cl,
**R⁵** H, Cl, C₁₋₃-Alkyl, -CF₃, -O-C₁₋₃-Alkyl, -C(O)-O-C₁₋₃-Alkyl oder -C(O)-NH₂,
**R⁶** H, F, Cl, Br, -CN, C₁₋₃-Alkyl, -CF₃, -COOH, -COO-C₁₋₃-Alkyl, -CH(OH)CH₃, -OH, -O-C₁₋₄-Alkyl, -OCF₃, -OCHF₂, -C(O)-CH₃, -C(O)-NH₂ oder Pyrrolyl,
**R⁷** H, F, Cl, C₁₋₃-Alkyl, -CF₃, -O-C₁₋₃-Alkyl, -C(O)-NH₂ oder -C(O)-Pyrrolidinyl und
**R⁸** H, F, Cl, Br, -CN, C₁₋₄-Alkyl, -CF₃, -O-C₁₋₃-Alkyl, -OCF₃, -C(O)-NH₂, -OCF₃ oder Pyrrolyl,
bedeuten,
deren Enantiomere, deren Diastereomere, deren Gemische und deren Salze, insbesondere deren physiologisch verträgliche Salze mit organischen oder anorganischen Säuren oder Basen.

15. Folgende Verbindungen der allgemeinen Formel **I** gemäß Anspruch 1:
| **Nr.** | **Struktur** |
|---|---|
| (1) | 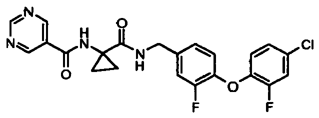 |
| (2) | 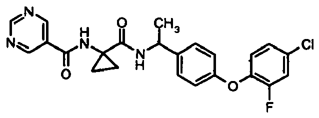 |
| (3) | 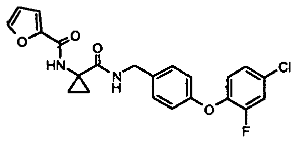 |
| (4) | 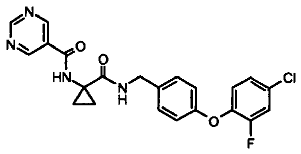 |
| (5) | 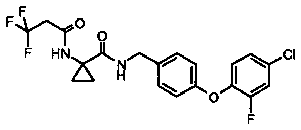 |
| (6) | 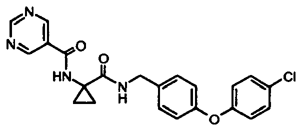 |
| (7) | 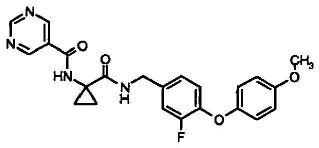 |
| (8) | 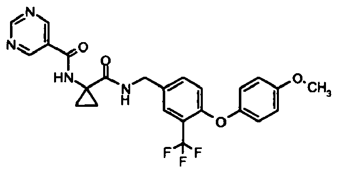 |
| (9) | 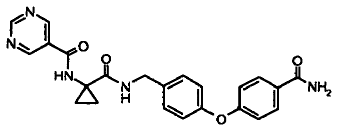 |
| (10) | 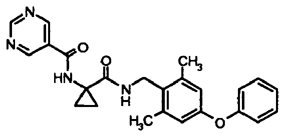 |
| (11) | 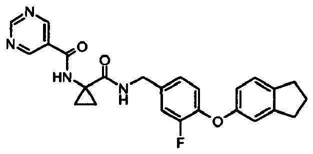 |
| (12) | 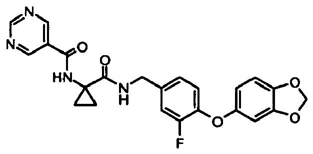 |
| (13) | 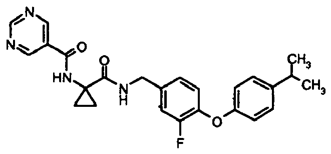 |
| (14) | 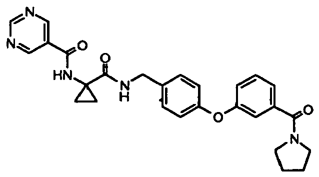 |
| (15) | 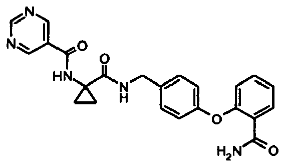 |
| (16) | 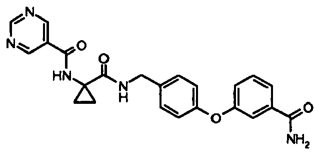 |
| (17) | 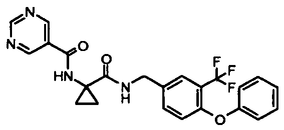 |
| (18) | 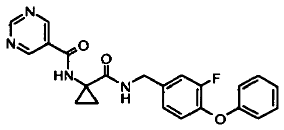 |
| (19) | 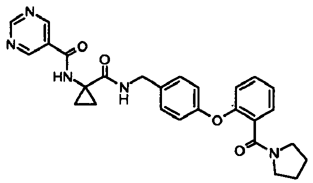 |
| (20) | 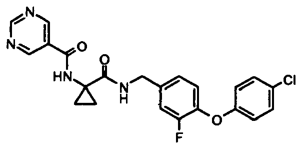 |
| (21) | 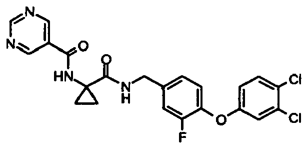 |
| (22) | 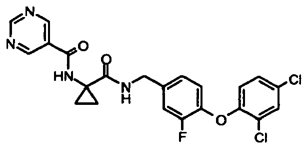 |
| (23) | 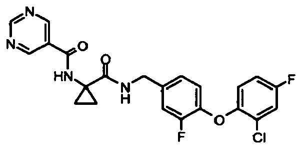 |
| (24) | 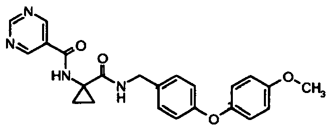 |
| (25) | 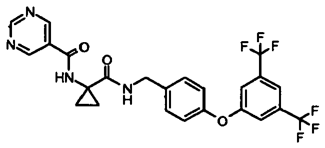 |
| (26) | 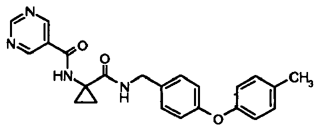 |
| (27) | 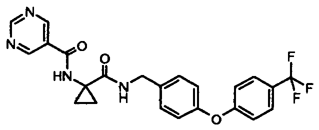 |
| (28) | 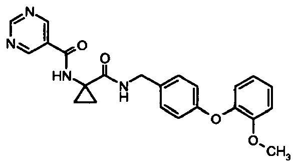 |
| (29) | 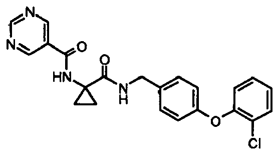 |
| (30) | 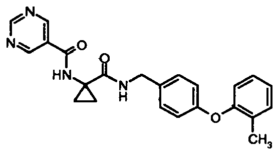 |
| (31) | 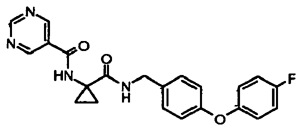 |
| (32) | 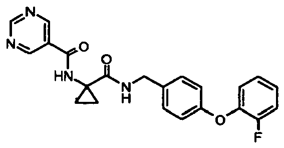 |
| (33) | 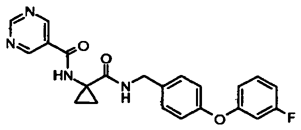 |
| (34) | 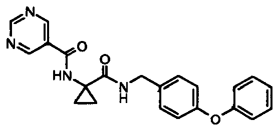 |
| (35) | 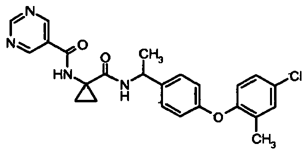 |
| (36) | 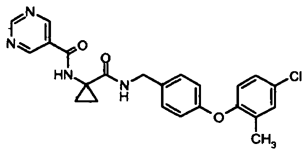 |
| (37) | 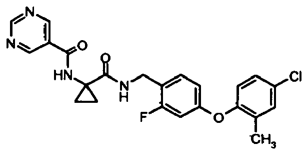 |
| (38) | 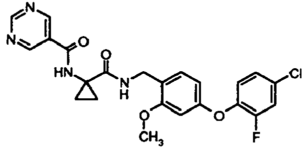 |
| (39) | 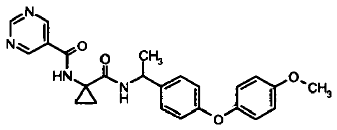 |
| (40) | 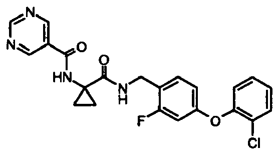 |
| (41) | 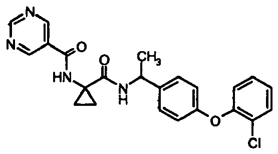 |
| (42) | 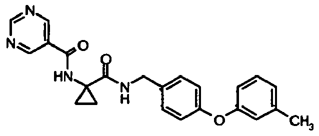 |
| (43) | 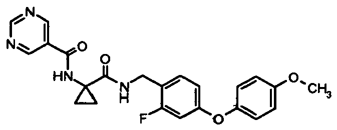 |
| (44) | 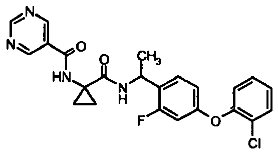 |
| (45) | 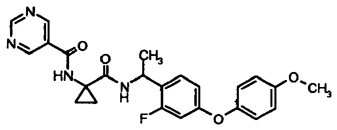 |
| (46) | 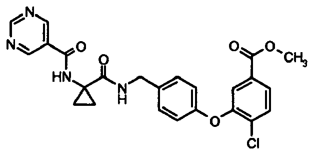 |
| (47) | 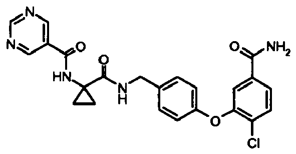 |
| (48) | 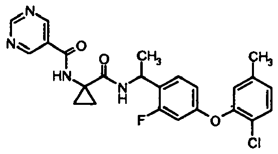 |
| (49) | 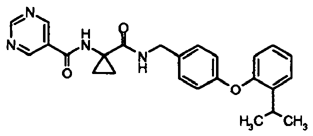 |
| (50) | 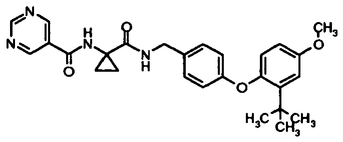 |
| (51) | 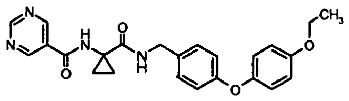 |
| (52) | 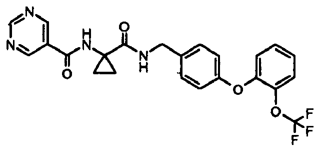 |
| (53) | 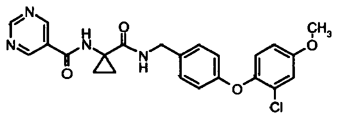 |
| (54) | 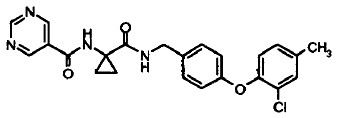 |
| (55) | 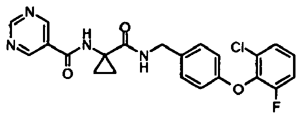 |
| (56) | 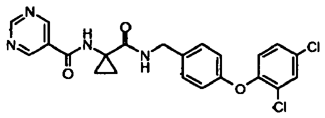 |
| (57) | 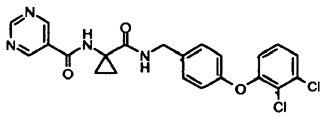 |
| (58) | 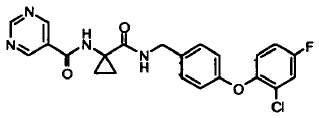 |
| (59) | 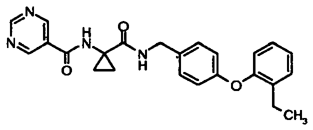 |
| (60) | 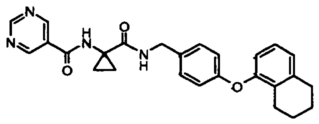 |
| (61) | 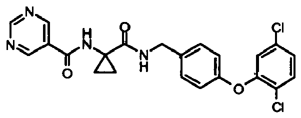 |
| (62) | 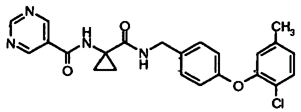 |
| (63) | 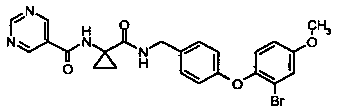 |
| (64) | 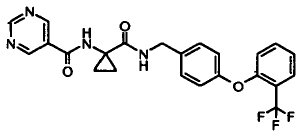 |
| (65) | 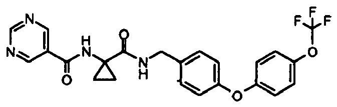 |
| (66) | 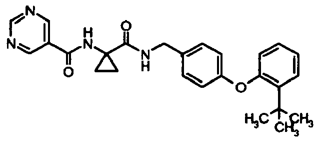 |
| (67) | 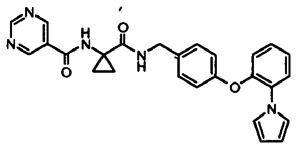 |
| (68) | 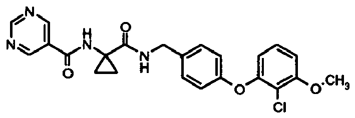 |
| (69) | 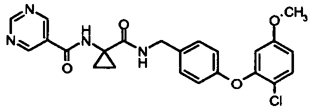 |
| (70) | 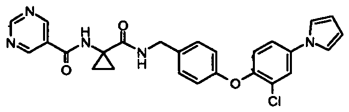 |
| (71) | 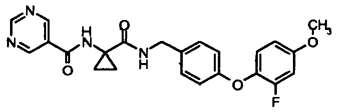 |
| (72) | 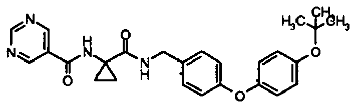 |
| (73) | 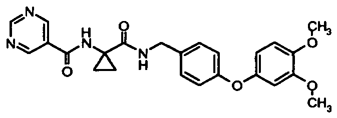 |
| (74) | 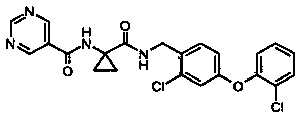 |
| (75) | 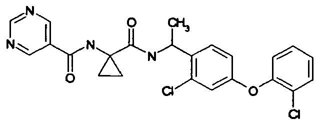 |
| (76) | 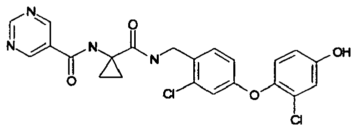 |
| (77) | 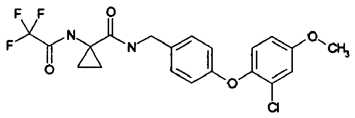 |
| (78) | 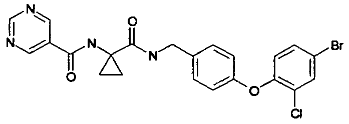 |
| (79) | 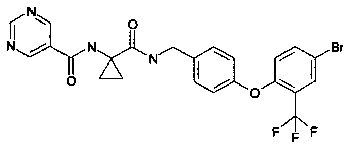 |
| (80) | 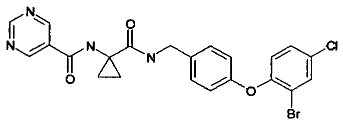 |
| (81) | 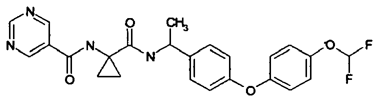 |
| (82) | 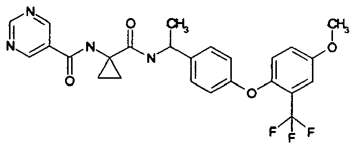 |
| (83) | 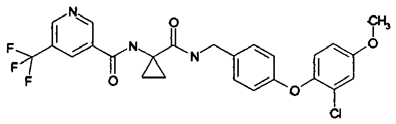 |
| (84) | 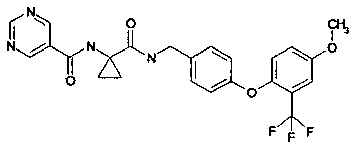 |
| (85) | 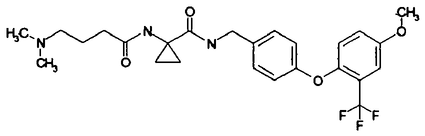 |
| (86) | 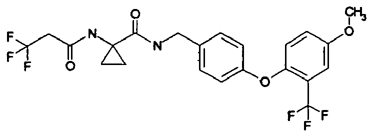 |
| (87) | 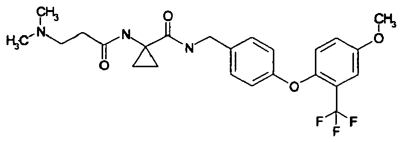 |
| (88) | 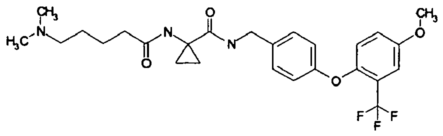 |
| (89) | 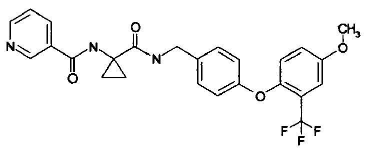 |
| (90) | 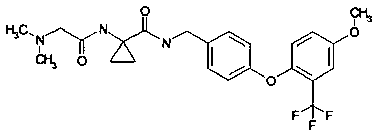 |
| (91) | 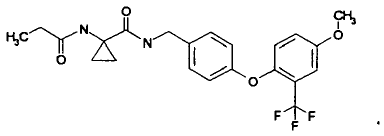 |
| (92) | 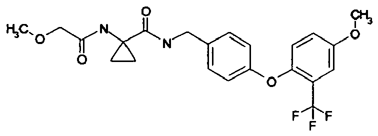 |
| (93) | 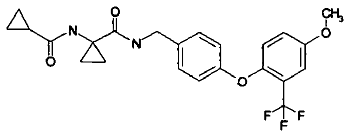 |
| (94) | 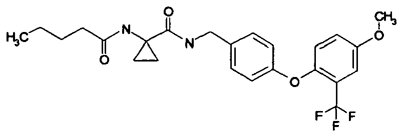 |
| (95) | 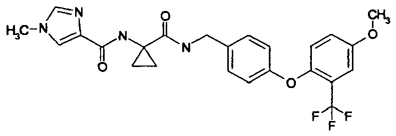 |
| (96) | 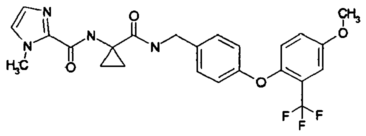 |
| (97) | 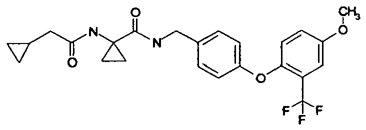 |
| (98) | 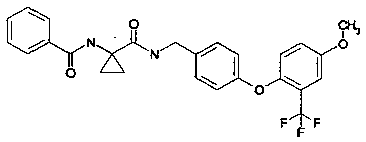 |
| (99) | 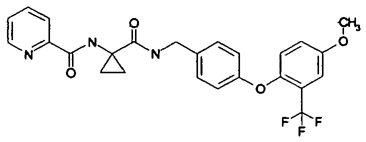 |
| (100) | 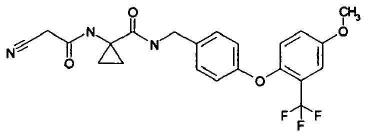 |
| (101) | 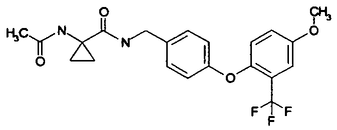 |
| (102) | 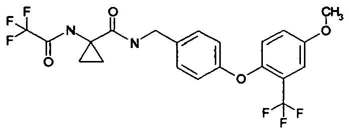 |
| (103) | 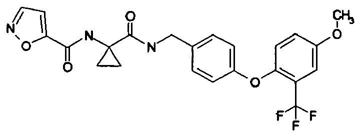 |
| (104) | 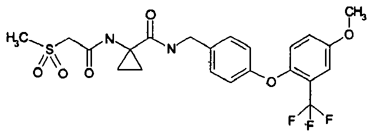 |
| (105) | 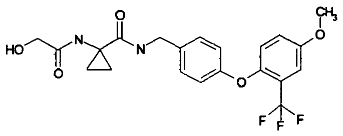 |
| (106) | 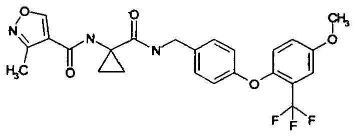 |
| (107) | 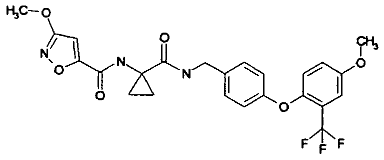 |
| (108) | 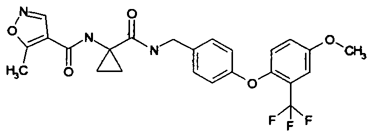 |
| (109) | 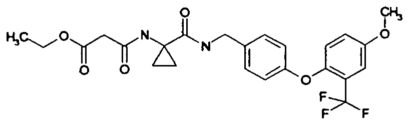 |
| (110) | 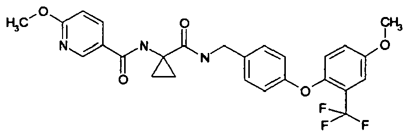 |
| (111) | 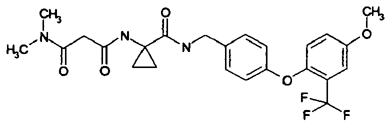 |
| (112) | 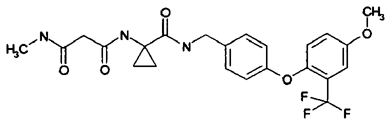 |
| (113) | 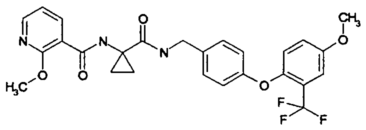 |
| (114) | 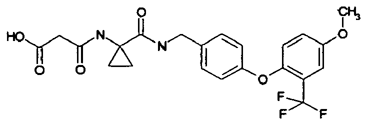 |
| (115) | 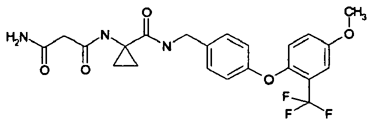 |
| (116) | 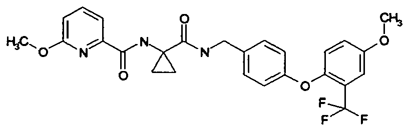 |
| (117) | 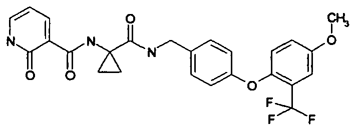 |
| (118) | 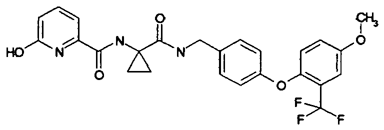 |
| (119) | 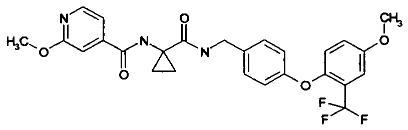 |
| (120) | 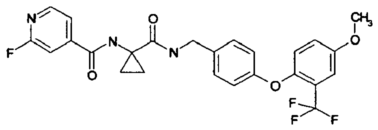 |
| (121) | 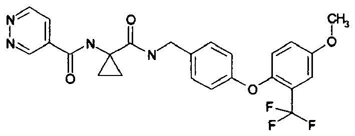 |
| (122) | 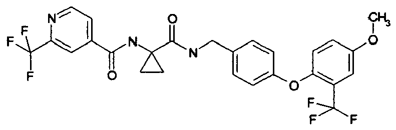 |
| (123) | 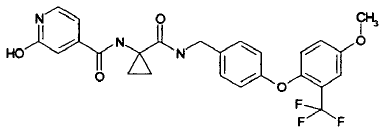 |
| (124) | 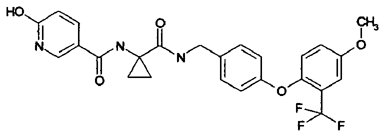 |
| (125) | 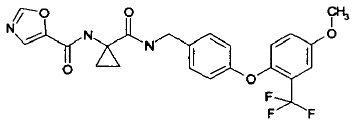 |
| (126) | 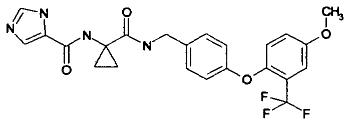 |
| (127) | 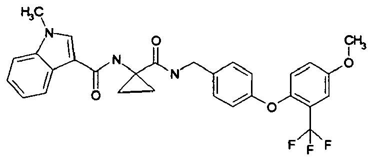 |
| (128) | 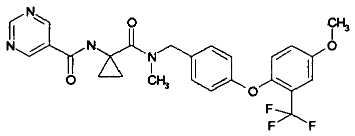 |
| (129) | 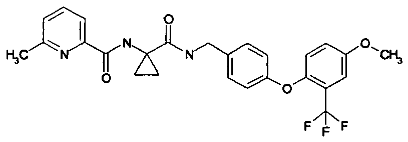 |
| (130) | 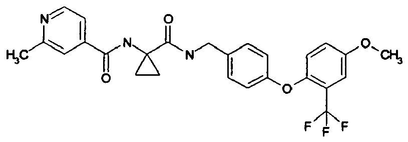 |
| (131) | 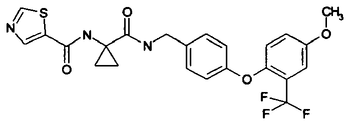 |
| (132) | 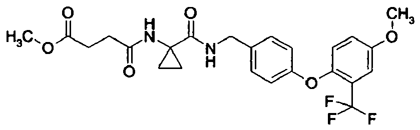 |
| (133) | 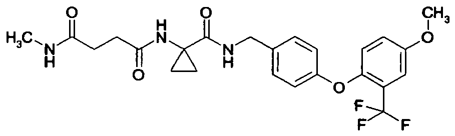 |
| (134) | 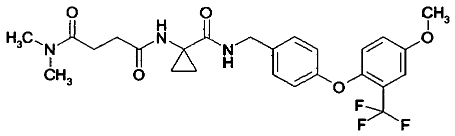 |
| (135) | 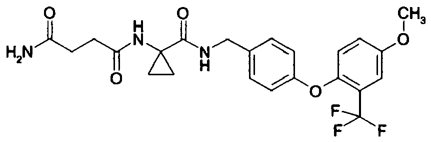 |
| (136) | 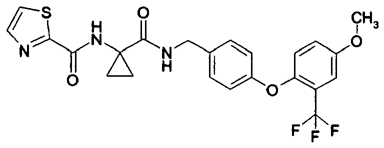 |
| (137) | 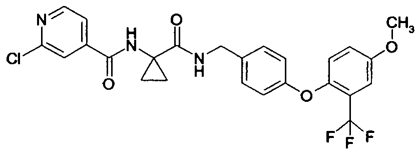 |
| (138) | 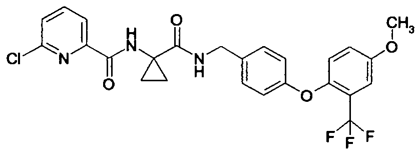 |
| (139) | 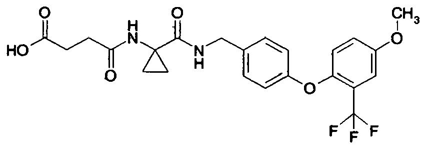 |
| (140) | 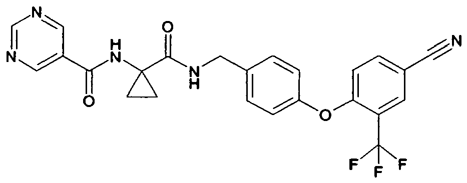 |
| (141) | 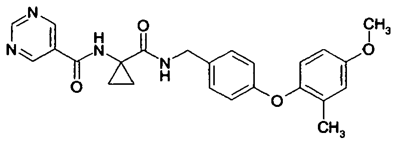 |
| (142) | 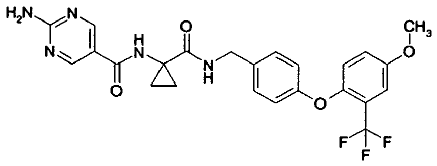 |
| (143) | 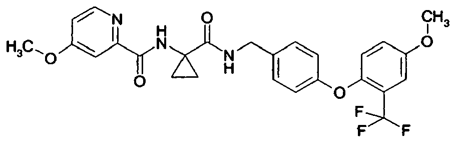 |
| (144) | 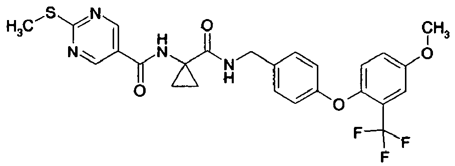 |
| (145) | 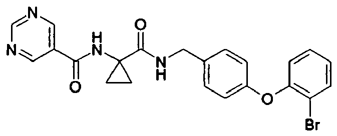 |
| (146) | 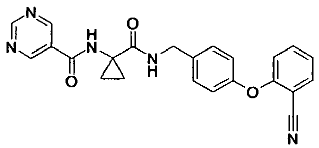 |
| (147) | 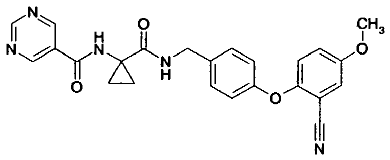 |
| (148) | 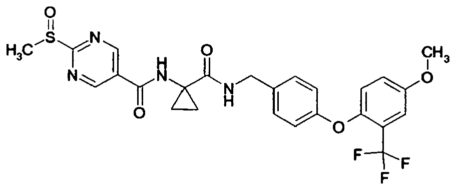 |
| (149) | 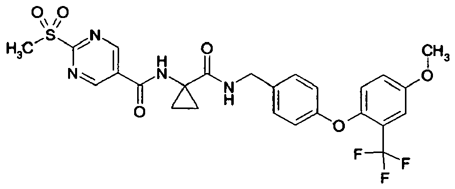 |
| (150) | 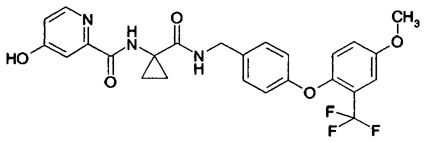 |
| (151) | 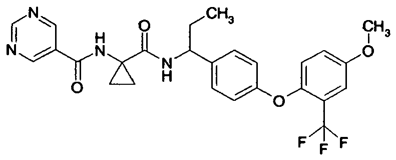 |
| (152) | 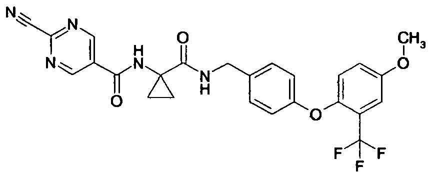 |
| (153) | 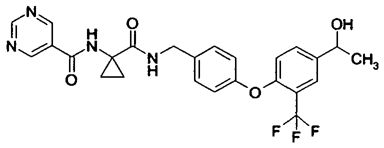 |
| (154) | 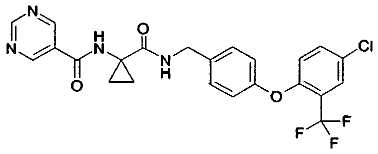 |
| (155) | 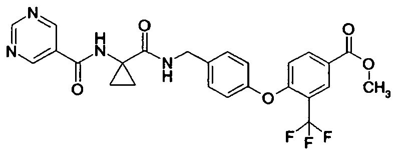 |
| (156) | 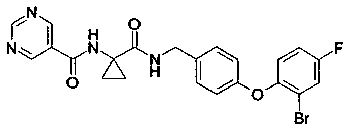 |
| (157) | 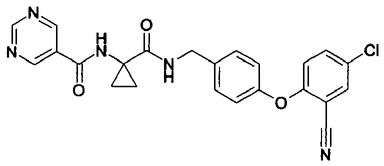 |
| (158) | 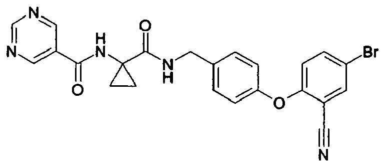 |
| (159) | 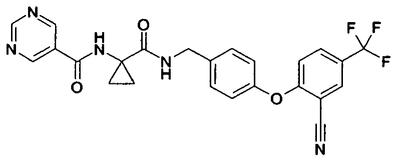 |
| (160) | 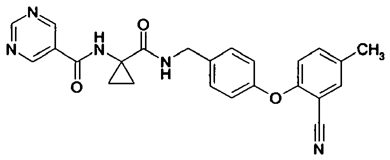 |
| (161) | 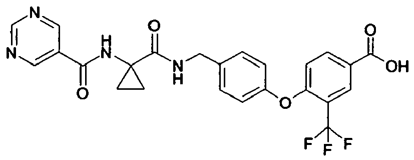 |
| (162) | 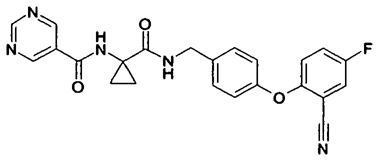 |
| (163) | 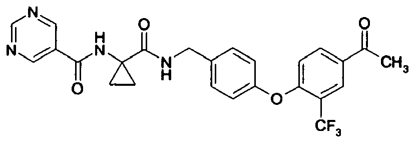 |
deren Enantiomere, deren Diastereomere, deren Gemische und deren Salze, insbesondere deren physiologisch verträgliche Salze mit organischen oder anorganischen Säuren oder Basen.

16. Physiologisch verträgliche Salze der Verbindungen nach einem der Ansprüche 1 bis 15 mit anorganischen oder organischen Säuren oder Basen.

17. Arzneimittel, enthaltend eine Verbindung nach mindestens einem der Ansprüche 1 bis 15 oder ein physiologisch verträgliches Salz gemäß Anspruch 16 neben gegebenenfalls einem oder mehreren inerten Trägerstoffen und/oder Verdünnungsmitteln.

18. Verwendung einer Verbindung nach mindestens einem der Ansprüche 1 bis 16 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

19. Verfahren zur Herstellung eines Arzneimittels gemäß Anspruch 17, **dadurch gekennzeichnet, dass** auf nichtchemischem Weg eine Verbindung nach mindestens einem der Ansprüche 1 bis 16 in einen oder mehrere inerte Trägerstoffe und/oder Verdünnungsmittel eingearbeitet wird.

## Claims

1. Compounds of general formula I 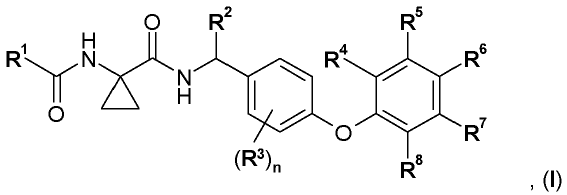 wherein
**R**¹ denotes (a) a C₁-₆-alkyl group optionally substituted by a group **R**^{1.1}, (b) a C₁₋₃-alkyl group wherein each methylene group is substituted by up to two fluorine atoms and each methyl group is substituted by up to three fluorine atoms, (c) a C₃₋₆-cycloalkyl group optionally substituted by a group **R**^{1.2}, (d) a C₂₋₆-alkenyl group, (e) a C₂₋₆-alkynyl group, (f) an aryl-C₀₋₂-alkylene group optionally substituted by 1, 2 or 3 groups **R^{1.3}**, (g) a five-membered heteroaryl group optionally substituted by 1, 2 or 3 groups **R^{1.4}**, which contains at least one N, O or S atom and optionally also contains one, two or three further N atoms and may additionally be benzo-fused, (h) a six-membered heteroaryl group optionally substituted by a group **R^{1.4}**, which contains one, two or three N atoms and may additionally be benzo-fused, (i) -O-**R^{1.1.1}** or (j) -N**R^{1.1.2}R^{1.1.4}**,
**R**^{1.1} denotes halogen, -NO₂, -CN, C₃₋₆-cycloalkyl, -O**R^{1.1.1}**, -**SR**^{**1**.}**^{1.1},** -C(O)**R^{1.1.1}**, -S(O)₂-**R^{1.1.2}**, -O-S(O)₂-**R^{1.1.1}**, -CO₂**R^{1.1.1}**, -O-C(O)-**R^{1.1.1}**, -N**R^{1.1.3}**R**^{1.1.4}**, -N**R**^{**1.1,3**-} C(O)-**R^{1.1.1}**, -NR^{**1.1,3**-}C(O)-**R^{1.1.1}**, -N**R^{1.1,3-}**CO₂-**R^{1.1.1}** or -C(O)-N**R^{1.1.3}R^{1.1.4},**
**R**^{1.1.1} denotes (a) H, (b) C₁₋₄-alkyl, (c) C₁₋₃-alkyl, wherein each methylene group is substituted by up to two fluorine atoms and each methyl group is substituted by up to three fluorine atoms, (d) a phenyl group optionally substituted by 1, 2 or 3 groups **R**^{1.1.1.1}, (e) C₃₋₆-cycloalkyl, or (f) a pyridyl group optionally substituted by 1, 2 or 3 groups **R**^{1.1.1.2},
**R**^{1.1.1.1} independently of one another denote (b) halogen, -NO₂, -CN, -OH, -O-C₁₋₄-alkyl, C₃₋₆-cycloalkyl, C₁₋₄-alkyl, or (c) C₁₋₃-alkyl, wherein each methylene group is substituted by up to two fluorine atoms and each methyl group is substituted by up to three fluorine atoms,
**R**^{1.1.1.2} independently of one another denote halogen or C₁₋₄-alkyl,
**R**^{1.1.2} denotes (a) C₁₋₄-alkyl, (b) C₁₋₃-alkyl, wherein each methylene group is substituted by up to two fluorine atoms and each methyl group is substituted by up to three fluorine atoms, (c) -O-C₁₋₄-alkyl, (d) a phenyl group optionally substituted by 1, 2 or 3 groups **R^{1.1.1.1}**,
**R^{1.1.3}**, **R^{1.1.4}** independently of one another denote (a) H, (b) a C₁₋₄-alkyl group optionally substituted by 1, 2 or 3 groups **R^{1.1.4.1}**, (c) a phenyl group optionally substituted by 1, 2 or 3 groups **R^{1.1.1.1}**, (d) C₃₋₆-cycloalkyl, or
**R^{1.1.3}** and **R^{1.1.4}** together with the N atom to which they are bound form a 4-, 5- or 6-membered heterocyclic ring, which may additionally contain a further heteroatom selected from N, O and S, or
**R^{1.1.3}** and **R^{1.1.4}** together with the N atom to which they are bound form a cyclic imide,
**R^{1.1.4.1}** independently of one another denote halogen, -NH₂, -NH(C₁₋₄-alkyl), -N(C₁₋₄-alkyl)₂ or -SO₂-**R^{1.1.2}**,
**R^{1.2}** denotes halogen, -NO₂, -CN or phenyl,
**R^{1.3}** denotes (a) halogen, -NO₂, -CN, -O**R^{1.1.1}**, -S**R^{1.1.1}**, -CO₂**R^{1.1.1}**, C₁₋₆-alkyl, or (b) C₁₋₃-alkyl, wherein each methylene group is substituted by up to two fluorine atoms and each methyl group is substituted by up to three fluorine atoms,
**R^{1.4}** independently of one another denote (a) halogen, -NO₂, -CN, -O**R^{1.1.1}**, -S**R^{1.1.1}**, -S(O)-**R^{1.1.2}**, -S(O)₂-**R^{1.1.2}**, -N**R^{1.1.3}R^{1.1.4}**, C₁₋₆-alkyl, (b) C₁₋₃-alkyl, wherein each methylene group is substituted by up to two fluorine atoms and each methyl group is substituted by up to three fluorine atoms, or (c) an oxo group,
**R**² denotes (a) H, C₁₋₄-alkyl or (b) C₁₋₃-alkyl, wherein each methylene group is substituted by up to two fluorine atoms and each methyl group is substituted by up to three fluorine atoms,
**R³** independently of one another denote (a) H, halogen, -CN, -OH, C₁₋₆-alkyl, C₃₋₇-cycloalkyl, -O-C₁₋₄-alkyl, -O-CF₃, -O-C₃₋₆-cycloalkyl, -N(C₁₋₃-alkyl)₂, -C(O)-NH₂, -(SO₂)NH₂, -SO₂-C₁₋₃-alkyl, or (b) C₁₋₃-alkyl, wherein each methylene group is substituted by up to two fluorine atoms and each methyl group is substituted by up to three fluorine atoms,
**R**⁴, **R⁵**, **R⁶**, **R⁷**, **R⁸** independently of one another denote (a) H, halogen, -CN, -OH, (b) C₁₋₆-alkyl, wherein two adjacent substituents together may denote a trimethylene or tetramethylene group, (c) C₁₋₃-alkyl, wherein each methylene group is substituted by up to two fluorine atoms and each methyl group is substituted by up to three fluorine atoms, (d) C₃₋₇-cycloalkyl, (e) -O-C₁₋₆-alkyl, wherein two adjacent substituents may denote a methylenedioxy or ethylenedioxy group, (f) -O-CF₃, -O-C₃-₇-cycloalkyl, (g) -NH₂, -NH(C₁₋₃-alkyl), -N(C₁₋₃-alkyl)₂, (h) -C(O)-**R^{8.1}**, (i) -SO₂-**R^{8.2}**, (j) a five-membered heteroaryl group optionally substituted by one or two C₁₋₃-alkyl groups which is selected from among pyrrolyl, oxazolyl, isoxazolyl, oxadiazolyl, thiazolyl, isothiazolyl, thiadiazolyl, imidazolyl, pyrazolyl, triazolyl and tetrazolyl, or (k) a six-membered heteroaryl group optionally substituted by one or two C₁₋₃-alkyl groups which is selected from among pyridyl, pyrimidinyl, pyrazinyl, pyridazinyl and triazinyl,
**R^{8.1}** denotes -NH₂, -NH(C₁₋₆-alkyl),-N(C₁₋₆-alkyl)₂, *N*-acetidinyl, *N*-pyrrolidinyl, *N*-piperidinyl, *N*-morpholinyl, -OH, -O-C₁₋₈-alkyl, -O-C₃₋₇-cycloalkyl,
**R^{8.2}** denotes -NH₂, -NH(C₁₋₆-alkyl),-N(C₁₋₆-alkyl)₂, *N*-acetidinyl, *N*-pyrrolidinyl, *N*-piperidinyl, *N*-morpholinyl and
**n** denotes one of the numbers 0, 1, 2, 3 or 4,
the enantiomers, the diastereomers, the mixtures thereof and the salts thereof, particularly the physiologically acceptable salts thereof with organic or inorganic acids or bases.

2. Compounds of general formula I according to claim 1, wherein **R²**, **R³**, **R⁴**, **R⁵**, **R⁶**, **R⁷**, **R⁸** and **n** are defined as in claim 1 and
**R¹** denotes (a) a C₁₋₆-alkyl group optionally substituted by a group **R^{1.1}**, (b) a C₁₋₃-alkyl group wherein each methylene group is substituted by up to two fluorine atoms and each methyl group is substituted by up to three fluorine atoms, (c) a C₃₋₆-cycloalkyl group optionally substituted by a group **R^{1.2}**, (d) a phenyl group optionally substituted by 1, 2 or 3 groups **R^{1.3}**, (e) a five-membered heteroaryl group optionally substituted by a group **R^{1.4}**, which contains at least one N, O or S atom and which optionally additionally contains one, two or three further N atoms, or (f) a six-membered heteroaryl group optionally substituted by a group **R^{1.4}**, which contains one, two or three N atoms,
**R^{1.1}** denotes C₃₋₆-cycloalkyl, -O**R**^{1.1.1}, -N**R^{1.1.3}R^{1.1.4}**, -C(O)-N**R^{1.1.3}R^{1.1.4}**, -CN, -CO₂**R^{1.1.1}**, -S(O)₂-C₁₋₆-alkyl or -O-S(O)₂-C₁₋₆-alkyl,
**R^{1.1.1}** denotes (a) H, (b) C₁₋₄-alkyl, (c) C₁₋₃-alkyl, wherein each methylene group is substituted by up to two fluorine atoms and each methyl group is substituted by up to three fluorine atoms, or (d) C₃₋₆-cycloalkyl,
**R^{1.1.3}**, **R^{1.1.4}** independently of one another denote (a) H,
**R^{1.2}** (b) C₁₋₄-alkyl or (c) C₃₋₆-cycloalkyl, denotes halogen, -NO₂, -CN or phenyl,
**R^{1.3}** denotes (a) halogen, -NO₂, -CN, -O**R^{1.1.1}**, -S**R^{1.1.1}**, -CO₂**R^{1.1.1}**, C₁₋₆-alkyl or (b) C₁₋₃-alkyl, wherein each methylene group is substituted by up to two fluorine atoms and each methyl group is substituted by up to three fluorine atoms, and
**R^{1.4}** denotes (a) halogen, -NO₂, -CN, -O**R^{1.1.1}**, -S**R^{1.1.1}**, -N**R^{1.1.3}R^{1.1.4}**, C₁₋₆-alkyl, (b) C₁₋₃-alkyl, wherein each methylene group is substituted by up to two fluorine atoms and each methyl group is substituted by up to three fluorine atoms, or (c) an oxo group,
the enantiomers, the diastereomers, the mixtures thereof and the salts thereof, particularly the physiologically acceptable salts thereof with organic or inorganic acids or bases.

3. Compounds of general formula I according to claim 1, wherein
**R¹** denotes (a) a C₁₋₆-alkyl group optionally substituted by a group **R^{1.1}**, (b) a C₁₋₃-alkyl group wherein each methylene group is substituted by up to two fluorine atoms and each methyl group is substituted by up to three fluorine atoms, (c) a C₃₋₆-cycloalkyl group optionally substituted by a group **R^{1.2}**, (d) a phenyl group optionally substituted by 1, 2 or 3 groups **R^{1.3}**, (e) a five-membered heteroaryl group optionally substituted by a group **R^{1.4}**, which contains at least one N, O or S atom and which optionally additionally contains one, two or three further N atoms, or
**R^{1.1}** (f) a six-membered heteroaryl group optionally substituted by a group **R^{1.4}**, which contains one, two or three N atoms, denotes C₃₋₆-cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂-C₁₋₆-alkyl or -O-S(O)₂-C₁₋₆-alkyl,
**R^{1.1.1}** denotes (a) H, (b) C₁₋₄-alkyl, (c) C₁₋₃-alkyl, wherein each methylene group is substituted by up to two fluorine atoms and each methyl group is substituted by up to three fluorine atoms, or (d) C₃₋₆-cycloalkyl,
**R^{1.1.3}**, **R^{1.1.4}** independently of one another denote (a) H, (b) C₁₋₄-alkyl or (c) C₃₋₆-cycloalkyl,
**R^{1.2}** denotes halogen, -NO₂, -CN or phenyl,
**R^{1.3}** denotes (a) halogen, -NO₂, -CN, -O**R^{1.1.1}**, -S**R^{1.1.1}**, -CO₂**R^{1.1.1}**, C₁₋₆-alkyl or (b) C₁₋₃-alkyl, wherein each methylene group is substituted by up to two fluorine atoms and each methyl group is substituted by up to three fluorine atoms,
**R^{1.4}** denotes (a) halogen, -NO₂, -CN, -O**R^{1.1.1}**, -S**R^{1.1.1}**, -N**R^{1.1.3}R^{1.1.4}**, C₁₋₆-alkyl, or (b) C₁₋₃-alkyl, wherein each methylene group is substituted by up to two fluorine atoms and each methyl group is substituted by up to three fluorine atoms, or (c) an oxo group
**R²** denotes H or C₁₋₃-alkyl,
**R³** independently of one another denote (a) H, halogen, C₁₋₃-alkyl, -O-C₁₋₃-alkyl or (b) C₁₋₃-alkyl, wherein each methylene group is substituted by up to two fluorine atoms and each methyl group is substituted by up to three fluorine atoms,
**R⁴** denotes H or halogen,
**R⁵** denotes (a) H, halogen, (b) C₁₋₃-alkyl, (c) C₁₋₃-alkyl, wherein each methylene group is substituted by up to two fluorine atoms and each methyl group is substituted by up to three fluorine atoms, (d) -O-C₁₋₃-alkyl, (e) -C(O)-O-C₁₋₃-alkyl or -C(O)-NH₂,
**R⁶** denotes (a) H, halogen, (b) C₁₋₃-alkylene-**R^{6.1}**, (c) C₁₋₃-alkyl, wherein each methylene group is substituted by up to two fluorine atoms and each methyl group is substituted by up to three fluorine atoms, (d) -OH, -O-C₁₋₄-alkyl, (e) -O-CHF₂, -O-CF₃, (f) -C(O)-O-**R^{6.2}**, -CN, -C(O)-CH₃, -C(O)-NH₂ or (g) pyrrolyl,
**R^{6.1}** denotes H, -OH,
**R^{6.2}** denotes H, C₁₋₃-alkyl,
**R⁷** denotes (a) H, halogen, (b) C₁₋₃-alkyl, (c) C₁₋₃-alkyl, wherein each methylene group is substituted by up to two fluorine atoms and each methyl group is substituted by up to three fluorine atoms, (d) -O-C₁₋₃-alkyl, (e) -C(O)-NH₂ or -C(O)-pyrrolidinyl, and
**R⁸** denotes (a) H, halogen, (b) C₁₋₄-alkyl, (c) C₁₋₃-alkyl, wherein each methylene group is substituted by up to two fluorine atoms and each methyl group is substituted by up to three fluorine atoms, (d) -O-C₁₋₃-alkyl, (e) -O-CF₃, (f) -CN, -C(O)-NH₂ or (g) pyrrolyl,
the enantiomers, the diastereomers, the mixtures thereof and the salts thereof, particularly the physiologically acceptable salts thereof with organic or inorganic acids or bases.

4. Compounds of general formula **I** according to claim 1, wherein **R²**, **R³**, **R⁴**, **R⁵**, **R⁶**, **R⁷**, **R⁸** and **n** are defined as in claim 1 and
**R¹** denotes (a) a C₁₋₆-alkyl group optionally substituted by a group **R^{1.1}**, (b) a C₁₋₃-alkyl group wherein each methylene group is substituted by up to two fluorine atoms and each methyl group is substituted by up to three fluorine atoms, (c) a phenyl group optionally substituted by 1, 2 or 3 groups **R^{1.3}**, (d) a five-membered heteroaryl group optionally substituted by a group **R^{1.4}**, which is selected from among furanyl, isoxazolyl, isothiazolyl, oxazolyl, thiazolyl, pyrazolyl, thiadiazolyl, oxadiazolyl, triazolyl, tetrazolyl,
**R^{1.1}** imidazolyl, indolyl, thienyl, pyrrolyl, benzimidazolyl, benzoxazolyl, benzothiazolyl and benzisoxazinyl, or (e) a six-membered heteroaryl group optionally substituted by a group **R^{1.4}**, which is selected from among pyridyl, pyrimidinyl, pyridazinyl, pyrazinyl, triazinyl, quinazolinyl and quinoxazinyl, denotes C₃₋₆-cycloalkyl, -O**R^{1.1.1}**, -N**R^{1.1.3}R^{1.1.4}**, -C(O)-N**R^{1.1.3}R^{1.1.4}**, -CN, -CO₂**R^{1.1.1}**, -S(O)₂-C_{1.6}-alkyl or -O-S(O)₂-C₁₋₆-alkyl,
**R^{1.1.1}** denotes (a) H, (b) C₁₋₄-alkyl, (c) C₁₋₃-alkyl, wherein each methylene group is substituted by up to two fluorine atoms and each methyl group is substituted by up to three fluorine atoms, or (d) C₃₋₆-cycloalkyl,
**R^{1.1.3}**, **R^{1.1.4}** independently of one another denote (a) H, (b) C₁₋₄-alkyl or (c) C₃₋₆-cycloalkyl,
**R^{1.3}** denotes (a) halogen, -NO₂, -CN, -OR^{1.1.1}, -SR^{1.1.1}, -CO₂R^{1.1.1}, C₁₋₆-alkyl or (b) C₁₋₃-alkyl, wherein each methylene group is substituted by up to two fluorine atoms and each methyl group is substituted by up to three fluorine atoms, and
**R^{1.4}** denotes (a) halogen, -NO₂, -CN, -O**R^{1.1.1}**, -S**R^{1.1.1}**, C₁₋₆-alkyl, (b) C₁₋₃-alkyl, wherein each methylene group is substituted by up to two fluorine atoms and each methyl group is substituted by up to three fluorine atoms, or (c) an oxo group,
the enantiomers, the diastereomers, the mixtures thereof and the salts thereof, particularly the physiologically acceptable salts thereof with organic or inorganic acids or bases.

5. Compounds of general formula **I** according to claim 1, wherein **R²**, **R³**, **R⁴**, **R⁵**, **R⁶, R⁷, R⁸** and **n** are defined as in claim 1 and
**R¹** denotes a group selected from 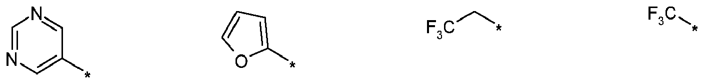 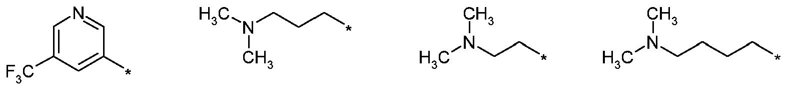 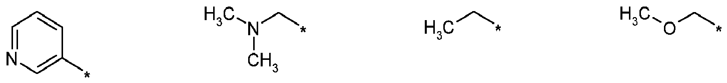 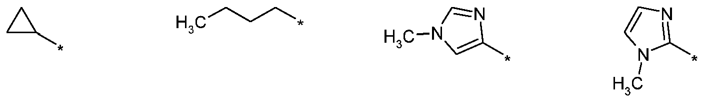 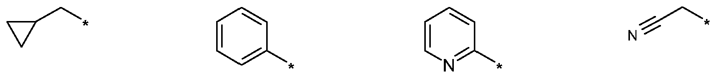 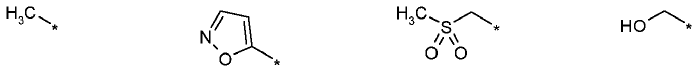 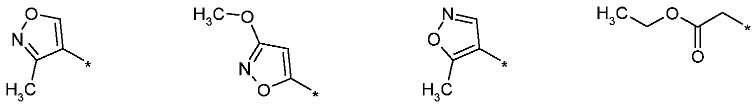 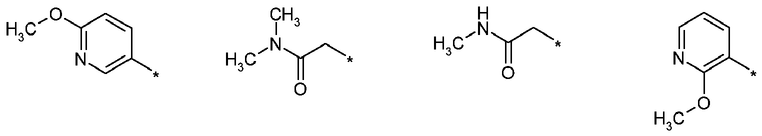 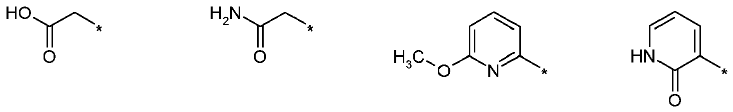 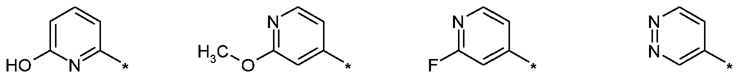 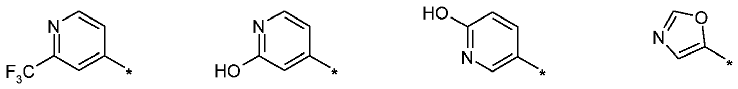 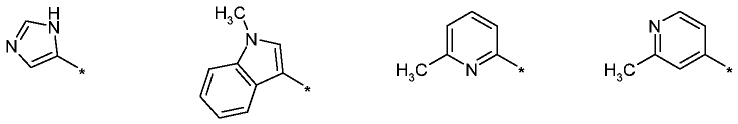 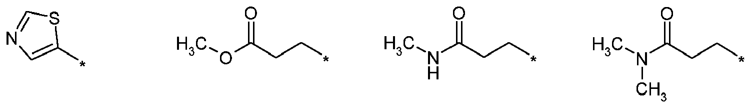 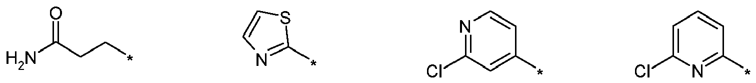 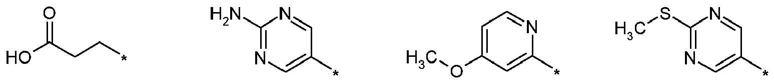 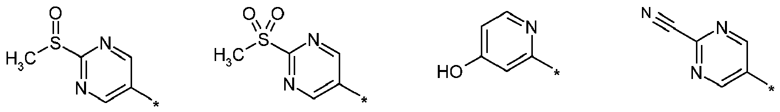
the enantiomers, the diastereomers, the mixtures thereof and the salts thereof, particularly the physiologically acceptable salts thereof with organic or inorganic acids or bases.

6. Compounds of general formula **I** according to claim 1, wherein **R¹**, **R³**, **R⁴**, **R⁵**, **R⁶**, **R⁷**, **R⁸ and n** are defined as in claim 1 and
**R²** denotes H or C₁₋₃-alkyl,
the enantiomers, the diastereomers, the mixtures thereof and the salts thereof, particularly the physiologically acceptable salts thereof with organic or inorganic acids or bases.

7. Compounds of general formula **I** according to claim 1, wherein **R¹**, **R²**, **R⁴**, **R⁵**, **R⁶**, **R⁷**, **R⁸** and **n** are defined as in claim 1 and
**R³** independently of one another denote (a) H, halogen, C₁₋₃-alkyl, -O-C₁₋₃-alkyl or (b) C₁₋₃-alkyl, wherein each methylene group is substituted by up to two fluorine atoms and each methyl group is substituted by up to three fluorine atoms,
the enantiomers, the diastereomers, the mixtures thereof and the salts thereof, particularly the physiologically acceptable salts thereof with organic or inorganic acids or bases.

8. Compounds of general formula **I** according to claim 1, wherein **R¹**, **R²**, **R³**, **R⁵**, **R⁶**, **R⁷**, **R⁸** and **n** are defined as in claim 1 and
**R⁴** denotes H or halogen,
the enantiomers, the diastereomers, the mixtures thereof and the salts thereof, particularly the physiologically acceptable salts thereof with organic or inorganic acids or bases.

9. Compounds of general formula **I** according to claim 1, wherein **R¹**, **R²**, **R³**, **R⁴**, **R⁶**, **R⁷**, **R⁸** and **n** are defined as in claim 1 and
**R⁵** denotes (a) H, halogen, (b) C₁₋₃-alkyl, (c) C₁₋₃-alkyl, wherein each methylene group is substituted by up to two fluorine atoms and each methyl group is substituted by up to three fluorine atoms, (d) -O-C₁₋₃-alkyl, (e) -C(O)-O-C₁₋₃-alkyl or -C(O)-NH₂,
the enantiomers, the diastereomers, the mixtures thereof and the salts thereof, particularly the physiologically acceptable salts thereof with organic or inorganic acids or bases.

10. Compounds of general formula **I** according to claim 1, wherein **R¹**, **R²**, **R³**, **R⁴**, **R⁵**, **R⁷**, **R⁸** and **n** are defined as in claim 1 and
**R⁶** denotes (a) H, halogen, (b) C₁₋₃-alkylene-**R^{6.1}**, (c) C₁₋₃-alkyl, wherein each methylene group is substituted by up to two fluorine atoms and each methyl group is substituted by up to three fluorine atoms, (d) -OH, -O-C₁₋₄-alkyl, (e) -OCHF₂, -O-CF₃, (f) -C(O)-O-**R^{6.2}**, -CN, -C(O)-CH₃, -C(O)-NH₂ or (g) pyrrolyl,
**R^{6.1}** denotes H or -OH and
**R^{6.2}** denotes H or C₁₋₃-alkyl,
the enantiomers, the diastereomers, the mixtures thereof and the salts thereof, particularly the physiologically acceptable salts thereof with organic or inorganic acids or bases.

11. Compounds of general formula **I** according to claim 1, wherein **R¹**, **R²**, **R³**, **R⁴**, **R⁵**, **R⁶**, **R⁸** and **n** are defined as in claim 1 and
**R⁷** denotes (a) H, halogen, (b) C₁₋₃-alkyl, (c) C₁₋₃-alkyl, wherein each methylene group is substituted by up to two fluorine atoms and each methyl group is substituted by up to three fluorine atoms, (d) -O-C₁₋₃-alkyl, (e) -C(O)-NH₂ or -C(O)-pyrrolidinyl,
the enantiomers, the diastereomers, the mixtures thereof and the salts thereof, particularly the physiologically acceptable salts thereof with organic or inorganic acids or bases.

12. Compounds of general formula **I** according to claim 1, wherein **R¹**, **R²**, **R³**, **R⁴**, **R⁵**, **R⁶**, **R⁷** and **n** are defined as in claim 1 and
**R⁸** denotes (a) H, halogen, (b) C₁₋₄-alkyl, (c) C₁₋₃-alkyl, wherein each methylene group is substituted by up to two fluorine atoms and each methyl group is substituted by up to three fluorine atoms, (d) -O-C₁₋₃-alkyl, (e) -O-CF₃, (f) -CN, -C(O)-NH₂ or (g) pyrrolyl,
the enantiomers, the diastereomers, the mixtures thereof and the salts thereof, particularly the physiologically acceptable salts thereof with organic or inorganic acids or bases.

13. Compounds of general formula I according to claim 1, wherein
**R¹** denotes a group selected from 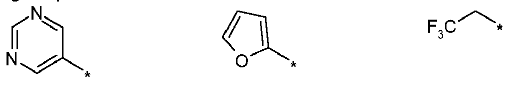
**R²** denotes H or -CH₃,
**R³** denotes H, F, -CF₃, -CH₃ or -O-CH₃,
**R⁴** denotes H or Cl,
**R⁵** denotes H, Cl, C₁₋₃-alkyl, -CF₃, -O-C₁₋₃-alkyl, -C(O)-O-C₁₋₃-alkyl or -C(O)-NH_{2,}
**R⁶** denotes H, F, Cl, C₁₋₃-alkyl, -CF₃, -O-C₁₋₄-alkyl, -OCF₃, -C(O)-NH₂ or pyrrolyl,
**R⁷** denotes H, F, Cl, C₁₋₃-alkyl, -CF₃, -O-C₁₋₃-alkyl, -C(O)-NH₂ or -C(O)-pyrrolidinyl,
**R⁸** denotes H, F, Cl, Br, C₁₋₄-alkyl, -CF₃, -O-C₁₋₃-alkyl, -OCF₃, -C(O)-NH₂, -OCF₃
or pyrrolyl,
the enantiomers, the diastereomers, the mixtures thereof and the salts thereof, particularly the physiologically acceptable salts thereof with organic or inorganic acids or bases.

14. Compounds of general formula I according to claim 1, wherein
**R¹** denotes a group selected from 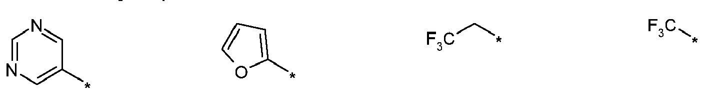 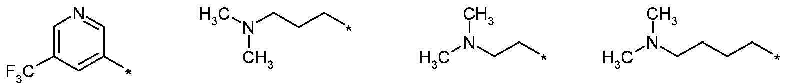 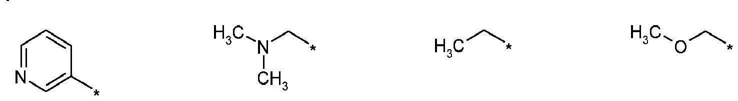 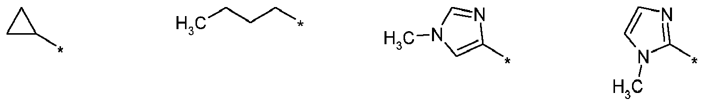 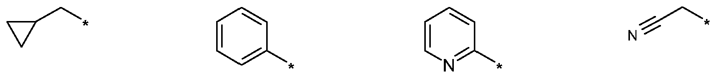 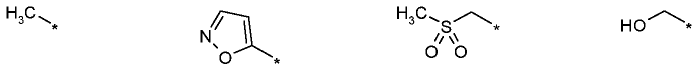 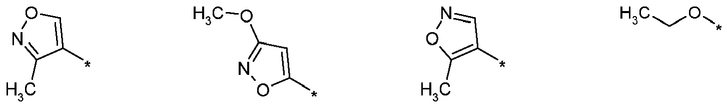 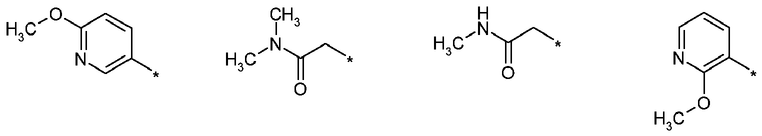 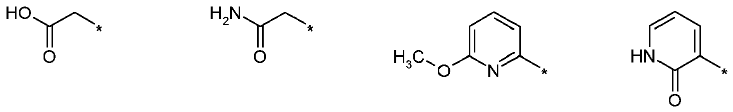 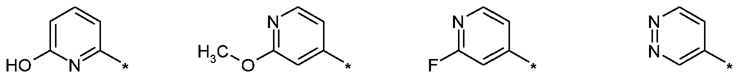 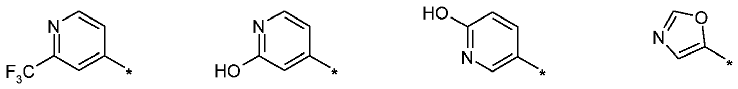 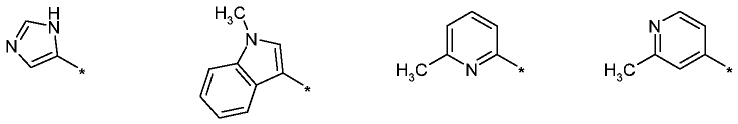 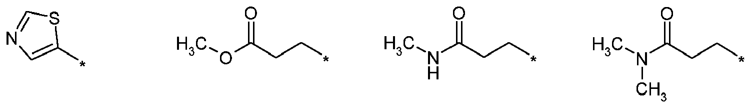 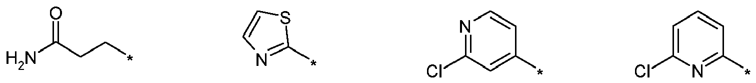 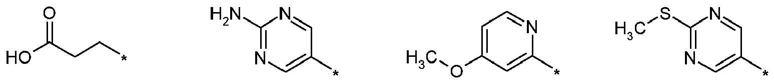 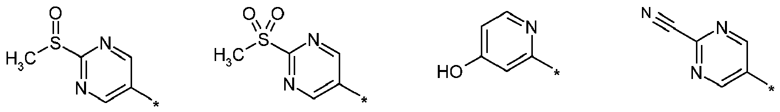
**R²** denotes H, -CH₃ or -C₂H₅,
**R³** denotes H, F, Cl, -CF₃, -CH₃ or -O-CH₃,
**R⁴** denotes H or Cl,
**R⁵** denotes H, Cl, C₁₋₃-alkyl, -CF₃, -O-C₁₋₃-alkyl, -C(O)-O-C₁₋₃-alkyl or -C(O)-NH_{2,}
**R⁶** denotes H, F, Cl, Br, -CN, C₁₋₃-alkyl, -CF₃, -COOH, -COO-C₁₋₃-alkyl, -CH(OH)CH₃, -OH, -O-C₁₋₄-alkyl, -OCF₃, -OCHF₂, -C(O)-CH₃, -C(O)-NH₂ or pyrrolyl,
**R⁷** denotes H, F, Cl, C₁₋₃-alkyl, -CF₃, -O-C₁₋₃-alkyl, -C(O)-NH₂ or -C(O)-pyrrolidinyl and
**R⁸** denotes H, F, Cl, Br, -CN, C₁₋₄-alkyl, -CF₃, -O-C₁-₃-alkyl, -OCF₃, -C(O)-NH₂, -OCF₃ or pyrrolyl,
the enantiomers, the diastereomers, the mixtures and the salts thereof, particularly the physiologically acceptable salts thereof with organic or inorganic acids or bases.

15. The following compounds of general formula I according to claim 1:
| **No.** | **Structure** |
|---|---|
| (1) | 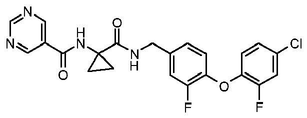 |
| (2) | 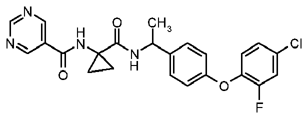 |
| (3) | 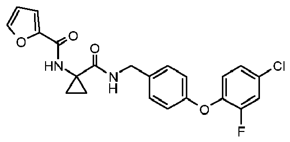 |
| (4) | 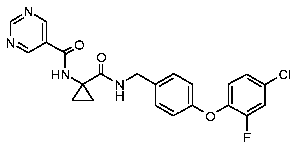 |
| (5) | 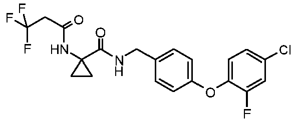 |
| (6) | 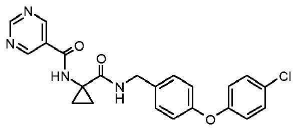 |
| (7) | 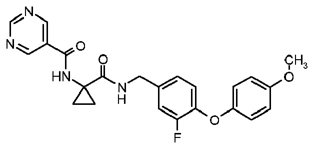 |
| (8) | 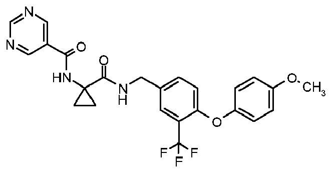 |
| (9) | 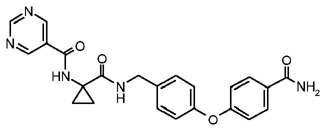 |
| (10) | 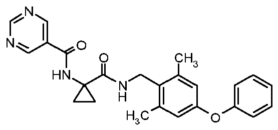 |
| (11) | 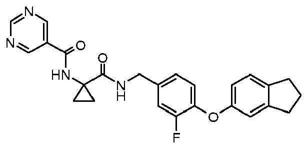 |
| (12) | 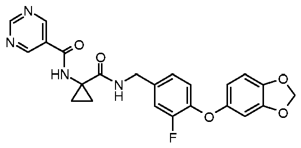 |
| (13) | 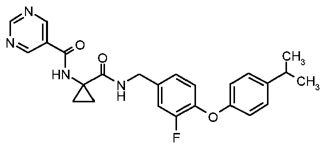 |
| (14) | 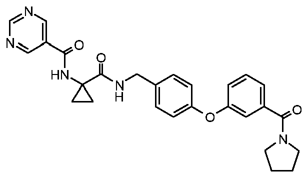 |
| (15) | 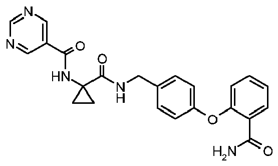 |
| (16) | 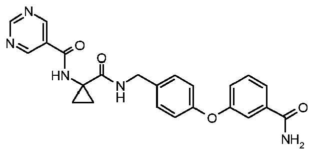 |
| (17) | 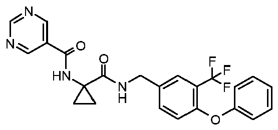 |
| (18) | 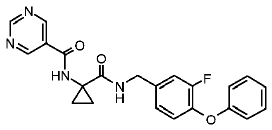 |
| (19) | 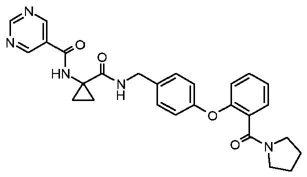 |
| (20) | 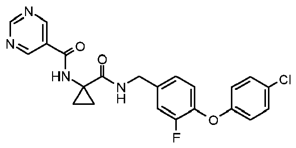 |
| (21) | 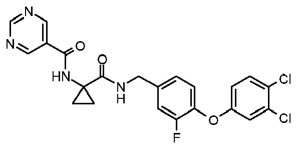 |
| (22) | 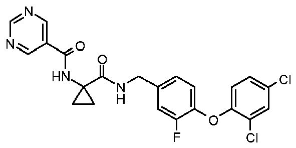 |
| (23) | 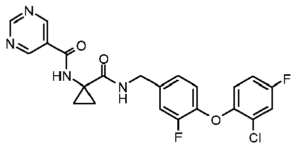 |
| (24) | 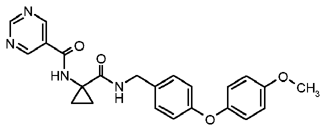 |
| (25) | 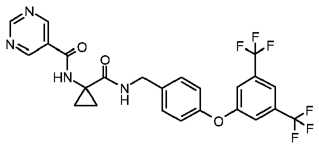 |
| (26) | 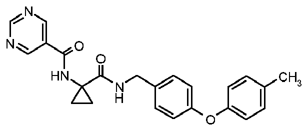 |
| (27) | 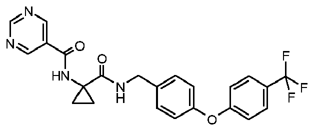 |
| (28) | 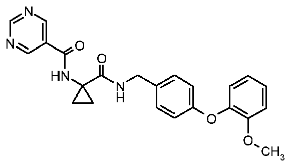 |
| (29) | 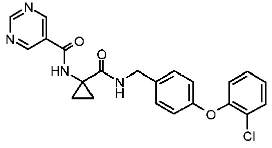 |
| (30) | 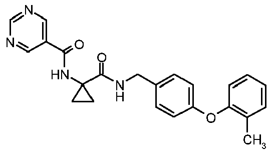 |
| (31) | 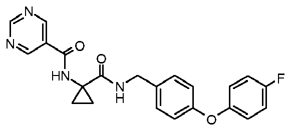 |
| (32) | 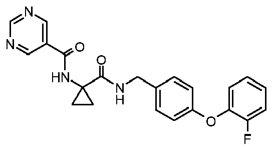 |
| (33) | 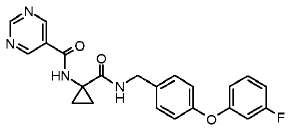 |
| (34) | 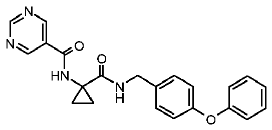 |
| (35) | 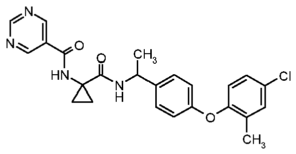 |
| (36) | 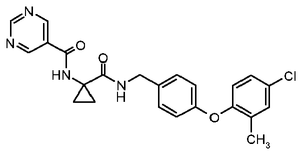 |
| (37) | 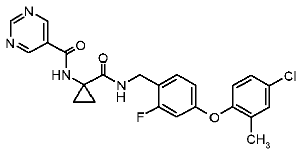 |
| (38) | 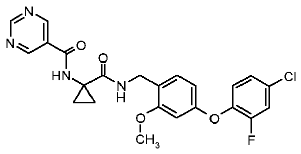 |
| (39) | 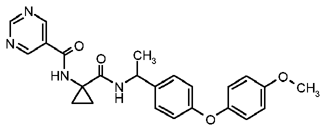 |
| (40) | 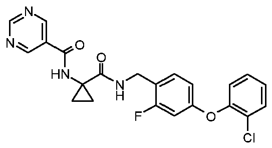 |
| (41) | 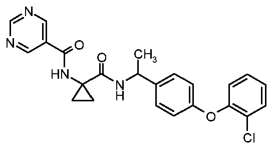 |
| (42) | 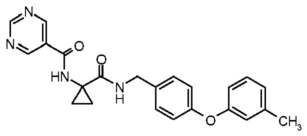 |
| (43) | 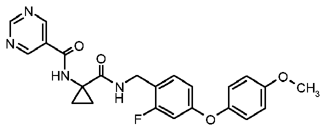 |
| (44) | 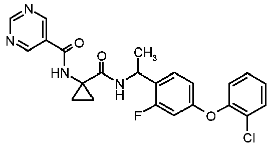 |
| (45) | 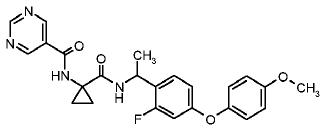 |
| (46) | 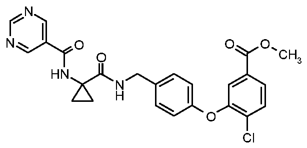 |
| (47) | 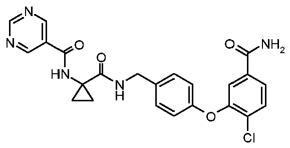 |
| (48) | 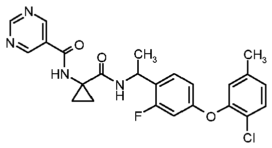 |
| (49) | 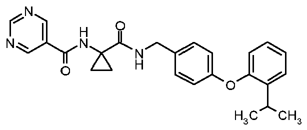 |
| (50) | 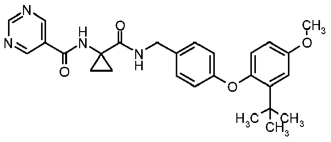 |
| (51) | 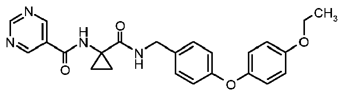 |
| (52) | 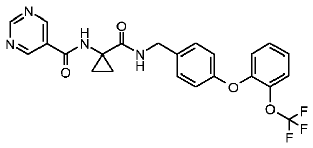 |
| (53) | 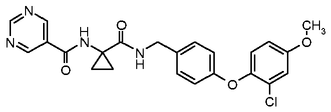 |
| (54) | 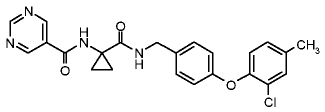 |
| (55) | 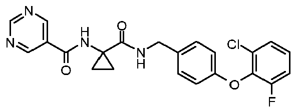 |
| (56) | 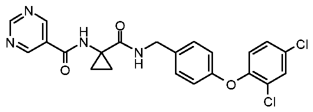 |
| (57) | 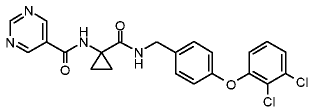 |
| (58) | 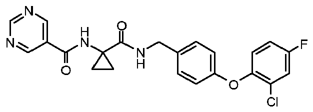 |
| (59) | 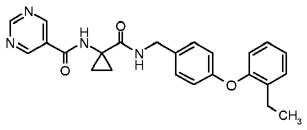 |
| (60) | 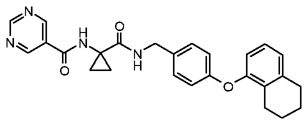 |
| (61) | 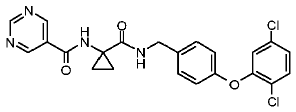 |
| (62) | 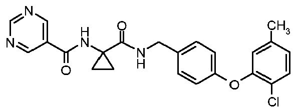 |
| (63) | 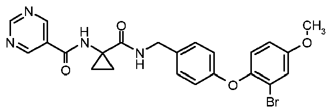 |
| (64) | 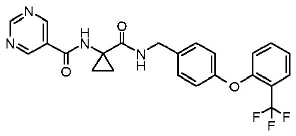 |
| (65) | 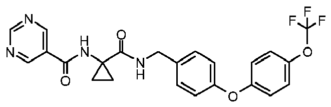 |
| (66) | 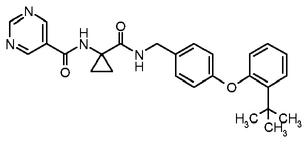 |
| (67) | 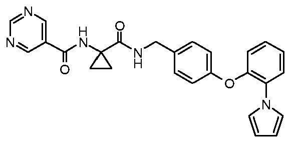 |
| (68) | 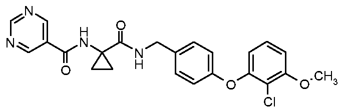 |
| (69) | 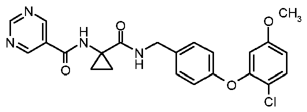 |
| (70) | 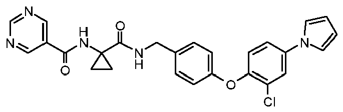 |
| (71) | 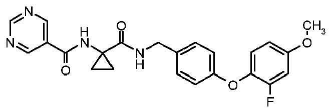 |
| (72) | 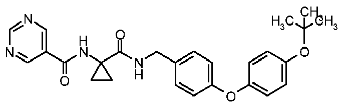 |
| (73) | 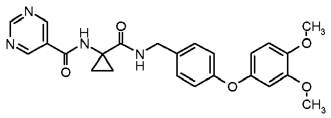 |
| (74) | 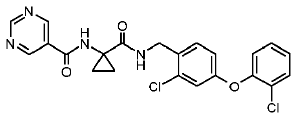 |
| (75) | 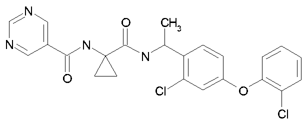 |
| (76) | 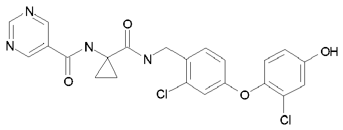 |
| (77) | 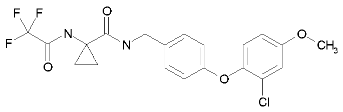 |
| (78) | 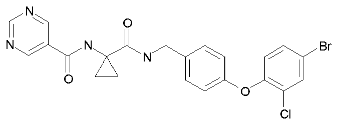 |
| (79) | 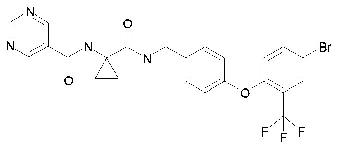 |
| (80) | 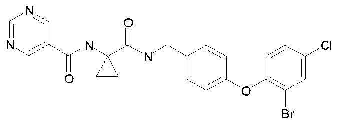 |
| (81) | 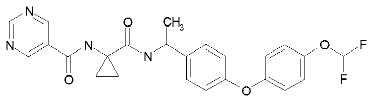 |
| (82) | 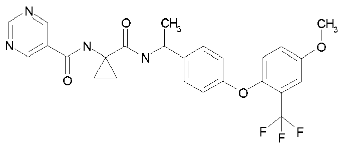 |
| (83) | 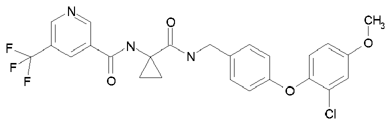 |
| (84) | 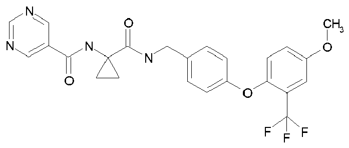 |
| (85) | 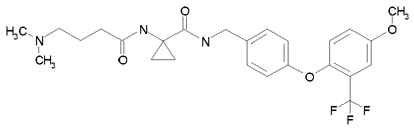 |
| (86) | 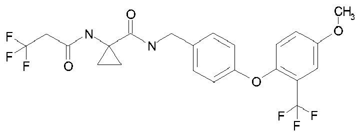 |
| (87) | 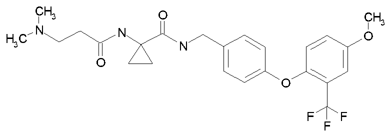 |
| (88) | 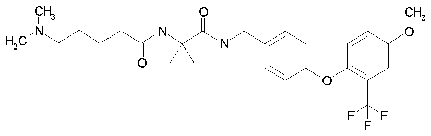 |
| (89) | 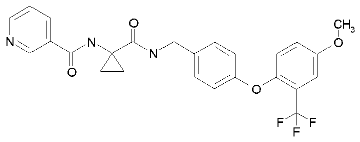 |
| (90) | 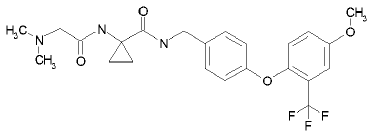 |
| (91) | 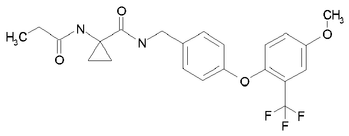 |
| (92) | 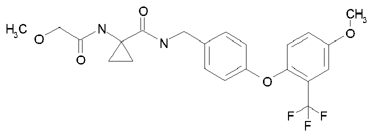 |
| (93) | 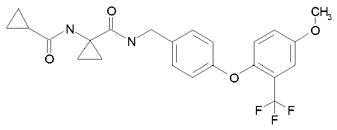 |
| (94) | 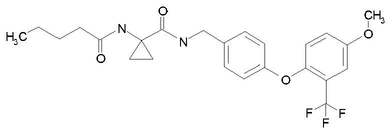 |
| (95) | 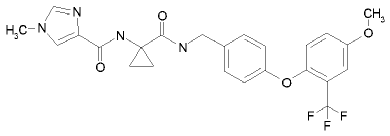 |
| (96) | 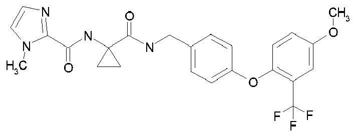 |
| (97) | 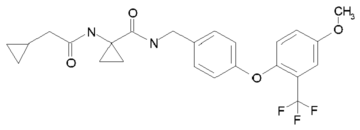 |
| (98) | 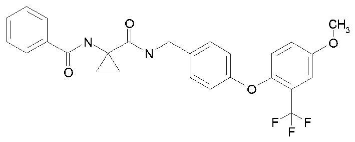 |
| (99) | 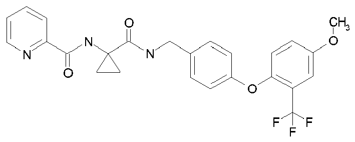 |
| (100) | 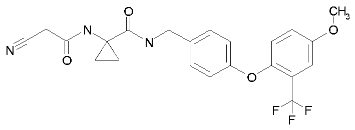 |
| (101) | 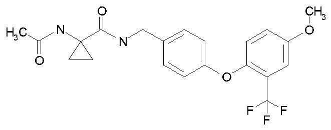 |
| (102) | 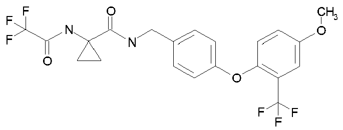 |
| (103) | 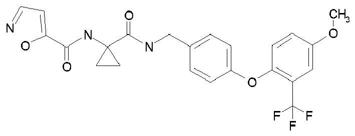 |
| (104) | 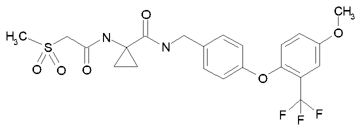 |
| (105) | 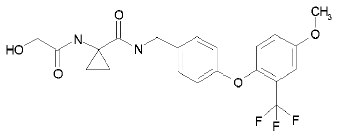 |
| (106) | 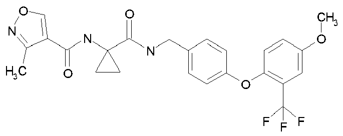 |
| (107) | 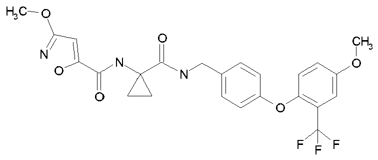 |
| (108) | 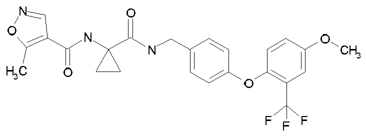 |
| (109) | 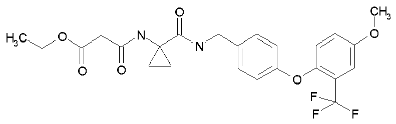 |
| (110) | 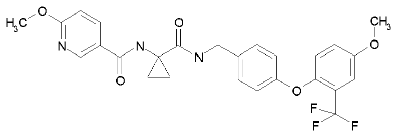 |
| (111) | 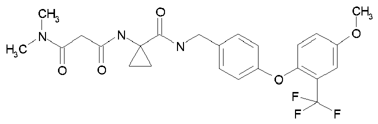 |
| (112) | 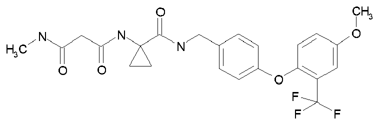 |
| (113) | 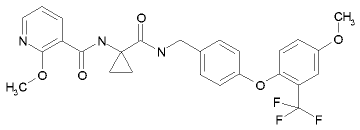 |
| (114) | 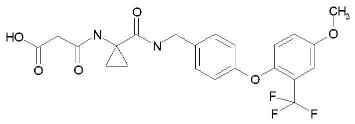 |
| (115) | 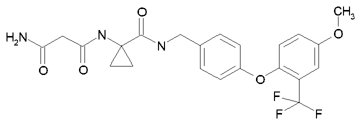 |
| (116) | 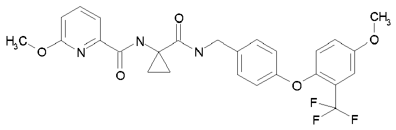 |
| (117) | 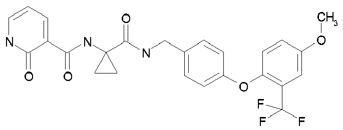 |
| (118) | 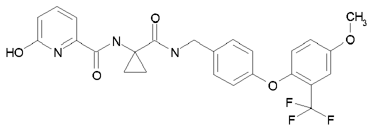 |
| (119) | 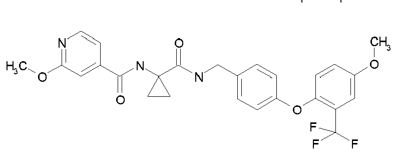 |
| (120) | 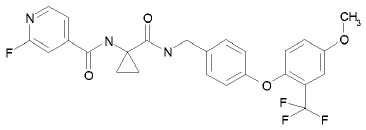 |
| (121) | 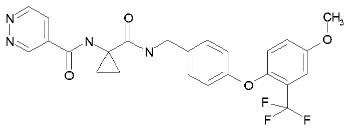 |
| (122) | 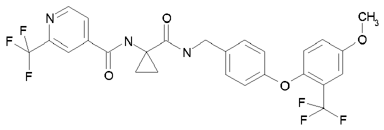 |
| (123) | 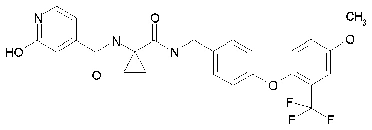 |
| (124) | 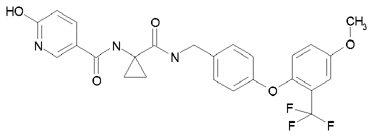 |
| (125) | 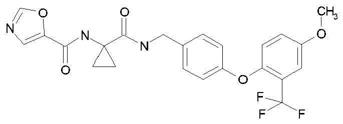 |
| (126) | 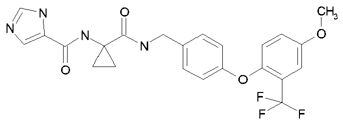 |
| (127) | 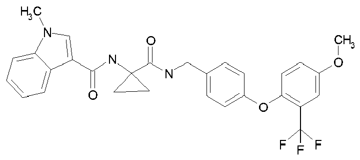 |
| (128) | 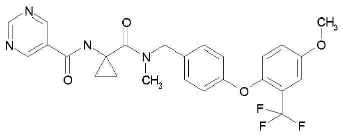 |
| (129) | 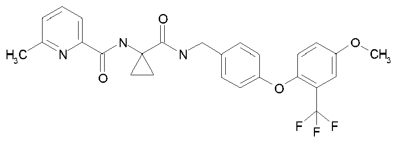 |
| (130) | 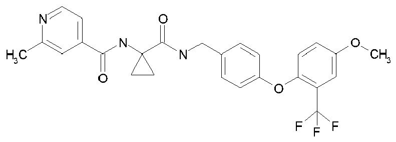 |
| (131) | 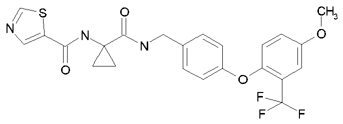 |
| (132) | 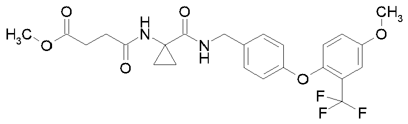 |
| (133) | 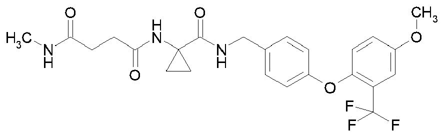 |
| (134) | 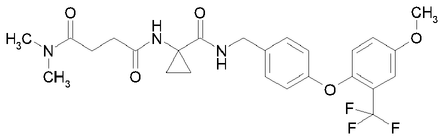 |
| (135) | 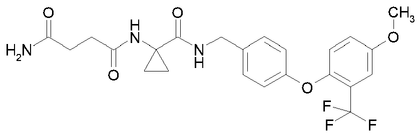 |
| (136) | 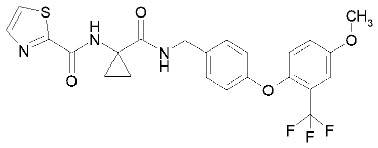 |
| (137) | 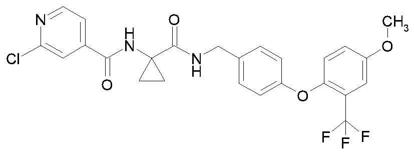 |
| (138) | 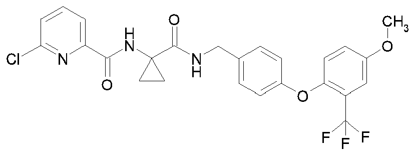 |
| (139) | 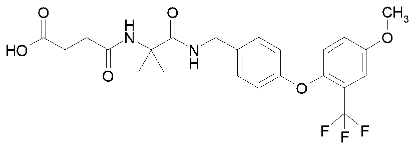 |
| (140) | 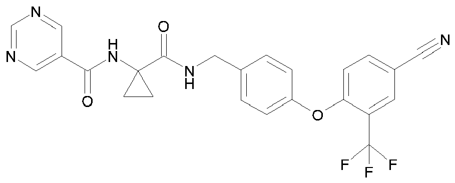 |
| (141) | 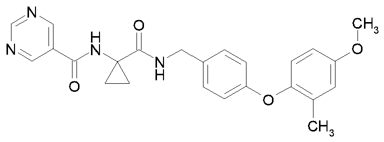 |
| (142) | 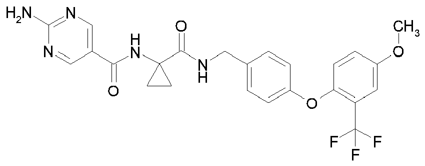 |
| (143) | 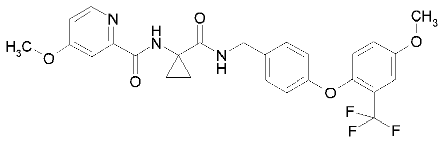 |
| (144) | 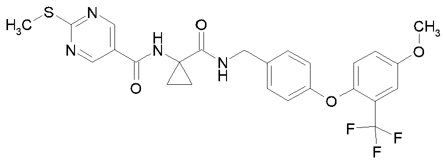 |
| (145) | 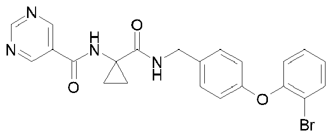 |
| (146) | 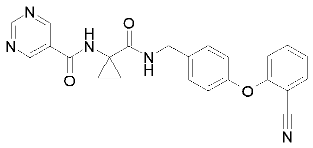 |
| (147) | 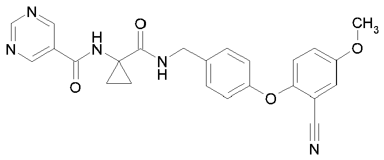 |
| (148) | 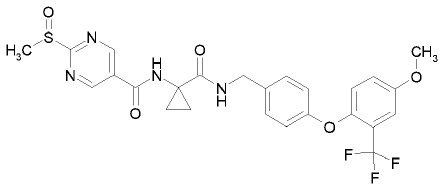 |
| (149) | 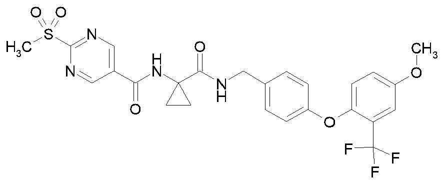 |
| (150) | 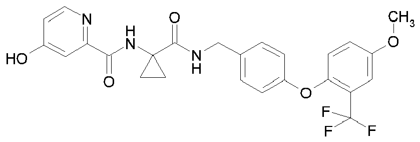 |
| (151) | 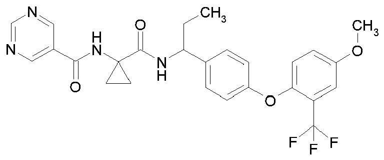 |
| (152) | 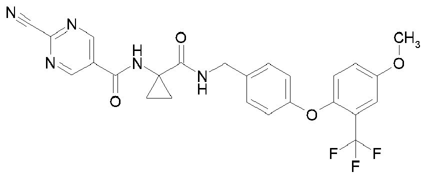 |
| (153) | 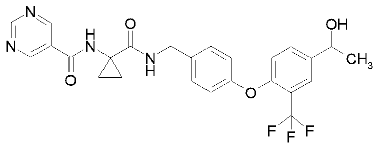 |
| (154) | 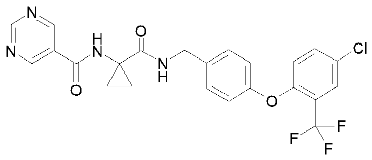 |
| (155) | 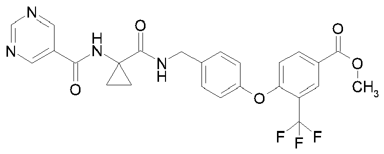 |
| (156) | 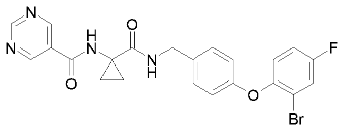 |
| (157) | 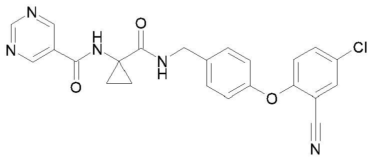 |
| (158) | 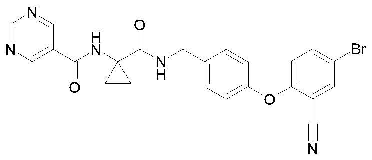 |
| (159) | 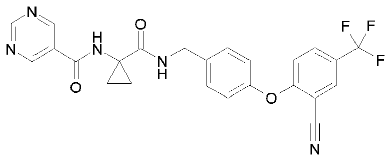 |
| (160) | 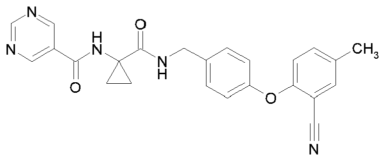 |
| (161) | 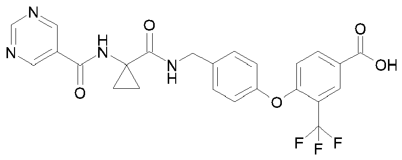 |
| (162) | 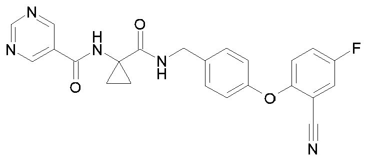 |
| (163) | 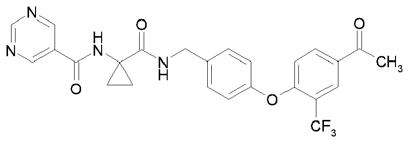 |
the enantiomers, the diastereomers, the mixtures thereof and the salts thereof, particularly the physiologically acceptable salts thereof with organic or inorganic acids or bases.

16. Physiologically acceptable salts of the compounds according to one of claims 1 to 15 with inorganic or organic acids or bases.

17. Medicaments, containing a compound according to at least one of claims 1 to 15 or a physiologically acceptable salt according to claim 16 optionally together with one or more inert carriers and/or diluents.

18. Use of a compound according to at least one of claims 1 to 16 for preparing a medicament for the acute and prophylactic treatment of acute pain, visceral pain, neuropathic pain, inflammatory/pain receptor-mediated pain, tumour pain and headache diseases.

19. Process for preparing a medicament according to claim 17, **characterised in that** a compound according to at least one of claims 1 to 16 is incorporated in one or more inert carriers and/or diluents by a non-chemical method.

## Revendications

1. Composés de formule générale I 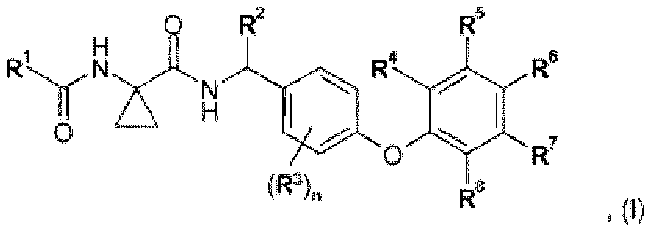 dans laquelle
R¹ désigne
(a) un groupe alkyle en C₁ à C₆ éventuellement substitué par un radical R^{1,1}
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
(c) un groupe cycloalkyle en C₃ à C₆ éventuellement substitué par un radical R^{1,2},
(d) un groupe alcényle en C₂ à C₆,
(e) un groupe alcinyle en C₂ à C₆,
(f) un groupe aryl-alkylène en C₀ à C₂ éventuellement substitué par 1, 2 ou 3 radicaux R_{1,3},
(g) un radical hétéroaryle à cinq chaînons éventuellement substitué par 1, 2 ou 3 radicaux R_{1,4} qui contient au moins un atome de N, O ou S et qui contient en outre éventuellement un, deux ou trois atomes de N et qui peut en outre être benzo-condensé,
(h) un radical hétéroaryle à six chaînons éventuellement substitué par un radical R^{1,4} qui contient un, deux ou trois atomes de N et qui peut en outre être baso-condensé,
(i) -O-R^{1,1,1} ou
(j) -NR^{1,1,2}R^{1,1,4},
R^{1,1} désigne un atome d'halogène, -NO₂, -CN, un groupe cycloalkyle en C₃ à C₆, -OR^{1,1,1}, -SR^{1,1,1}, - C(O)R^{1,1,1}, -S(O)₂-R^{1,1,2}, -O-S(O)₂-R^{1,1,1}, -CO₂R^{1,1,1}, -OC(O)-R^{1,1,1}, -NR^{1,1,3}R^{1,1,4}, -NR^{1,1,3}-C(O)-R^{1,1,1}, -NR^{1,1,3}-C(O)-R^{1,1,1}, -NR^{1,1,3}-CO₂-R^{1,1,1} ou -C(O)-NR^{1,1,3}R^{1,1,4},
R^{1,1,1} désigne
(a) H,
(b) un groupe alkyle en C₁ à C₄,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe phényle éventuellement substitué par 1, 2 ou 3 radicaux R^{1,1,1,1},
(e) un groupe cycloalkyle en C₃ à C₆ ou
(f) un groupe pyridyle substitué éventuellement par 1, 2 ou 3 radicaux R^{1,1,1,2},
les R^{1,1,1,1} désignent indépendamment les uns des autres
(a) un atome d'halogène, -NO₂, -CN, -ON, un groupe -O-alkyle en C₁ à C₄, cycloalkyle en C₃ à C₆, alkyle en C₁ à C₄ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
les R^{1,1,1,2} désignent indépendamment les uns des autres, un atome d'halogène ou un groupe alkyle en C₁ à C₄,
R^{1,1,2} désigne
(a) un groupe alkyle en C₁ à C₄,
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
(c) un groupe -O-alkyle en C₁ à C₄ ou
(d) un groupe phényle éventuellement substitué par 1, 2 ou 3 radicaux R^{1,1,1,1},
les R^{1,1,3},
R^{1,1,4} désignent indépendamment les uns des autres
(a) H
(b) un groupe alkyle en C₁ à C₄ éventuellement substitué par 1, 2 ou 3 radicaux R^{1,1,4,1},
(c) un groupe phényle éventuellement substitué par 1, 2 ou 3 radicaux R^{1,1,1,1},
(d) un groupe cycloalkyle en C₃ à C₆, ou
les R^{1,1,3} et R^{1,1,4} conjointement avec l'atome de N auquel ils sont liés forment un cycle hétérocyclique de 4, 5 ou 6 chaînons qui peut contenir un autre hétéroatome choisi parmi N, O et S, ou
les R^{1,1,3} et R^{1,1,4} conjointement avec l'atome de N auquel ils sont liés forment un imide cyclique,
les R^{1,1,4,1} indépendamment les uns des autres désignent un atome d'halogène, -NH₂, un groupe - NH(alkyle en C₁ à C₄), -N (alkyle en C₁ à C₄)₂ ou -SO₂-R^{1,1,2},
R^{1,2} désigne un atome d'halogène, -NO₂, -CN ou un groupe phényle,
R^{1,3} désigne (a) un atome d'halogène, -NO₂, -CN, - OR^{1,1,1}, -SR^{1,1,1}, -CO₂R^{1,1,1}, un groupe alkyle en C₁ à C₆ ou (b) un groupe alkyle en C₁ à C₃ dans lequel chaque groupe méthylène est substitué par jusqu'à deux atomes de fluor et chaque groupe méthyle est substitué par jusqu'à trois atomes de fluor,
les R^{1,4} désignent indépendamment les uns des autres
(a) un atome d'halogène, -NO₂, -CN, -OR^{1,1,1}, - SR^{1,1,1}, -S(O)-R^{1,1,2}, -S(O)₂-R^{1,1,2}, -NR^{1,1,3}R^{1,1,4}, un groupe alkyle en C₁ à C₆,
(b) un groupe alkyle en C₁ à C₃ dans lequel chaque groupe méthylène est substitué par jusqu'à deux atomes de fluor et chaque groupe méthyle est substitué par jusqu'à trois atomes de fluor, ou
(c) un groupe oxo,
R² désigne
(a) H, un groupe alkyle en C₁ à C₄ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
les R³ désignent indépendamment les uns des autres
(a) H, un atome d'halogène, -CN, -OH, un groupe alkyle en C₁ à C₆, cycloalkyle en C₃ à C₇, -O-alkyle en C₁ à C₄, -O-CF₃, -O-cycloalkyle en C₃ à C₆, -N (alkyle en C₁ à C₃) ₂, -C(O)-NH₂, -(SO₂)NH₂, -SO₂-alkyle en C₁ à C₃ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
les R⁴, R⁵,
R⁶, R⁷,
R⁸ désignent indépendamment les uns des autres
(a) H, un atome d'halogène, -CN, -OH,
(b) un groupe alkyle en C₁ à C₆ dans lequel deux substituants voisins peuvent représenter conjointement un groupe triméthylène ou tétraméthylène,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe cycloalkyle en C₃ à C₇,
(e) un groupe -O-alkyle en C₁ à C₆, dans lequel deux substituants voisins peuvent représenter un groupe méthylènedioxy ou un groupe éthylènedioxy,
(f) -O-CF₃, -O-cycloalkyle en C₃ à C₇,
(g) -NH₂, un groupe -NH (alkyle en C₁ à C₃), - N(alkyle en C₁ à C₃) ₂,
(h) -C(O)-R^{8,1},
(i) -SO₂-R^{8,2},
(j) un groupe hétéroaryle à cinq chaînons éventuellement substitué par un ou deux groupes alkyle en C₁ à C₃ qui est choisi dans le groupe comprenant les groupes pyrrolyle, oxazolyle, isoxazolyle, oxadiazolyle, thiazolyle, isothiazolyle, thiadiazolyle, imidazolyle, pyrazolyle, triazolyle et tétrazolyle, ou
(k) un groupe héréroaryle à six chaînons substitué éventuellement par un ou deux groupes alkyle en C₁ à C₃, qui est choisi dans le groupe comprenant les groupes pyridyle, pyrimidinyle, pyrazinyle, pyridazinyle et triazinyle,
R^{8,1} désigne les groupes -NH₂, -NH(alkyle en C₁ à C₆), - (alkyle en C₁ à C₆) ₂, N-acétidinyle, N-pyrrolidinyle, N-pipéridinyle, N-morpholinyle, -OH, -O-alkyle en C₁ à C₈ ou -O-cycloalkyle en C₃ à C₇,
R^{8,2} désigne les groupes -NH₂, -NH (alkyle en C₁ à C₆), -N(alkyle en C₁ à C₆)₂, N-acétidinyle, N-pyrrolidinyle, N-pipéridinyle ou N-morpholinyle et
n désigne l'un des chiffres 0, 1, 2, 3 ou 4,
leurs énantiomères, leurs diastéréomères, leurs mélanges et leurs sels, en particulier leurs sels physiologiquement acceptables avec des acides ou bases organiques ou anorganiques.

2. Composés de formule générale I selon la revendication 1, dans laquelle R², R³, R⁴, R⁵, R⁶, R⁷, R⁸ et n sont tels que définis dans la revendication 1 et
R¹ désigne
(a) un groupe alkyle en C₁ à C₆ éventuellement substitué par un radical R^{1,1},
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
(c) un groupe cycloalkyle en C₃ à C₆ éventuellement substitué par un radical R^{1,2},
(d) un groupe phényle éventuellement substitué par 1, 2 ou 3 radicaux R^{1,3},
(e) un radical hétéroaryle à cinq chaînons éventuellement substitué par un radical R^{1,4} qui contient au moins un atome de N, O ou S et qui contient en outre éventuellement un, deux ou trois atomes de N, ou
(f) un radical hétéroaryle à six chaînons éventuellement substitué par un radical R^{1,4} qui contient un, deux ou trois atomes de N,
R^{1,1} désigne un groupe cycloalkyle en C₃ à C₆, - OR^{1,1,1}, -NR^{1,1}, 3R^{1,1,4}, -C(O)-NR^{1,1}, 3R^{1,1,4}, -CN, -CO₂R^{1,1,1}, - S(O)₂-alkyle en C₁ à C₆ ou -O-S-(O)₂-alkyle en C₁ à C₆,
R^{1,1,1} désigne
(a) H,
(b) un groupe alkyle en C₁ à C₄,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe cycloalkyle en C₃ à C₆,
les R^{1,1,3},
R^{1,1,4} désignent indépendamment les uns des autres
(a) H
(b) un groupe alkyle en C₁ à C₄ ou,
(c) un groupe cycloalkyle en C₃ à C₆,
R^{1,2} désigne un atome d'halogène, -NO₂, -CN ou un groupe phényle,
R^{1,3} désigne
(a) un atome d'halogène, -NO₂, -CN, - OR^{1,1,1}, -SR^{1,1,1}, -CO₂R^{1,1,1}, un groupe alkyle en C₁ à C₆ ou
(b) un groupe alkyle en C₁ à C₃ dans lequel chaque groupe méthylène est substitué par jusqu'à deux atomes
de fluor et chaque groupe méthyle est substitué par jusqu'à trois atomes de fluor,
les R^{1,4} désignent
(a) un atome d'halogène, -NO₂, -CN, -OR^{1,1,1}, - SR^{1,1,1}, -NR^{1,1,3}R^{1,1,4}, un groupe alkyle en C₁ à C₆,
(b) un groupe alkyle en C₁ à C₃ dans lequel chaque groupe méthylène est substitué par jusqu'à deux atomes de fluor et chaque groupe méthyle est substitué par jusqu'à trois atomes de fluor, ou
(c) un groupe oxo,
leurs énantiomères, leurs diastéréomères, leurs mélanges et leurs sels, en particulier leurs sels physiologiquement acceptables avec des acides ou bases organiques ou anorganiques.

3. Composés de formule générale I selon la revendication 1, dans laquelle
R¹ désigne
(a) un groupe alkyle en C₁ à C₆ éventuellement substitué par un radical R^{1,1},
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
(c) un groupe cycloalkyle en C₃ à C₆ éventuellement substitué par un radical R^{1,2},
(d) un groupe phényle éventuellement substitué par 1, 2 ou 3 radicaux R^{1,3},
(e) un radical hétéroaryle à cinq chaînons éventuellement substitué par un radical R^{1,4} qui contient au moins un atome de N, O ou S et qui contient en outre éventuellement un, deux ou trois atomes de N, ou
(f) un radical hétéroaryle à six chaînons éventuellement substitué par un radical R^{1,4} qui contient un, deux ou trois atomes de N,
R^{1,1} désigne un groupe cycloalkyle en C₃ à C₆, - OR^{1,1,1}, -NR^{1,1,3}R^{1,1,4}, -C (O) -NR^{1,1,3}R^{1,1,4}, -CN, -CO₂R^{1,1,1}, - S(O)₂-alkyle en C₁ à C₆ ou -O-S-(O)₂-alkyle en C₁ à C₆,
R^{1,1,1} désigne
(a) H,
(b) un groupe alkyle en C₁ à C₄,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe cycloalkyle en C₃ à C₆,
les R^{1,1,3},
R^{1,1,4} désignent indépendamment les uns des autres
(a) H
(b) un groupe alkyle en C₁ à C₄ ou,
(c) un groupe cycloalkyle en C₃ à C₆,
R^{1,2} désigne un atome d'halogène, -NO₂, -CN ou un groupe phényle,
R^{1,3} désigne
(a) un atome d'halogène, -NO₂, -CN, - OR^{1,1,1}, -SR^{1,1,1}, -CO₂R^{1,1,1}, un groupe alkyle en C₁ à C₆ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
les R^{1,4} désignent
(a) un atome d'halogène, -NO₂, -CN, -OR^{1,1,1}, - SR^{1,1,1}, -NR^{1,1,3}R^{1,1,4}, un groupe alkyle en C₁ à C₆,
(b) un groupe alkyle en C₁ à C₃ dans lequel chaque groupe méthylène est substitué par jusqu'à deux atomes de fluor et chaque groupe méthyle est substitué par jusqu'à trois atomes de fluor, ou
(c) un groupe oxo,
R² désigne H ou un groupe alkyle en C₁ à C₃,
les R³ désignent, indépendamment les uns des autres
(a) H, un atome d'halogène, un groupe alkyle en C₁ à C₃, -O-alkyle en C₁ à C₃ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
R⁴ désigne H ou un atome d'halogène,
R⁵ désigne
(a) H, un atome d'halogène,
(b) un groupe alkyle en C₁ à C₃,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe -O-alkyle en C₁ à C₃,
(e) un groupe -C (O) -O-alkyle en C₁ à C₃ ou -C (O) - NH₂,
R⁶ désigne
(a) H, un atome d'halogène,
(b) un groupe alkylène en C₁ à C₃-R^{6,1},
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe -OH, -O-alkyle en C₁ à C₄,
(e) -O-CHF₂, -O-CF₃,
(f) -C(O)-O-R^{8,2}, -CN, -C(O)-CH₃, -C(O)-NH₂ ou
(g) un groupe pyrrolyle
R^{6,1} désigne H, -OH,
R^{6,2} désigne H, un groupe alkyle en C₁ à C₃,
R⁷ désigne
(a) H, un atome d'halogène,
(b) un groupe alkyle en C₁ à C₃,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) -O-alkyle en C₁ à C₃,
(e) -C(O)-NH₂ ou -C(O)pyrrolidinyle et
R⁸ désigne
(a) H, un atome d'halogène,
(b) un groupe alkyle en C₁ à C₄,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) -O-alkyle en C₁ à C₃,
(e) -O-CF₃,
(f) -CN, -C(O)-NH₂ ou
(g) un groupe pyrrolyle,
leurs énantiomères, leurs diastéréomères, leurs mélanges et leurs sels, en particulier leurs sels physiologiquement acceptables avec des acides ou bases organiques ou anorganiques.

4. Composés de formule générale I selon la revendication 1, dans laquelle R², R³, R⁴, R⁵, R⁶, R⁷, R⁸ et n sont tels que définis dans la revendication 1 et
R¹ désigne
(a) un groupe alkyle en C₁ à C₆ éventuellement substitué par un radical R^{1,1},
(b) un groupe alkyle en C₁ à C₃, dans lequel chaque groupe méthylène est substitué par jusqu'à deux atomes de fluor et chaque groupe méthyle est substitué par jusqu'à trois atomes de fluor,
(c) un groupe phényle éventuellement substitué par 1, 2 ou 3 radicaux R^{1,3},
(d) un radical hétéroaryle à cinq chaînons éventuellement substitué par un radical R^{1,4} qui est choisi dans le groupe comprenant les groupes furanyle, isoxazolyle, isothiazolyle, oxazolyle, thiazolyle, pyrazolyle, thiadiazolyle, oxadiazolyle, triazolyle, tétrazolyle, imidazolyle, indolyle, thiényle, pyrrolyle, benzimidazolyle, benzoxazolyle, benzthiazolyle et benzisoxazinyle, ou
(e) un radical hétéroaryle à six chaînons éventuellement substitué par un radical R^{1,4} qui est choisi dans le groupe comprenant les groupes pyridyle, pyrimidinyle, pyridazinyle, pyrazinyle, triazinyle, quinasolinyle et quinoxazinyle,
R^{1,1} désigne un groupe cycloalkyle en C₃ à C₆, - OR^{1,1,1}, -NR^{1,1,3}R^{1,1,4}, -C(O)-NR^{1,1,3}R^{1,1,4}, -CN, -CO₂R^{1,1,1}, - S(O)₂-alkyle en C₁ à C₆ ou -O-S-(O)₂-alkyle en C₁ à C₆,
R^{1,1,1} désigne
(a) H,
(b) un groupe alkyle en C₁ à C₄,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) un groupe cycloalkyle en C₃ à C₆,
les R^{1,1,3},
R^{1,1,4} désignent indépendamment les uns des autres
(a) H
(b) un groupe alkyle en C₁ à C₄ ou,
(c) un groupe cycloalkyle en C₃ à C₆,
R^{1,3} désigne
(a) un atome d'halogène, -NO₂, -CN, - OR^{1,1,1}, -SR^{1,1,1}, -CO₂R^{1,1,1}, un groupe alkyle en C₁ à C₆ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
R^{1,4} désigne
(a) un atome d'halogène, -NO₂, -CN, - OR^{1,1,1}, -SR^{1,1,1}, un groupe alkyle en C₁ à C₆,
(b) un groupe alkyle en C₁ à C₃ dans lequel chaque groupe méthylène est substitué par jusqu'à deux atomes de fluor et chaque groupe méthyle est substitué par jusqu'à trois atomes de fluor, ou
(c) un groupe oxo,
leurs énantiomères, leurs diastéréomères, leurs mélanges et leurs sels, en particulier leurs sels physiologiquement acceptables avec des acides ou bases organiques ou anorganiques.

5. Composés de formule générale I selon la revendication 1, dans laquelle les R², R³, R⁴, R⁵, R⁶, R⁷, R⁸ et n sont tels que définis dans la revendication 1 et
R¹ est un groupe choisi parmi 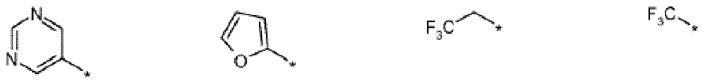 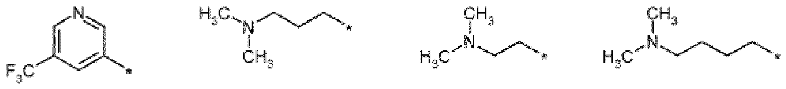 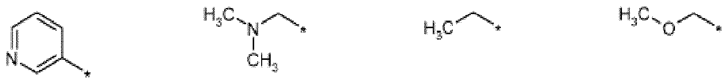 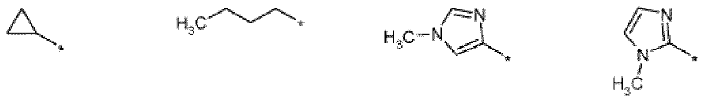 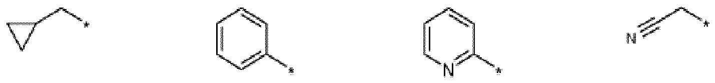 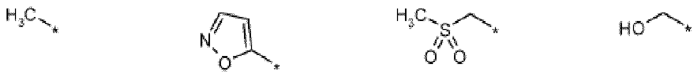 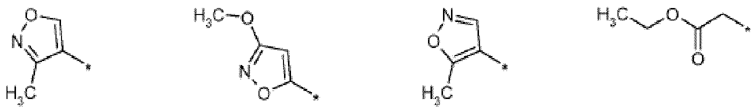 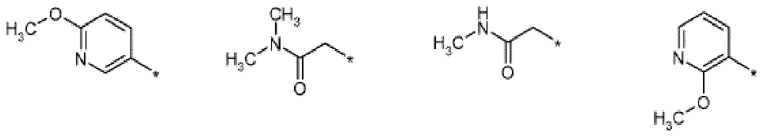 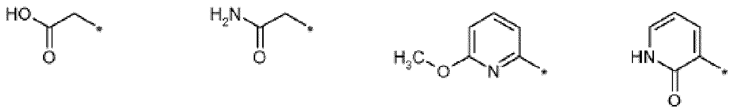 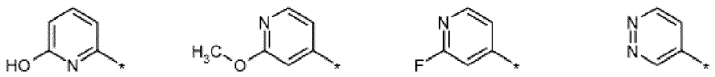 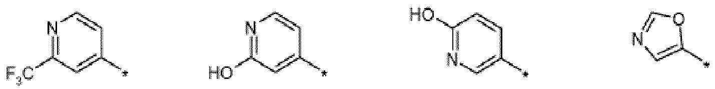 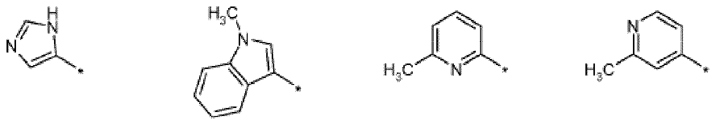 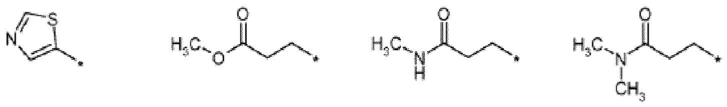 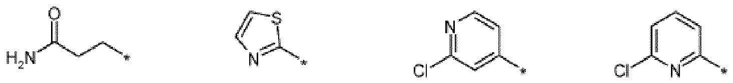 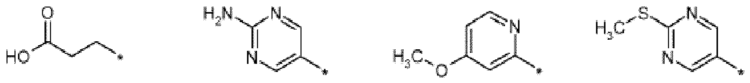 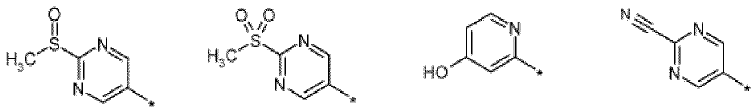 leurs énantiomères, leurs diastéréomères, leurs mélanges et leurs sels, en particulier leurs sels physiologiquement acceptables avec des acides ou bases organiques ou anorganiques.

6. Composés de formule générale I selon la revendication 1, dans laquelle R¹, R³, R⁴, R⁵, R⁶, R⁷, R⁸ et n sont tels que définis dans la revendication 1 et
R² désigne H ou un groupe alkyle en C₁ à C₃
leurs énantiomères, leurs diastéréomères, leurs mélanges et leurs sels, en particulier leurs sels physiologiquement acceptables avec des acides ou bases organiques ou anorganiques.

7. Composés de formule générale I selon la revendication 1, dans laquelle R¹, R², R⁴, R⁵, R⁶, R⁷, R⁸ et n sont tels que définis dans la revendication 1 et
les R³ désignent, indépendamment les uns des autres
(a) H, un atome d'halogène, un groupe alkyle en C₁ à C₃, -O-alkyle en C₁ à C₃ ou
(b) un groupe alkyle en C₁ à C₃ dans lequel chaque groupe méthylène est substitué par jusqu'à deux atomes de fluor et chaque groupe méthyle est substitué par jusqu'à trois atomes de fluor,
leurs énantiomères, leurs diastéréomères, leurs mélanges et leurs sels, en particulier leurs sels physiologiquement acceptables avec des acides ou bases organiques ou anorganiques.

8. Composés de formule générale I selon la revendication 1, dans laquelle R¹, R², R³, R⁵, R⁶, R⁷, R⁸ et n sont tels que définis dans la revendication 1 et
R⁴ désigne H ou un atome d'halogène,
leurs énantiomères, leurs diastéréomères, leurs mélanges et leurs sels, en particulier leurs sels physiologiquement acceptables avec des acides ou bases organiques ou anorganiques.

9. Composés de formule générale I selon la revendication 1, dans laquelle R¹, R², R³, R⁴, R⁶, R⁷, R⁸ et n sont tels que définis dans la revendication 1 et
R⁵ désigne
(a) H, un atome d'halogène,
(b) un groupe alkyle en C₁ à C₃,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) -O-alkyle en C₁ à C₃,
(e) -C (O) -O-alkyle en C₁ à C₃ ou -C(O)-NH₂
leurs énantiomères, leurs diastéréomères, leurs mélanges et leurs sels, en particulier leurs sels physiologiquement acceptables avec des acides ou bases organiques ou anorganiques.

10. Composés de formule générale I selon la revendication 1, dans laquelle R¹, R², R³, R⁴, R⁵, R⁷, R⁸ et n sont tels que définis dans la revendication 1 et
R⁶ désigne
(a) H, un atome d'halogène,
(b) un groupe alkylène en C₁ à C₃-R^{6,1},
(c) un groupe alkyle en C₁ à C₃, dans lequel chaque groupe méthylène est substitué par jusqu'à deux atomes de fluor et chaque groupe méthyle est substitué par jusqu'à trois atomes de fluor,
(d) -OH, -O-alkyle en C₁ à C₄,
(e) -OCHF₂, -O-CF₃,
(f) -C(O)-O-R^{6,2}, -CN, -C(O)-CH₃, -C(O)-NH₂ ou
(g) un groupe pyrrolyle,
R^{6,1} désigne H ou -OH, et
R^{6,2} désigne H ou un groupe alkyle en C₁ à C₃,
leurs énantiomères, leurs diastéréomères, leurs mélanges et leurs sels, en particulier leurs sels physiologiquement acceptables avec des acides ou bases organiques ou anorganiques.

11. Composés de formule générale I selon la revendication 1, dans laquelle R¹, R², R³, R⁴, R⁵, R⁶, R⁸ et n sont tels que définis dans la revendication 1 et
R⁷ désigne
(a) H, un atome d'halogène,
(b) un groupe alkyle en C₁ à C₃,
(c) un groupe alkyle en C₁ à C₃, dans lequel chaque groupe méthylène est substitué par jusqu'à deux atomes de fluor et chaque groupe méthyle est substitué par jusqu'à trois atomes de fluor,
(d) -O-alkyle en C₁ à C₃,
(e) -C(O)-NH₂ ou -C (O) -pyrrolidinyle,
leurs énantiomères, leurs diastéréomères, leurs mélanges et leurs sels, en particulier leurs sels physiologiquement acceptables avec des acides ou bases organiques ou anorganiques.

12. Composés de formule générale I selon la revendication 1, dans laquelle R¹, R², R³, R⁴, R⁵, R⁶, R⁷ et n sont tels que définis dans la revendication 1 et R⁸ désigne
(a) H, un atome d'halogène,
(b) un groupe alkyle en C₁ à C₄,
(c) un groupe alkyle en C₁ à C₃ dans lequel chaque groupe méthylène est substitué par jusqu'à deux atomes de fluor et chaque groupe méthyle est substitué par jusqu'à trois atomes de fluor,
(d) -O-alkyle en C₁ à C₃,
(e) -O-CF₃,
(f) -CN, -C(O) -NH₂ ou
(g) un groupe pyrrolyle,
leurs énantiomères, leurs diastéréomères, leurs mélanges et leurs sels, en particulier leurs sels physiologiquement acceptables avec des acides ou bases organiques ou anorganiques.

13. Composés de formule générale I selon la revendication 1, dans laquelle
R¹ est un groupe choisi parmi 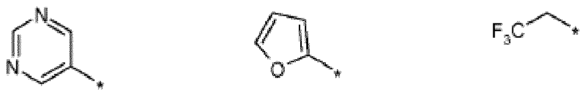 R² est H ou -CH₃,
R³ est H, F, -CF₃, -CH₃ ou -O-CH₃,
R⁴ est H ou Cl,
R⁵ est H, Cl, un groupe alkyle en C₁ à C₃, -CF₃, un groupe -O-alkyle en C₁ à C₃, -C(O)-O-alkyle en C₁ à C₃ ou C(O) -NH₂,
R⁶ est H, Cl, un groupe alkyle en C₁ à C₃, -CF₃, un groupe -O-alkyle en C₁ à C₄, -OCF₃, -C(O)-NH₂ ou pyrrolyle,
R⁷ est H, F, Cl, un groupe alkyle en C₁ à C₃, -CF₃, un groupe -O-alkyle en C₁ à C₃, -C(O)-NH₂ ou -C(O)-pyrrolidinyle et
R⁸ est H, F, Cl, Br, un groupe alkyle en C₁ à C₄, - CF₃, -O-alkyle en C₁ à C₃, -OCF₃, -C(O) -NH₂, -OCF₃ ou pyrrolyle,
leurs énantiomères, leurs diastéréomères, leurs mélanges et leurs sels, en particulier leurs sels physiologiquement acceptables avec des acides ou bases organiques ou anorganiques.

14. Composés de formule générale I selon la revendication 1, dans laquelle
R¹ est un groupe choisi parmi 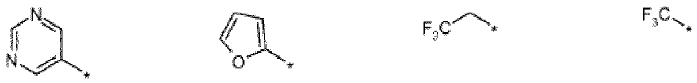 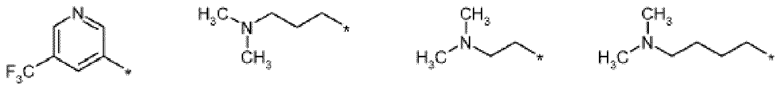 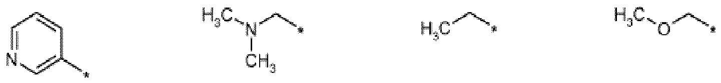 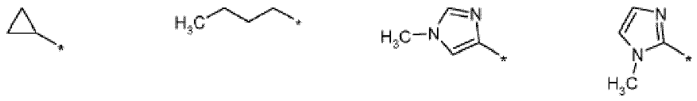 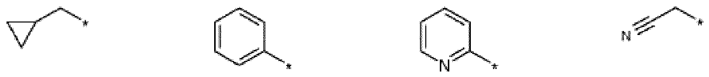 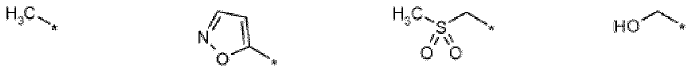 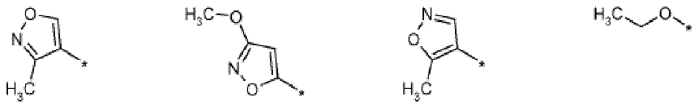 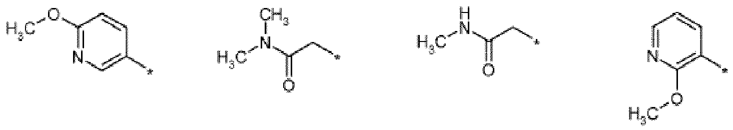 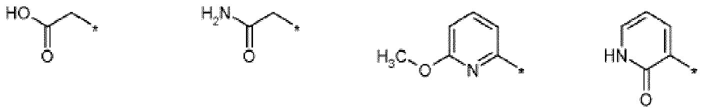 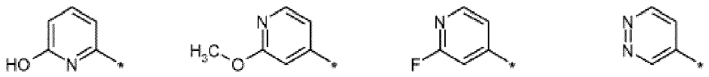 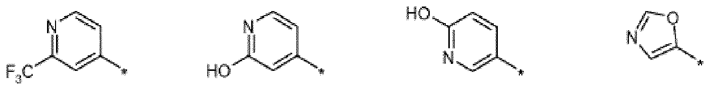 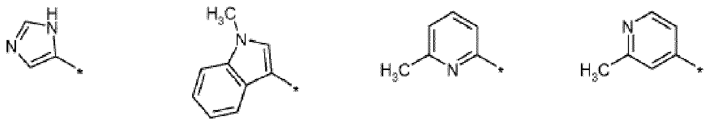 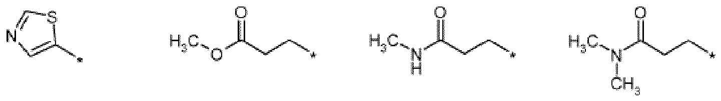 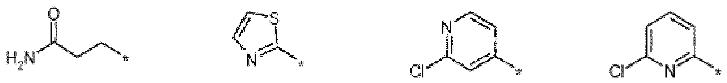 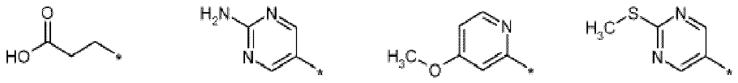 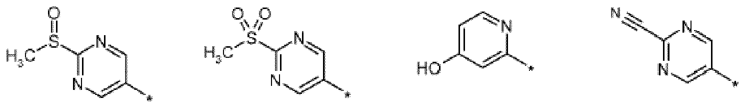 R² est H, -CH₃ ou -C₂H₅,
R³ est H, F, Cl, -CF₃, -CH₃ ou -O-CH₃, R⁴ est H ou Cl,
R⁵ est H, Cl, un groupe alkyle en C₁ à C₃, -CF₃, - O-alkyle en C₁ à C₃, -C (O) -O-alkyle en C₁ à C₃ ou -C (O) - NH₂,
R⁶ est H, F, Cl, Br, -CN, un groupe alkyle en C₁ à C₃, -CF₃, -COOH, -COO-alkyle en C₁ à C₃, -CH (OH) CH3, -OH, -O-alkyle en C₁ à C₄, -OCF₃, -OCHF₂, -C(O)-CH₃, -C(O)-NH₂ ou pyrrolyle,
R⁷ désigne H, F, Cl, un groupe alkyle en C₁ à C₃, - CF₃, -O-alkyle en C₁ à C₃, -C(O)-NH₂ ou -C (O) - pyrrolidinyle et
R⁸ désigne H, F, Cl, Br, -CN, un groupe alkyle en C₁ à C₄, -CF₃, -O-alkyle en C₁ à C₃, -OCF₃, -C(O)-NH₂, - OCF₃ ou pyrrolyle,
leurs énantiomères, leurs diastéréomères, leurs mélanges et leurs sels, en particulier leurs sels physiologiquement acceptables avec des acides ou bases organiques ou anorganiques.

15. Composés suivants de formule générale I selon la revendication 1 :
| N° | Structure |
|---|---|
| (1) | 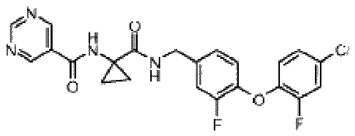 |
| (2) | 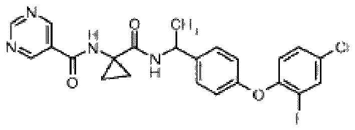 |
| (3) | 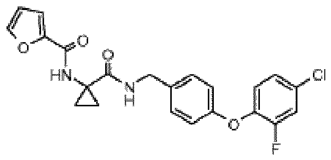 |
| (4) | 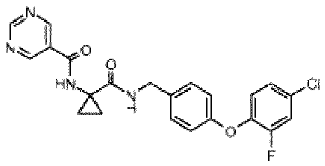 |
| (5) | 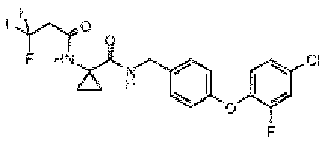 |
| (6) | 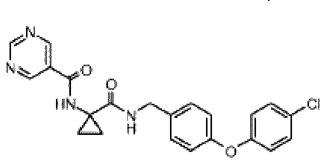 |
| (7) | 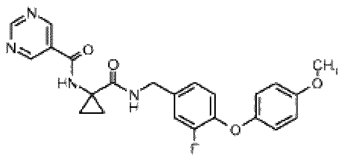 |
| (8) | 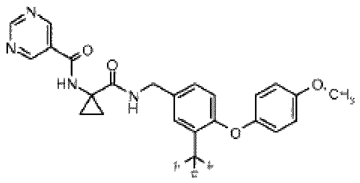 |
| (9) | 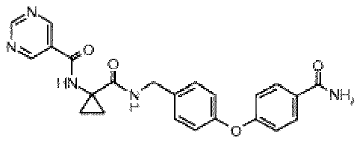 |
| (10) | 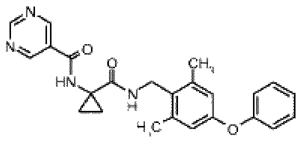 |
| (11) | 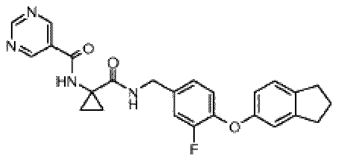 |
| (12) | 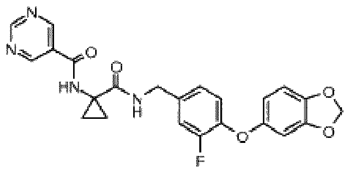 |
| (13) | 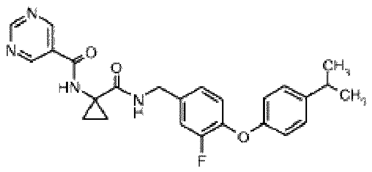 |
| (14) | 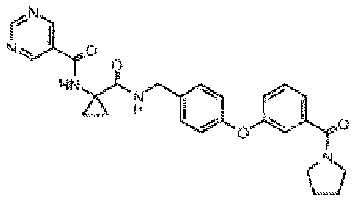 |
| (15) | 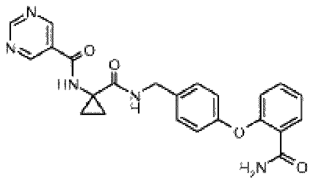 |
| (16) | 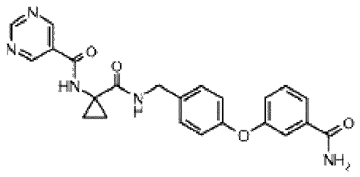 |
| (17) | 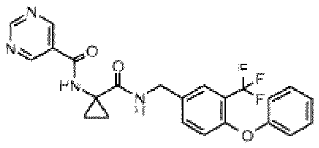 |
| (18) | 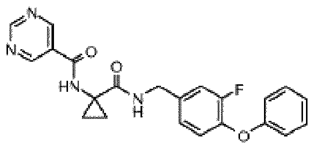 |
| (19) | 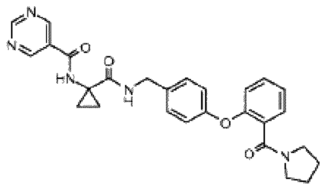 |
| (20) | 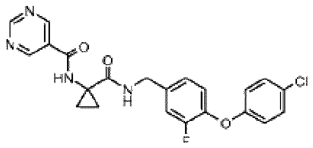 |
| (21) | 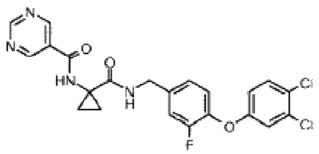 |
| (22) | 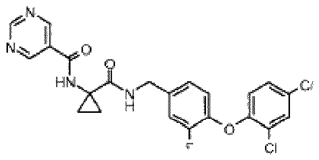 |
| (23) | 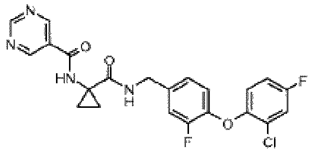 |
| (24) | 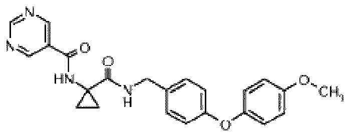 |
| (25) | 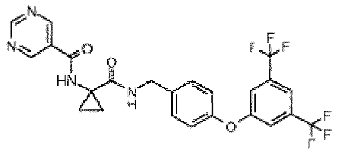 |
| (26) | 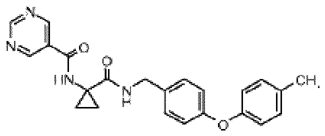 |
| (27) | 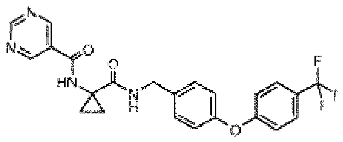 |
| (28) | 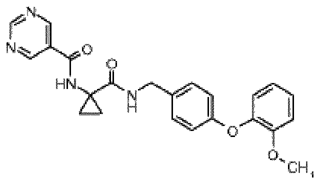 |
| (29) | 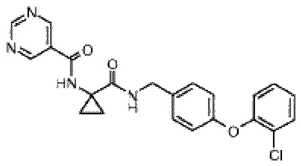 |
| (30) | 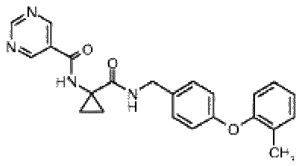 |
| (31) | 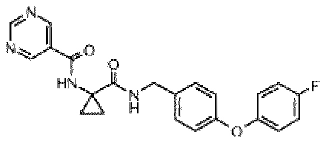 |
| (32) | 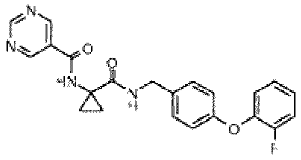 |
| (33) | 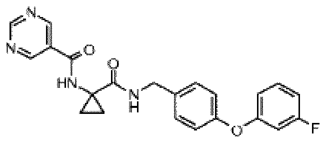 |
| (34) | 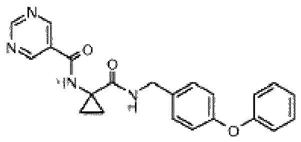 |
| (35) | 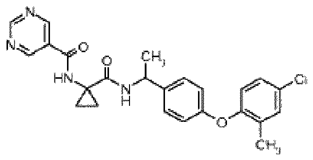 |
| (36) | 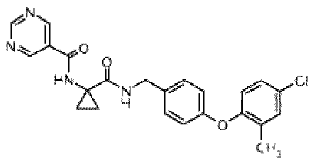 |
| (37) | 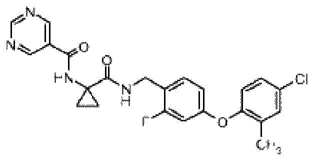 |
| (38) | 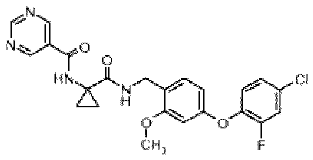 |
| (39) | 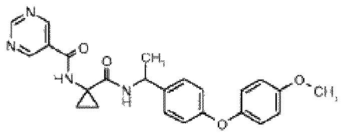 |
| (40) | 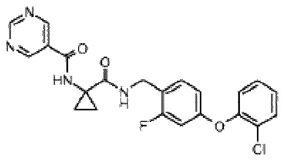 |
| (41) | 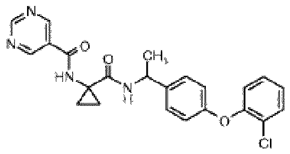 |
| (42) | 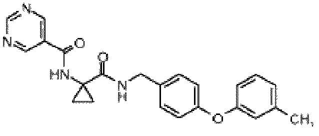 |
| (43) | 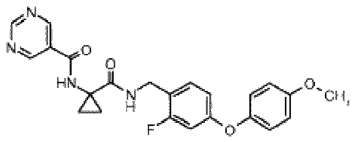 |
| (44) | 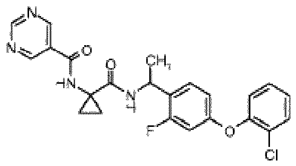 |
| (45) | 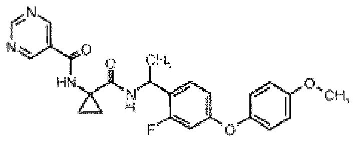 |
| (46) | 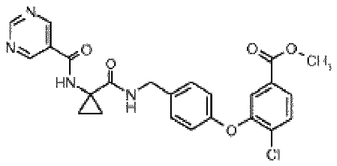 |
| (47) | 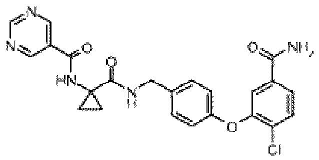 |
| (48) | 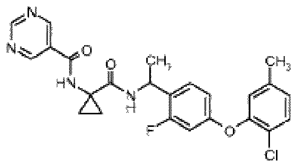 |
| (49) | 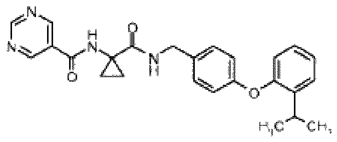 |
| (50) | 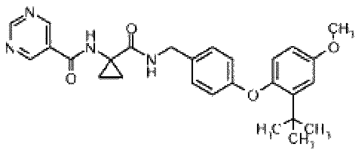 |
| (51) | 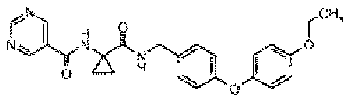 |
| (52) | 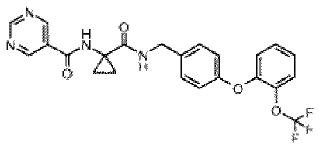 |
| (53) | 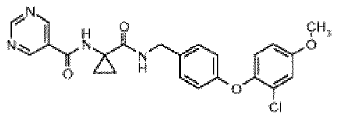 |
| (54) | 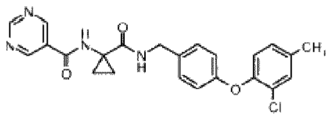 |
| (55) | 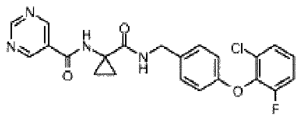 |
| (56) | 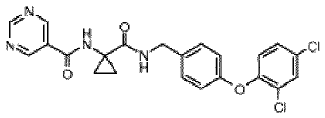 |
| (57) | 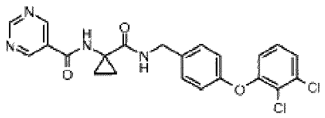 |
| (58) | 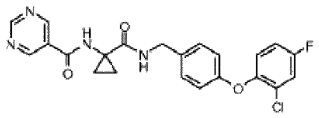 |
| (59) | 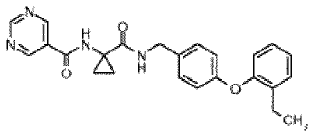 |
| (60) | 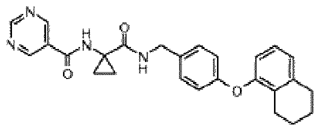 |
| (61) | 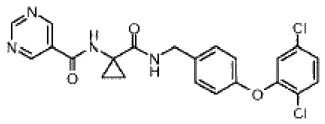 |
| (62) | 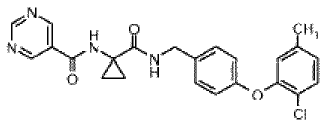 |
| (63) | 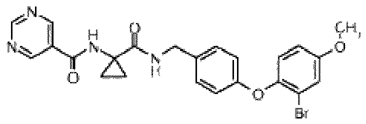 |
| (64) | 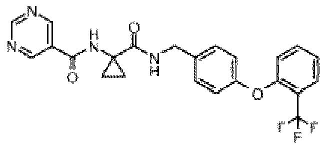 |
| (65) | 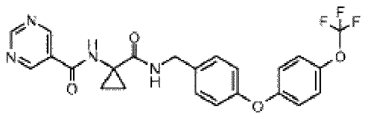 |
| (66) | 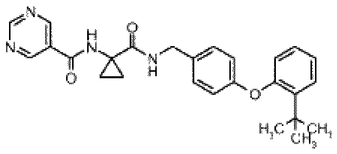 |
| (67) | 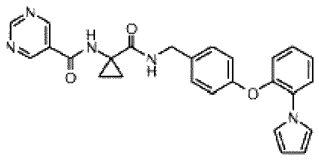 |
| (68) | 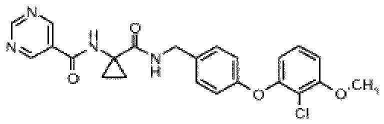 |
| (69) | 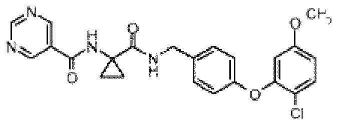 |
| (70) | 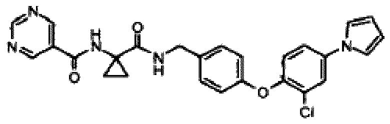 |
| (71) | 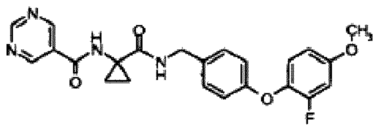 |
| (72) | 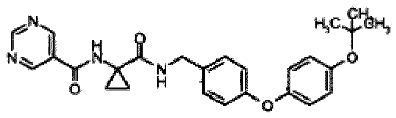 |
| (73) | 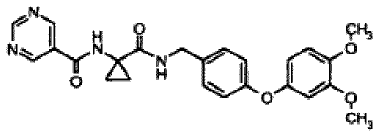 |
| (74) | 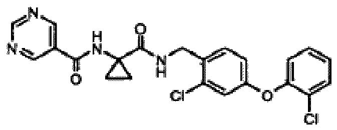 |
| (75) | 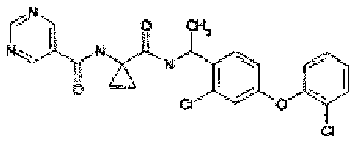 |
| (76) | 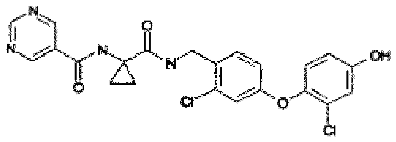 |
| (77) | 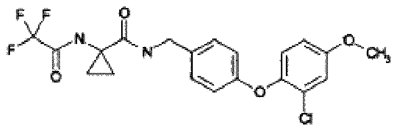 |
| (78) | 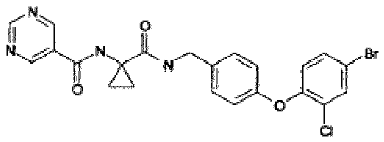 |
| (79) | 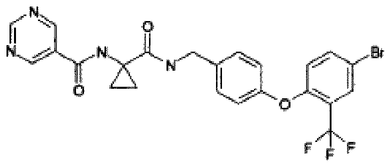 |
| (80) | 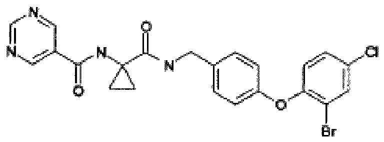 |
| (81) | 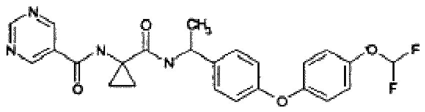 |
| (82) | 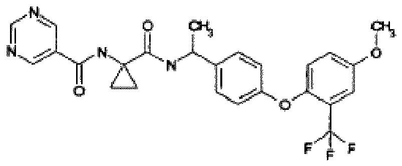 |
| (83) | 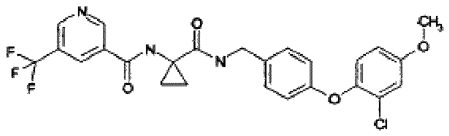 |
| (84) | 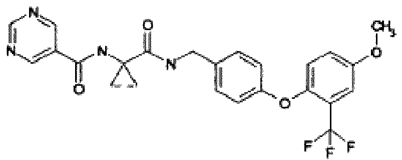 |
| (85) | 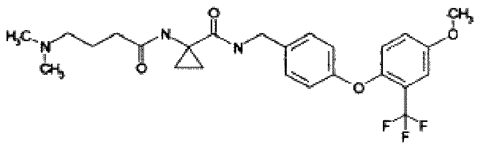 |
| (86) | 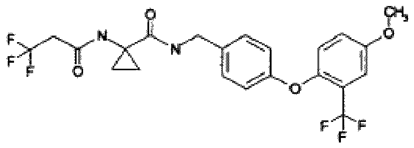 |
| (87) | 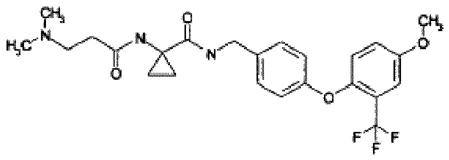 |
| (88) | 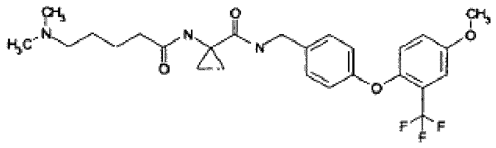 |
| (89) | 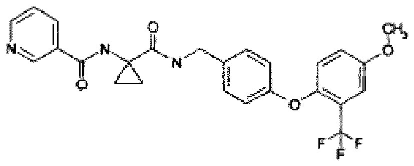 |
| (90) | 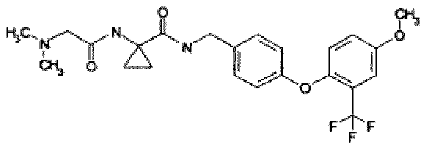 |
| (91) | 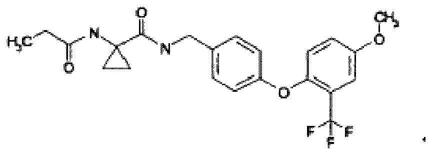 |
| (92) | 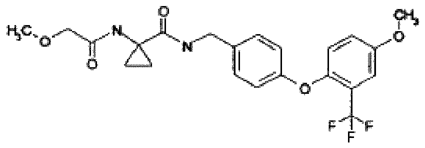 |
| (93) | 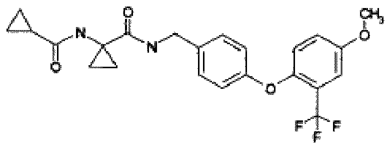 |
| (94) | 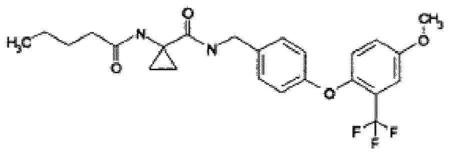 |
| (95) | 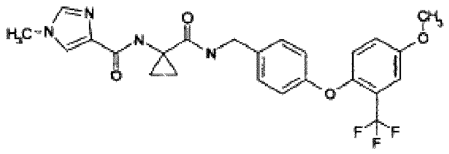 |
| (96) | 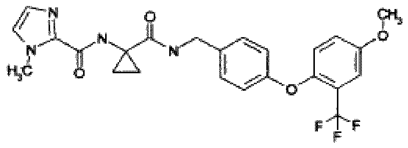 |
| (97) | 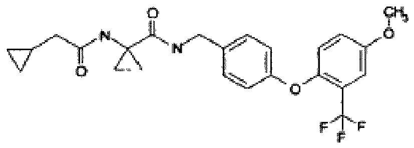 |
| (98) | 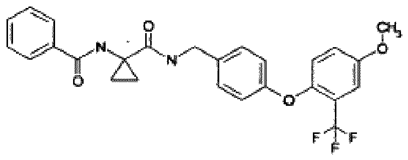 |
| (99) | 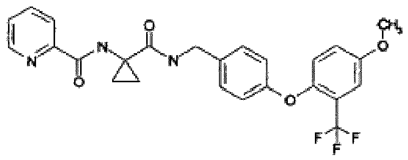 |
| (100) | 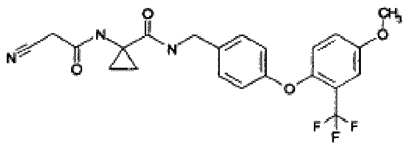 |
| (101) | 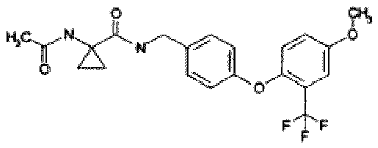 |
| (102) | 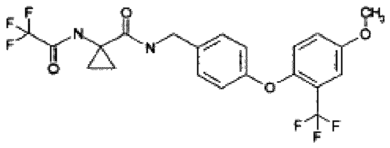 |
| (103) | 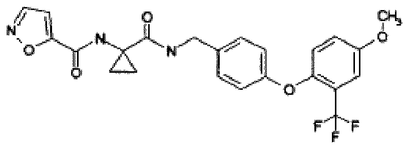 |
| (104) | 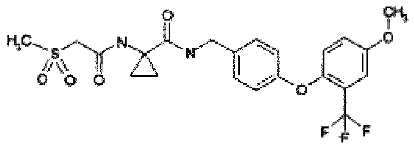 |
| (105) | 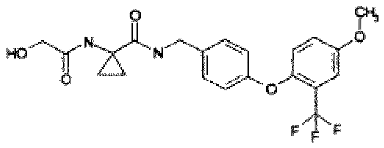 |
| (106) | 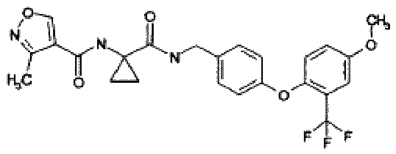 |
| (107) | 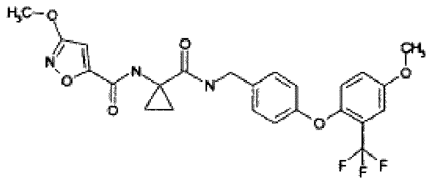 |
| (108) | 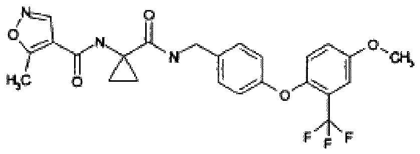 |
| (109) | 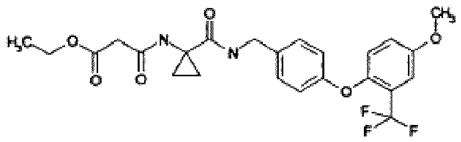 |
| (110) | 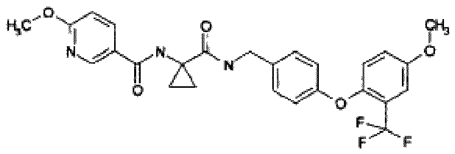 |
| (111) | 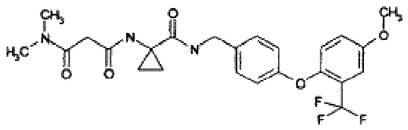 |
| (112) | 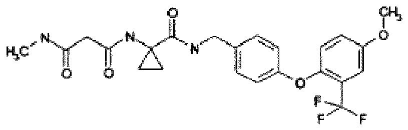 |
| (113) | 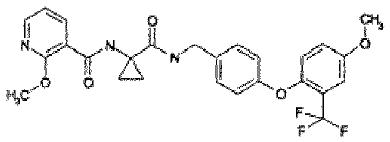 |
| (114) | 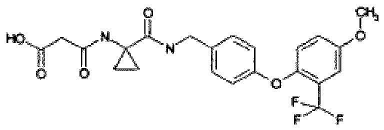 |
| (115) | 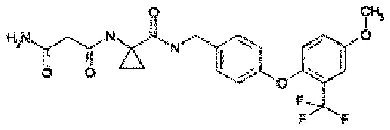 |
| (116) | 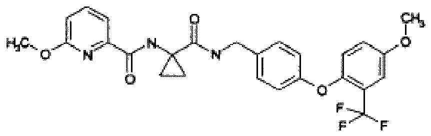 |
| (117) | 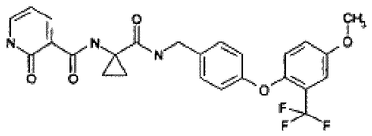 |
| (118) | 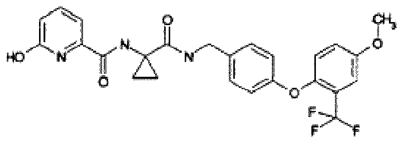 |
| (119) | 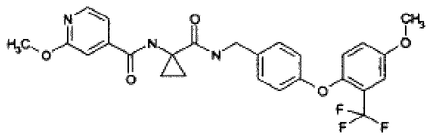 |
| (120) | 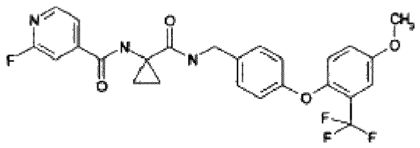 |
| (121) | 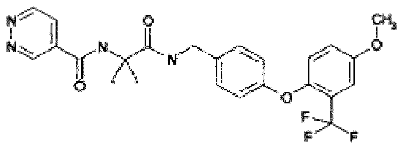 |
| (122) | 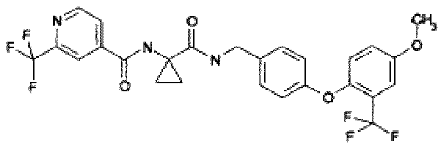 |
| (123) | 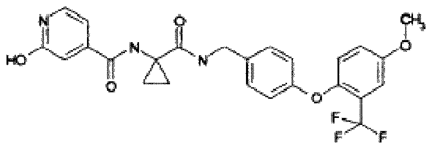 |
| (124) | 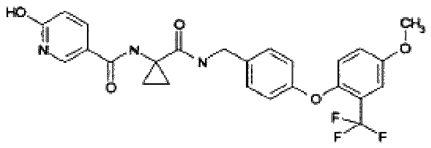 |
| (125) | 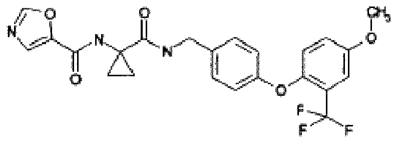 |
| (126) | 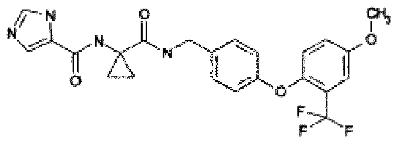 |
| (127) | 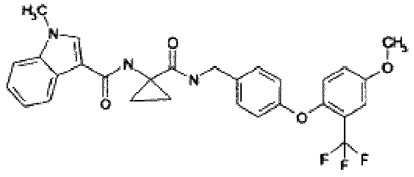 |
| (128) | 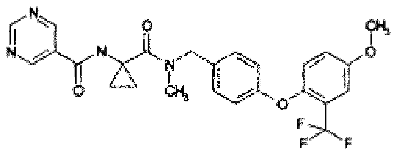 |
| (129) | 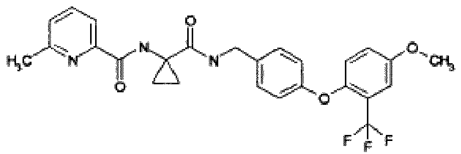 |
| (130) | 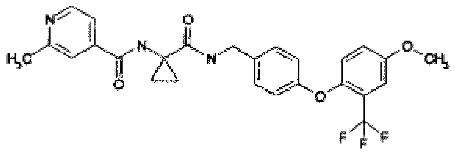 |
| (131) | 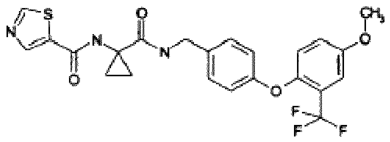 |
| (132) | 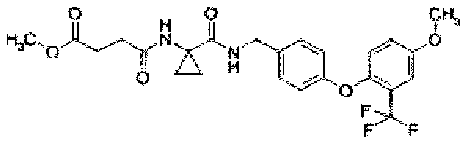 |
| (133) | 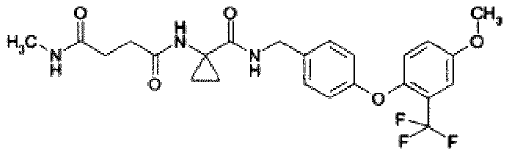 |
| (134) | 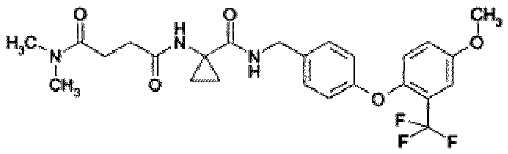 |
| (135) | 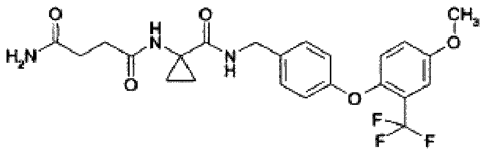 |
| (136) | 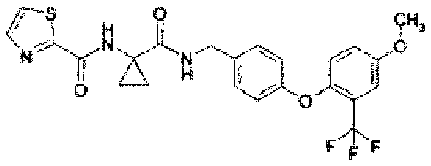 |
| (137) | 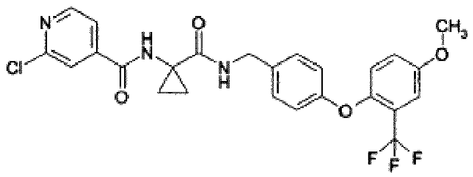 |
| (138) | 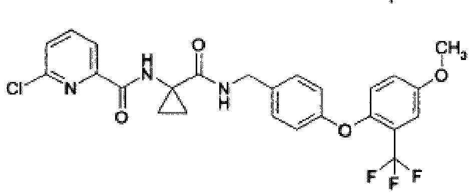 |
| (139) | 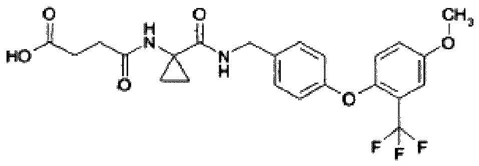 |
| (140) | 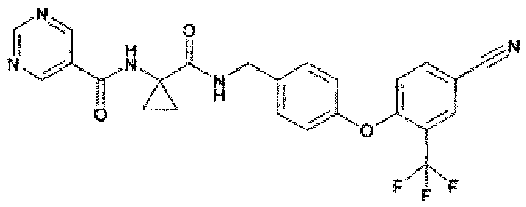 |
| (141) | 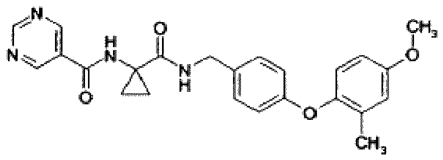 |
| (142) | 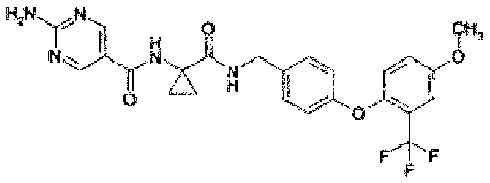 |
| (143) | 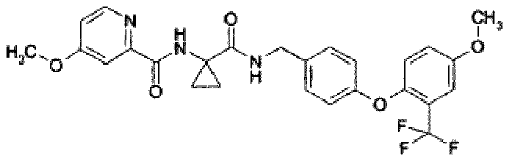 |
| (144) | 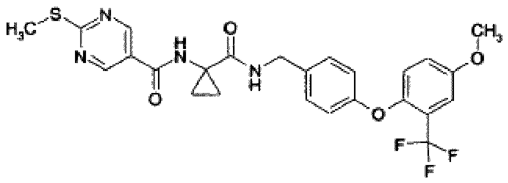 |
| (145) | 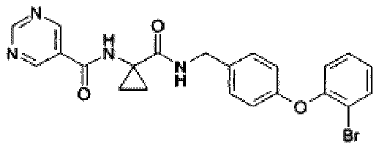 |
| (146) | 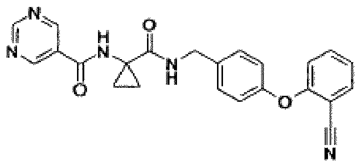 |
| (147) | 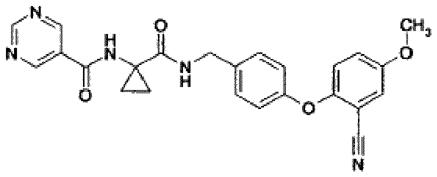 |
| (148) | 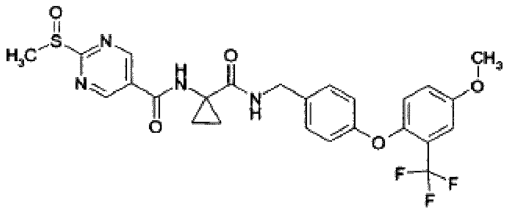 |
| (149) | 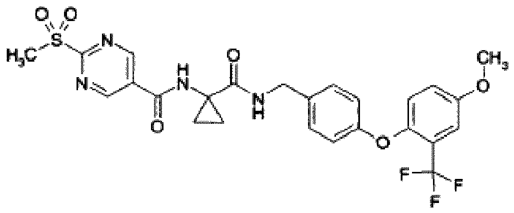 |
| (150) | 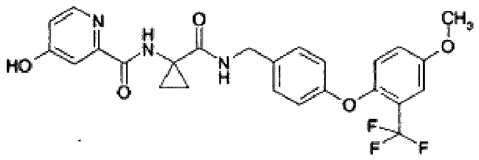 |
| (151) | 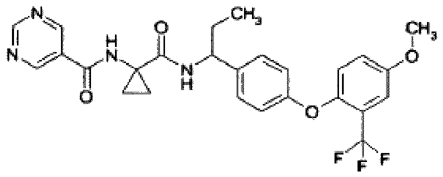 |
| (152) | 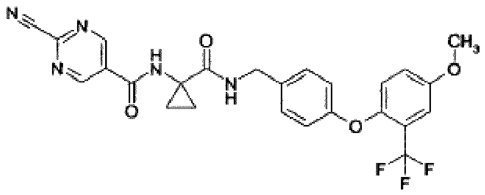 |
| (153) | 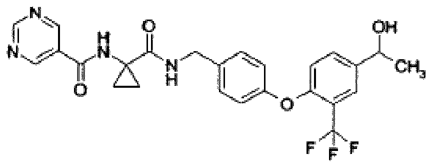 |
| (154) | 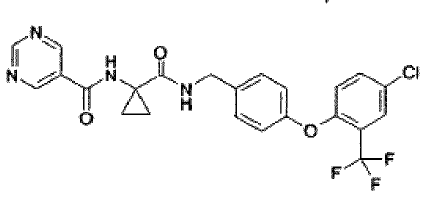 |
| (155) | 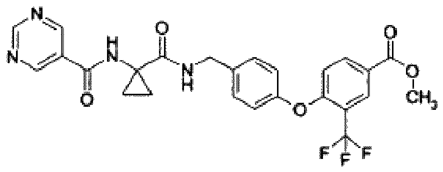 |
| (156) | 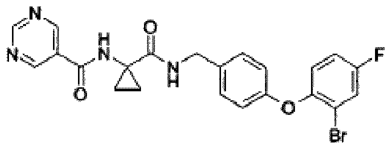 |
| (157) | 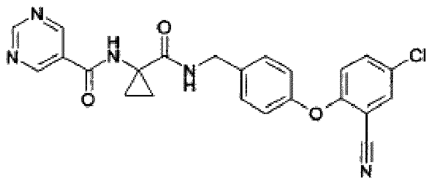 |
| (158) | 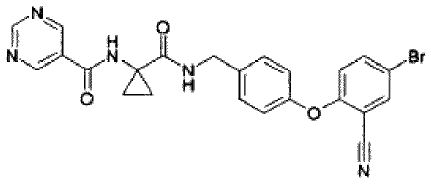 |
| (159) | 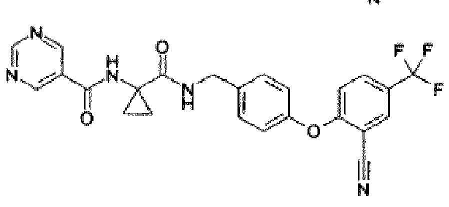 |
| (160) | 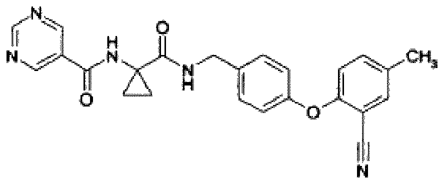 |
| (161) | 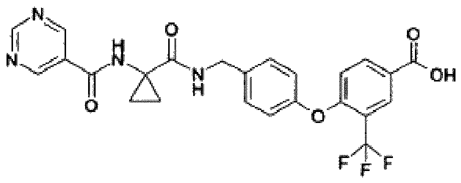 |
| (162) | 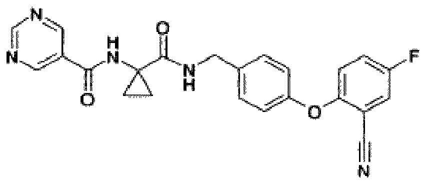 |
| (163) | 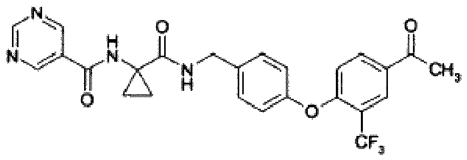 |
leurs énantiomères, leurs diastéréomères, leurs mélanges et leurs sels, en particulier leurs sels physiologiquement acceptables avec des acides ou bases organiques ou anorganiques.

16. Sels physiologiquement acceptables des composés selon l'une des revendications 1 à 15, avec des acides ou bases anorganiques ou organiques.

17. Médicament contenant un composé selon au moins l'une des revendications 1 à 15, ou sel physiologiquement acceptable selon la revendication 16, éventuellement, parallèlement à un ou plusieurs véhicules et/ou diluants inertes.

18. Utilisation d'un composé selon au moins l'une des revendications 1 à 16 pour la production d'un médicament destiné au traitement aigu et prophylactique des douleurs aiguës, des douleurs viscérales, des douleurs neuropathiques, des douleurs inflammatoires ou induites par les récepteurs de la douleur, des douleurs tumorales et des céphalées.

19. Procédé de production d'un médicament selon la revendication 17, **caractérisé en ce que**, de façon non chimique, un composé selon au moins l'une des revendications 1 à 16 est incorporé dans un ou plusieurs véhicules et/ou diluants inertes.
